# EUROPEAN PATENT APPLICATION

(11) **EP 4 806 892 A1**
(43) Date of publication of application: **16.09.2026**
(21) Application number: 24888114.6
(22) Date of filing: 08.11.2024
(51) Int. Cl.: C07D 403/04, A61K 31/506, A61P 35/00

(54) **CEREBLON E3 UBIQUITIN LIGASE INHIBITOR**

(30) Priority: 08.11.2023 CN 202311479565; 26.12.2023 CN 202311804797; 06.09.2024 CN 202411250508
(71) Applicant: GAN & LEE PHARMACEUTICALS CO., LTD., Beijing 101109 (CN)
(72) Inventor: XU, Fuming, Beijing 101109 (CN); CHEN, Wenmin, Beijing 101109 (CN); ZHOU, Guan, Beijing 101109 (CN); XUE, Wei, Beijing 101109 (CN); DING, Yongzheng, Beijing 101109 (CN); LIU, Xiaobing, Beijing 101109 (CN); LI, Xiaoguang, Beijing 101109 (CN)
(74) Representative: Kraus & Lederer PartGmbB
(86) International application number: PCT/CN2024/131100
(87) International publication number: WO 2025/098503

(57) **Abstract**

The present invention relates to a new human cereblon (CRBN) E3 ubiquitin ligase inhibitor, which can be used for preparing PROTAC molecules. The CRBN E3 ubiquitin ligase inhibitor and the PROTAC molecules can be used for treating cancer and other diseases.

## Description

### TECHNICAL FIELD

The present invention relates to the field of biopharmaceuticals, and particularly related to cerebellar protein E3 ubiquitin ligase inhibitors and the proteolysis targeting chimera (PROTAC) compounds comprising the same, as well as their pharmaceutical uses.

### BACKGROUND

Cereblon (CRBN) is a brain-associated protein with protease activity, which can interact with DNA damage-binding protein 1 (DDB1), Cullin 4 (Cullin4, Cul4A or Cul4B), and Regulator of Cullins 1 (RoC1) to form a functional E3 ubiquitin ligase complex (CRBN-CRL4). Upon substrate binding, this complex mediates proteolysis through the ubiquitin-proteasome pathway. Immune modulating drugs (IMiDs) such as thalidomide, lenalidomide, and pomalidomide recruit novel substrates when bound to CRBN, causing them to bind to CRBN-CRL4 complexes. This leads to increased ubiquitination and proteasome-dependent degradation, thereby treating cancer and other diseases.

On this basis, CRBN ligands have been extensively applied in protein degradation, leading to the development of a series of CRBN-based protein degradation-targeting chimeras (PROTACs). Due to the inherent effects of CRBN ligands on their target sites, which may induce additional degradation of domain proteins, the design and synthesis of novel CRBN ligands hold significant importance for the further advancement of PROTACs molecules.

### SUMMARY

In view of the above, the present disclosure provides a series of structurally novel CRBN ligands and further applies them in the synthesis of corresponding PROTAC molecules.In a first aspect, the present invention provides a compound of Formula I, or an isomer, an isotopic derivative, a polymorph, a prodrug, or a pharmaceutically acceptable salt or a solvate thereof, 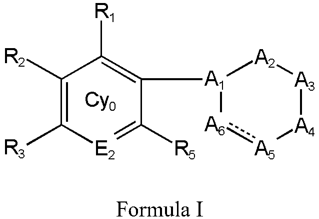
wherein, A₁ is selected from CRₐ and N;
A₂ and A₄ are each independently selected from C(O) and C(Rₐ)₂;
E₂ is selected from CR₄ and N;
A₃ is NRₐ; preferably, A₃ is NH;
the bond between A₅ and A₆ is a single bond or a double bond; when A₅ and A₆ are connected by a single bond, A₅ and A₆ are each independently selected from NRₐ and C(Rₐ)₂; when A₅ and A₆ are connected by a double bond, A₅ and A₆ are each independently selected from N and CRₐ; preferably, A₆ and A₅ are connected by a single bond and are both C(H)₂;
R₁ and R₂, together with the atoms to which they are attached, form a ring Cy₁ fused to Cy₀; R₃, R₄, and R₅, at each occurrence, are each independently selected from H, deuterium atom, halogen, C₁₋₆ alkyl, C₁₋₆ deuterated alkyl, carboxyl, C₁₋₆ heteroalkyl, alkenyl, alkynyl, C₁₋₆ alkoxy, C₁₋₆ haloalkyl, C₁₋₆ haloalkoxy, hydroxyl, C₁₋₆ hydroxyalkyl, nitro, cyano, amino, C₁₋₆ alkylamino, C₁₋₆ alkylcarbonyl, C₁₋₆ alkoxycarbonyl, C₁₋₆ alkylaminocarbonyl, cycloalkyl, heterocyclyl, aryl, and heteroaryl, wherein the C₁₋₆ alkyl, C₁₋₆ heteroalkyl, alkenyl, alkynyl, C₁₋₆ alkoxy, C₁₋₆ haloalkyl, C₁₋₆ haloalkoxy, C₁₋₆ hydroxyalkyl, C₁₋₆ alkylamino, C₁₋₆ alkylcarbonyl, C₁₋₆ alkoxycarbonyl, C₁₋₆ alkylaminocarbonyl, cycloalkyl, heterocyclyl, aryl, and heteroaryl are each independently optionally substituted with one or more substituents selected from halogen, alkyl, heteroalkyl, alkoxy, haloalkyl, hydroxyl, hydroxyalkyl, cyano, amino, nitro, cycloalkyl, heterocyclyl, alkenyl, alkynyl, alkylamino, alkylcarbonyl, alkoxycarbonyl, alkylaminocarbonyl, aryl, and heteroaryl; preferably, R₃, R₄, and R₅, at each occurrence, are each independently selected from H, deuterium atom, halogen, C₁₋₆ alkyl, C₁₋₆ deuterated alkyl, C₁₋₆ heteroalkyl, alkenyl, alkynyl, C₁₋₆ alkoxy, C₁₋₆ haloalkyl, C₁₋₆ haloalkoxy, hydroxyl, C₁₋₆ hydroxyalkyl, and amino; more preferably, R₃, R₄, and R₅, at each occurrence, are each independently selected from H, deuterium atom, F, Cl, Br, I, C₁₋₆ alkyl, C₁₋₆ deuterated alkyl, C₁₋₆ alkoxy, C₁₋₆ haloalkyl, C₁₋₆ haloalkoxy, and hydroxyl; or
R₂ and R₃, together with the atoms to which they are attached, form a ring Cy₂ fused to Cy₀; R₁, R₄, and R₅, at each occurrence, are each independently selected from H, deuterium atom, halogen, C₁₋₆ alkyl, C₁₋₆ deuterated alkyl, carboxyl, C₁₋₆ heteroalkyl, alkenyl, alkynyl, C₁₋₆ alkoxy, C₁₋₆ haloalkyl, C₁₋₆ haloalkoxy, hydroxyl, C₁₋₆ hydroxyalkyl, nitro, cyano, amino, C₁₋₆ alkylamino, C₁₋₆ alkylcarbonyl, C₁₋₆ alkoxycarbonyl, C₁₋₆ alkylaminocarbonyl, cycloalkyl, heterocyclyl, aryl, and heteroaryl, wherein the C₁₋₆ alkyl, C₁₋₆ heteroalkyl, alkenyl, alkynyl, C₁₋₆ alkoxy, C₁₋₆ haloalkyl, C₁₋₆ haloalkoxy, C₁₋₆ hydroxyalkyl, C₁₋₆ alkylamino, C₁₋₆ alkylcarbonyl, C₁₋₆ alkoxycarbonyl, C₁₋₆ alkylaminocarbonyl, cycloalkyl, heterocyclyl, aryl, and heteroaryl are each independently optionally substituted with one or more substituents selected from halogen, alkyl, heteroalkyl, alkoxy, haloalkyl, hydroxyl, hydroxyalkyl, cyano, amino, nitro, cycloalkyl, heterocyclyl, alkenyl, alkynyl, alkylamino, alkylcarbonyl, alkoxycarbonyl, alkylaminocarbonyl, aryl, and heteroaryl; preferably, R₁, R₄, and R₅, at each occurrence, are each independently selected from H, deuterium atom, halogen, C₁₋₆ alkyl, C₁₋₆ deuterated alkyl, C₁₋₆ heteroalkyl, alkenyl, alkynyl, C₁₋₆ alkoxy, C₁₋₆ haloalkyl, C₁₋₆ haloalkoxy, hydroxyl, C₁₋₆ hydroxyalkyl, and amino; more preferably, R₁, R₄, and R₅, at each occurrence, are each independently selected from H, deuterium atom, F, Cl, Br, I, C₁₋₆ alkyl, C₁₋₆ deuterated alkyl, C₁₋₆ alkoxy, C₁₋₆ haloalkyl, C₁₋₆ haloalkoxy, and hydroxyl; or
when R₄ is present, R₃ and R₄, together with the atoms to which they are attached, form a ring Cy₃ fused to Cy₀; R₁, R₂, and R₅, at each occurrence, are each independently selected from H, deuterium atom, halogen, C₁₋₆ alkyl, C₁₋₆ deuterated alkyl, carboxyl, C₁₋₆ heteroalkyl, alkenyl, alkynyl, C₁₋₆ alkoxy, C₁₋₆ haloalkyl, C₁₋₆ haloalkoxy, hydroxyl, C₁₋₆ hydroxyalkyl, nitro, cyano, amino, C₁₋₆ alkylamino, C₁₋₆ alkylcarbonyl, C₁₋₆ alkoxycarbonyl, C₁₋₆ alkylaminocarbonyl, cycloalkyl, heterocyclyl, aryl, and heteroaryl, wherein the C₁₋₆ alkyl, C₁₋₆ heteroalkyl, alkenyl, alkynyl, C₁₋₆ alkoxy, C₁₋₆ haloalkyl, C₁₋₆ haloalkoxy, C₁₋₆ hydroxyalkyl, C₁₋₆ alkylamino, C₁₋₆ alkylcarbonyl, C₁₋₆ alkoxycarbonyl, C₁₋₆ alkylaminocarbonyl, cycloalkyl, heterocyclyl, aryl, and heteroaryl are each independently optionally substituted with one or more substituents selected from halogen, alkyl, heteroalkyl, alkoxy, haloalkyl, hydroxyl, hydroxyalkyl, cyano, amino, nitro, cycloalkyl, heterocyclyl, alkenyl, alkynyl, alkylamino, alkylcarbonyl, alkoxycarbonyl, alkylaminocarbonyl, aryl, and heteroaryl; preferably, R₁, R₂, and R₅, at each occurrence, are each independently selected from H, deuterium atom, halogen, C₁₋₆ alkyl, C₁₋₆ deuterated alkyl, C₁₋₆ heteroalkyl, alkenyl, alkynyl, C₁₋₆ alkoxy, C₁₋₆ haloalkyl, C₁₋₆ haloalkoxy, hydroxyl, C₁₋₆ hydroxyalkyl, and amino; more preferably, R₁, R₂, and R₅, at each occurrence, are each independently selected from H, deuterium atom, F, Cl, Br, I, C₁₋₆ alkyl, C₁₋₆ deuterated alkyl, C₁₋₆ alkoxy, C₁₋₆ haloalkyl, C₁₋₆ haloalkoxy, and hydroxyl; or
when R₄ is present, R₄ and R₅, together with the atoms to which they are attached, form a ring Cy₄ fused to Cy₀; R₁, R₂, and R₃, at each occurrence, are each independently selected from H, deuterium atom, halogen, C₁₋₆ alkyl, C₁₋₆ deuterated alkyl, carboxyl, C₁₋₆ heteroalkyl, alkenyl, alkynyl, C₁₋₆ alkoxy, C₁₋₆ haloalkyl, C₁₋₆ haloalkoxy, hydroxyl, C₁₋₆ hydroxyalkyl, nitro, cyano, amino, C₁₋₆ alkylamino, C₁₋₆ alkylcarbonyl, C₁₋₆ alkoxycarbonyl, C₁₋₆ alkylaminocarbonyl, cycloalkyl, heterocyclyl, aryl, and heteroaryl, wherein the C₁₋₆ alkyl, C₁₋₆ heteroalkyl, alkenyl, alkynyl, C₁₋₆ alkoxy, C₁₋₆ haloalkyl, C₁₋₆ haloalkoxy, C₁₋₆ hydroxyalkyl, C₁₋₆ alkylamino, C₁₋₆ alkylcarbonyl, C₁₋₆ alkoxycarbonyl, C₁₋₆ alkylaminocarbonyl, cycloalkyl, heterocyclyl, aryl, and heteroaryl are each independently optionally substituted with one or more substituents selected from halogen, alkyl, heteroalkyl, alkoxy, haloalkyl, hydroxyl, hydroxyalkyl, cyano, amino, nitro, cycloalkyl, heterocyclyl, alkenyl, alkynyl, alkylamino, alkylcarbonyl, alkoxycarbonyl, alkylaminocarbonyl, aryl, and heteroaryl; preferably, R₁, R₂, and R₃, at each occurrence, are each independently selected from H, deuterium atom, halogen, C₁₋₆ alkyl, C₁₋₆ deuterated alkyl, C₁₋₆ heteroalkyl, C₂₋₆ alkenyl, C₂₋₆ alkynyl, C₁₋₆ alkoxy, C₁₋₆ haloalkyl, C₁₋₆ haloalkoxy, hydroxyl, C₁₋₆ hydroxyalkyl, and amino; more preferably, R₁, R₂, and R₃, at each occurrence, are each independently selected from H, deuterium atom, F, Cl, Br, I, C₁₋₆ alkyl, C₁₋₆ deuterated alkyl, C₁₋₆ alkoxy, C₁₋₆ haloalkyl, C₁₋₆ haloalkoxy, and hydroxyl;
exactly one of Cy₁, Cy₂, Cy₃, and Cy₄ is present, and is selected from structures 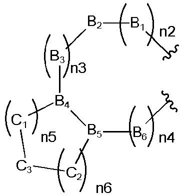 and 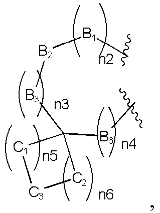 wherein,
B₂ is selected from C(Rₐ)₂, NRₐ, O, and S; and when exactly one of Cy₁, Cy₂, Cy₃, and Cy₄ is present as the structure 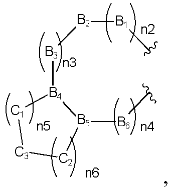 B₂ is selected from NRₐ, O, and S;
B₄ and B₅ are each independently selected from CRₐ and N;
B₁ and B₃, at each occurrence, are each independently selected from C(Rₐ)₂ and C(O);
B₆, at each occurrence, is selected from C(Rₐ)₂ and NRₐ;
C₃ is selected from C(O) and NRₛ₁;
Rₛ₁ is selected from H, deuterium atom, F, Cl, Br, I, C₁₋₆ alkyl, deuterated C₁₋₆ alkyl, carboxyl, C₁₋₆ heteroalkyl, C₂₋₆ alkenyl, C₂₋₆ alkynyl, -C(O)-C₁₋₆ alkyl, C₁₋₆ alkoxy, C₁₋₆ haloalkyl, C₁₋₆ haloalkoxy, hydroxyl, C₁₋₆ hydroxyalkyl, nitro, cyano, and amino; preferably, Rₛ₁ is selected from H, deuterium atom, F, Cl, Br, I, C₁₋₆ alkyl, deuterated C₁₋₆ alkyl, carboxyl, C₁₋₆ heteroalkyl, C₂₋₆ alkenyl, C₂₋₆ alkynyl, -C(O)-C₁₋₆ alkyl, C₁₋₆ alkoxy, C₁₋₆ haloalkyl, C₁₋₆ haloalkoxy, hydroxyl, and C₁₋₆ hydroxyalkyl; more preferably, Rₛ₁ is selected from H, deuterium atom, F, Cl, Br, I, C₁-₃ alkyl, deuterated C₁-₃ alkyl, carboxyl, -C(O)-C₁-₃ alkyl, C₁-₃ alkoxy, C₁-₃ haloalkyl, and C₁-₃ haloalkoxy;
n2, n3, n4, n5, and n6 are each independently selected from 0, 1, 2, and 3; and n5 and n6 are not both 0;
preferably, when exactly one of Cy₁, Cy₂, Cy₃, and Cy₄ is present as the structure 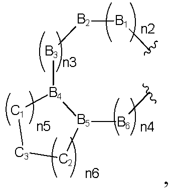 and when B₂ is selected from O and S, n2+n3+n4 is greater than or equal to 2;
C₁ and C₂, at each occurrence, are each independently C(Rₐ)₂;
Rₐ, at each occurrence, is each independently selected from H, deuterium atom, F, Cl, Br, I, C₁₋₆ alkyl, deuterated C₁₋₆ alkyl, carboxyl, C₁₋₆ heteroalkyl, C₂₋₆ alkenyl, C₂₋₆ alkynyl, -C(O)-C₁₋₆ alkyl, C₁₋₆ alkoxy, C₁₋₆ haloalkyl, C₁₋₆ haloalkoxy, hydroxyl, C₁₋₆ hydroxyalkyl, nitro, cyano, amino, C₁₋₆ alkylamino, C₁₋₆ alkylcarbonyl, C₁₋₆ alkoxycarbonyl, C₁₋₆ alkylaminocarbonyl, 3-10-membered cycloalkyl, 3-10-membered heterocyclyl, 6-10-membered aryl, and 5-10-membered heteroaryl, wherein the C₁₋₆ alkyl, C₁₋₆ heteroalkyl, C₂₋₆ alkenyl, C₂₋₆ alkynyl, -C(O)-C₁₋₆ alkyl, C₁₋₆ alkoxy, C₁₋₆ haloalkyl, C₁₋₆ haloalkoxy, C₁₋₆ hydroxyalkyl, C₁₋₆ alkylamino, C₁₋₆ alkylcarbonyl, C₁₋₆ alkoxycarbonyl, C₁₋₆ alkylaminocarbonyl, 3-10-membered cycloalkyl, 3-10-membered heterocyclyl, 6-10-membered aryl, and 5-10-membered heteroaryl are each independently optionally substituted with one or more substituents selected from halogen, alkyl, heteroalkyl, alkoxy, haloalkyl, hydroxyl, hydroxyalkyl, cyano, amino, nitro, cycloalkyl, heterocyclyl, alkenyl, alkynyl, alkylamino, alkylcarbonyl, alkoxycarbonyl, alkylaminocarbonyl, aryl, and heteroaryl; preferably, Rₐ, at each occurrence, is each independently selected from H, deuterium atom, F, Cl, Br, I, C₁₋₆ alkyl, C₁₋₆ deuterated alkyl, C₁₋₆ heteroalkyl, C₂₋₆ alkenyl, C₂₋₆ alkynyl, C₁₋₆ alkoxy, C₁₋₆ haloalkyl, C₁₋₆ haloalkoxy, hydroxyl, C₁₋₆ hydroxyalkyl, and amino; more preferably, Rₐ, at each occurrence, is each independently selected from H, deuterium atom, F, Cl, Br, I, C₁-₃ alkyl, C₁-₃ deuterated alkyl, C₁-₃ alkoxy, C₁-₃ haloalkyl, C₁-₃ haloalkoxy, and hydroxyl;
in the structures of Cy₁, Cy₂, Cy₃, or Cy₄, 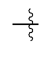 represents the point of attachment to Cy₀;
the compound of Formula I is not the following structures:

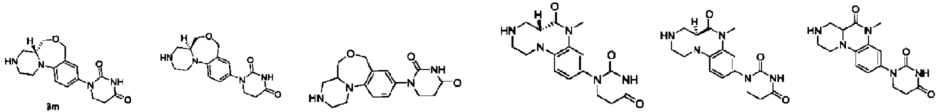

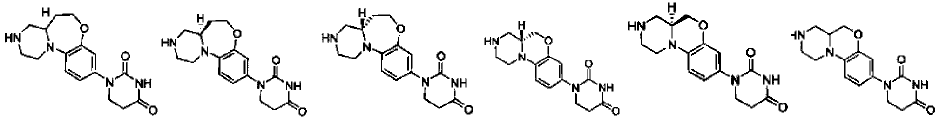

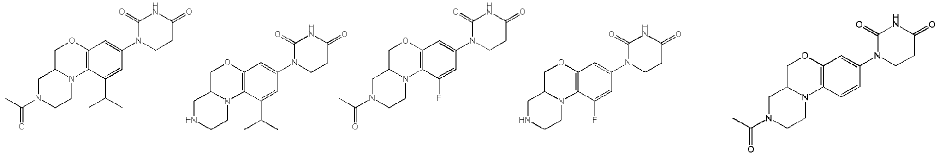

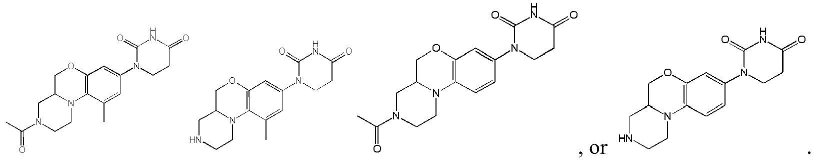

In a second aspect, the present invention provides a compound of Formula 2, or an isomer, an isotopic derivative, a polymorph, a prodrug, or a pharmaceutically acceptable salt or a solvate thereof, 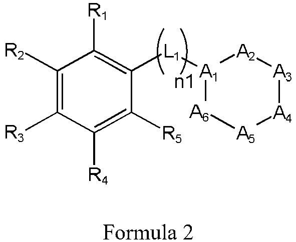 wherein,
A₁ is selected from CRₐ and N;
A₂ and A₄ are each independently selected from C(O) and C(Rₐ)₂;
A₃ is NRₐ;
A₅ and A₆ are each independently selected from a single bond, NRₐ, and C(Rₐ)₂; preferably, A₅ and A₆ are each independently C(Rₐ)₂;
L₁, at each occurrence, is each independently selected from a single bond, alkylene, haloalkylene, -O-, - N(R_{b})C(O)-, -C(O)-, -OC(O)-, -NR_{b}-, -S-, -SO-, -SO₂-, cycloalkylene, heterocyclylene, arylene, and heteroarylene, wherein the cycloalkylene, heterocyclylene, arylene, and heteroarylene are optionally substituted with 0-6 R_{b} groups; preferably, L₁ is a single bond;
n1 is selected from 1, 2, 3, 4, and 5;
R₁ and R₂, together with the atoms to which they are attached, form a 3-10-membered heterocycloalkyl, a 3-10-membered cycloalkyl, a 6-10-membered aryl, a 6-10-membered heteroaryl, a 5-15-membered fused ring group or heterofused ring group, or a 5-15-membered spiro ring or heterospiro ring group; the 3-10-membered heterocycloalkyl, 3-10-membered cycloalkyl, 6-10-membered aryl, 6-10-membered heteroaryl, 5-15-membered fused ring group or heterofused ring group, or 5-15-membered spiro ring or heterospiro ring group are optionally substituted with 1, 2, or more substituents selected from halogen, carboxyl, alkoxy, alkyl, hydroxyl, amino, haloalkyl, 3-10-membered heterocycloalkyl, 3-10-membered cycloalkyl, 6-10-membered aryl, and 6-10-membered heteroaryl; when R₅ is present, R₃, R₄, and R₅, at each occurrence, are each independently selected from H, deuterium atom, carboxyl, halogen, alkyl, deuterated alkyl, heteroalkyl, alkenyl, alkynyl, alkoxy, haloalkyl, haloalkoxy, hydroxyl, hydroxyalkyl, nitro, cyano, amino, alkylamino, alkylcarbonyl, alkoxycarbonyl, alkylaminocarbonyl, cycloalkyl, heterocyclyl, aryl, and heteroaryl; preferably, R₁ and R₂, together with the atoms to which they are attached, form a 5-15-membered heterofused ring group, or a 5-15-membered spiro ring or heterospiro ring group; the 5-15-membered heterofused ring group, or 5-15-membered spiro ring or heterospiro ring group are optionally substituted with 1, 2, or more substituents selected from halogen, carboxyl, alkoxy, alkyl, hydroxyl, amino, haloalkyl, 3-10-membered heterocycloalkyl, 3-10-membered cycloalkyl, 6-10-membered aryl, and 6-10-membered heteroaryl; R₃, R₄, and R₅, at each occurrence, are each independently selected from H, deuterium atom, carboxyl, halogen, alkyl, deuterated alkyl, heteroalkyl, alkenyl, alkynyl, alkoxy, haloalkyl, haloalkoxy, hydroxyl, hydroxyalkyl, nitro, cyano, amino, alkylamino, alkylcarbonyl, alkoxycarbonyl, alkylaminocarbonyl, cycloalkyl, heterocyclyl, aryl, and heteroaryl; or
R₂ and R₃, together with the atoms to which they are attached, form a 3-10-membered heterocycloalkyl, a 3-10-membered cycloalkyl, a 6-10-membered aryl, a 6-10-membered heteroaryl, a 5-15-membered fused ring group or heterofused ring group, or a 5-15-membered spiro ring or heterospiro ring group; the 3-10-membered heterocycloalkyl, 3-10-membered cycloalkyl, 6-10-membered aryl, 6-10-membered heteroaryl, 5-15-membered fused ring group or heterofused ring group, or 5-15-membered spiro ring or heterospiro ring group are optionally substituted with 1, 2, or more substituents selected from halogen, carboxyl, alkoxy, alkyl, hydroxyl, amino, haloalkyl, 3-10-membered heterocycloalkyl, 3-10-membered cycloalkyl, 6-10-membered aryl, and 6-10-membered heteroaryl; R₁, R₄, and R₅, at each occurrence, are each independently selected from H, deuterium atom, carboxyl, halogen, alkyl, deuterated alkyl, heteroalkyl, alkenyl, alkynyl, alkoxy, haloalkyl, haloalkoxy, hydroxyl, hydroxyalkyl, nitro, cyano, amino, alkylamino, alkylcarbonyl, alkoxycarbonyl, alkylaminocarbonyl, cycloalkyl, heterocyclyl, aryl, and heteroaryl; preferably, R₂ and R₃, together with the atoms to which they are attached, form a 5-15-membered fused ring group or heterofused ring group, or a 5-15-membered spiro ring or heterospiro ring group; the 5-15-membered heterofused ring group, or 5-15-membered spiro ring or heterospiro ring group are optionally substituted with 1, 2, or more substituents selected from halogen, carboxyl, alkoxy, alkyl, hydroxyl, amino, haloalkyl, 3-10-membered heterocycloalkyl, 3-10-membered cycloalkyl, 6-10-membered aryl, and 6-10-membered heteroaryl; R₁, R₄, and R₅, at each occurrence, are each independently selected from H, deuterium atom, carboxyl, halogen, alkyl, deuterated alkyl, heteroalkyl, alkenyl, alkynyl, alkoxy, haloalkyl, haloalkoxy, hydroxyl, hydroxyalkyl, nitro, cyano, amino, alkylamino, alkylcarbonyl, alkoxycarbonyl, alkylaminocarbonyl, cycloalkyl, heterocyclyl, aryl, and heteroaryl; or
R₃ and R₄, together with the atoms to which they are attached, form a 3-10-membered heterocycloalkyl, a 3-10-membered cycloalkyl, a 6-10-membered aryl, a 6-10-membered heteroaryl, a 5-15-membered fused ring group or heterofused ring group, or a 5-15-membered spiro ring or heterospiro ring group; the 3-10-membered heterocycloalkyl, 3-10-membered cycloalkyl, 6-10-membered aryl, 6-10-membered heteroaryl, 4-15-membered fused ring group or heterofused ring group, and 5-15-membered spiro ring or heterospiro ring group are optionally substituted with 1, 2, or more substituents selected from halogen, carboxyl, alkoxy, alkyl, hydroxyl, amino, haloalkyl, 3-10-membered heterocycloalkyl, 3-10-membered cycloalkyl, 6-10-membered aryl, and 6-10-membered heteroaryl; R₁, R₂, and R₅, at each occurrence, are each independently selected from H, deuterium atom, carboxyl, halogen, alkyl, deuterated alkyl, heteroalkyl, alkenyl, alkynyl, alkoxy, haloalkyl, haloalkoxy, hydroxyl, hydroxyalkyl, nitro, cyano, amino, alkylamino, alkylcarbonyl, alkoxycarbonyl, alkylaminocarbonyl, cycloalkyl, heterocyclyl, aryl, and heteroaryl; preferably, R₃ and R₄, together with the atoms to which they are attached, form a 5-15-membered heterofused ring group, or a 5-15-membered spiro ring or heterospiro ring group; the 5-15-membered heterofused ring group, or 5-15-membered spiro ring or heterospiro ring group are optionally substituted with 1, 2, or more substituents selected from halogen, carboxyl, alkoxy, alkyl, hydroxyl, amino, haloalkyl, 3-10-membered heterocycloalkyl, 3-10-membered cycloalkyl, 6-10-membered aryl, and 6-10-membered heteroaryl; R₁, R₄, and R₅, at each occurrence, are each independently selected from H, deuterium atom, carboxyl, halogen, alkyl, deuterated alkyl, heteroalkyl, alkenyl, alkynyl, alkoxy, haloalkyl, haloalkoxy, hydroxyl, hydroxyalkyl, nitro, cyano, amino, alkylamino, alkylcarbonyl, alkoxycarbonyl, alkylaminocarbonyl, cycloalkyl, heterocyclyl, aryl, and heteroaryl; or
R₄ and R₅, together with the atoms to which they are attached, form a 3-10-membered heterocycloalkyl, a 3-10-membered cycloalkyl, a 6-10-membered aryl, a 6-10-membered heteroaryl, a 5-15-membered fused ring group or heterofused ring group, or a 5-15-membered spiro ring or heterospiro ring group; the 3-10-membered heterocycloalkyl, 3-10-membered cycloalkyl, 6-10-membered aryl, 6-10-membered heteroaryl, 5-15-membered fused ring group or heterofused ring group, or 5-15-membered spiro ring or heterospiro ring group are optionally substituted with 1, 2, or more substituents selected from halogen, carboxyl, alkoxy, alkyl, hydroxyl, amino, haloalkyl, 3-10-membered heterocycloalkyl, 3-10-membered cycloalkyl, 6-10-membered aryl, and 6-10-membered heteroaryl; R₁, R₂, and R₃, at each occurrence, are each independently selected from H, deuterium atom, carboxyl, halogen, alkyl, deuterated alkyl, heteroalkyl, alkenyl, alkynyl, alkoxy, haloalkyl, haloalkoxy, hydroxyl, hydroxyalkyl, nitro, cyano, amino, alkylamino, alkylcarbonyl, alkoxycarbonyl, alkylaminocarbonyl, cycloalkyl, heterocyclyl, aryl, and heteroaryl; preferably, R₄ and R₅, together with the atoms to which they are attached, form a 5-15-membered heterofused ring group, or a 5-15-membered spiro ring or heterospiro ring group; the 5-15-membered heterofused ring group, or 5-15-membered spiro ring or heterospiro ring group are optionally substituted with 1, 2, or more substituents selected from halogen, carboxyl, alkoxy, alkyl, hydroxyl, amino, haloalkyl, 3-10-membered heterocycloalkyl, 3-10-membered cycloalkyl, 6-10-membered aryl, and 6-10-membered heteroaryl; R₁, R₂, and R₃, at each occurrence, are each independently selected from H, deuterium atom, carboxyl, halogen, alkyl, deuterated alkyl, heteroalkyl, alkenyl, alkynyl, alkoxy, haloalkyl, haloalkoxy, hydroxyl, hydroxyalkyl, nitro, cyano, amino, alkylamino, alkylcarbonyl, alkoxycarbonyl, alkylaminocarbonyl, cycloalkyl, heterocyclyl, aryl, and heteroaryl;
Rₐ and R_{b}, at each occurrence, are each independently selected from H, deuterium atom, halogen, alkyl, deuterated alkyl, carboxyl, heteroalkyl, alkenyl, alkynyl, alkoxy, haloalkyl, haloalkoxy, hydroxyl, hydroxyalkyl, nitro, cyano, amino, alkylamino, alkylcarbonyl, alkoxycarbonyl, alkylaminocarbonyl, cycloalkyl, heterocyclyl, aryl, and heteroaryl, wherein the alkyl, heteroalkyl, alkenyl, alkynyl, alkoxy, cycloalkyl, heterocyclyl, aryl, and heteroaryl are each independently optionally substituted with one or more substituents selected from halogen, alkyl, heteroalkyl, alkoxy, haloalkyl, hydroxyl, hydroxyalkyl, cyano, amino, nitro, cycloalkyl, heterocyclyl, alkenyl, alkynyl, alkylamino, alkylcarbonyl, alkoxycarbonyl, alkylaminocarbonyl, aryl, and heteroaryl; preferably, Rₐ and R_{b}, at each occurrence, are each independently selected from H, F, Cl, Br, I, C₁-₃ alkyl, C₁-₃ alkoxy, hydroxyl, amino, and -C(O)-C₁-₃ alkyl;
the compound is not the following structures:

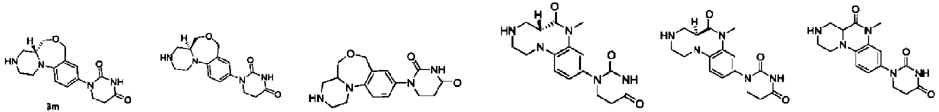

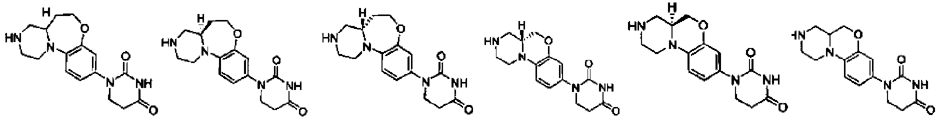

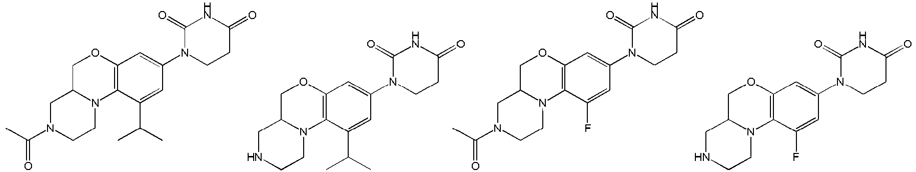

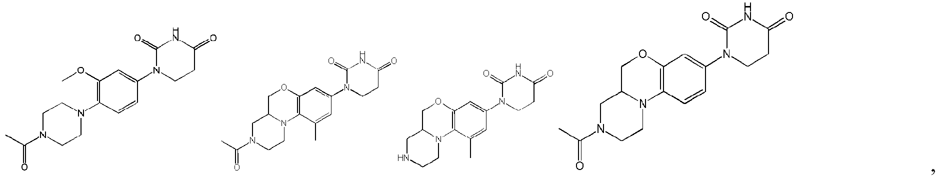

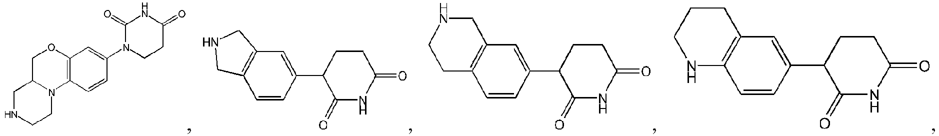

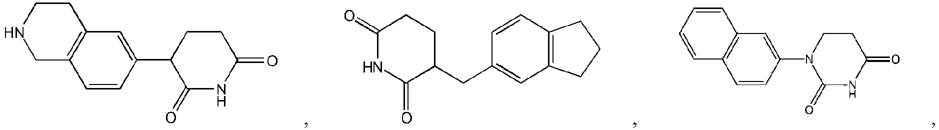

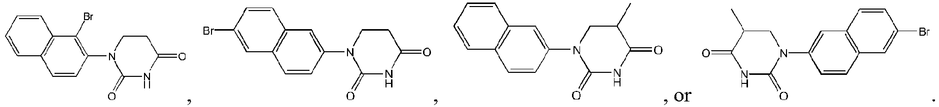

In one embodiment, wherein, at least one of A₂ and A₄ is C(O); preferably, both A and A₄ are C(O); and/or
A₃ is NH; and/or
A₅ and A₆ are each independently selected from a single bond and C(H)₂; preferably, A₅ and A₆ are C(H)₂; and/or
L₁, at each occurrence, is each independently selected from a single bond, C₁₋₆ alkylene, C₁₋₆ haloalkylene, -O-, -N(R_{b})C(O)-, -C(O)-, -OC(O)-, -NR_{b}-, -S-, -SO-, -SO₂-, C₃-₁₅ cycloalkylene, C₃-₁₅ heterocyclylene, C₆-₁₅ arylene, and C₅-₁₅ heteroarylene, wherein the cycloalkylene, heterocyclylene, arylene, and heteroarylene are optionally substituted with 0-6 R_{b} groups; preferably, L₁ is a single bond; and/or
n1 is selected from 1, 2, 3; and/or
R₁ and R₂, together with the atoms to which they are attached, form a 3-10-membered saturated or unsaturated heterocycloalkyl containing 1, 2, or 3 N, O, S atoms, a 6-10-membered aryl, 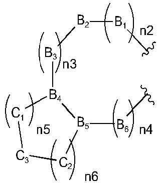 or 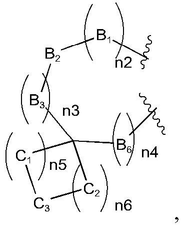 wherein the 3-10-membered saturated or unsaturated heterocycloalkyl containing 1, 2, or 3 N, O, S atoms, and 6-10-membered aryl are optionally substituted with 1, 2, or more substituents selected from halogen, carboxyl, C₁₋₆ alkoxy, C₁₋₆ alkyl, hydroxyl, amino, C₁₋₆ haloalkyl, 3-10-membered heterocycloalkyl, 3-10-membered cycloalkyl, 6-10-membered aryl, and 6-10-membered heteroaryl; R₃, R₄, and R₅, at each occurrence, are each independently selected from H, deuterium atom, carboxyl, halogen, C₁₋₆ alkyl, C₁₋₆ deuterated alkyl, C₁₋₆ heteroalkyl, alkenyl, alkynyl, C₁₋₆ alkoxy, C₁₋₆ haloalkyl, C₁₋₆ haloalkoxy, hydroxyl, C₁₋₆ hydroxyalkyl, nitro, cyano, amino, C₁₋₆ alkylamino, C₁₋₆ alkylcarbonyl, C₁₋₆ alkoxycarbonyl, C₁₋₆ alkylaminocarbonyl, cycloalkyl, heterocyclyl, aryl, and heteroaryl; preferably, R₁ and R₂, together with the atoms to which they are attached, form 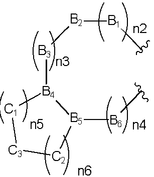 or 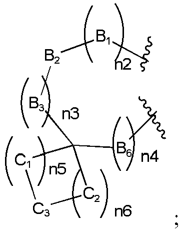
R₂ and R₃, together with the atoms to which they are attached, form a 3-10-membered saturated or unsaturated heterocycloalkyl containing 1, 2, or 3 N, O, S atoms, a 6-10-membered aryl, 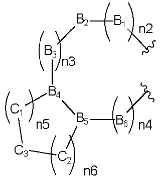 or 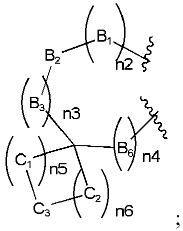 wherein the 3-10-membered saturated or unsaturated heterocycloalkyl containing 1, 2, or 3 N, O, S atoms, and 6-10-membered aryl are optionally substituted with 1, 2, or more substituents selected from halogen, carboxyl, alkoxy, alkyl, hydroxyl, amino, haloalkyl, 3-10-membered heterocycloalkyl, 3-10-membered cycloalkyl, 6-10-membered aryl, and 6-10-membered heteroaryl; when R₅ is present, R₁, R₄, and R₅, at each occurrence, are each independently selected from H, deuterium atom, carboxyl, halogen, C₁₋₆ alkyl, C₁₋₆ deuterated alkyl, C₁₋₆ heteroalkyl, alkenyl, alkynyl, C₁₋₆ alkoxy, C₁₋₆ haloalkyl, C₁₋₆ haloalkoxy, hydroxyl, C₁₋₆ hydroxyalkyl, nitro, cyano, amino, C₁₋₆ alkylamino, C₁₋₆ alkylcarbonyl, C₁₋₆ alkoxycarbonyl, C₁₋₆ alkylaminocarbonyl, cycloalkyl, heterocyclyl, aryl, and heteroaryl; preferably, R₂ and R₃, together with the atoms to which they are attached, form 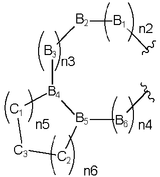 or 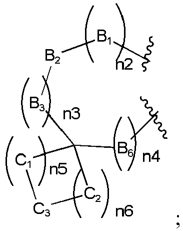 or
R₃ and R₄, together with the atoms to which they are attached, form a 3-10-membered saturated or unsaturated heterocycloalkyl containing 1, 2, or 3 N, O, S atoms, a 6-10-membered aryl ring, 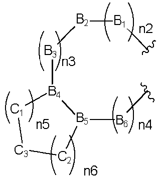 or 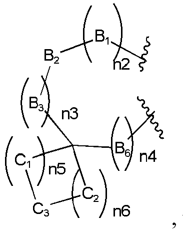 wherein the 3-10-membered saturated or unsaturated heterocycloalkyl containing 1, 2, or 3 N, O, S atoms, and 6-10-membered aryl are optionally substituted with 1, 2, or more substituents selected from halogen, carboxyl, alkoxy, alkyl, hydroxyl, amino, haloalkyl, 3-10-membered heterocycloalkyl, 3-10-membered cycloalkyl, 6-10-membered aryl, and 6-10-membered heteroaryl; when R₅ is present, R₁, R₂, and R₅, at each occurrence, are each independently selected from H, deuterium atom, carboxyl, halogen, C₁₋₆ alkyl, C₁₋₆ deuterated alkyl, C₁₋₆ heteroalkyl, alkenyl, alkynyl, C₁₋₆ alkoxy, C₁₋₆ haloalkyl, C₁₋₆ haloalkoxy, hydroxyl, C₁₋₆ hydroxyalkyl, nitro, cyano, amino, C₁₋₆ alkylamino, C₁₋₆ alkylcarbonyl, C₁₋₆ alkoxycarbonyl, C₁₋₆ alkylaminocarbonyl, cycloalkyl, heterocyclyl, aryl, and heteroaryl; preferably, R₃ and R₄, together with the atoms to which they are attached, form 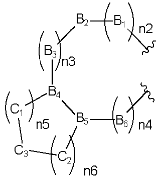 or 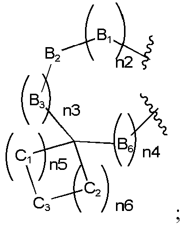 or
R₄ and R₅, together with the atoms to which they are attached, form a 3-10-membered saturated or unsaturated heterocycloalkyl containing 1, 2, or 3 N, O, S atoms, a 6-10-membered aryl, 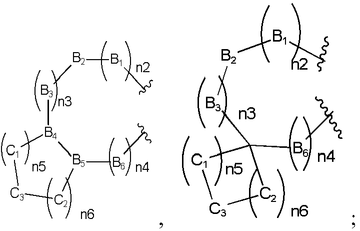 the 3-10-membered saturated or unsaturated heterocycloalkyl containing 1, 2, or 3 N, O, S atoms, and 6-10-membered aryl are optionally substituted with 1, 2, or more substituents selected from halogen, carboxyl, alkoxy, alkyl, hydroxyl, amino, haloalkyl, 3-10-membered heterocycloalkyl, 3-10-membered cycloalkyl, 6-10-membered aryl, and 6-10-membered heteroaryl; R₁, R₂, and R₃, at each occurrence, are each independently selected from H, deuterium atom, carboxyl, halogen, C₁₋₆ alkyl, C₁₋₆ deuterated alkyl, C₁₋₆ heteroalkyl, alkenyl, alkynyl, C₁₋₆ alkoxy, C₁₋₆ haloalkyl, C₁₋₆ haloalkoxy, hydroxyl, C₁₋₆ hydroxyalkyl, nitro, cyano, amino, C₁₋₆ alkylamino, C₁₋₆ alkylcarbonyl, C₁₋₆ alkoxycarbonyl, C₁₋₆ alkylaminocarbonyl, cycloalkyl, heterocyclyl, aryl, and heteroaryl; preferably, R₄ and R₅ together with the atoms to which they are attached form 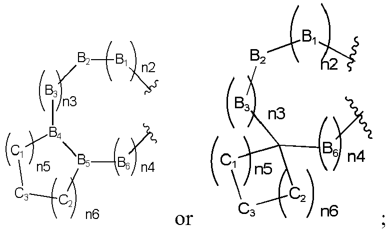
B₂, at each occurrence, is selected from C(Rₐ)₂, NRₐ, O, and S;
B₄ and B₅, at each occurrence, are each independently selected from CRₐ and N;
C₃, at each occurrence, is selected from C(O), CR_{c}R_{d}, and NRₐ;
R_{c} and R_{d}, at each occurrence, are each independently selected from H, deuterium atom, halogen, C₁₋₆ alkyl, C₁₋₆ deuterated alkyl, carboxyl, C₁₋₆ heteroalkyl, alkenyl, alkynyl, C₁₋₆ alkoxy, C₁₋₆ haloalkyl, C₁₋₆ haloalkoxy, hydroxyl, C₁₋₆ hydroxyalkyl, nitro, cyano, amino, C₁₋₆ alkylamino, C₁₋₆ alkylcarbonyl, C₁₋₆ alkoxycarbonyl, C₁₋₆ alkylaminocarbonyl, cycloalkyl, heterocyclyl, aryl, and heteroaryl, wherein the alkyl, heteroalkyl, alkenyl, alkynyl, alkoxy, cycloalkyl, heterocyclyl, aryl, and heteroaryl are each independently optionally substituted with one or more substituents selected from halogen, alkyl, heteroalkyl, alkoxy, haloalkyl, hydroxyl, hydroxyalkyl, cyano, amino, nitro, cycloalkyl, heterocyclyl, alkenyl, alkynyl, alkylamino, alkylcarbonyl, alkoxycarbonyl, alkylaminocarbonyl, aryl, and heteroaryl; or R_{c} and R_{d}, together with the carbon atom to which they are attached, form a 3-10-membered heterocycloalkyl, a 3-10-membered cycloalkyl, a 6-10-membered aryl, or a 6-10-membered heteroaryl, wherein the 3-10-membered heterocycloalkyl, 3-10-membered cycloalkyl, 6-10-membered aryl, and 6-10-membered heteroaryl are optionally substituted with 1, 2, or more substituents selected from halogen, carboxyl, alkoxy, alkyl, hydroxyl, amino, haloalkyl, 3-10-membered heterocycloalkyl, 3-10-membered cycloalkyl, 6-10-membered aryl, and 6-10-membered heteroaryl;
n2, n3, n4, n5, and n6 are each independently selected from 0, 1, 2, and 3;
B₁, B₃, B₆, C₁, C₂, D₁, and D₂, at each occurrence, are each independently selected from C(Rₐ)₂;
D₃ is selected from C(O) and NRₐ; and/or
Rₐ and R_{b}, at each occurrence, are each independently selected from H, deuterium atom, F, Cl, Br, I, C₁₋₆ alkyl, deuterated C₁₋₆ alkyl, carboxyl, C₁₋₆ heteroalkyl, C₂₋₆ alkenyl, C₂₋₆ alkynyl, C₁₋₆ alkoxy, C₁₋₆ haloalkyl, C₁₋₆ haloalkoxy, hydroxyl, C₁₋₆ hydroxyalkyl, nitro, cyano, amino, C₁₋₆ alkylamino, C₁₋₆ alkylcarbonyl, C₁₋₆ alkoxycarbonyl, C₁₋₆ alkylaminocarbonyl, 3-10-membered cycloalkyl, 3-10-membered heterocyclyl, 6-10-membered aryl, and 5-10-membered heteroaryl, wherein the alkyl, heteroalkyl, alkenyl, alkynyl, alkoxy, cycloalkyl, heterocyclyl, aryl, and heteroaryl are each independently optionally substituted with one or more substituents selected from halogen, alkyl, heteroalkyl, alkoxy, haloalkyl, hydroxyl, hydroxyalkyl, cyano, amino, nitro, cycloalkyl, heterocyclyl, alkenyl, alkynyl, alkylamino, alkylcarbonyl, alkoxycarbonyl, alkylaminocarbonyl, aryl, and heteroaryl; 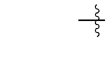 represents the point of attachment.

In one embodiment, the compound is selected from the following structures: 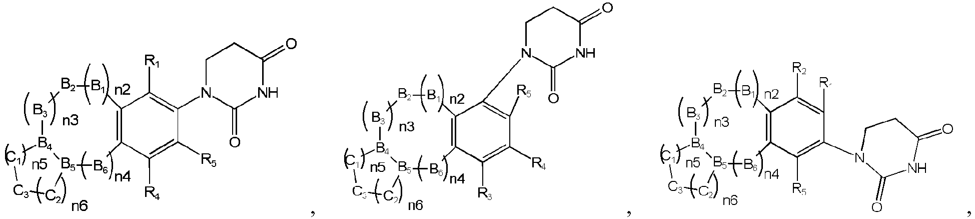 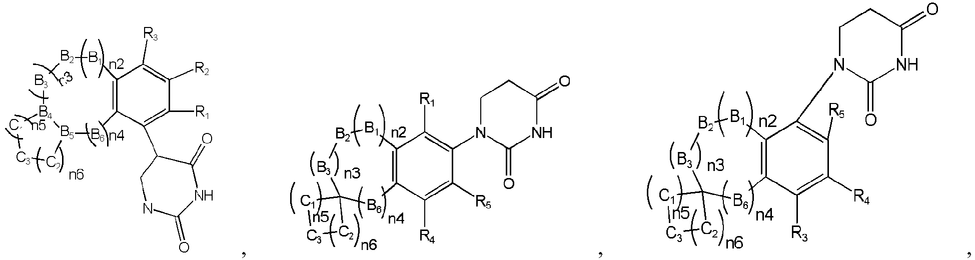 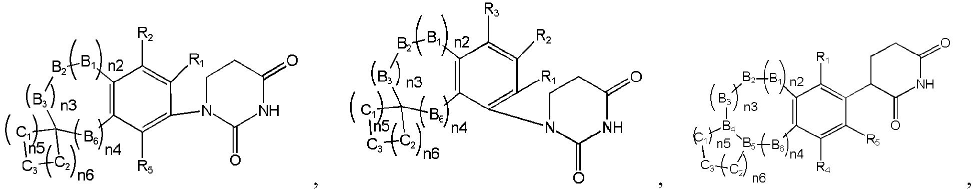 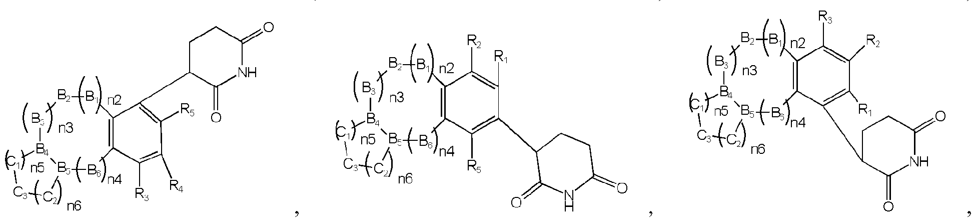 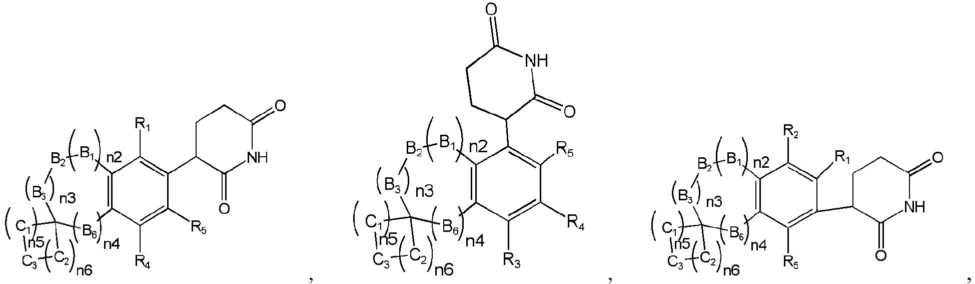 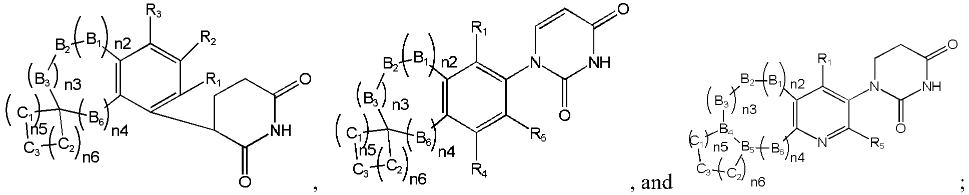
wherein, R₁, R₂, R₃, R₄, R₅, B₁, B₂, B₃, B₄, B₅, B₆, C₁, C₂, C₃, Rₐ, n2, n3, n4, n5, and n6 are as defined above;
preferably:
   R₁, R₂, R₃, R₄, and R₅ are each independently selected from H, F, Cl, Br, I, C₁-₃ alkyl, C₁-₃ alkoxy, hydroxyl, and amino;
   B₂, at each occurrence, is selected from C(Rₐ)₂, NRₐ, O, and S;
   B₁ and B₃, at each occurrence, are each independently selected from C(Rₐ)₂ and CO;
   B₆, at each occurrence, is selected from C(Rₐ)₂ and NRₐ;
   One of B₄ and B₅ is N, and the other is CRₐ;
   C₁ and C₂, at each occurrence, are each independently C(Rₐ)₂;
   C₃ is selected from C(O) and NRₐ;
   Rₐ, at each occurrence, is each independently selected from H, F, Cl, Br, I, C₁-₃ alkyl, C₁-₃ alkoxy, hydroxyl, amino, and -C(O)-C₁-₃ alkyl; and
   n2, n3, n4, n5, and n6 are each independently selected from 0, 1, 2, and 3.

In some embodiments, the compound is 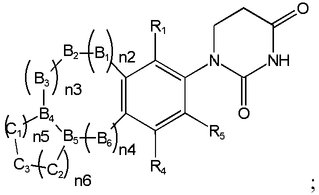 wherein each group is as defined in any one of the preceding embodiments.

In some embodiments, the compound is 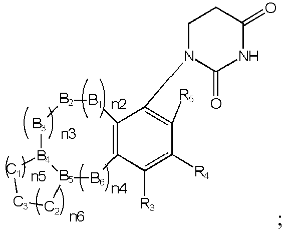 wherein each group is as defined in any one of the preceding embodiments.

In some embodiments, the compound is 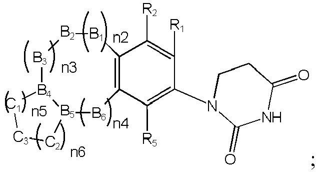 wherein each group is as defined in any one of the preceding embodiments.

In some embodiments, the compound is 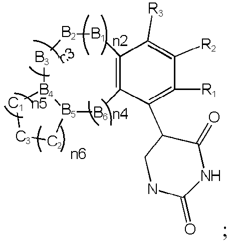 wherein each group is as defined in any one of the preceding embodiments.

In some embodiments, the compound is 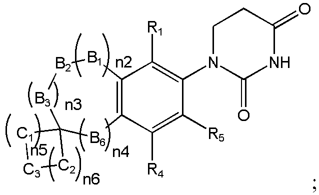 wherein each group is as defined in any one of the preceding embodiments.

In some embodiments, the compound is 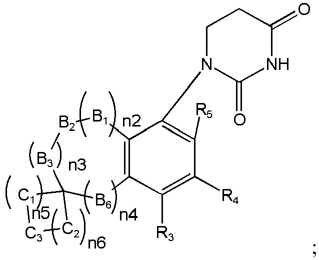 wherein each group is as defined in any one of the preceding embodiments.

In some embodiments, the compound is 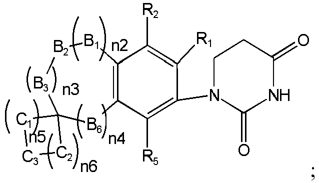 wherein each group is as defined in any one of the preceding embodiments.

In some embodiments, the compound is 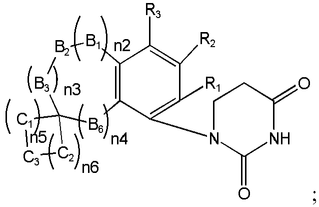 wherein each group is as defined in any one of the preceding embodiments.

In some embodiments, the compound is 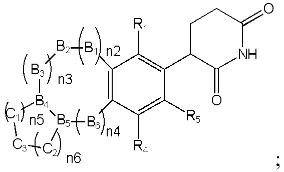 wherein each group is as defined in any one of the preceding embodiments.

In some embodiments, the compound is 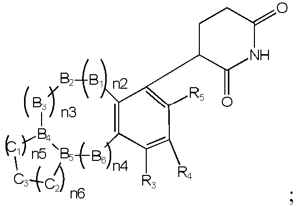 wherein each group is as defined in any one of the preceding embodiments.

In some embodiments, the compound is 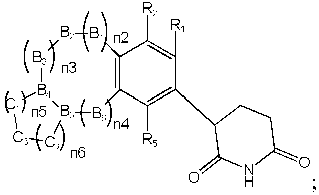 wherein each group is as defined in any one of the preceding embodiments.

In some embodiments, the compound is 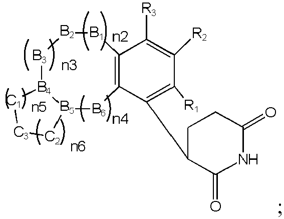 wherein each group is as defined in any one of the preceding embodiments.

In some embodiments, the compound is 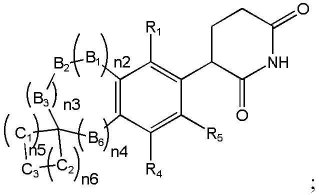 wherein each group is as defined in any one of the preceding embodiments.

In some embodiments, the compound is 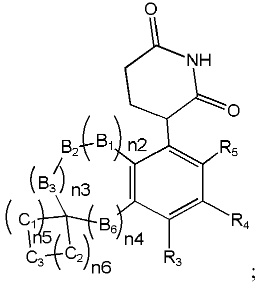 wherein each group is as defined in any one of the preceding embodiments.

In some embodiments, the compound is 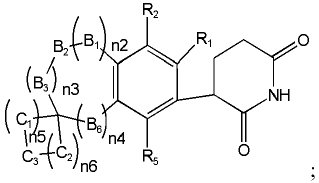 wherein each group is as defined in any one of the preceding embodiments.

In some embodiments, the compound is 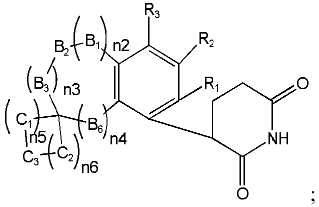 wherein each group is as defined in any one of the preceding embodiments.

In some embodiments, the compound is 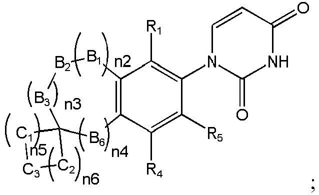 wherein each group is as defined in any one of the preceding embodiments.

In some embodiments, the compound is 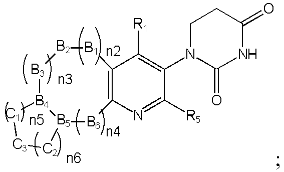 wherein each group is as defined in any one of the preceding embodiments.

In some embodiments, the compound is selected from any one of the following structures: 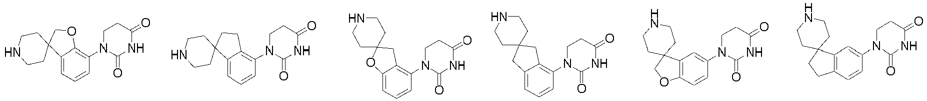 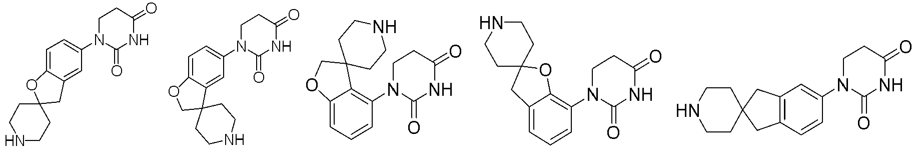 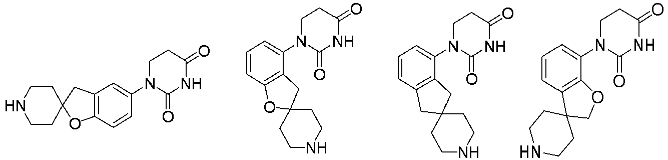 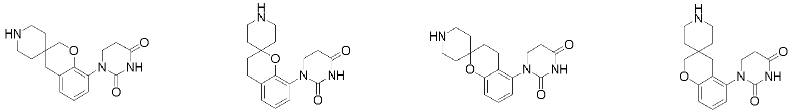 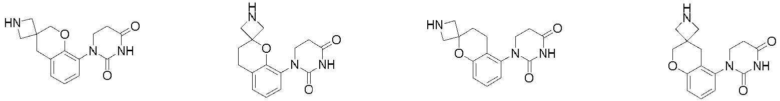 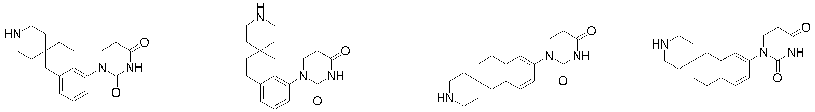 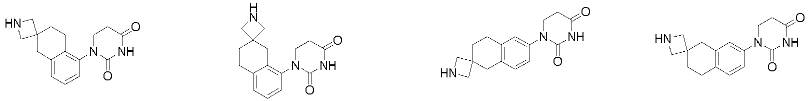 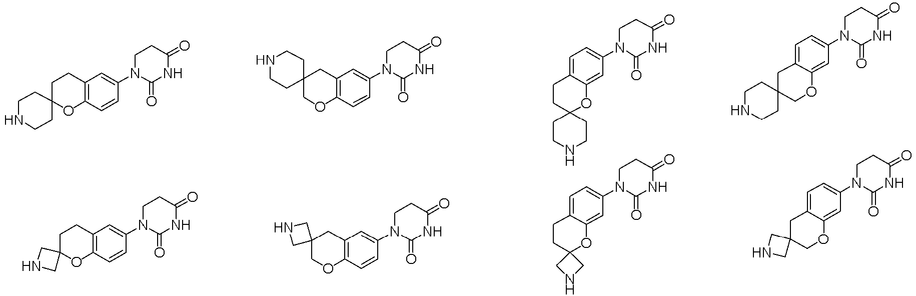 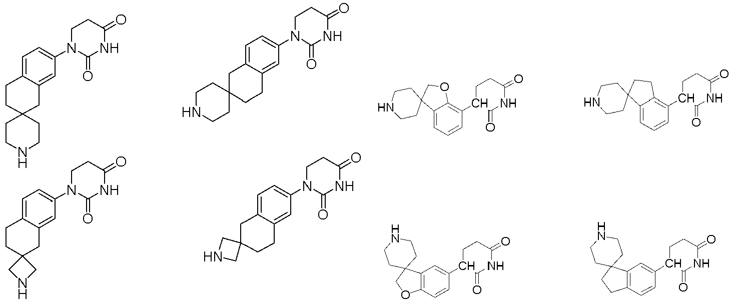 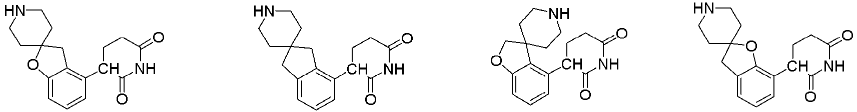 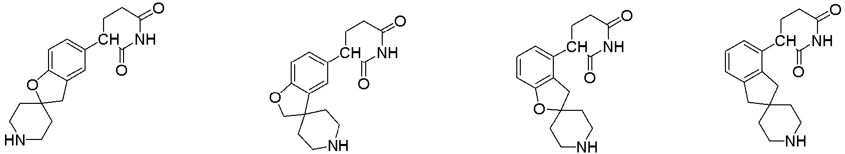 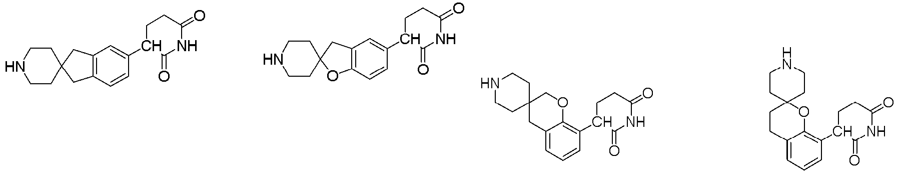 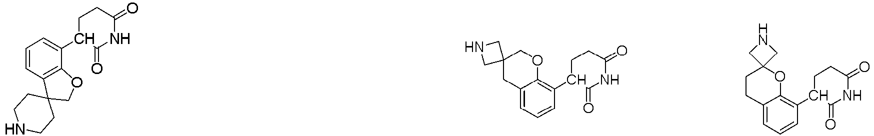 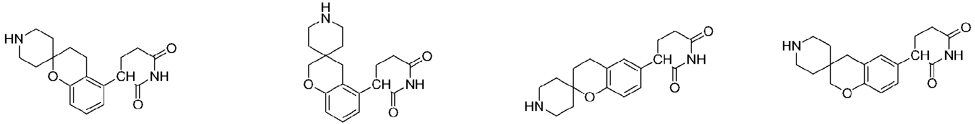 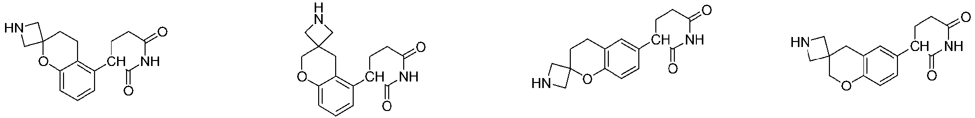 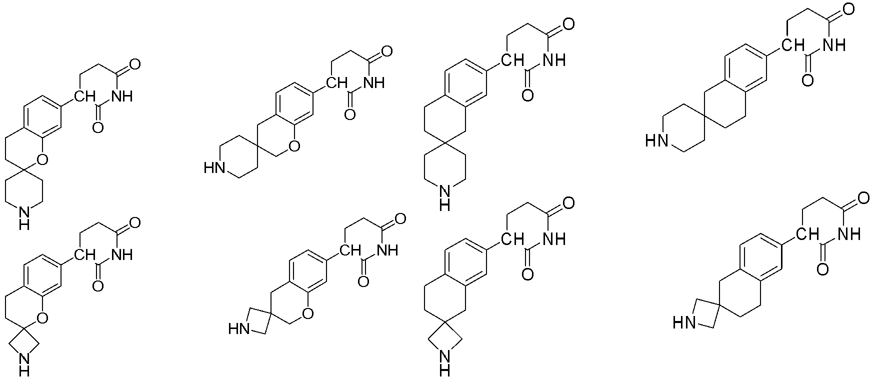 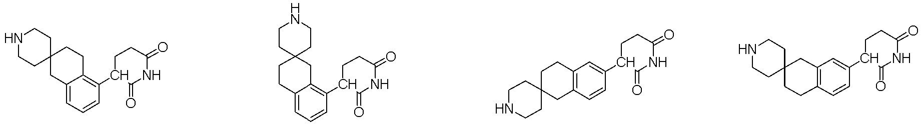 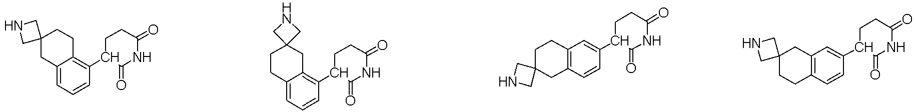 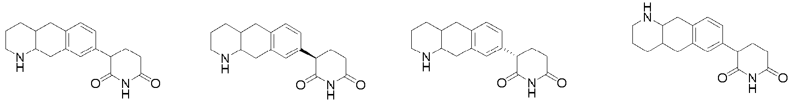 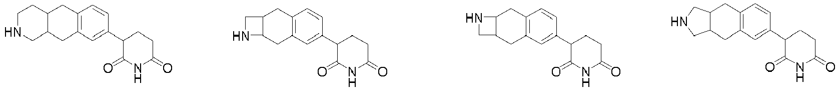 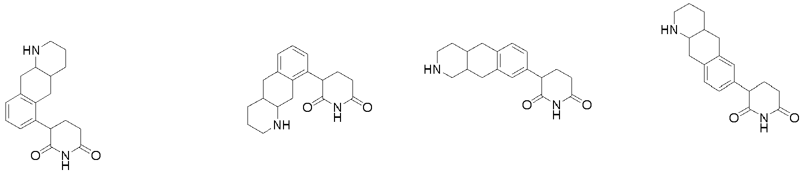 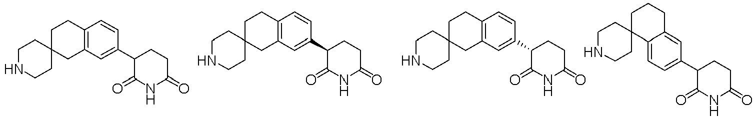 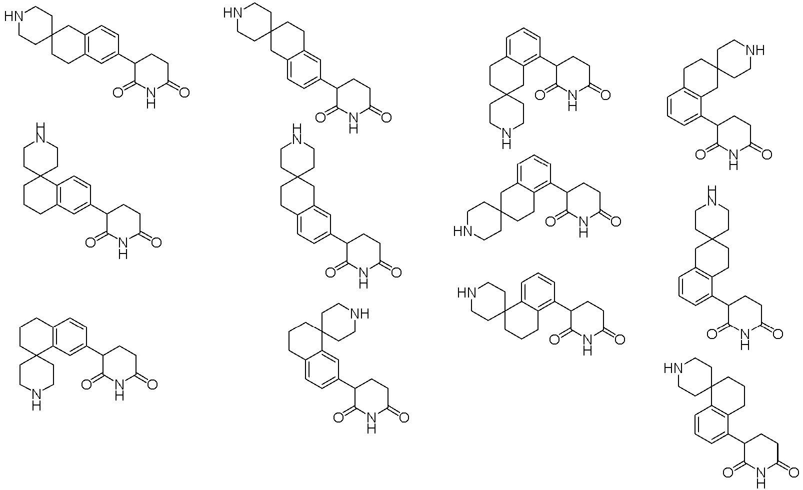 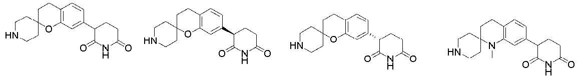 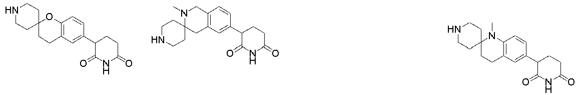 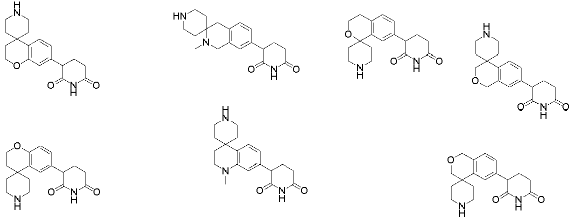 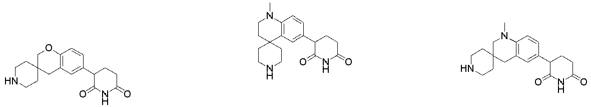 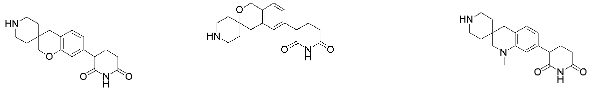 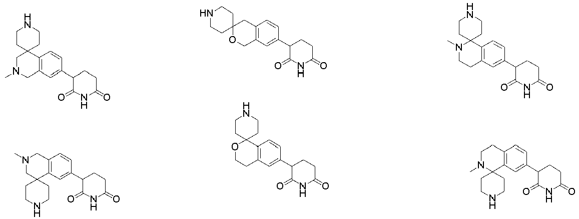 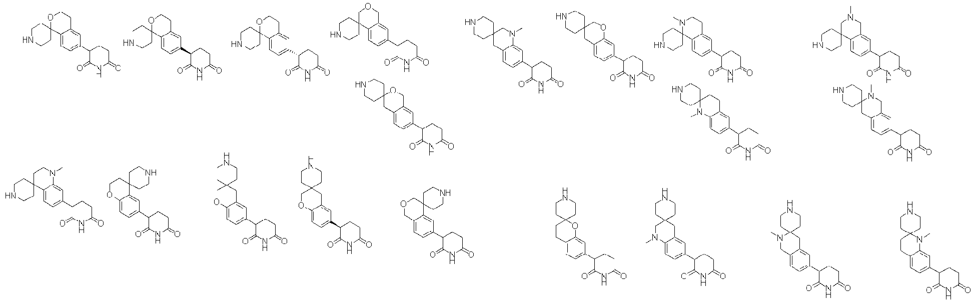 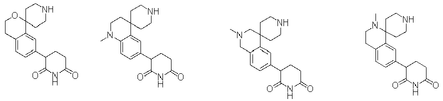 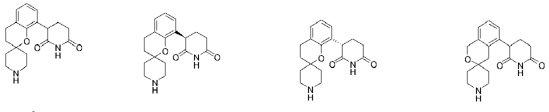 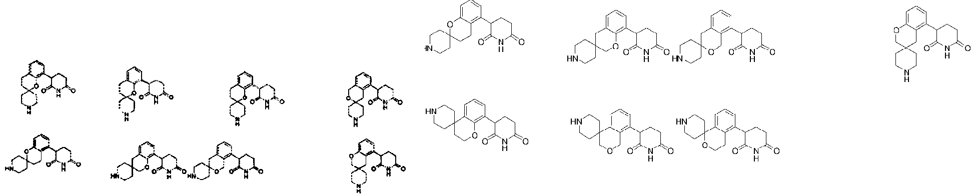 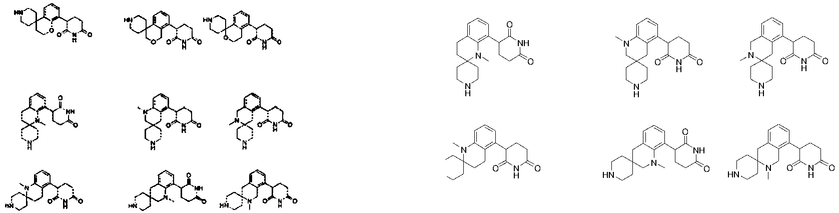 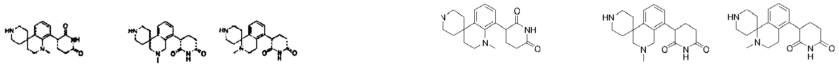 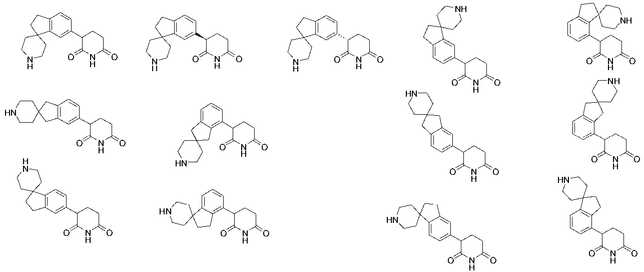 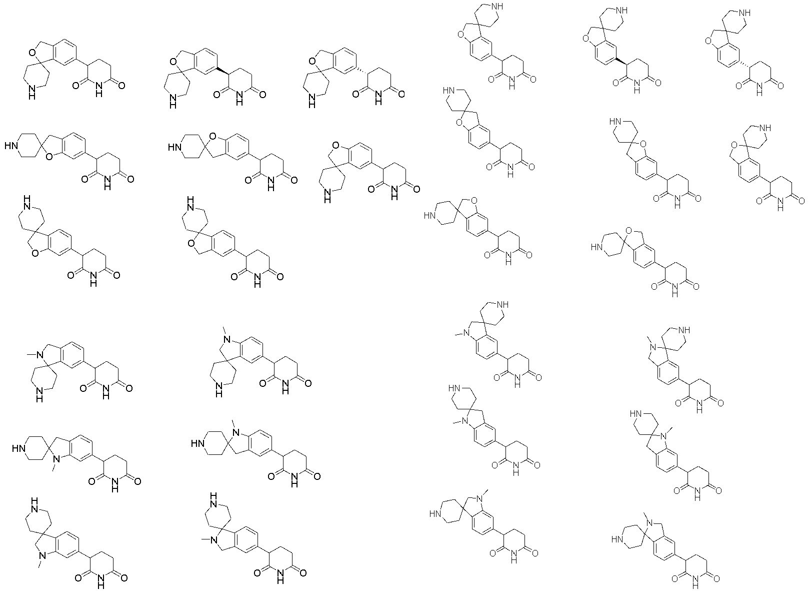 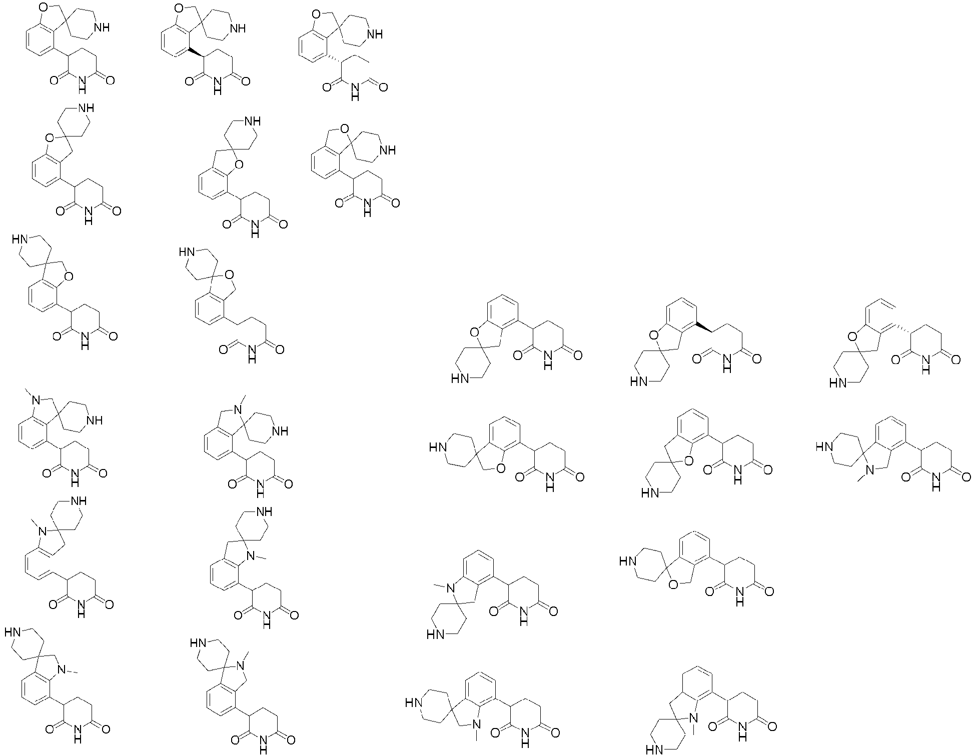 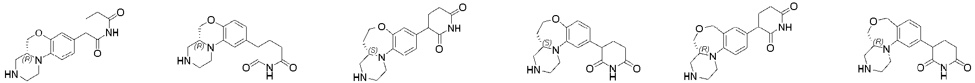 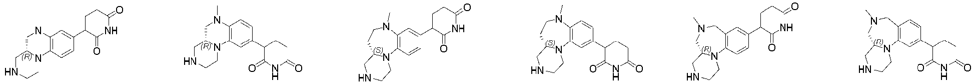 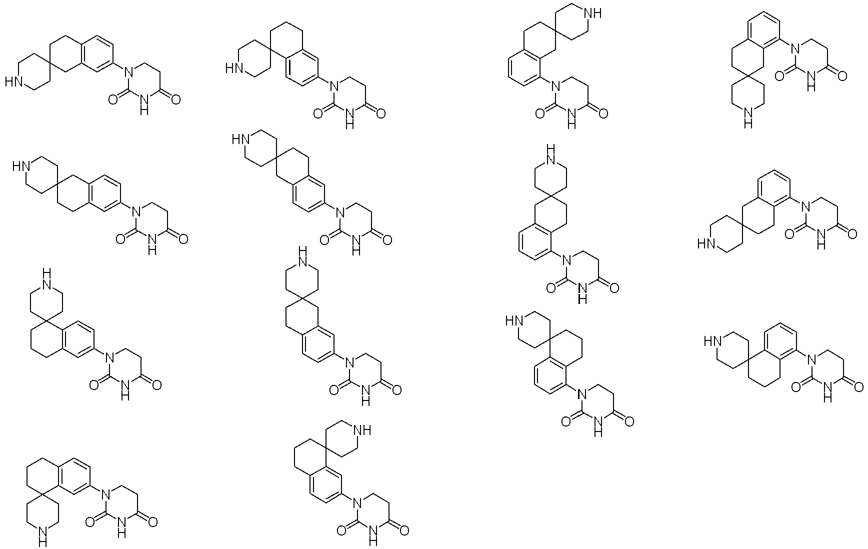 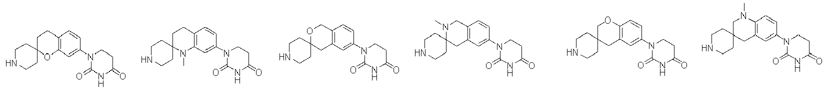 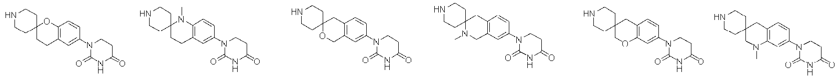 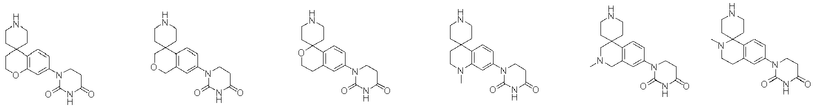 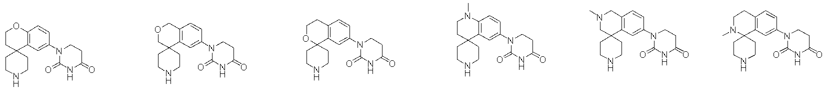 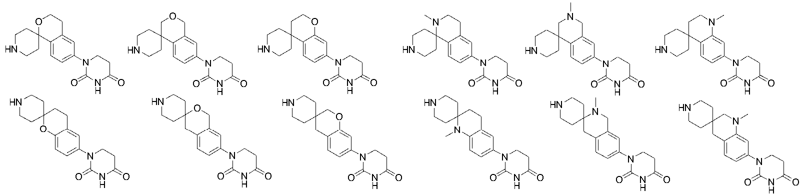 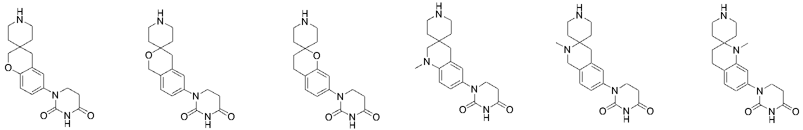 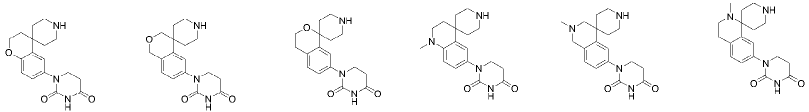 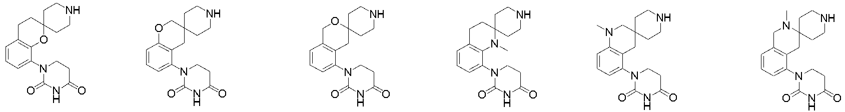 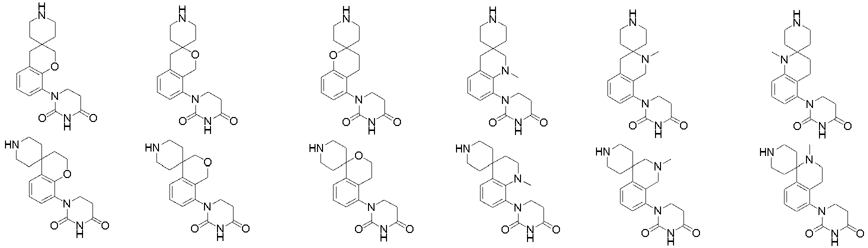 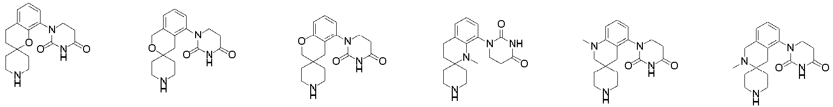 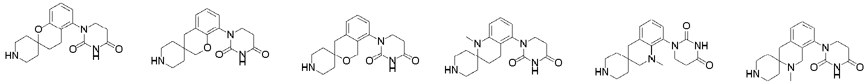 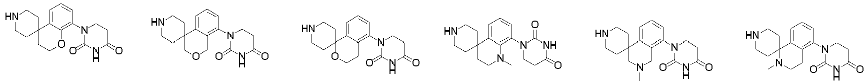 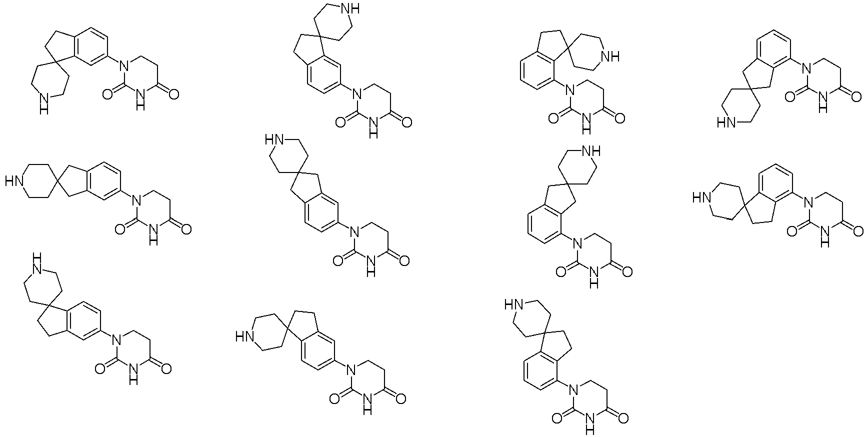 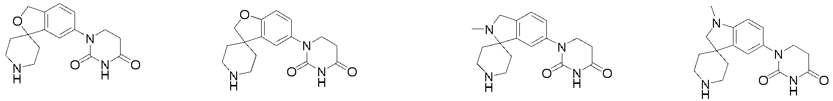 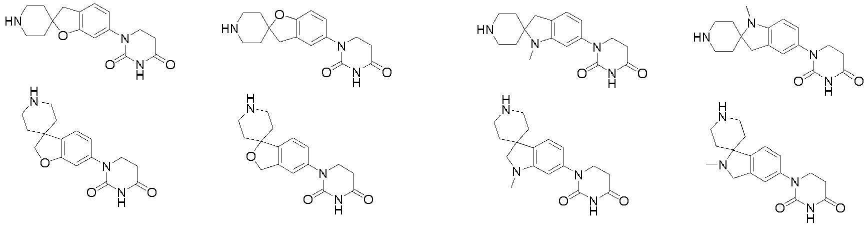 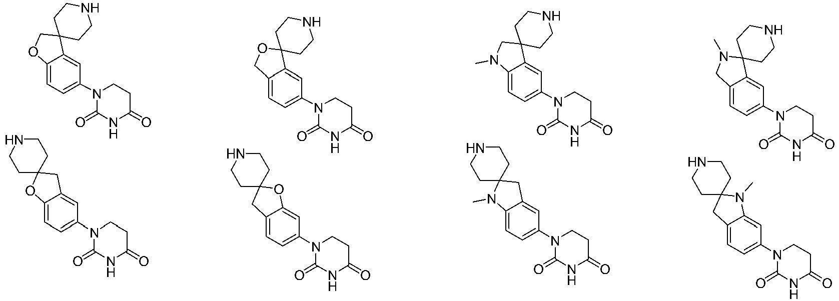 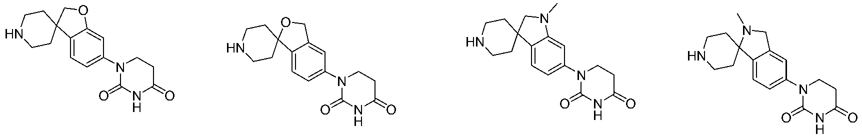 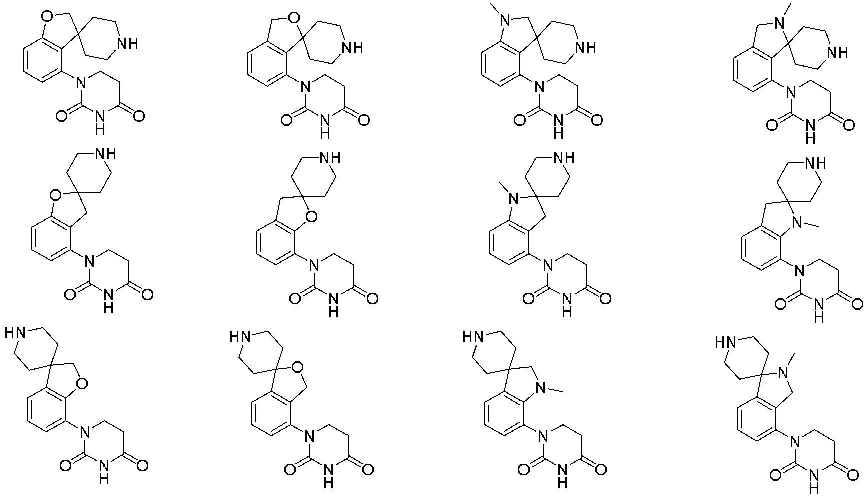 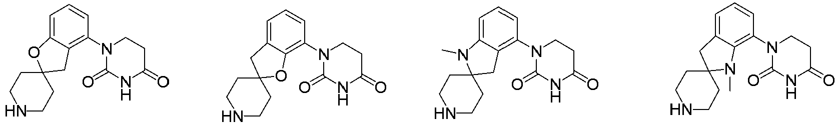 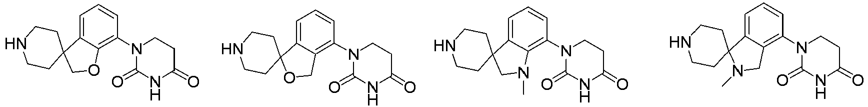 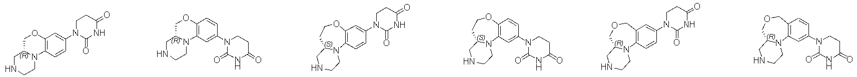 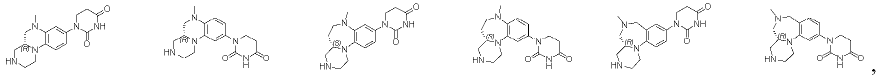 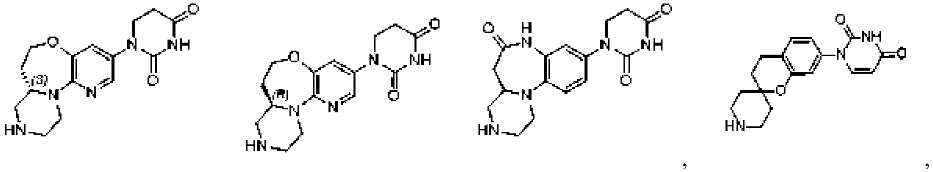 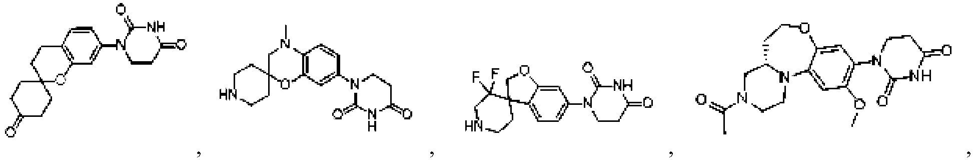 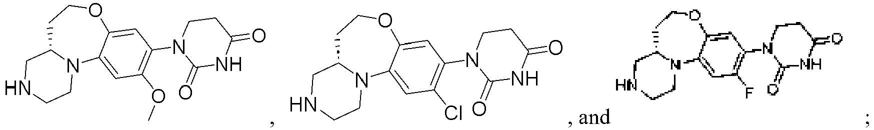 preferably, the compound is selected from: 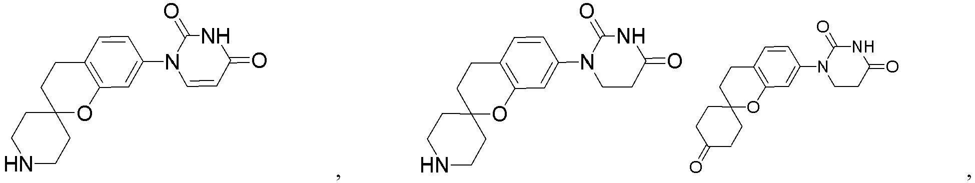 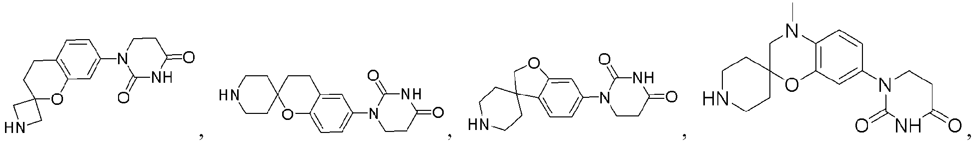 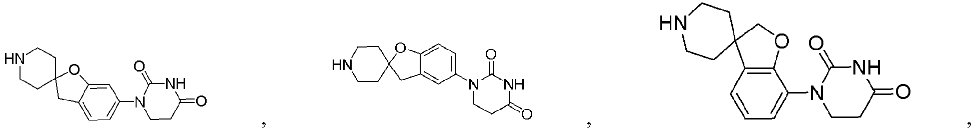 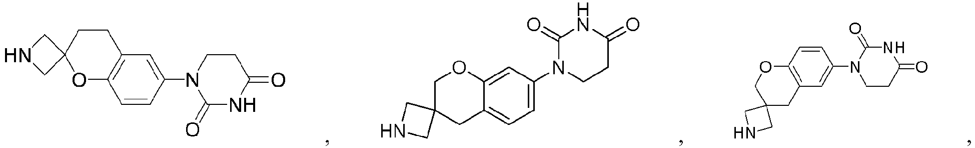 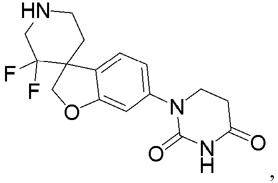 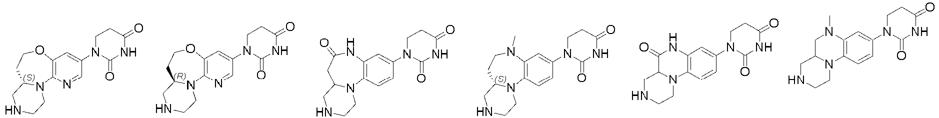 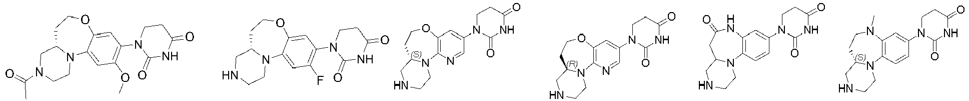 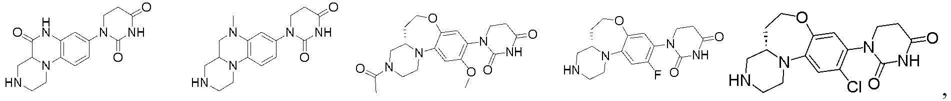 and

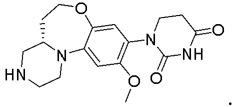

In a third aspect, the present invention provides a compound represented by Formula II, or an isomer, an isotopic a derivative, a polymorph, a prodrug, or a pharmaceutically acceptable salt or a solvate thereof,

PTM-L-CLM (Formula II)

wherein, PTM is a target protein binding moiety;
L is a bond or a chemical linking moiety that covalently connects the PTM and CLM moieties;

In some embodiments, the compound of Formula (II) is preferably a proteolysis targeting chimera (PROTAC), wherein the proteolysis targeting chimera comprises a target protein binding moiety, an E3 ligase ligand moiety, and a linker moiety; the PTM is the target protein binding moiety, the CLM is the E3 ligase ligand moiety, and the L is the linker moiety.

In some embodiments, the target protein is selected from B7.1, B7, TNFR2, NADPH oxidase, BclIBax and other partners in the apoptosis pathway, C5a receptor, HMG-CoA reductase, PDE V phosphodiesterase type, PDEIV phosphodiesterase type 4, PDEI I, PDEI II, PDE III, squalene epoxidase, CXCR1, CXCR2, nitric oxide (NO) synthase, cyclooxygenase 1, cyclooxygenase 2, 5HT receptor, dopamine receptor, G protein (i.e., Gq), histamine receptor, 5-lipoxygenase, tryptase serine protease, thymidylate synthase, purine nucleoside phosphorylase, GAPDH trypanosome, glycogen phosphorylase, carbonic anhydrase, chemokine receptor, JAW STAT, RXR and analogs thereof, HIV1 protease, HIV1 integrase, influenza neuraminidase, hepatitis B reverse transcriptase, sodium channel, multi-drug resistance bacteria, P-glycoprotein, tyrosine kinase, CD23, CD124, tyrosine kinase p56lck, CD4, CD5, IL-2 receptor, IL-1 receptor, TNF-αR, ICAM1, Ca²⁺ channel, VCAM, VLA-4 integrin, selectin, CD40/CD40L, inosine monophosphate dehydrogenase, p38 MAP kinase, Ras/Raf/MEWERK pathway, interleukin-1 converting enzyme, caspase, HCV NS3 protease, HCV NS3 RNA helicase, glycinamide ribonucleotide formyltransferase, rhinovirus 3C protease, herpes simplex virus-I (HSV-I) protease, cytomegalovirus (CMV) protease, poly(ADP-ribose) polymerase, cyclin-dependent kinase 4/6, vascular endothelial growth factor, oxytocin receptor, microsomal transfer protein inhibitors, bile acid transport inhibitors, 5α reductase inhibitors, angiotensin II, glycine receptor, norepinephrine reuptake receptor, endothelin receptor, neuropeptide Y and receptor thereof, adenosine receptor, adenosine kinase and AMP deaminase, purinergic receptor (P2Y1, P2Y2, P2Y4, P2Y6, P2X1-7), farnesyl transferase, geranylgeranyl transferase, TrkA receptor of NGF, β-amyloid, tyrosine kinase Flk-II/KDR, vitronectin receptor, integrin receptor, Her-2/neu, telomerase inhibitor, cytosolic phospholipase A2, ecdysone 20-monooxygenase, ion channel of GABA-gated chloride channel, acetylcholinesterase, voltage-sensitive sodium channel protein, calcium release channel and chloride channel, acetyl-CoA carboxylase, adenylosuccinate synthetase, protoporphyrinogen oxidase, enolpyruvylshikimate phosphate synthase, MYC protein, androgen receptor, estrogen receptor, Aurora kinase A, Bcr-Abl protein, epidermal growth factor receptor, anaplastic lymphoma kinase, Bruton's tyrosine kinase, bromodomain protein 4, and interleukin-1 receptor-associated kinase 4 (IRAK4); preferably, the target protein is selected from the androgen receptor, estrogen receptor, and interleukin-1 receptor-associated kinase 4 (IRAK4).

In some embodiments, CLM is a cereblon E3 ubiquitin ligase binding moiety selected from any one of the following structures: 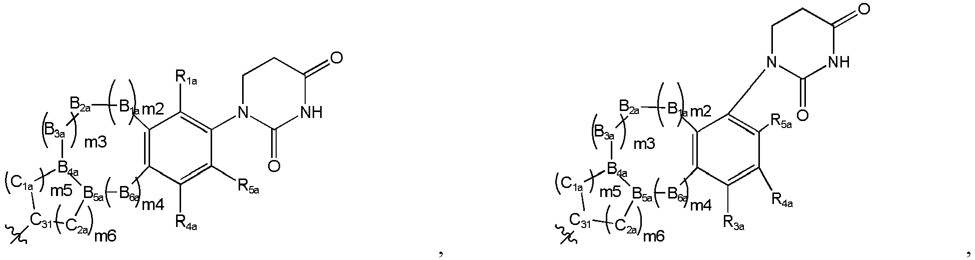 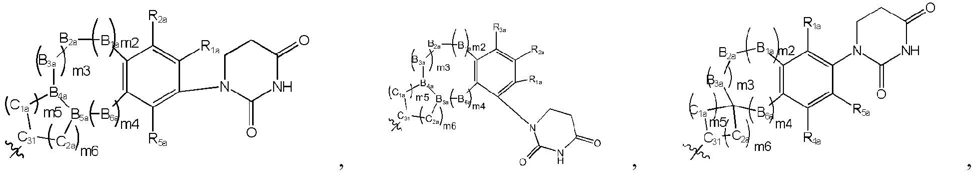 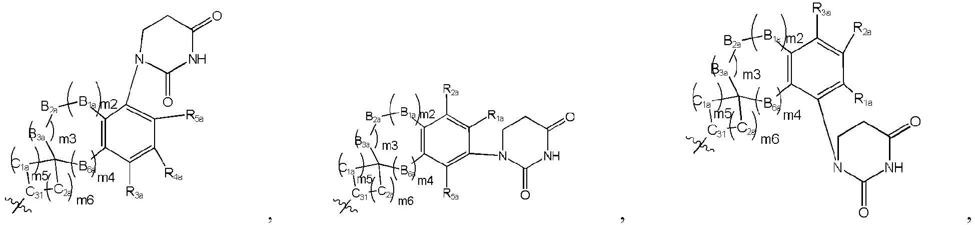 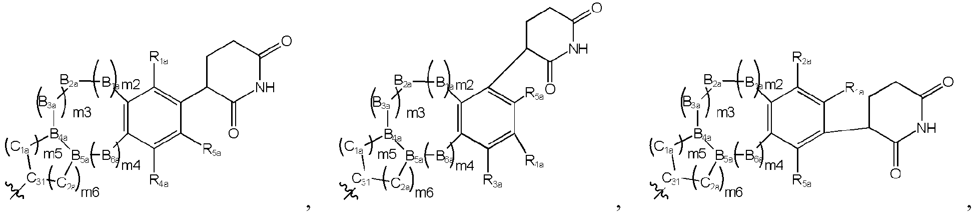 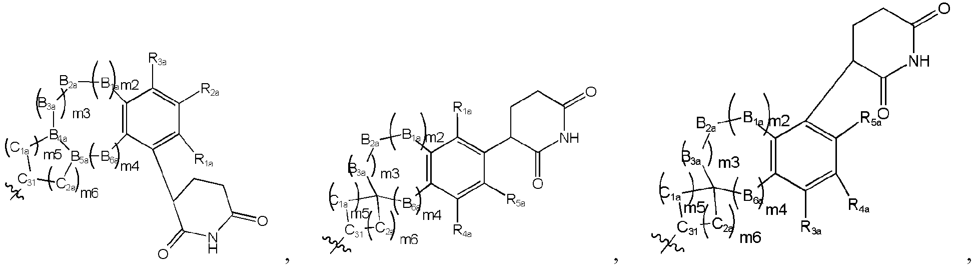 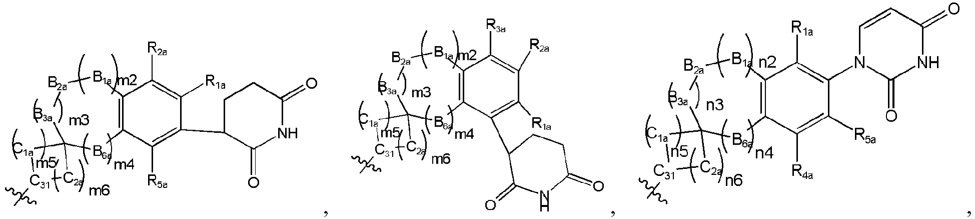 and 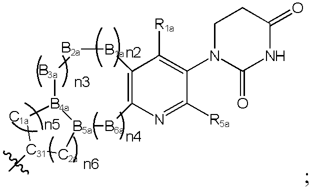
R₁ₐ, R₂ₐ, R₃ₐ, R₄ₐ, and R₅ₐ, at each occurrence, are each independently selected from H, deuterium atom, halogen, C₁₋₆ alkyl, C₁₋₆ deuterated alkyl, carboxyl, C₁₋₆ heteroalkyl, alkenyl, alkynyl, C₁₋₆ alkoxy, C₁₋₆ haloalkyl, C₁₋₆ haloalkoxy, hydroxyl, C₁₋₆ hydroxyalkyl, nitro, cyano, amino, C₁₋₆ alkylamino, C₁₋₆ alkylcarbonyl, C₁₋₆ alkoxycarbonyl, C₁₋₆ alkylaminocarbonyl, cycloalkyl, heterocyclyl, aryl, and heteroaryl, wherein the C₁₋₆ alkyl, C₁₋₆ heteroalkyl, alkenyl, alkynyl, C₁₋₆ alkoxy, C₁₋₆ haloalkyl, C₁₋₆ haloalkoxy, C₁₋₆ hydroxyalkyl, C₁₋₆ alkylamino, C₁₋₆ alkylcarbonyl, C₁₋₆ alkoxycarbonyl, C₁₋₆ alkylaminocarbonyl, cycloalkyl, heterocyclyl, aryl, and heteroaryl are each independently optionally substituted with one or more substituents selected from halogen, alkyl, heteroalkyl, alkoxy, haloalkyl, hydroxyl, hydroxyalkyl, cyano, amino, nitro, cycloalkyl, heterocyclyl, alkenyl, alkynyl, alkylamino, alkylcarbonyl, alkoxycarbonyl, alkylaminocarbonyl, aryl, and heteroaryl; preferably, R₁ₐ, R₂ₐ, R₃ₐ, R₄ₐ, and R₅ₐ, at each occurrence, are each independently selected from H, deuterium atom, halogen, C₁₋₆ alkyl, C₁₋₆ deuterated alkyl, C₁₋₆ heteroalkyl, C₂₋₆ alkenyl, C₂₋₆ alkynyl, C₁₋₆ alkoxy, C₁₋₆ haloalkyl, C₁₋₆ haloalkoxy, hydroxyl, C₁₋₆ hydroxyalkyl, and amino; more preferably, R₁ₐ, R₂ₐ, R₃ₐ, R₄ₐ, and R₅ₐ, at each occurrence, are each independently selected from H, deuterium atom, F, Cl, Br, I, C₁-₃ alkyl, C₁-₃ deuterated alkyl, C₁-₃ alkoxy, C₁-₃ haloalkyl, C₁-₃ haloalkoxy, amino, and hydroxyl; further preferably, R₁ₐ, R₂ₐ, R₃ₐ, R₄ₐ, and R₅ₐ, at each occurrence, are each independently selected from H, F, Cl, Br, I, C₁-₃ alkyl, C₁-₃ alkoxy, hydroxyl, and amino;
B₂ₐ is selected from C(Rₐₐ)₂, NRₐₐ, O, and S; and when B₄ₐ and B₅ₐ are present, B₂ₐ is selected from NRₐₐ, O, and S;
B₄ₐ and B₅ₐ are each independently selected from CRₐₐ and N; preferably, one of B₄ₐ and B₅ₐ is N, and the other is CRₐₐ;
B₁ₐ and B₃ₐ, at each occurrence, are each independently selected from C(Rₐₐ)₂ and C(O);
B₆ₐ, at each occurrence, is selected from C(Rₐₐ)₂ and NRₐₐ;
C₁ₐ and C₂ₐ, at each occurrence, are each independently C(Rₐₐ)₂;
m2, m3, m4, m5, and m6 are each independently selected from 0, 1, 2, and 3; and m5 and m6 are not both 0;
C₃₁ is selected from N and CR_{d};
Rₐₐ and R_{d}, at each occurrence, are each independently selected from H, deuterium atom, F, Cl, Br, I, C₁₋₆ alkyl, deuterated C₁₋₆ alkyl, carboxyl, C₁₋₆ heteroalkyl, C₂₋₆ alkenyl, C₂₋₆ alkynyl, -CO-C₁₋₆ alkyl, C₁₋₆ alkoxy, C₁₋₆ haloalkyl, C₁₋₆ haloalkoxy, hydroxyl, C₁₋₆ hydroxyalkyl, nitro, cyano, amino, C₁₋₆ alkylamino, C₁₋₆ alkylcarbonyl, C₁₋₆ alkoxycarbonyl, C₁₋₆ alkylaminocarbonyl, 3-10-membered cycloalkyl, 3-10-membered heterocyclyl, 6-10-membered aryl, and 5-10-membered heteroaryl, wherein the C₁₋₆ alkyl, C₁₋₆ heteroalkyl, C₂₋₆ alkenyl, C₂₋₆ alkynyl, -CO-C₁₋₆ alkyl, C₁₋₆ alkoxy, C₁₋₆ haloalkyl, C₁₋₆ haloalkoxy, C₁₋₆ hydroxyalkyl, C₁₋₆ alkylamino, C₁₋₆ alkylcarbonyl, C₁₋₆ alkoxycarbonyl, C₁₋₆ alkylaminocarbonyl, 3-10-membered cycloalkyl, 3-10-membered heterocyclyl, 6-10-membered aryl, and 5-10-membered heteroaryl are each independently optionally substituted with one or more substituents selected from halogen, alkyl, heteroalkyl, alkoxy, haloalkyl, hydroxyl, hydroxyalkyl, cyano, amino, nitro, cycloalkyl, heterocyclyl, alkenyl, alkynyl, alkylamino, alkylcarbonyl, alkoxycarbonyl, alkylaminocarbonyl, aryl, and heteroaryl; preferably, Rₐₐ and R_{d}, at each occurrence, are each independently selected from H, deuterium atom, F, Cl, Br, I, C₁₋₆ alkyl, deuterated C₁₋₆ alkyl, carboxyl, C₁₋₆ heteroalkyl, C₂₋₆ alkenyl, C₂₋₆ alkynyl, -CO-C₁₋₆ alkyl, C₁₋₆ alkoxy, C₁₋₆ haloalkyl, C₁₋₆ haloalkoxy, hydroxyl, C₁₋₆ hydroxyalkyl, nitro, cyano, amino, C₁₋₆ alkylamino, C₁₋₆ alkylcarbonyl, C₁₋₆ alkoxycarbonyl, and C₁₋₆ alkylaminocarbonyl; more preferably, Rₐₐ and R_{d}, at each occurrence, are each independently selected from H, F, Cl, Br, I, C₁₋₆ alkyl, carboxyl, C₁₋₆ heteroalkyl, C₂₋₆ alkenyl, C₂₋₆ alkynyl, -CO-C₁₋₆ alkyl, C₁₋₆ alkoxy, C₁₋₆ haloalkyl, C₁₋₆ haloalkoxy, hydroxyl, C₁₋₆ hydroxyalkyl, nitro, cyano, and amino; further preferably, Rₐₐ and R_{d}, at each occurrence, are each independently selected from H, F, Cl, Br, I, C₁-₃ alkyl, C₁-₃ alkoxy, hydroxyl, amino, and -C(O)-C₁-₃ alkyl;
the PTM is not 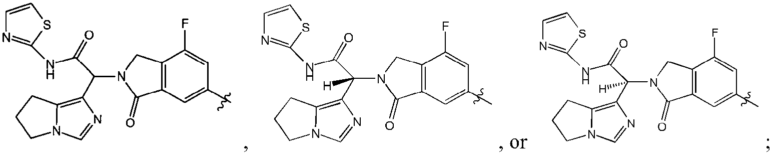 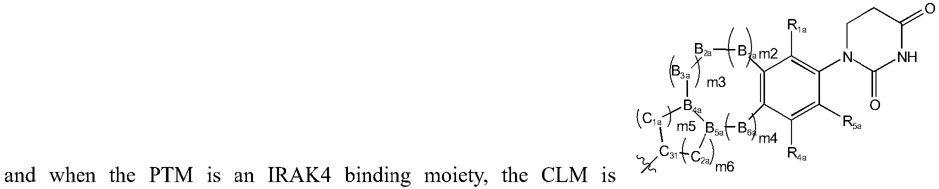 or 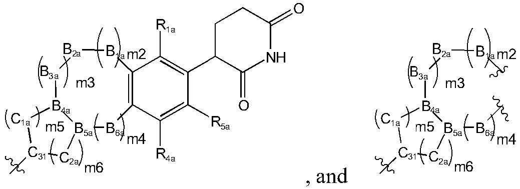 is not

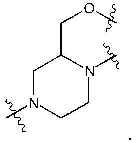

In one embodiment, the CLM is selected from any one of the following structures: 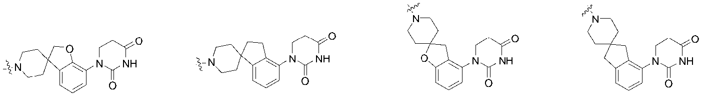 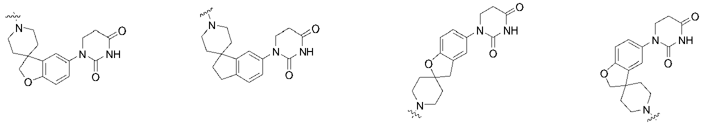 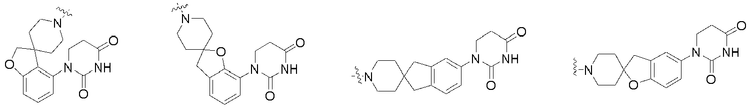 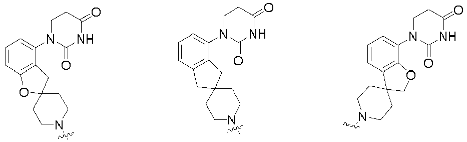 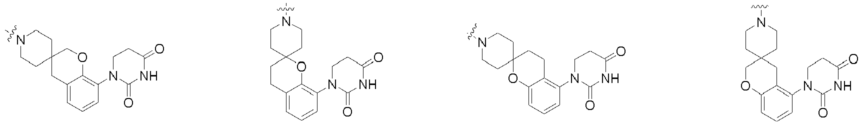 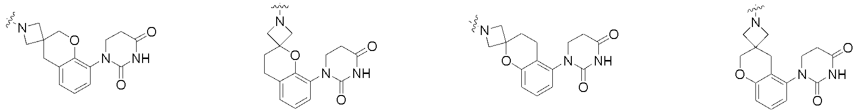 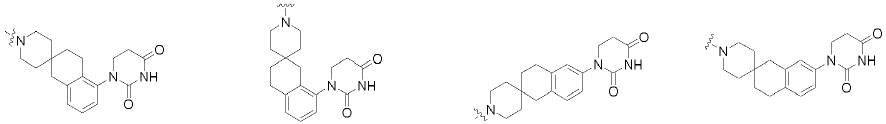 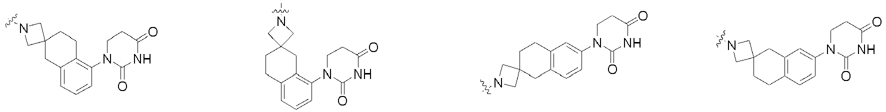 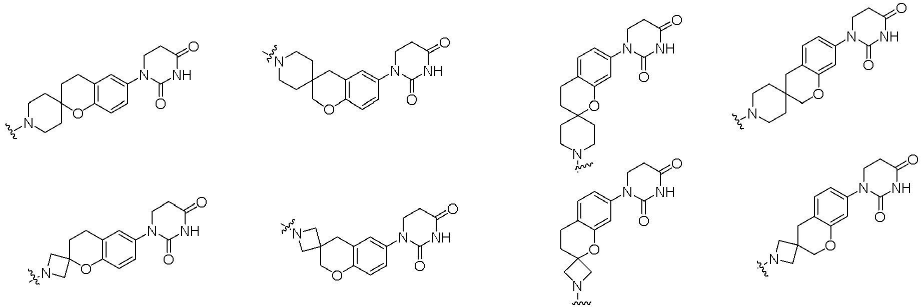 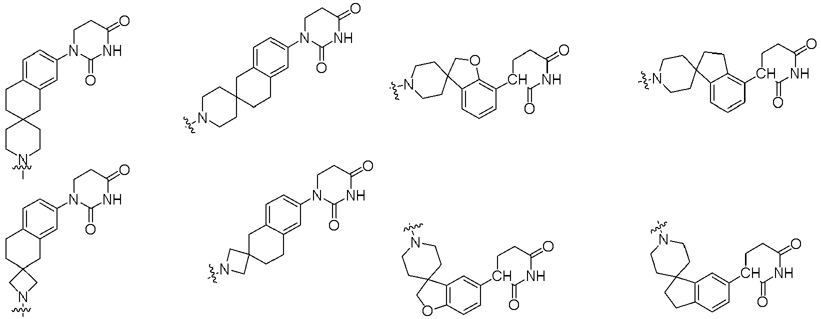 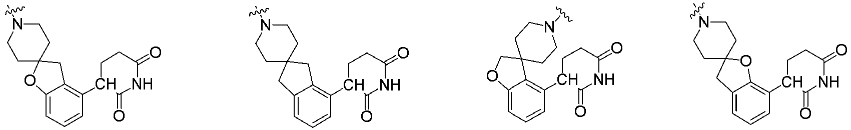 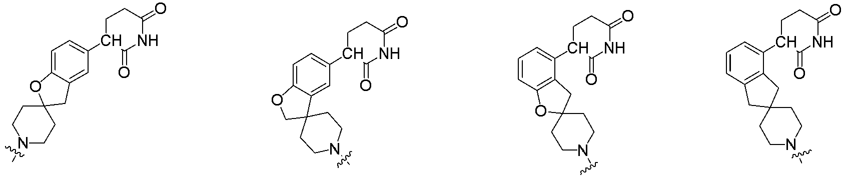 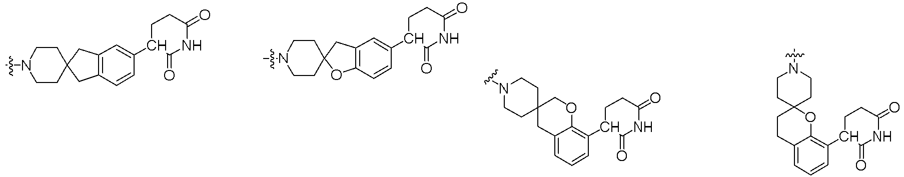 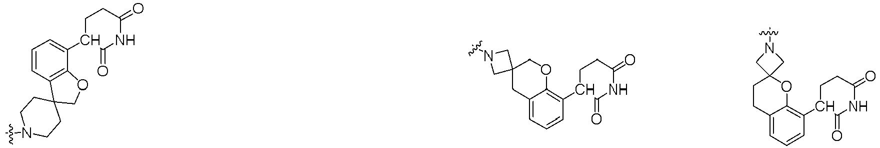 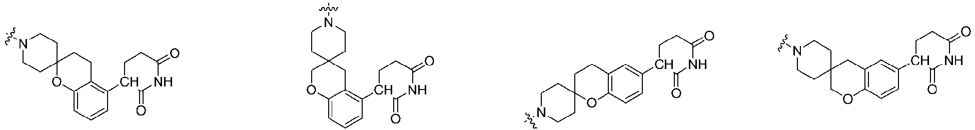 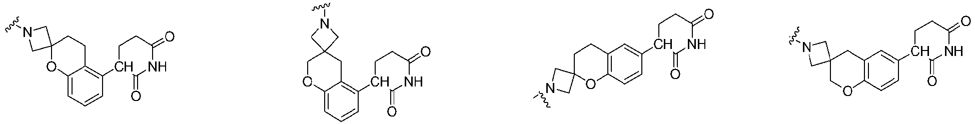 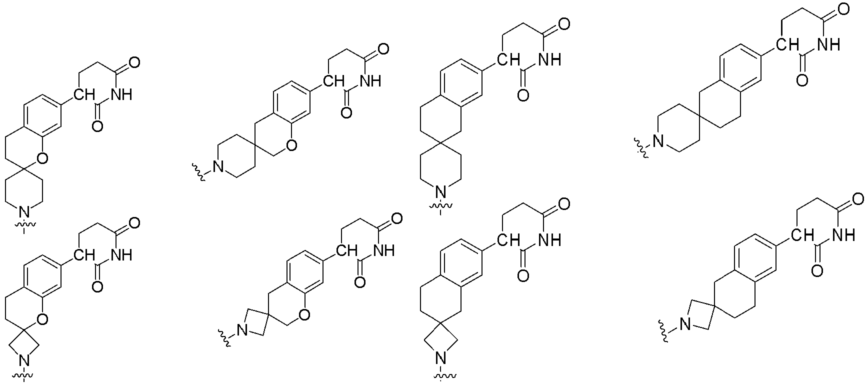 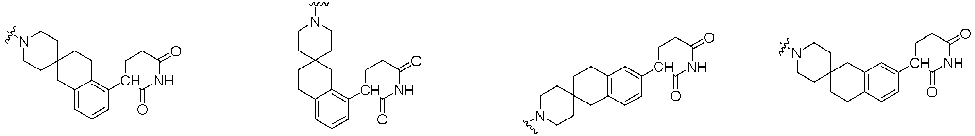 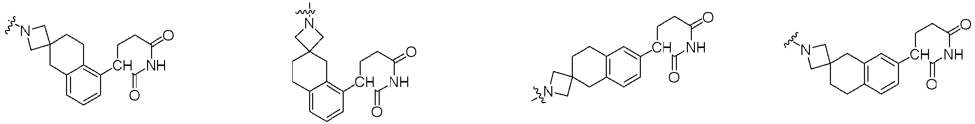 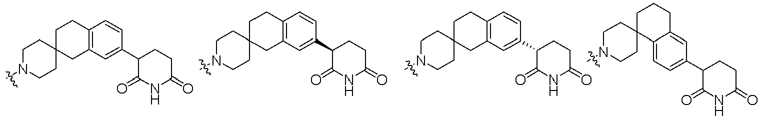 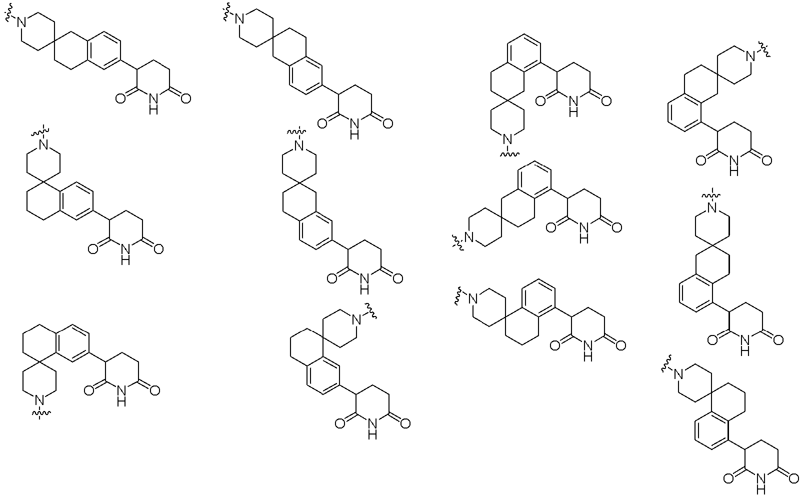 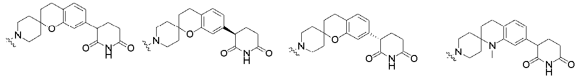 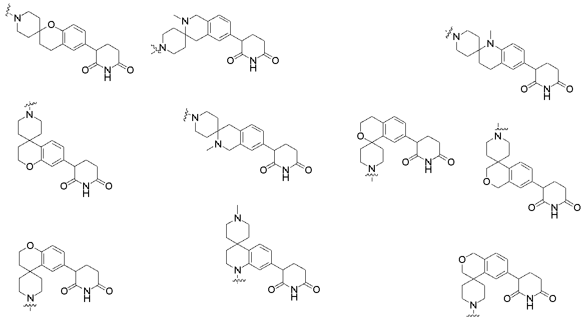 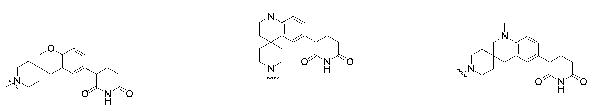 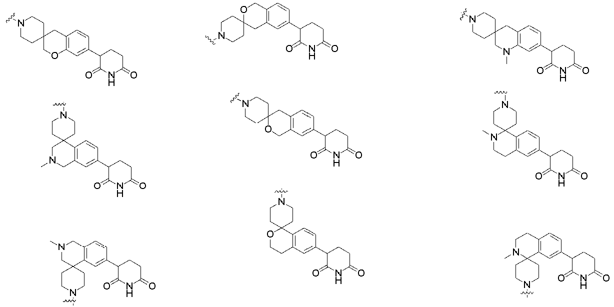 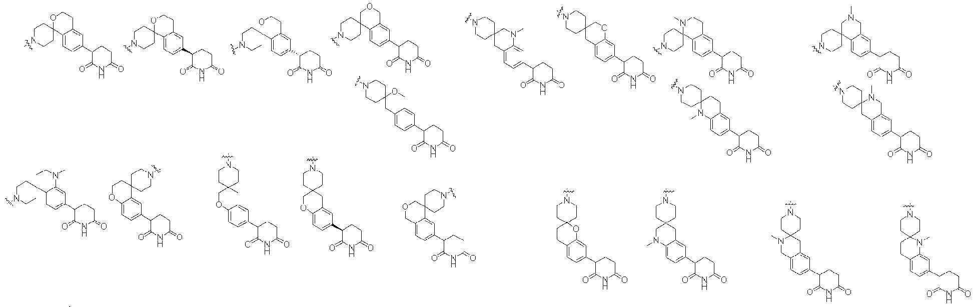 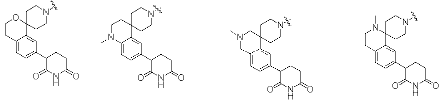 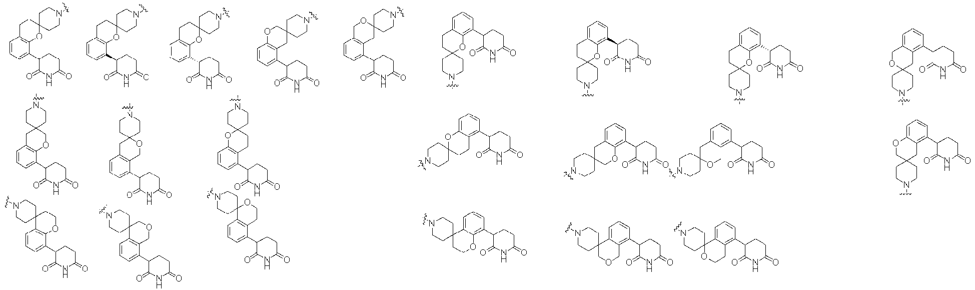 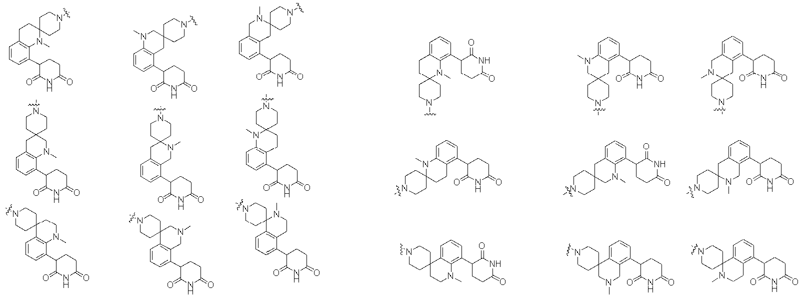 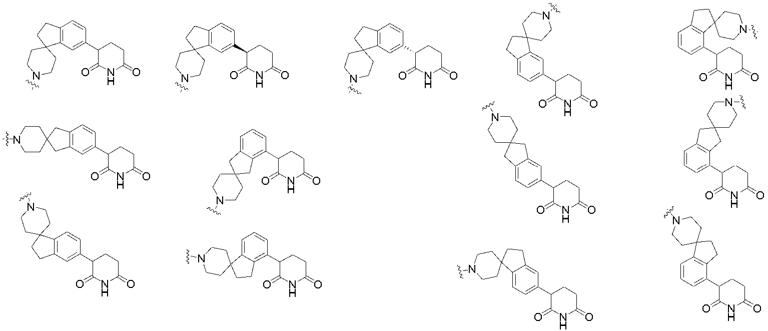 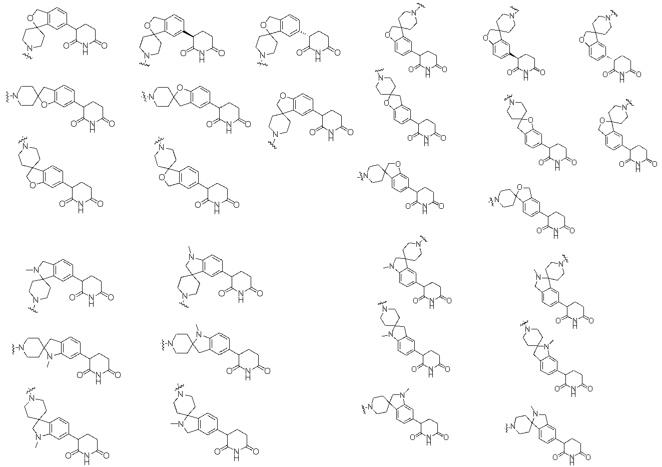 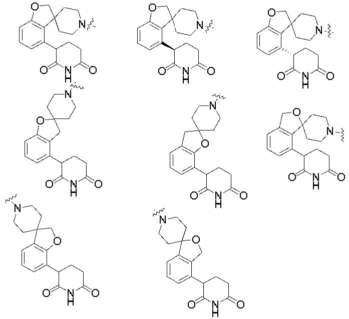 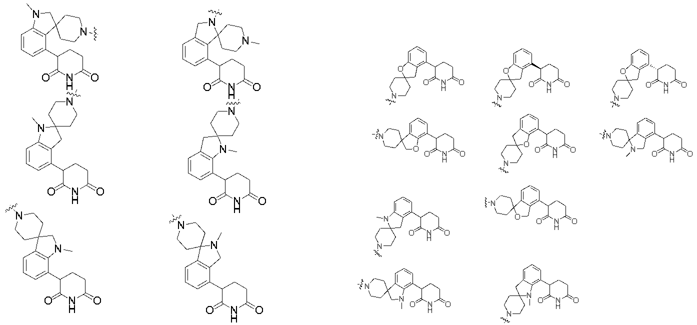 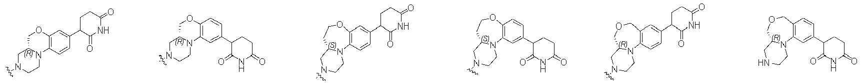 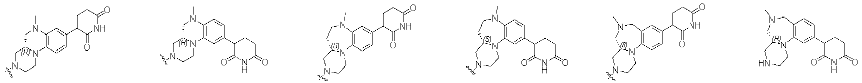 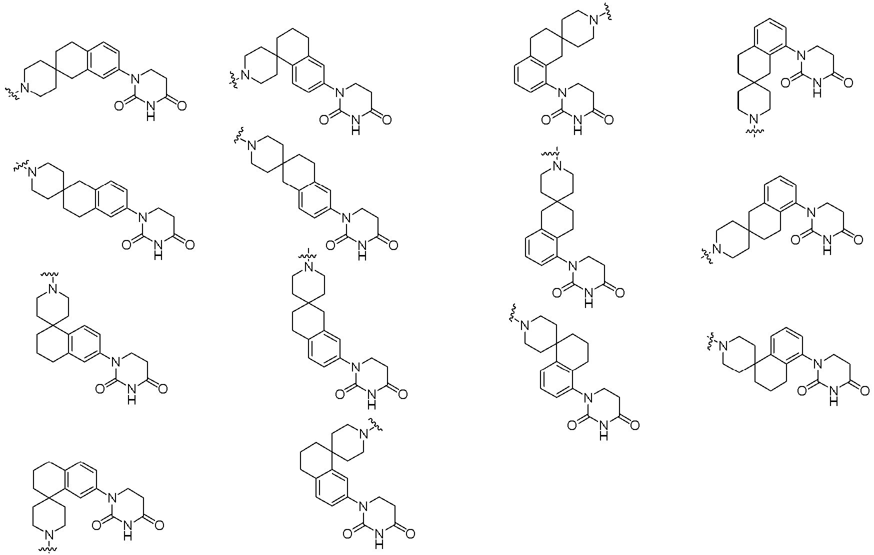 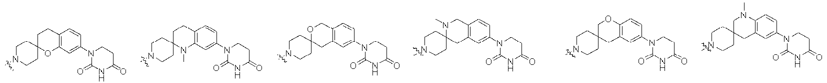 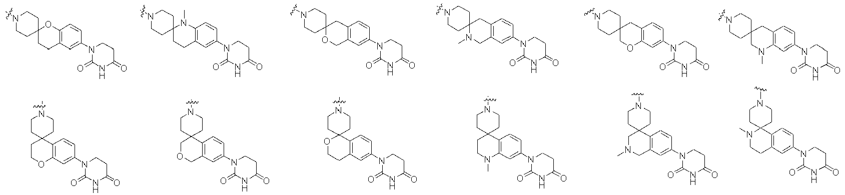 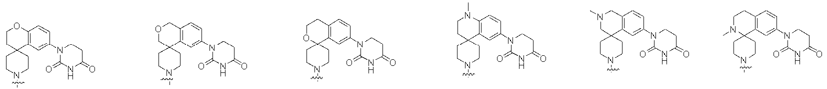 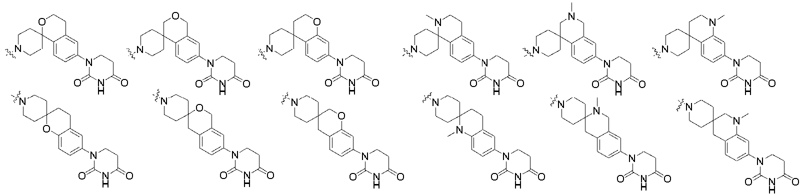 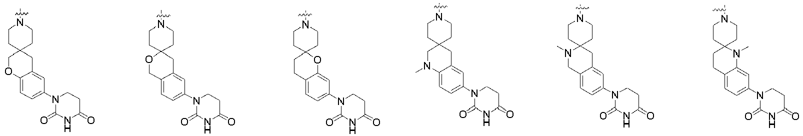 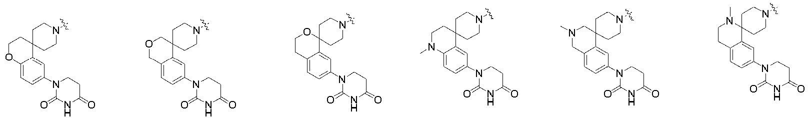 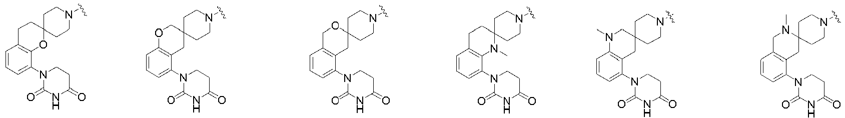 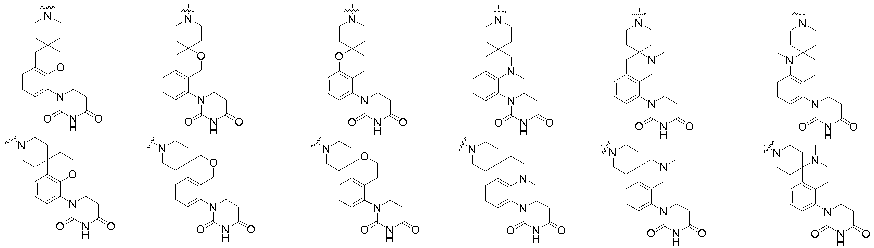 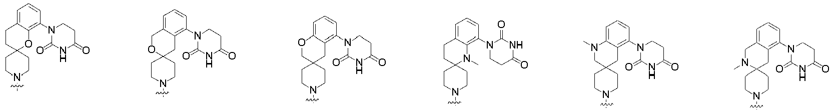 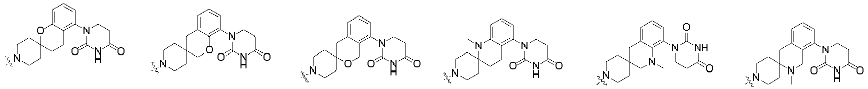 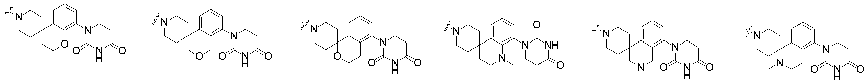 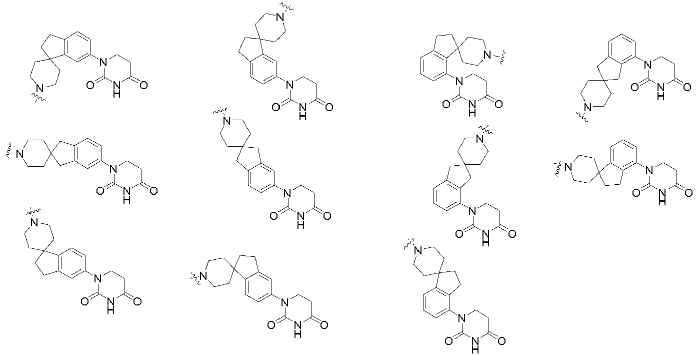 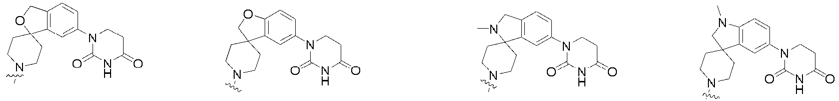 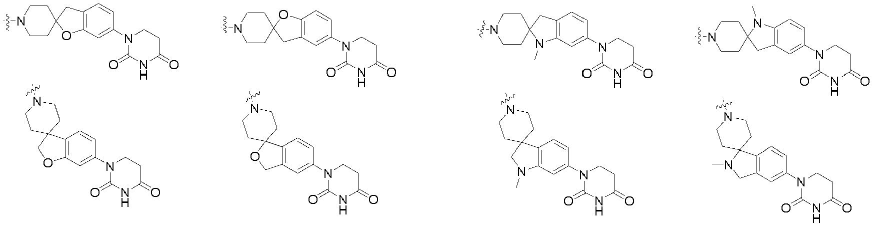 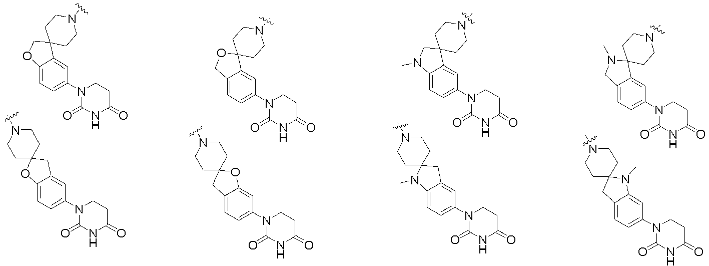 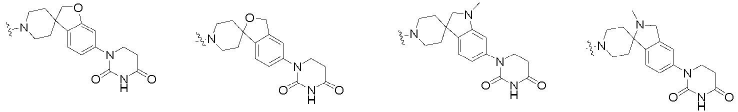 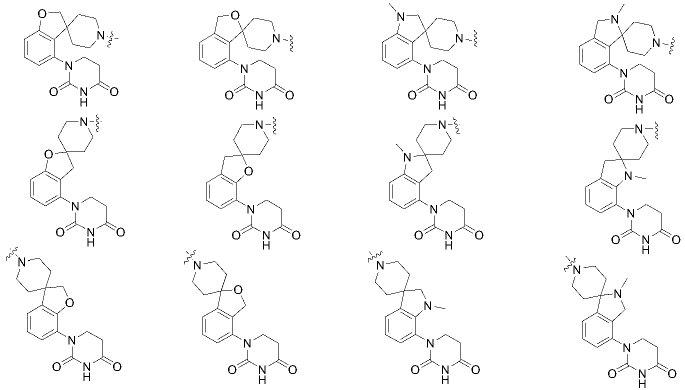 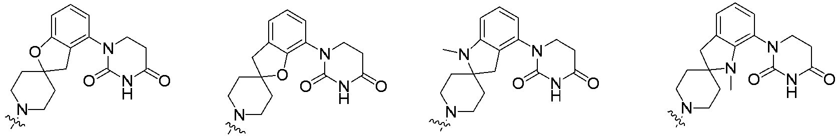 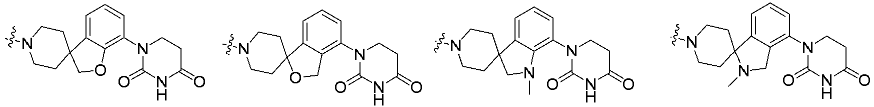 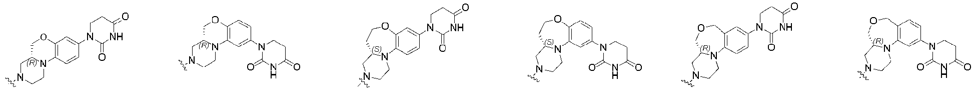 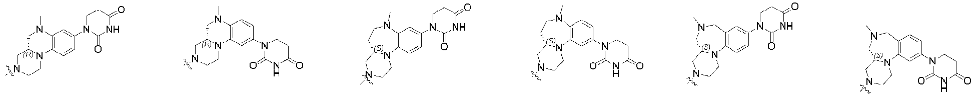 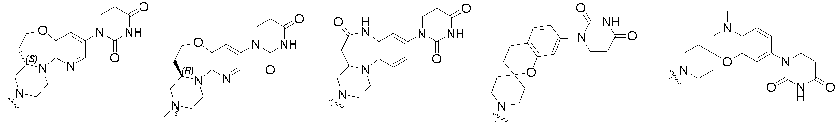 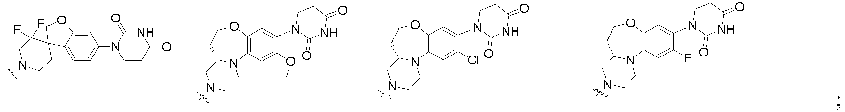 preferably, the CLM is selected from:

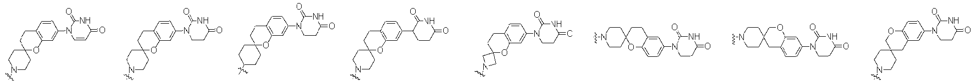

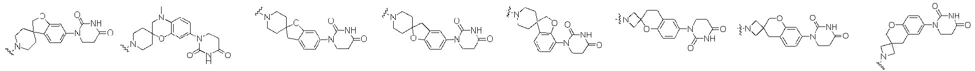

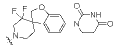

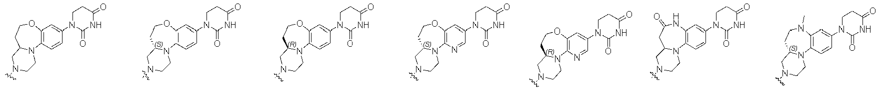

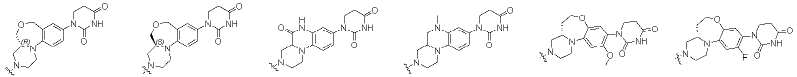

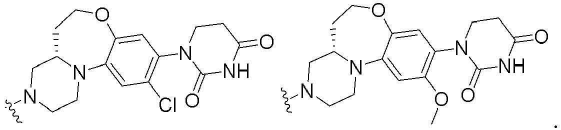

In one embodiment,
wherein, L is a bond or -(B^{L})_{q}-;
B^{L}, at each occurrence, is the same or different and is independently selected from CR^{L1}R^{L2}, O, S, SO, SO₂, NR^{L3}, SO₂NR^{L3}, SONR^{L3}, CONR^{L3}, NR^{L3}CONR^{L4}, NR^{L3}SO₂NR^{L4}, CO, CR^{L1}=CR^{L2}, C≡C, SiR^{L1}R^{L2}, P(O)R^{L1}, P(O)OR^{L1}, NR^{L3}C(=NCN)NR^{L4}, NR^{L3}C(=NCN), NR^{L3}C(=CNO₂)NR^{L4}, cycloalkylene, heterocyclylene, arylene, or heteroarylene, wherein the cycloalkylene, heterocyclylene, arylene, and heteroarylene are optionally substituted with 0-6 R^{L1} and/or R^{L2} groups;
R^{L1}, R^{L2}, R^{L3}, and R^{L4}, at each occurrence, are independently selected from H, halogen, C₁-₈ alkyl, O-C₁-₈ alkyl, S-C₁-₈ alkyl, NH-C₁-₈ alkyl, N(C₁-₈ alkyl)₂, C₃-₁₁ cycloalkyl, aryl, heteroaryl, C₃-₁₁ heterocyclyl, O-C₃-₈ cycloalkyl, O-C₃-₁₁ heterocyclyl, O-aryl, O-heteroaryl, S-C₃-₈ cycloalkyl, NH-C₃-₈ cycloalkyl, N(C₃-₈ cycloalkyl)₂, N(C₃-₈ cycloalkyl)(C₁-₈ alkyl), NH-C₃-₈ heterocyclyl, N(C₃-₈ heterocyclyl)₂, N(C₃-₈ heterocyclyl)(C₁-₈ alkyl), NH-aryl, N(aryl)(C₁-₈ alkyl), NH-heteroaryl, N(heteroaryl)(C₁-₈ alkyl), OH, NH₂, SH, SO₂, P(O)(O-C₁-₈ alkyl)(C₁-₈ alkyl), P(O)(O-C₁-₈ alkyl)₂, C≡C-C₁-₈ alkyl, C≡CH, CHCH-(C₁-₈ alkyl), C(C₁-₈ alkyl)=CH-(C₁-₈ alkyl), C(C₁-₈ alkyl)= C(C₁-₈ alkyl)₂, Si(OH)₃, Si(C₁-₈ alkyl)₃, Si(OH)(C₁-₈ alkyl)₂, CO-C₁-₈ alkyl, CO₂H, CN, CF₃, CHF₂, CH₂F, NO₂, SF₅, SO₂NH-C₁-₈ alkyl, SO₂N(C₁-₈ alkyl)₂, SONH-C₁-₈ alkyl, SON(C₁-₈ alkyl)₂, CONH-C₁-₈ alkyl, CON(C₁-₈ alkyl)₂, N(C₁-₈ alkyl)CONH(C₁-₈ alkyl), N(C₁-₈ alkyl)CON(C₁-₈ alkyl)₂, NHCONH(C₁-₈ alkyl), NHCON(C₁-₈ alkyl)₂, NHCONH₂, N(C₁-₈ alkyl)SO₂NH(C₁-₈ alkyl), N(C₁-₈ alkyl)SO₂N(C₁-₈ alkyl)₂, NHSO₂NH(C₁-₈ alkyl), NHSO₂N(C₁-₈ alkyl)₂, and NHSO₂NH₂, wherein the C₁-₈ alkyl, C₃-₁₁ cycloalkyl, C₃-₁₁ heterocyclyl, C₆-₁₀ aryl, and C₅-₁₀ heteroaryl are each independently optionally substituted with one or more substituents selected from halogen, alkyl, heteroalkyl, alkenyl, alkynyl, alkoxy, hydroxyl, haloalkyl, hydroxyalkyl, cyano, amino, nitro, cycloalkyl, heterocyclyl, halocycloalkyl, haloheteroalkyl, alkylamino, aryl, heteroaryl, haloaryl, and haloheteroaryl; and
q is an integer greater than or equal to 1; preferably, q is 1, 2, 3, 4, 5, 6, 7, 8, 9, 10, 11, 12, 13, 14, 15, 16, 17, 18, 19, or 20.

In one embodiment, wherein B^{L} is selected from one or more of the following structures: -O-, -S-, -SO-, -SO₂-, -CH₂ -, -CO-, -NH-, 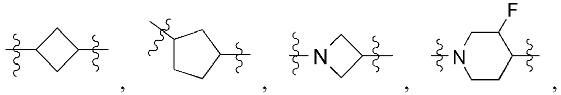 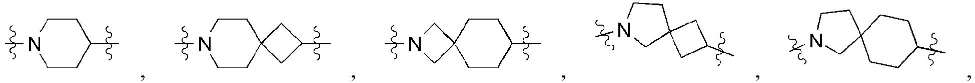 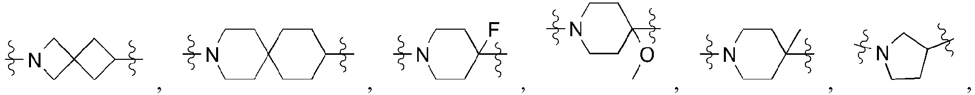 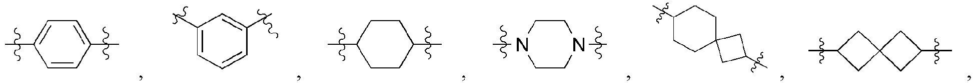 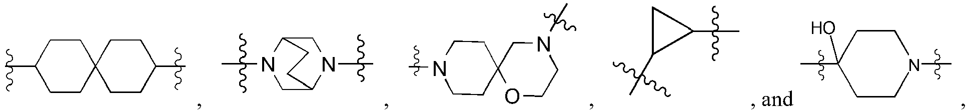 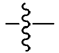 is the point of attachment.

In one embodiment, wherein L is selected from the following structures:
a covalent bond, -(CH₂)ⱼ-, -(CH₂)ₚ-NH-(CH₂)ₛ-, -(CH₂)_{y}-NH-(CH₂)ⱼ-NH-(CH₂)ₛ-, -(CH₂)ₚ-CO-(CH₂)ₛ-, -(CH₂)ₚ-O-(CH₂)ₛ-, -(CH₂)_{y}-CO-(CH₂)ⱼ-CO-(CH₂)ₛ-, -(CH₂)_{y}-O-(CH₂)-O-(CH₂)ₛ-, -(CH₂)_{y}-O-(CH₂)-CO-(CH₂)ₛ-, -(CH₂)_{y}-CO-(CH₂)-O-(CH₂)ₛ-, -(CH₂)ₚ-NH-(CH₂)_{y}-O-(CH₂)ⱼ-CO-(CH₂)ₛ-, -(CH₂)_{y}-CO-(CH₂)ⱼ-O-(CH₂)ₛ-NH-(CH₂)ₚ-, 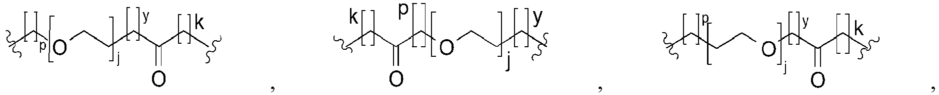 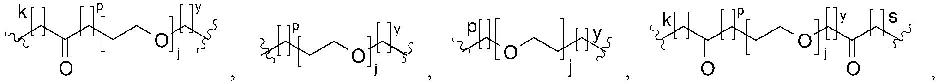 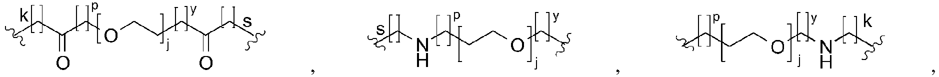 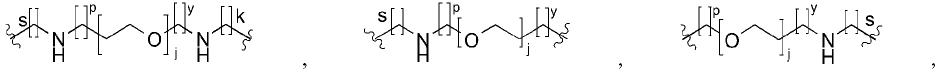 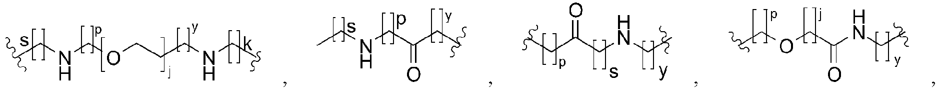 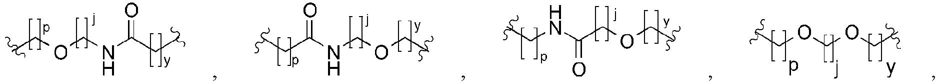 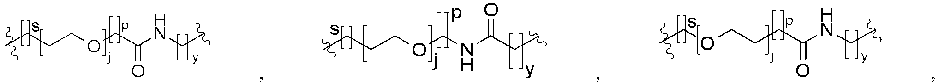 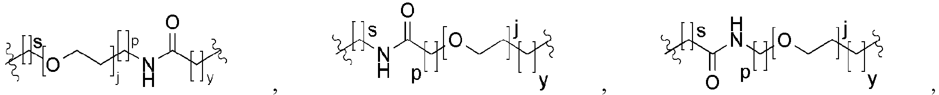 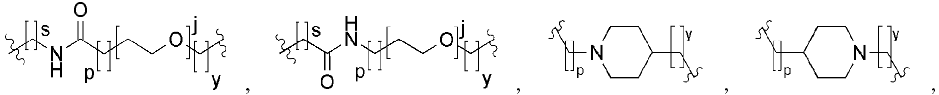 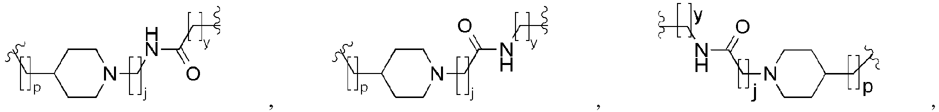 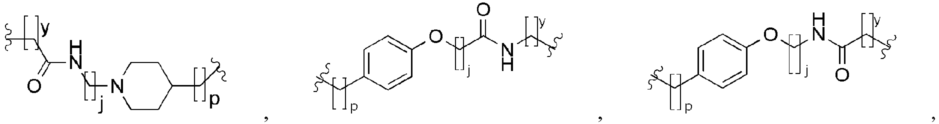 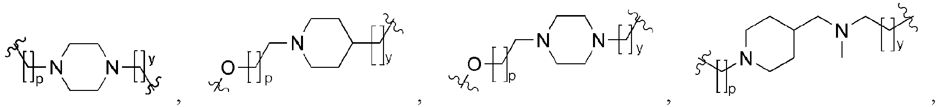 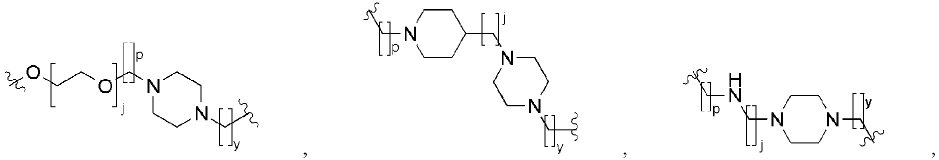 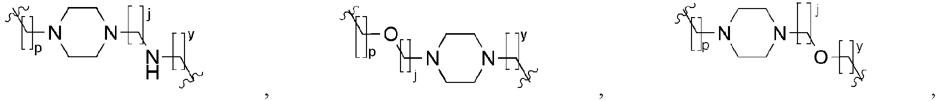 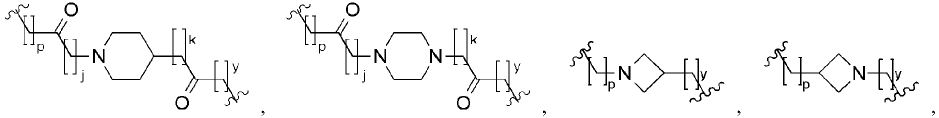 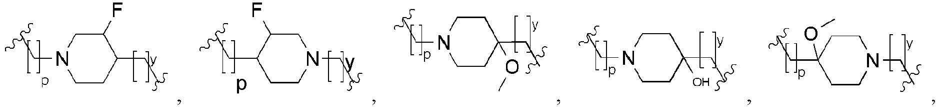 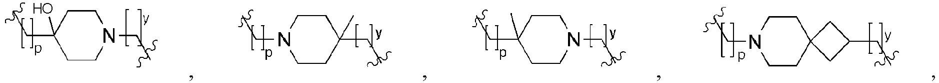 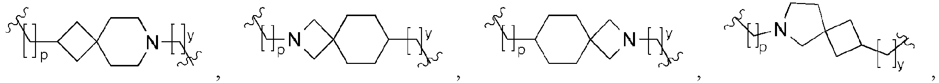 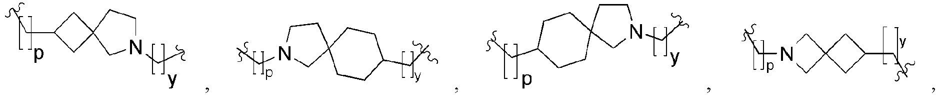 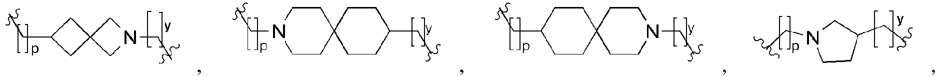 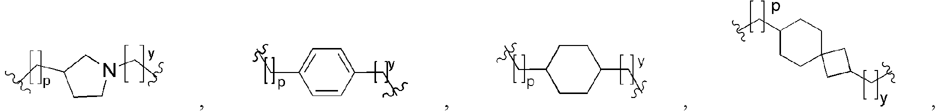 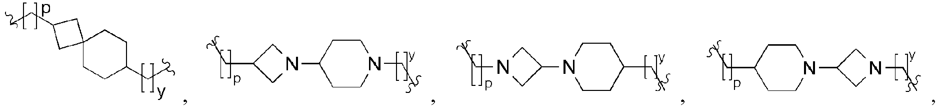 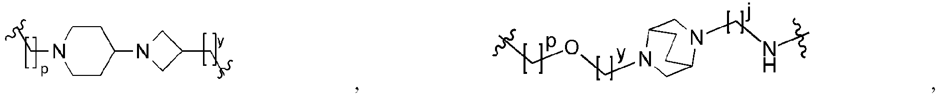 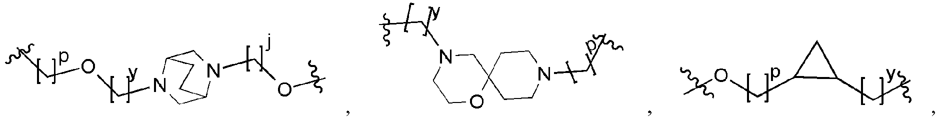 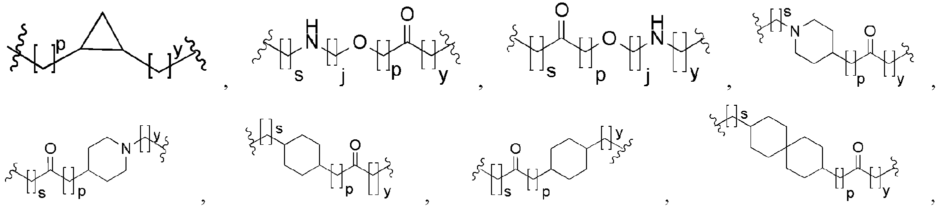 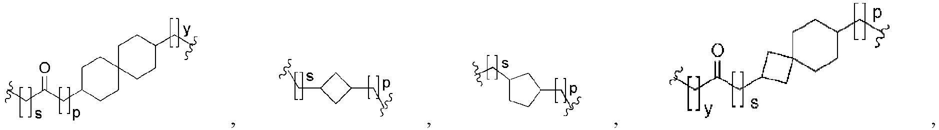 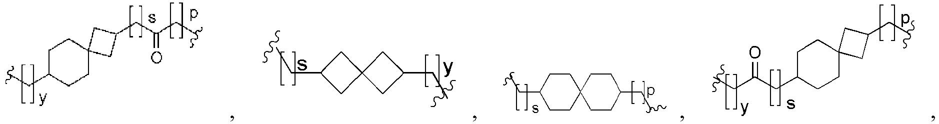 and 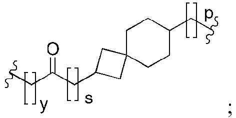
wherein, j is 1, 2, 3, 4, 5, 6, 7, 8, 9, or 10;
k, s, p, and y are 0, 1, 2, 3, 4, 5, 6, 7, 8, 9, or 10; 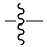 is the point of attachment for the CLM or PTM;
preferably, L is selected from the following structures: a covalent bond, -CH₂-, -(CH₂)₂-, -(CH₂)₃-, -(CH₂)₄-, - (CH₂)₅-, -(CH₂)₆-, -(CH₂)₇-, -(CH₂)₈-, -NH-CH₂-, -NH-(CH₂)₂-, -NH-(CH₂)₃-, -NH-(CH₂)₄-, -NH-(CH₂)₅-, -NH-(CH₂)₆-, -NH-(CH₂)₇-, -NH-(CH₂)₈-, -CO-NH-CH₂-, -CO-NH-(CH₂)₂-, -CO-NH-(CH₂)₃-, -CO-NH-(CH₂)₄-, -CO-NH-(CH₂)₅-, -CO-NH-(CH₂)₆-, -CO-NH-(CH₂)₇-, -CO-NH-(CH₂)₈-, -CH₂-NH-, -(CH₂)₂-NH-, -(CH₂)₃-NH-, - (CH₂)₄-NH-, -(CH₂)₅-NH-, -(CH₂)₆-NH-, -(CH₂)₇-NH-, -(CH₂)₈-NH-, -NH-CH₂-NH-, -NH-(CH₂)₂-NH-, -NH-(CH₂)₃-NH-, -NH-(CH₂)₄-NH-, -NH-(CH₂)₅-NH-, -NH-(CH₂)₆-NH-, -NH-(CH₂)₇-NH-, -NH-(CH₂)₈-NH-, -(CH₂-CH₂-O)-CH₂-CH₂-, -(CH₂-CH₂-O)₂-CH₂-CH₂-, -(CH₂-CH₂-O)₃-CH₂-CH₂-, -NH-(CH₂-CH₂-O)-CH₂-CH₂-, -NH-(CH₂-CH₂-O)₂-CH₂-CH₂-, -NH-(CH₂-CH₂-O)₃-CH₂-CH₂-, -CO-NH-(CH₂-CH₂-O)-CH₂-CH₂-, -CO-NH-(CH₂-CH₂-O)₂-CH₂-CH₂-, -CO-NH-(CH₂-CH₂-O)₃-CH₂-CH₂-, -(CH₂-CH₂-O)-CH₂-CH₂-NH-, -(CH₂-CH₂-O)₂-CH₂-CH₂-NH-, -(CH₂-CH₂-O)₃-CH₂-CH₂-NH-, -NH-(CH₂-CH₂-O)-CH₂-CH₂-NH-, -NH-(CH₂-CH₂-O)₂-CH₂-CH₂-NH-, -NH-(CH₂-CH₂-O)₃-CH₂-CH₂-NH-, -CO-NH-(CH₂-CH₂-O)-CH₂-CH₂-NH-, -CO-NH-(CH₂-CH₂-O)₂-CH₂-CH₂-NH-, -CO-NH-(CH₂-CH₂-O)₃-CH₂-CH₂-NH-, -CH₂-CH₂-(O-CH₂-CH₂)-, -CH₂-CH₂-(O-CH₂-CH₂)₂-, -CH₂-CH₂-(O-CH₂-CH₂)₃-, - NH-CH₂-CH₂-(O-CH₂-CH₂)-, -NH-CH₂-CH₂-(O-CH₂-CH₂)₂-, -NH-CH₂-CH₂-(O-CH₂-CH₂)₃-, -CO-NH-CH₂-CH₂-(O-CH₂-CH₂)-, -CO-NH-CH₂-CH₂-(O-CH₂-CH₂)₂-, -CO-NH-CH₂-CH₂-(O-CH₂-CH₂)₃-, -CH₂-CH₂-(O-CH₂-CH₂)-NH-, -CH₂-CH₂-(O-CH₂-CH₂)₂-NH-, -CH₂-CH₂-(O-CH₂-CH₂)₃-NH-, -NH-CH₂-CH₂-(O-CH₂-CH₂)-NH-, -NH-CH₂-CH₂-(O-CH₂-CH₂)₂-NH-, -NH-CH₂-CH₂-(O-CH₂-CH₂)₃-NH-, -NH-CH₂-CH₂-O-CH₂-CH₂-CO-, -CO-CH₂-CH₂-O-CH₂-CH₂-NH-, -NH-(CH₂)₄-CO-, -NH-(CH₂)₅-CO-, -NH-(CH₂)₆-CO-, -CO-(CH₂)₄-NH-, -CO-(CH₂)₅-NH-, -CO-(CH₂)₆-NH-, -NH-(CH₂-CH₂-O)-(CH₂)₃-, -NH-(CH₂-CH₂-O)-(CH₂)₄-, -NH-(CH₂-CH₂-O)-(CH₂)₅-, -NH-(CH₂-CH₂-O)-(CH₂)₆-, -(CH₂)₃-(O-CH₂-CH₂)-NH-, -(CH₂)₄-(O-CH₂-CH₂)-NH-, -(CH₂)₅-(O-CH₂-CH₂)-NH-, -(CH₂)₆-(O-CH₂-CH₂)-NH-, -CH₂-CH₂-O-(CH₂)₂-CO-, -CH₂-CH₂-O-(CH₂)₃-CO-, -CH₂-CH₂-O-(CH₂)₄-CO-, -CO-(CH₂)₂-O-CH₂-CH₂-, -CO-(CH₂)₃-O-CH₂-CH₂-, -CO-(CH₂)₄-O-CH₂-CH₂-, -CO-(CH₂)₂-, -CO-(CH₂)₃-, -CO-(CH₂)₄-, -CO-(CH₂)₅-, -CO-(CH₂)₆-, -(CH₂)₂-CO-, -(CH₂)₃-CO-, -(CH₂)₄-CO-, -(CH₂)₅-CO-, -(CH₂)₆-CO-, -CO-(CH₂)₂-CO-, -CO-(CH₂)₃-CO-, -CO-(CH₂)₄-CO-, -CO-(CH₂)₅-CO-, -CO-(CH₂)₆-CO-, -CH₂-CO-CH₂-, -CH₂-CO-(CH₂)₂-, -CH₂-CO-(CH₂)₃-, - CH₂-CO-(CH₂)₄-, -(CH₂)₂-CO-CH₂-, -(CH₂)₂-CO-(CH₂)₂-, -(CH₂)₂-CO-(CH₂)₃-, -(CH₂)₂-CO-(CH₂)₄-, -(CH₂)₃-CO-CH₂-, -(CH₂)₃-CO-(CH₂)₂-, -(CH₂)₃-CO-(CH₂)₃-, -(CH₂)₃-CO-(CH₂)₄-, -(CH₂)₄-CO-CH₂-, -(CH₂)₄-CO-(CH₂)₂-, - (CH₂)₄-CO-(CH₂)₃-, -(CH₂)₄-CO-(CH₂)₄-, -CH₂-O-CH₂-, -CH₂-O-(CH₂)₂-, -CH₂-O-(CH₂)₃-, -CH₂-O-(CH₂)₄-, - (CH₂)₂-O-CH₂-, -(CH₂)₂-O-(CH₂)₂-, -(CH₂)₂-O-(CH₂)₃-, -(CH₂)₂-O-(CH₂)₄-, -(CH₂)₃-O-CH₂-, -(CH₂)₃-O-(CH₂)₂-, - (CH₂)₃-O-(CH₂)₃-, -(CH₂)₃-O-(CH₂)₄-, -(CH₂)₄-O-CH₂-, -(CH₂)₄-O-(CH₂)₂-, -(CH₂)₄-O-(CH₂)₃-, -(CH₂)₄-O-(CH₂)₄-,

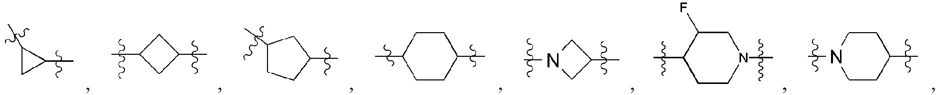

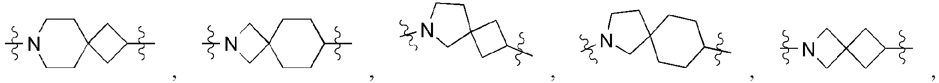

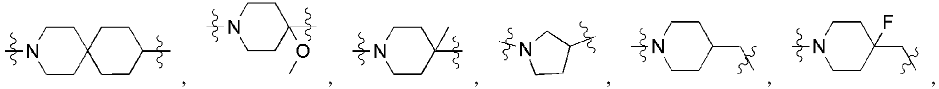

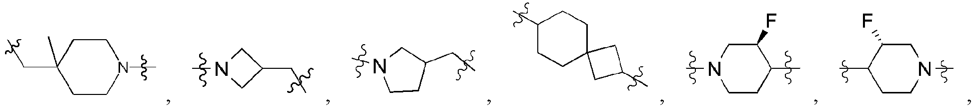

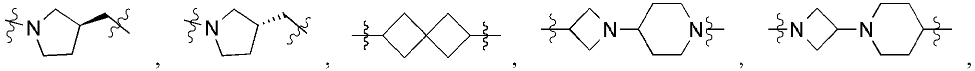

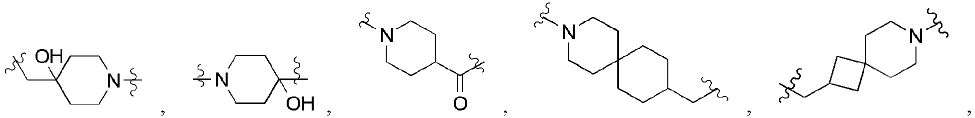

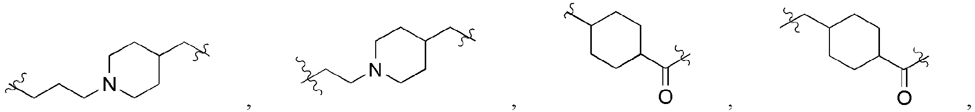

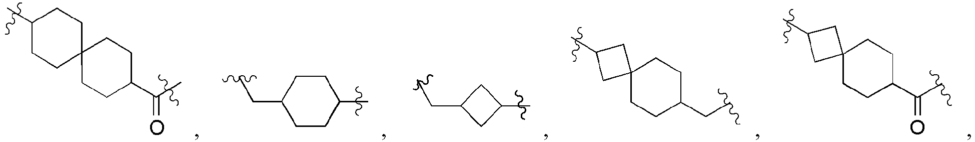

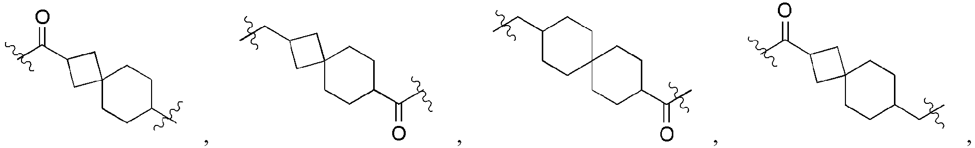

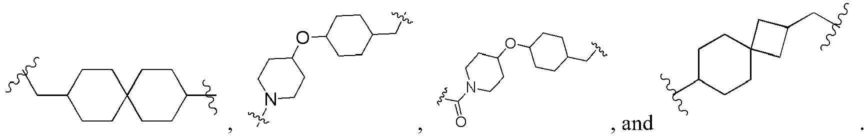

In one embodiment, wherein the PTM is a moiety that binds to an androgen receptor; preferably, the PTM is selected from the following structures: 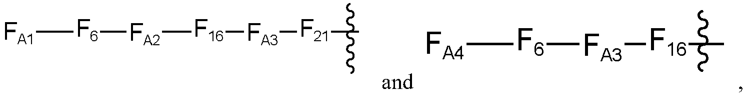 wherein,
F₆, F₁₆, and F₂₁, at each occurrence, are each independently selected from a single bond, NH, SO, S, O, SO₂, alkylene, haloalkylene, heteroalkylene, alkyleneoxy, heteroalkyleneoxy, alkenylene, alkynylene, C(=O), OC(=O), C(=O)O, C(=O)NH, and NHC(=O), or a combination of one or more thereof; wherein the alkylene, alkyleneoxy, and alkenylene are optionally substituted with 0, 1, 2, 3, 4, 5, or 6 R_{c} substituents; preferably, F₆, F₁₆, and F₂₁, at each occurrence, are each independently selected from a single bond, NH, SO, S, O, SO₂, C(=O), OC(=O), and NHC(=O);
F_{A1} and F_{A3} are each independently selected from 6-10-membered aryl and 5-10-membered heteroaryl; the aryl and heteroaryl are optionally substituted with 0, 1, 2, 3, 4, 5, or 6 R_{da} substituents; preferably, F_{A1} is phenyl, optionally substituted with 0, 1, 2, 3, 4, 5, or 6 R_{da} substituents, and F_{A3} is phenyl or a 6-membered heteroaryl containing 1, 2, 3 N, O, S heteroatoms, optionally substituted with 0, 1, 2, 3, 4, 5, or 6 R_{da} substituents;
F_{A2} is 3-15-membered cycloalkylene, 5-15-membered spirocycloalkylene, 3-15-membered heterocycloalkylene, or 5-15-membered spiroheterocycloalkylene, optionally substituted with 0, 1, 2, 3, 4, 5, or 6 R_{ca} substituents; preferably, F_{A2} is 4-6-membered cycloalkylene, 7-11-membered spirocycloalkylene, 4-6-membered heterocycloalkylene, or 7-11-membered spiroheterocycloalkylene, optionally substituted with 0, 1, 2, 3, 4, 5, or 6 R_{ca} substituents; preferably, F_{A2} is 4-, 5-, or 6-membered cycloalkylene, 10-membered spiroheterocycloalkylene containing 1, 2, 3 N atoms, optionally substituted with 0, 1, 2, 3, 4, 5, or 6 R_{ca} substituents;
F_{A4} is 6-10-membered aryl-fused 7-15-membered spiroheterocyclyl, optionally substituted with 0, 1, 2, 3, 4, 5, or 6 R_{da} substituents; preferably, F_{A4} is phenyl-fused 7-15-membered spiroheterocyclyl, the 7-15-membered spiroheterocyclyl containing 1, 2, 3, or 4 heteroatoms each independently selected from N, O, S, optionally substituted with 0, 1, 2, 3, 4, 5, or 6 R_{da} substituents;
R_{da}, at each occurrence, is independently selected from H, halogen, C₁₋₆ alkyl, C₁₋₆ haloalkyl, C₁₋₆ heteroalkyl, C₁₋₆ alkoxy, C₁₋₆ heteroalkoxy, C₃-₇ cycloalkyl, C₃-₇ heterocyclyl, C₆-₈ aryl, C₅-₈ heteroaryl, OH, NH₂, CN, and NO₂; preferably, R_{da}, at each occurrence, is independently selected from H, F, Cl, Br, I, CH₃, OCH₃, CF₃, OH, NH₂, CN, and NO₂;
R_{ca}, at each occurrence, is independently selected from H, halogen, C₁₋₆ alkyl, C₁₋₆ haloalkyl, C₁₋₆ heteroalkyl, C₁₋₆ alkoxy, C₁₋₆ heteroalkoxy, C₃-₇ cycloalkyl, C₃-₇ heterocyclyl, C₆-₈ aryl, C₅-₈ heteroaryl, oxo (=O), thioxo (=S), OH, NH₂, CN, and NO₂; preferably, R_{ca}, at each occurrence, is independently selected from H, F, Cl, Br, I, CH₃, OCH₃, CF₃, OH, NH₂, CN, and NO₂;
preferably, the PTM is selected from:

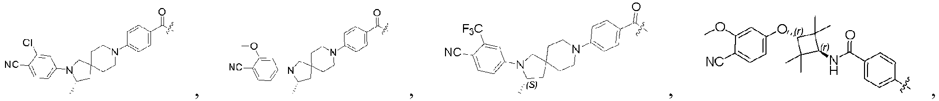

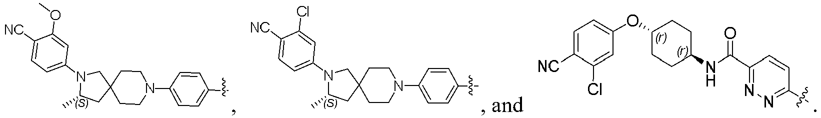

In one embodiment, the structure is selected from:

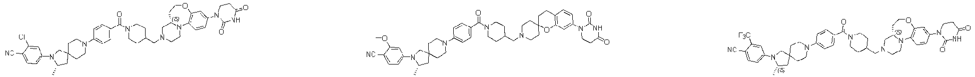

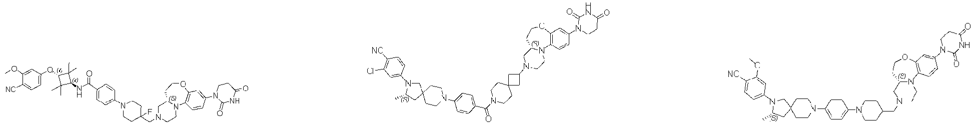

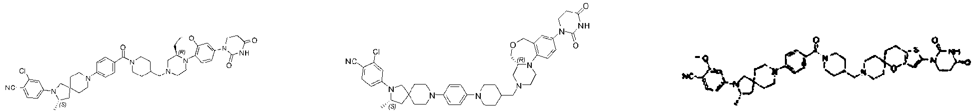

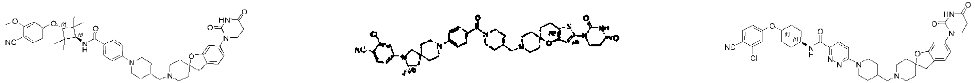

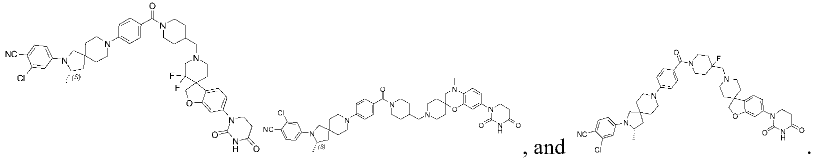

In one embodiment, the PTM is a moiety that binds to an estrogen receptor, preferably, the PTM is selected from the following structures: 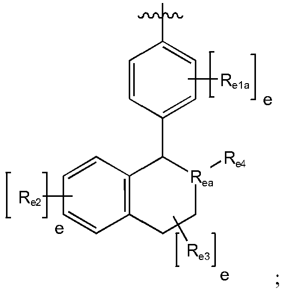
Rₑₐ is selected from CRₑ₁ₐ and N;
Rₑ₄ is selected from 6-10-membered aryl, 5-10-membered heteroaryl, C₁-C₆ alkyl, and C₁-C₆ haloalkyl, wherein the 6-10-membered aryl and 5-10-membered heteroaryl are optionally substituted with 0, 1, 2, 3, 4, or 5 Rₑ₁ₐ substituents; preferably, Rₑ₄ is selected from phenyl and difluoroethyl, wherein the phenyl is optionally substituted with 0, 1, 2, 3, 4, or 5 Rₑ₁ₐ substituents;
Rₑ₁ₐ, Rₑ₂, and Rₑ₃, at each occurrence, are each independently selected from H, F, Cl, Br, I, C₁-C₆ alkyl, C₁-C₆ heteroalkyl, C₂-C₆ alkenyl, C₂-C₆ alkynyl, C₁-C₆ alkoxy, hydroxyl, nitro, cyano, and amino; preferably, Rₑ₁ₐ and Rₑ₂, at each occurrence, are each independently selected from hydroxyl, H, F, Cl, Br, I, and C₁-C₃ alkyl;
e, at each occurrence, is selected from 0, 1, 2, 3, and 4;
preferably, the PTM is selected from:

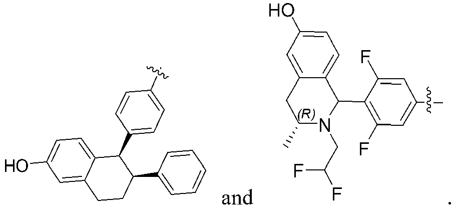

In one embodiment, wherein the compound is selected from:

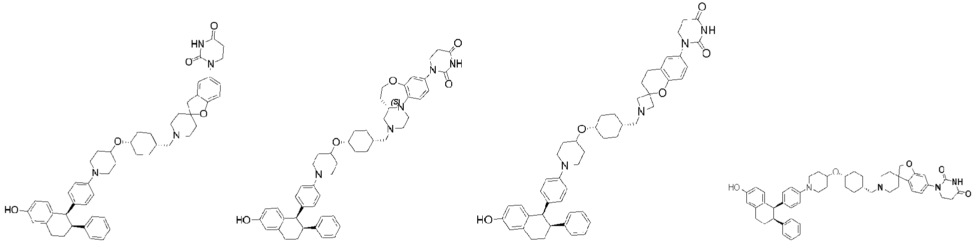

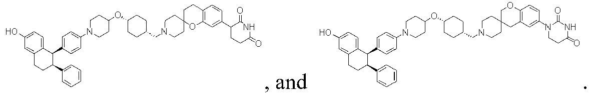

In one embodiment, the PTM is a moiety that binds to the IRAK4 receptor, preferably, the PTM has the structure: 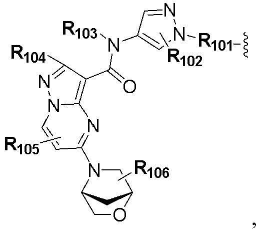 wherein,
R₁₀₁ is selected from a single bond, 3-10-membered heterocycloalkylene, 3-10-membered cycloalkylene, 6-10-membered arylene, and 5-10-membered heteroarylene, wherein the 3-10-membered heterocycloalkyl, 3-10-membered cycloalkyl, 6-10-membered aryl, and 5-10-membered heteroaryl are optionally substituted with one, two, or more substituents selected from halogen, carboxyl, alkoxy, alkyl, hydroxyl, amino, and haloalkyl; preferably, R₁₀₁ is selected from a single bond, 5-7-membered heterocycloalkylene, 5-7-membered cycloalkylene, 6-membered arylene, and 5-8-membered heteroarylene, wherein the 5-7-membered heterocycloalkylene, 5-7-membered cycloalkylene, 6-membered arylene, and 5-8-membered heteroarylene are optionally substituted with one, two, or three substituents selected from F, Cl, Br, I, carboxyl, C₁-₃ alkoxy, C₁-₃ alkyl, hydroxyl, amino, and C₁-₃ haloalkyl;
R₁₀₂, R₁₀₃, R₁₀₄, R₁₀₅, and R₁₀₆ are selected from H, deuterium atom, halogen, C₁₋₆ alkyl, C₁₋₆ deuterated alkyl, carboxyl, C₁₋₆ heteroalkyl, alkenyl, alkynyl, C₁₋₆ alkoxy, C₁₋₆ haloalkyl, C₁₋₆ haloalkoxy, hydroxyl, C₁₋₆ hydroxyalkyl, nitro, cyano, amino, C₁₋₆ alkylamino, C₁₋₆ alkylcarbonyl, C₁₋₆ alkoxycarbonyl, and C₁₋₆ alkylaminocarbonyl; preferably, R₁₀₂, R₁₀₃, R₁₀₄, R₁₀₅, and R₁₀₆ are selected from H, F, Cl, Br, I, C₁-₃ alkyl, carboxyl, C₁-₃ heteroalkyl, C₁-₃ alkoxy, and C₁-₃ haloalkyl; more preferably, R₁₀₂ is C₁-₃ fluoroalkyl; even more preferably, the PTM has the structure:

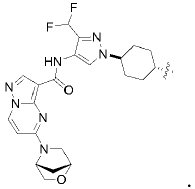

In one embodiment, the compound is selected from: 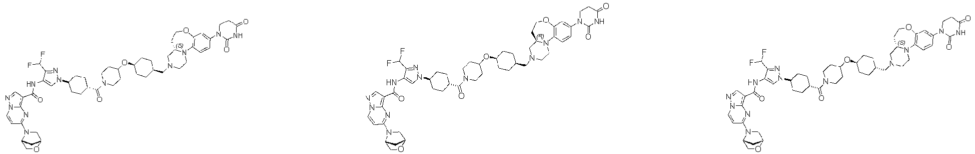 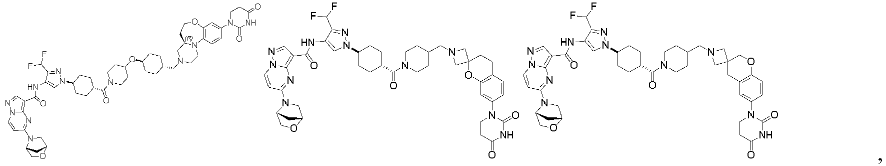 and

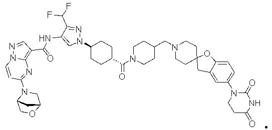

In a fourth aspect, the present invention provides a pharmaceutical composition comprising the compound according to any one of the preceding items.

In some embodiments, the compound or pharmaceutical composition is used for treating or preventing a condition that is treated by degrading a target protein that binds to a target protein ligand; preferably, the target protein is cyclin-dependent kinase 4/6, androgen receptor, estrogen receptor, Aurora kinase A, Bcr-Abl protein, epidermal growth factor receptor, anaplastic lymphoma kinase, Bruton's tyrosine kinase, bromodomain protein 4, or interleukin-1 receptor-associated kinase 4 (IRAK4).

In some embodiments, the compound or pharmaceutical composition is used for treating or preventing a condition that is treated by binding to cereblon in vivo.

In some embodiments, the compound or pharmaceutical composition is used for treating or preventing a condition associated with the accumulation and/or aggregation of a target protein; preferably, the target protein is cyclin-dependent kinase 4/6, androgen receptor, estrogen receptor, Aurora kinase A, Bcr-Abl protein, epidermal growth factor receptor, anaplastic lymphoma kinase, Bruton's tyrosine kinase, bromodomain protein 4, or interleukin-1 receptor-associated kinase 4 (IRAK4).

In some embodiments, the condition is a tumor or cancer; preferably, the tumor or cancer is breast cancer, ductal breast carcinoma, prostate cancer, mantle cell lymphoma, chronic myeloid leukemia, acute myeloid leukemia, myelomonocytic leukemia, non-small cell lung cancer, lung adenocarcinoma, and/or cervical cancer.

In a fifth aspect, the present invention provides the use of the compound according to any one of the preceding items or the pharmaceutical composition in the manufacture of a medicament for treating or preventing a condition that is treated by binding to cereblon in vivo.

In the fifth aspect, the present invention also provides a method for treating or preventing a condition that is treated by binding to cereblon in vivo, comprising administering a therapeutically effective amount of the aforementioned compound or pharmaceutical composition to a subject in need thereof.

In a sixth aspect, the present invention provides the use of the compound according to any one of the preceding items or the pharmaceutical composition in the manufacture of a medicament for treating or preventing a condition that is treated by degrading a target protein that binds to a target protein ligand, wherein the target protein is cyclin-dependent kinase 4/6, androgen receptor, estrogen receptor, Aurora kinase A, Bcr-Abl protein, epidermal growth factor receptor, anaplastic lymphoma kinase, Bruton's tyrosine kinase, bromodomain protein 4, or interleukin-1 receptor-associated kinase 4 (IRAK4).

In the sixth aspect, the present invention also provides a method for treating or preventing a condition that is treated by degrading a target protein that binds to a target protein ligand, comprising administering a therapeutically effective amount of the aforementioned compound or pharmaceutical composition to a subject in need thereof; preferably, the target protein is cyclin-dependent kinase 4/6, androgen receptor, estrogen receptor, Aurora kinase A, Bcr-Abl protein, epidermal growth factor receptor, anaplastic lymphoma kinase, Bruton's tyrosine kinase, bromodomain protein 4, or interleukin-1 receptor-associated kinase 4 (IRAK4).

In a seventh aspect, the present invention provides the use or method of the compound according to any one of the preceding items or the pharmaceutical composition in treating or preventing a condition associated with the accumulation and/or aggregation of a target protein, wherein the target protein is preferably cyclin-dependent kinase 4/6, androgen receptor, estrogen receptor, Aurora kinase A, Bcr-Abl protein, epidermal growth factor receptor, anaplastic lymphoma kinase, Bruton's tyrosine kinase, bromodomain protein 4, or interleukin-1 receptor-associated kinase 4 (IRAK4).

In the seventh aspect, the present invention also provides a method for treating or preventing a condition associated with the accumulation and/or aggregation of a target protein, comprising administering a therapeutically effective amount of the aforementioned compound or pharmaceutical composition to a subject in need thereof; preferably, the target protein is cyclin-dependent kinase 4/6, androgen receptor, estrogen receptor, Aurora kinase A, Bcr-Abl protein, epidermal growth factor receptor, anaplastic lymphoma kinase, Bruton's tyrosine kinase, bromodomain protein 4, or interleukin-1 receptor-associated kinase 4 (IRAK4). In one embodiment, the condition is a tumor or cancer, preferably the tumor or cancer is breast cancer, ductal breast carcinoma, prostate cancer, mantle cell lymphoma, chronic myeloid leukemia, acute myeloid leukemia, myelomonocytic leukemia, non-small cell lung cancer, lung adenocarcinoma, and/or cervical cancer.

In an eighth aspect, the present invention provides a method for inducing degradation of a target protein in a cell, comprising administering an effective amount of the aforementioned compound or the aforementioned pharmaceutical composition to an organism in need thereof; preferably, the target protein is cyclin-dependent kinase 4/6, androgen receptor, estrogen receptor, Aurora kinase A, Bcr-Abl protein, epidermal growth factor receptor, anaplastic lymphoma kinase, Bruton's tyrosine kinase, bromodomain protein 4, or interleukin-1 receptor-associated kinase 4 (IRAK4).

### DETAILED DESCRIPTION

### I. Definitions

In the present invention, unless otherwise specified, the scientific and technical terms used herein have the meanings commonly understood by those skilled in the art. Furthermore, the relevant terminology and laboratory procedures described herein are terms and standard procedures widely used in the relevant field. Additionally, to facilitate a better understanding of the invention, definitions and explanations of the relevant terms are provided below.

In this document, references to "some examples," "some implementations," or "some embodiments" describe a subset of all possible examples; however, it should be understood that "some examples" may constitute the same or different subsets of all possible examples and may be combined with one another without conflict.

Unless otherwise specified, the terms "comprising," "including," "having," and "containing", as well as their grammatical equivalents in this document are generally to be understood as open-ended and non-limiting; for example, they do not exclude other elements or steps not listed herein.

In this specification and in the claims of this application, the phrase "and/or" is to be interpreted as meaning "either one or both" of the elements; that is, the elements may coexist in some cases or exist separately in other cases.

When a numerical range is listed, it is understood to include each value and any subranges within that range. For example, "C₁₋₆ alkyl" includes C₁, C₂, C₃, C₄, C₅, C₆, C₁₋₆, C₁₋₅, C₁₋₄, C₁₋₃, C₁₂, C₂₋₆, C₂₋₅, C₂₋₄, C₂₋₃, C₃₋₆, C₃₋₅, C₃₋₄, C₄₋₆, C₄₋₅ and C₅₋₆ alkyl groups.

The term "alkyl" as considered herein refers to a saturated aliphatic hydrocarbon group, which is a straight-chain or branched group comprising 1 to 20 carbon atoms, preferably an alkyl group containing 1 to 12 (e.g., 1, 2, 3, 4, 5, 6, 7, 8, 9, 10, 11, and 12) carbon atoms, and more preferably an alkyl group containing 1 to 6 carbon atoms. Non-limiting examples include methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, n-heptyl, 2-methylhexyl, 3-methylhexyl, 4-methylhexyl, 5-methylhexyl, 2,3-dimethylpentyl, 2,4-dimethylpentyl, 2,2-dimethylpentyl, 3,3-dimethylpentyl, 2-ethylpentyl, 3-ethylpentyl, n-octyl, 2,3-dimethylhexyl, 2,4-dimethylhexyl, 2,5-dimethylhexyl, 2,2-dimethylhexyl, 3,3-dimethylhexyl, 4,4-dimethylhexyl, 2-ethylhexyl, 3-ethylhexyl, 4-ethylhexyl, 2-methyl-2-ethylpentyl, 2-methyl-3-ethylpentyl, n-nonyl, 2-methyl-2-ethylhexyl, 2-methyl-3-ethylhexyl, 2,2-diethylpentyl, n-decyl, 3,3-diethylhexyl, 2,2-diethylhexyl, and their various branched-chain isomers. More preferably, a lower alkyl group containing 1 to 6 carbon atoms. Non-limiting examples include methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, etc. Alkyl groups may be substituted or unsubstituted. When substituted, substituents may be attached at any available bonding site. Wherein the substituent is independently and optionally selected from one or more substituents selected from H, D, halogen, alkyl, alkoxy, haloalkyl, hydroxy, hydroxyalkyl, cyano, amino, nitro, cycloalkyl, heterocyclyl, aryl, or heteroaryl groups.

The term "heteroalkyl" refers to an alkyl group in which one or more -CH₂- moieties are substituted by a heteroatom selected from NH, O, and S, or one or more -CH- moieties are substituted by an N atom; wherein said alkyl group is as defined above; The heteroalkyl group may be substituted or unsubstituted. When substituted, the substituent may be attached at any available bonding site. Wherein the substituent is preferably independently selected from one or more substituents selected from H atoms, D atoms, halogen, alkyl, alkoxy, haloalkyl, hydroxy, hydroxyalkyl, cyano, amino, nitro, cycloalkyl, heteroaryl, aryl, or heteroaryl.

The term "alkoxy" refers to -O-(alkyl) and -O-(unsubstituted cycloalkyl), where alkyl or cycloalkyl is defined herein. Non-limiting examples of alkoxy groups include: methoxy, ethoxy, propoxy, butoxy, cyclopropoxy, cyclobutoxy, cyclopentoxy, and cyclohexyloxy. The alkoxy group may be optionally substituted or unsubstituted. When substituted, the substituent is preferably one or more of the following groups, each independently selected from H atoms, D atoms, halogen, alkyl, alkoxy, haloalkyl, hydroxyl, hydroxyalkyl, cyano, amino, nitro, cycloalkyl, heterocyclyl, aryl or heteroaryl groups.

The term "alkene" refers to an alkyl compound comprising a carbon-carbon double bond, wherein the alkyl is defined as described above. The alkenyl group is preferably a "C₂₋₈ alkenyl" having 2 to 8 carbon atoms and at least one (e.g., 1 to 2) carbon-carbon double bond. More preferably, it is a C₂₋₆ alkenyl group (i.e., an alkenyl group having 2 to 6 carbon atoms and 1 to 2 carbon-carbon double bonds). More preferably, it is a C₂₋₄ alkenyl group (i.e., an alkenyl group having 2 to 4 carbon atoms and 1 to 2 carbon-carbon double bonds). Specific examples include, but are not limited to, vinyl, 1-propyl, 2-propyl, 1-, 2-, or 3-butyl, pentyl, hexyl, and butadienyl groups. The alkene may be substituted or unsubstituted. When substituted, the substituent is preferably one or more of the following groups, each independently selected from hydrogen, alkyl, alkoxy, halogen, haloalkyl, hydroxy, hydroxyalkyl, cyano, amino, nitro, cycloalkyl, heterocyclyl, aryl, and heteroaryl groups.

The term "alkyne" refers to an alkyl compound containing a carbon-carbon triple bond in their molecules, where the definition of alkyl is as described above. The alkyne group is preferably a "C₂₋₈ alkyne group" having 2 to 8 carbon atoms and at least one (e.g., 1 to 2) carbon-carbon triple bond. Preferably, it is a C₂₋₆ alkyne group (i.e., an alkyne group having 2 to 6 carbon atoms and 1 to 2 carbon-carbon triple bonds). More preferably, it is a C₂₋₄ alkyne group (i.e., an alkyne group having 2 to 4 carbon atoms and 1 to 2 carbon-carbon triple bonds). Specific examples include, but are not limited to, acetylene groups, 1-propynyl groups, 2-propynyl groups, and 1-, 2-, or 3-butynyl groups. The alkyne group may be substituted or unsubstituted. When substituted, the substituent is preferably one or more of the following groups, each independently selected from hydrogen, alkyl, alkoxy, halogen, haloalkyl, hydroxy, hydroxyalkyl, cyano, amino, nitro, cycloalkyl, heterocyclyl, aryl, and heteroaryl groups.

The term "cycloalkyl" refers to a saturated or partially unsaturated monocyclic cyclic hydrocarbon substituent, wherein the naphthenyl ring contains 3 to 20 carbon atoms, preferably 3 to 12 carbon atoms, more preferably 3 to 8 (e.g., 3, 4, 5, 6, 7, and 8) carbon atoms, and most preferably 4 to 7 carbon atoms. Non-limiting examples of single-ring cycloalkanyl groups include cyclopropyl, cyclobutyl, cyclopentyl, cyclopentenyl, cyclohexyl, cyclohexenyl, cyclohexadienyl, cycloheptyl, cycloheptatriene, cyclooctyl, etc.

Cycloalkyl may be substituted or unsubstituted. When substituted, substituents may be attached at any available connection point, wherein the substituent is preferably independently selected from one or more of hydrogen, halogen, alkyl, alkoxy, haloalkyl, hydroxy, hydroxyalkyl, cyano, amino, nitro, cycloalkyl, heterocyclyl, aryl, or heteroaryl groups.

The term "heterocyclyl group" refers to a saturated or partially unsaturated monocyclic cyclic hydrocarbon substituent comprising 3 to 20 ring atoms, wherein one or more ring atoms are heteroatoms selected from nitrogen, oxygen, or S(O)m (where m is an integer from 0 to 2), but excluding ring segments consisting of -O-O-, -O-S-, or - S-S-, with the remaining ring atoms being carbon. Preferably, it comprises 3 to 12 ring atoms (e.g., 3, 4, 5, 6, 7, 8, 9, 10, 11, and 12), of which 1 to 4 (e.g., 1, 2, 3, and 4) are heteroatoms; more preferably, it comprises 3 to 8 ring atoms, of which 1 to 3 are heteroatoms; even more preferably, it comprises 3 to 6 ring atoms, of which 1 to 3 are heteroatoms; Most preferably, it comprises 5 or 6 ring atoms, of which 1 to 3 are heteroatoms. Non-limiting examples of single-ring heterocyclic groups include pyrrolidine, tetrahydropyran, 1,2,3,6-tetrahydropyridine, piperidine, piperazine, morpholine, thiomorpholine, and pyrrolidine, etc.

The heterocyclyl group may be substituted or unsubstituted. When substituted, substituents may be attached at any available connection point. Wherein the substituent is preferably independently substituted by one or more substituents selected from hydrogen, halogen, alkyl, alkoxy, haloalkyl, hydroxy, hydroxyalkyl, cyano, amino, nitro, cycloalkyl, heterocyclyl, aryl, or heteroaryl groups. The term "aryl" refers to a 6- to 14-membered monocyclic or fused polycyclic (where fused polycyclic rings share adjacent carbon atom pairs) group possessing a conjugated π-electron system, preferably 6 to 10 members, such as phenyl and naphthyl. The aromatic ring may be fused to a heteroaromatic, heterocyclic, or cycloalkyl ring as described above, provided that the ring connected to the parent structure is an aromatic ring. The aryl may be substituted or unsubstituted. When substituted, the substituent may be attached at any available connection point. Wherein the substituent is preferably independently substituted by one or more substituents selected from hydrogen, halogen, alkyl, alkoxy, haloalkyl, hydroxy, hydroxyalkyl, cyano, amino, nitro, cycloalkyl, heterocyclyl, aryl, or heteroaryl groups.

The term "heteroaromatic" refers to a heteroaromatic system comprising 1 to 4 heteroatoms (e.g., 1, 2, 3, and 4) and 5 to 14 ring atoms, wherein the heteroatoms are selected from oxygen, sulfur, and nitrogen. The heteroaromatic group is preferably 5 to 10 rings (e.g., 5, 6, 7, 8, 9, or 10 rings), and more preferably 5 or 6 rings, such as furan, thiophene, pyridine, pyrrolyl, N-alkylpyrrolyl, pyrimidine, pyrazinyl, diazolyl, imidazolyl, pyrazolyl, triazolyl, tetrazolyl, etc. The heteroaromatic ring described herein includes heteroaromatic rings fused to aromatic, heterocyclic, or cycloalkyl rings as described above, wherein the ring connected to the parent structure is the heteroaromatic ring. The heteroaryl group may be substituted or unsubstituted. When substituted, the substituent may be attached at any available connection point. Wherein the substituent is preferably independently substituted by one or more substituents selected from hydrogen, halogen, alkyl, alkoxy, haloalkyl, hydroxy, hydroxyalkyl, cyano, amino, nitro, cycloalkyl, heterocyclyl, aryl, or heteroaryl groups. The term "haloalkyl" refers to an alkyl group substituted by one or more halogens, where the alkyl group is defined as above.

The term "hydroxyalkyl" refers to an alkyl group, as defined above, substituted by one or more hydroxyl groups.

The term "halogen" refers to fluorine, chlorine, bromine, or iodine.

The term "amino" refers to -NH₂.

The term "cyano" refers to -CN.

The term "nitro" refers to -NO₂.

The term "ubiquitin ligase" refers to a family of proteins that catalyze the transfer of ubiquitin to specific substrate proteins, thereby targeting these substrate proteins for degradation. For example, cerebellum is an E3 ubiquitin ligase protein that, either alone or in combination with E2 ubiquitin ligases, causes ubiquitination of lysine residues on target proteins. This subsequently targets specific protein substrates for degradation by the proteasome. Therefore, E3 ubiquitin ligases, either acting independently or as part of a complex with E2 ubiquitin ligases, which are responsible for transferring ubiquitin to target proteins. Generally speaking, ubiquitin ligases participate in polyubiquitination, whereby the second ubiquitin is attached to the first ubiquitin, the third ubiquitin is attached to the second ubiquitin, and so on. Polyubiquitinated proteins are targeted for degradation by the proteasome. However, there exist some ubiquitination events restricted to monoubiquitination, in which only a single ubiquitin is added to the substrate molecule by the ubiquitin ligase. Unconjugated ubiquitinated proteins are not targeted to the proteasome for degradation, but may instead undergo changes in their cellular localization or function, such as through binding to other proteins possessing ubiquitin-binding domains. To complicate matters further, different lysine residues on ubiquitin can be targeted by E3 ligases to form chains. The most common lysine is Lys48 on the ubiquitin chain. This lysine is used to form polyubiquitin, which is recognized by the proteasome.

The term "target protein" refers to proteins and peptides possessing any biological function or activity (including structural, regulatory, hormonal, enzymatic, genetic, immune, contractile, storage, transport, and signal transduction functions). In some embodiments, target proteins include structural proteins, receptors, enzymes, cell surface proteins, and proteins involved in cellular integration functions, encompassing those associated with the following activities: catalytic activity, aromatase activity, motility activity, helicase activity, metabolic processes (anabolism and catabolism), antioxidant activity, proteolysis, biosynthesis, proteins with kinase activity, oxidoreductase activity, transferase activity, hydrolase activity, lyase activity, isomerase activity, ligase activity, enzyme regulatory factor activity, signal transduction factor activity, structural molecule activity, binding activity (proteins, lipids-carbohydrates), receptor activity, cell motility, membrane fusion, cell communication, biological process regulation, development, cell differentiation, stimulus response, behavioral proteins, cell adhesion proteins, proteins involved in cell death, proteins involved in transport (including protein transport activity, nuclear transport, ion transport activity, channel transport activity, carrier activity), permease activity, secretory activity, electron transport activity, pathogens, chaperone activity, nucleic acid binding activity, transcription factor activity, extracellular structure and biological origin activity, translational factor activity. The proteins described include those derived from eukaryotes and prokaryotes, with eukaryotes and prokaryotes comprising microorganisms, viruses, fungi, parasites, and numerous others. This includes humans, microorganisms, viruses, fungi, and parasites serving as targets for drug therapies, other animals such as domesticated animals, microorganisms used to determine targets for antibiotics and other antimicrobial agents, plants, viruses, and numerous other entities.

The term "isomer" refers to that the compounds of the present invention may possess an asymmetric center and may exist as racemates, racemic mixtures, or individual diastereomers. All such isomers, including stereoisomers and geometric isomers, are comprised within the scope of the present invention. In the present invention, when a compound or its salt exists in stereoisomeric form (e.g., containing one or more asymmetric carbon atoms), the individual stereoisomers (enantiomers and diastereomers) as well as mixtures thereof are included within the scope of the invention. The invention also comprises individual isomers of the compound or salt, as well as mixtures of isomers with one or more of their chiral centers reversed. The scope of the present invention includes mixtures of stereoisomers, as well as purified enantiomers or mixtures enriched in enantiomers or diastereomer mixtures. The present invention comprises mixtures of stereoisomers comprising all possible combinations of enantiomers and diastereomers. The present invention comprises all combinations and subsets of stereoisomers of all specific groups defined above. The present invention also includes geometric isomers of the compound or its salts, wherein the geometric isomers include cis-trans isomers.

The term "isotope derivative" refers to a compound differing structurally only in the presence of one or more isotopically enriched atoms. For example, structures according to the present disclosure comprise compounds wherein hydrogen atoms are substituted with deuterium or tritium, fluorine atoms are substituted with ¹⁸F-fluorine (¹⁸F isotope), or carbon atoms are substituted with ¹¹C-, ¹³C-, or ¹⁴C-enriched carbon (¹¹C-, ¹³C-, or ¹⁴C-carbon labeling; ¹¹C-, ¹³C-, or ¹⁴C-isotopes) instead of carbon atoms. Such compounds can serve as analytical tools or probes in biological assays, or as in vivo diagnostic imaging tracers for disease detection, or as tracers for pharmacodynamic, pharmacokinetic, or receptor studies.

"Optional" or "optionally" means that the event or circumstance described subsequently may or may not occur; the description encompasses both instances where the event or circumstance occurs and those where it does not. For example, "the cyclopropyl group may optionally be substituted" means that the cyclopropyl group may be substituted but need not necessarily be present. This description encompasses both cases where the cyclopropyl group is substituted and where it is not substituted.

"Substituted" refers to one or more hydrogen atoms in the group, preferably up to 5, and more preferably 1 to 3 hydrogen atoms, each of which is independently replaced by a corresponding number of substituents. It goes without saying that substituents occupy only their possible chemical positions, and those skilled in the art can readily determine (through experimentation or theory) which substituents are possible or impossible without undue effort.

"Pharmaceutical salts" or "pharmaceutically acceptable salt" refers to salts of the compounds disclosed herein that are safe and effective for use in mammals and possess the desired biological activity.

In this application, a wavy line drawn on a functional group, regardless of its form, indicates that this is the point of attachment to the rest of the molecule. If no wavy line is drawn on a functional group, it means that any position within that group could be attached to another part of the molecule.

Unless otherwise defined, when a group described in the present invention is substituted by a substituent, it means that all instances of that same group appearing in the present invention may be substituted by a substituent; that is, it indicates that the group may be substituted when it exists in isolation, and also indicates that the group may be substituted when it exists in combination with other groups. For example, R is -C₁₋₆ alkyl, C₆₋₁₀ aryl, C₃₋₆ monocyclic cycloalkyl, -C(O)C₁₋₆ alkyl, - C₁₋₄-alkyl-C₆₋₁₀-aryl, or -S(O)₂-C₃₋₆ monocyclic alkyl, wherein the C₁₋₆-alkyl, C₆₋₁₀-aryl, and C₃₋₆ monocyclic alkyl are optionally substituted; this description also includes the -C(O)C₁₋₆-alkyl, -C₁₋₄-alkyl-C₆₋₁₀-aryl, and -S(O)₂- C₃₋₆ monocyclic cycloalkyl, wherein the C₁₋₆-alkyl, C₆₋₁₀-aryl, and C₃₋₆ monocyclic cycloalkyl groups are optionally substituted.

Unless otherwise defined, the term "...the same or different" as used in the present invention means that, when there is more than one identical substituent group in a general formula, such groups may be the same or different. In this invention, " 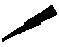 " and " 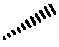 " denote the absolute configuration of a stereocenter. A bond " 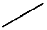 " includes the possible " 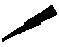 " and " 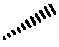 " configurations, provided that the chemical formula permits.

When 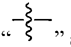 appears in a ring structure and the exact position of the bond is unspecified, it indicates that the bond can be formed with any atom on the same ring as 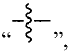 provided that the atom's valence permits.

### II. Examples

The following examples are provided by way of illustration and not limitation.

The abbreviations used herein are as follows:
MeOH is methanol; H₂SO₂ is concentrated sulfuric acid; NIS is N-iodosuccinimide; TFA is trifluoroacetic acid; Pd is palladium; Sn is tin; Pyrrolidine is pyrrolidine; OH is hydroxy; Boc is tert-butoxycarbonyl; NaBH₄ is sodium borohydride; Et₃SiH is triethylsilane; H₂ is hydrogen; LiOH is lithium hydroxide; NaOAc is sodium acetate; AcOH is acetic acid; PE is petroleum ether; EA is ethyl acetate; CDCl₃ is deuterated chloroform; HNO₃ is nitric acid; HBF₄ is tetrafluoroboric acid; NaNO₂ is sodium nitrite; NBS is N-bromosuccinimide; KOtBu is potassium tert-butoxide; NaH is sodium hydride; B₂pin₂ is bis(pinacolato)diboron; Oxone is potassium peroxymonosulfate; Cbz is benzyloxycarbonyl; PPh₃ is triphenylphosphine; CBr₄ is carbon tetrabromide; Zn is zinc; NH₄Cl is ammonium chloride; B is boron; Br is bromine; (TMS)₃SiH is tris(trimethylsilyl)silane; AIBN is azobisisobutyronitrile; DMF is N,N-dimethylformamide; K₂CO₃ is potassium carbonate; TBAB is tetrabutylammonium bromide; Bn is benzyl; LiAlH₄ is lithium aluminum hydride; BH₃ is borane; THF is tetrahydrofuran; H₂O₂ is hydrogen peroxide; DMP is Dess-Martin periodinane; PBr₃ is phosphorus tribromide; Pd(OAc)₂ is palladium(II) acetate; BF₃-Et₂O is boron trifluoride diethyl etherate; S is sulfur; MsCl is methanesulfonyl chloride; TEA is triethylamine; DBU is 1,8-diazabicyclo[5.4.0]undec-7-ene; NaHCO₃ is sodium bicarbonate; Malonic acid is malonic acid; PPA is polyphosphoric acid; HBr is hydrogen bromide; Tf₂O is trifluoromethanesulfonic anhydride; Pd/C is palladium on carbon; DMSO is dimethyl sulfoxide; UPLC is ultra performance liquid chromatography; MgCl₂ is magnesium chloride; NADPH is nicotinamide adenine dinucleotide phosphate; NaS₂O₃ is sodium thiosulfate; Pd(dppf)Cl₂ or PdCl₂(dppf) is [1,1'-bis(diphenylphosphino)ferrocene]dichloropalladium(II); Boc₂O is di-tert-butyl dicarbonate; AIBN is azobisisobutyronitrile; DIPEA or DIEA is N,N-diisopropylethylamine; TsOH is p-toluenesulfonic acid; CCl₄ is carbon tetrachloride; CAN is ceric ammonium nitrate; IBX is 2-iodoxybenzoic acid; HATU is 2-(7-aza-1H-benzotriazole-1-yl)-1,1,3,3-tetramethyluronium hexafluorophosphate.

The following examples are directed to the intermediate compounds and final products identified in the specification and synthetic schemes. The following examples are used to provide a detailed description of the preparation of the compounds of the present invention, but the chemical reactions described are disclosed based on their general applicability to the preparation of the compounds of the present invention. Sometimes, the reactions may not be applicable to every compound within the scope of the present invention as described. Those skilled in the art can readily identify compounds where this occurs. In these cases, the reactions can be successfully carried out by routine modifications known to those skilled in the art. In all preparation methods, all starting materials are known or can be readily prepared using known starting materials.

The starting materials, chemical reagents, and solvents used in the present disclosure are all commercially available and were purchased from companies such as Energy Chemical, Shanghai BiDe Pharmaceutical, Beijing InnoChem, Jiangsu Aikon, Sinopharm Chemical Reagent, Beijing J&K Scientific, Yunnan Xinlanjing, etc.

The structures of the compounds were determined by nuclear magnetic resonance (NMR) and/or mass spectrometry (MS). NMR was measured using a Bruker AVANCE-400/600 NMR spectrometer, using deuterated solvents such as deuterated dimethyl sulfoxide (DMSO-d₆), deuterated chloroform (CDCl₃), and deuterated methanol (CD₃OD), with tetramethylsilane (TMS) as the internal standard. MS was measured using a Waters Acquity UPLC^{®} Plus device. High performance liquid phase preparative separation was performed using a Waters 2489 device. Medium pressure rapid preparative chromatography was performed using a COMBIFLASH NEXTGEN 300+ device. Silica gel 60 thin-layer chromatography silica gel plates (aluminum plates, with fluorescence) were used. The silica gel (100-200 mesh, 200-300 mesh) used for thin-layer chromatography was purchased from InnoChem.

The progress of reactions in the examples was monitored by thin-layer chromatography (TLC). The developing solvent systems used for monitoring the reactions and the eluent systems used for purifying compounds by column chromatography included: petroleum ether/ethyl acetate system and dichloromethane/methanol system.

### Example 1: Synthesis of Compound A1

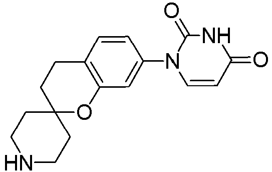

### 1-(spiro[chroman-2,4'-piperidin]-7-yl)pyrimidine-2,4(1H,3H)-dione

### Synthetic Scheme

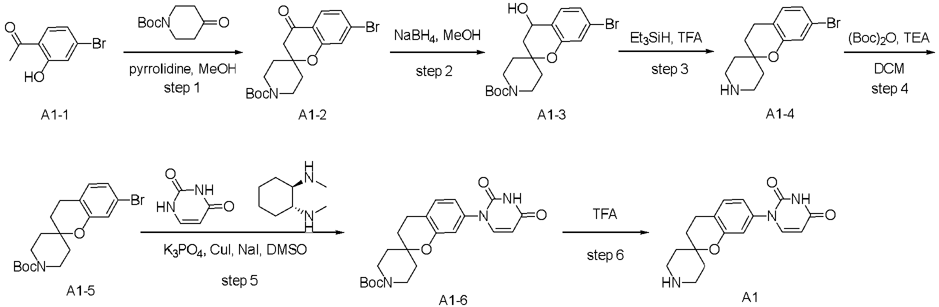

### Step 1: tert-Butyl 7-bromo-4-oxospiro[chroman-2,4'-piperidine]-1'-carboxylate (Compound A1-2)

Compound A1-1 (25 g, 116.3 mmol, 1.0 eq), tert-butyl 4-oxopiperidine-1-carboxylate (23.16 g, 116.3 mmol, 1.0 eq) and pyrrolidine (4.13 g, 58.1 mmol, 0.5 eq) were dissolved in methanol (250 mL). The mixture was heated to 70°C and stirred for 16 hours. After cooling the reaction mixture to room temperature, it was filtered to obtain a yellow solid Compound A1-2 (40 g, 86.83%).

¹H NMR (400 MHz, Chloroform-d) δ 7.73 (d, *J* = 8.4 Hz, 1H), 7.23 (d, *J =* 1.8 Hz, 1H), 7.17 (dd, *J =* 8.4, 1.8 Hz, 1H), 3.90 (s, 2H), 3.21 (t, *J =* 12.6 Hz, 2H), 2.73 (s, 2H), 2.02 (dd, *J =* 14.4, 2.8 Hz, 2H), 1.66 - 1.58 (m, 2H), 1.48 (s, 9H).

LCMS (ESI): [M-t-Bu+H]⁺ = 340.20.

### Step 2: tert-Butyl 7-bromo-4-hydroxyspiro[chroman-2,4'-piperidine]-1'-carboxylate (Compound A1-3)

Compound A1-2 (40 g, 100.9 mmol, 1.0 eq) was dissolved in methanol (400 mL). The reaction mixture was cooled to 0°C, and sodium borohydride (5.73 g, 151.4 mmol, 1.5 eq) was added. The mixture was stirred at room temperature overnight. After concentration under reduced pressure, water was added, and the mixture was extracted three times with ethyl acetate. The organic layers were washed with saturated brine, combined, and dried over anhydrous sodium sulfate. The organic layer was concentrated to obtain a crude white solid Compound A1-3 (40 g, 99.49%).LCMS (ESI): [M-Boc+H]⁺ = 298.20.

### Step 3: 7-Bromospiro[chroman-2,4'-piperidine] (Compound A1-4)

Compound A1-3 (40.0 g, 100.4 mmol, 1.0 eq) was dissolved in trifluoroacetic acid (200 mL), and triethylsilane (17.52 g, 150.6 mmol, 1.5 eq) was added. The mixture was heated to 50°C and stirred overnight. After cooling the reaction mixture to room temperature, it was concentrated under reduced pressure to obtain crude Compound A1-4 (28.0 g), which was used directly in the next step.

LCMS (ESI): [M+H]⁺ = 282.20.

### Step 4: tert-Butyl 7-bromospiro[chroman-2,4'-piperidine]-1'-carboxylate (Compound A1-5)

Compound A1-4 (28.0 g, 99.2 mmol, 1.0 eq) was dissolved in dichloromethane (300 mL). The reaction mixture was cooled to 0°C, and triethylamine (30.12 g, 297.7 mmol, 3.0 eq) and di-tert-butyl dicarbonate (32.48 g, 148.8 mmol, 1.5 eq) were added. The mixture was stirred at room temperature overnight. After concentration under reduced pressure, the crude product was purified by column chromatography to obtain a white solid Compound A1-5 (32 g, 84.36%).

¹H NMR (400 MHz, Chloroform-d) δ 7.03 (d, *J =* 2.0 Hz, 1H), 7.00 - 6.91 (m, 2H), 3.90 (s, 2H), 3.21 (s, 2H), 2.74 (t*, J* = 6.8 Hz, 2H), 1.80 (q, *J* = 7.7, 7.3 Hz, 4H), 1.59 - 1.50 (m, 2H), 1.49 (s, 9H).

### Step 5: tert-Butyl 7-(2,4-dioxo-3,4-dihydropyrimidin-1(2H)-yl)spiro[chroman-2,4'-piperidine]-1'-carboxylate (Compound A1-6)

Compound A1-5 (100 mg, 0.26 mmol, 1.0 eq), uracil (29 mg, 0.26 mmol, 1.0 eq), copper(I) iodide (5 mg, 0.03 mmol, 0.1 eq), sodium iodide (4 mg, 0.03 mmol, 0.1 eq), potassium phosphate (117 mg, 0.55 mmol, 2.1 eq), and trans-(1R,2R)-N,N'-dimethyl-1,2-cyclohexanediamine (37 mg, 0.26 mmol, 1.0 eq) were dissolved in dimethyl sulfoxide (1 mL). The mixture was stirred at 110°C overnight. The reaction solution was concentrated under reduced pressure, and the crude product was purified by column chromatography to obtain a light blue solid Compound A1-6(20 mg, 18.49%).

¹H NMR (600 MHz, DMSO-d₆) δ 11.39 (d, *J* = 2.2 Hz, 1H), 7.67 (d, *J* = 7.9 Hz, 1H), 7.18 (d, *J* = 8.7 Hz, 1H), 6.89 - 6.82 (m, 2H), 5.63 (dd, *J* = 7.9, 2.3 Hz, 1H), 3.71 (d, *J* = 13.0 Hz, 2H), 3.16 (s, 2H), 2.77 (t, *J* = 6.8 Hz, 2H), 1.82 (t*, J* = 6.7 Hz, 2H), 1.69 (d*, J* = 13.6 Hz, 2H), 1.56 (td, *J =* 13.3, 12.7, 4.6 Hz, 2H), 1.41 (s, 9H).

LCMS (ESI) [M-t-Bu+H]⁺ = 358.30.

### Step 6: 1-(spiro[chroman-2,4'-piperidin]-7-yl)pyrimidine-2,4(1H,3H)-dione (Compound A1)

Compound A1-6 (20 mg, 0.05 mmol, 1.0 eq) was dissolved in dichloromethane (1 mL), and trifluoroacetic acid (0.1 mL) was added. The mixture was stirred at room temperature for 12 hours. The reaction mixture was concentrated, and the crude product was purified by HPLC to obtain a white solid Compound A1 (2 mg, 13.19%).

¹H NMR (400 MHz, DMSO-d₆) δ 11.43 (d, *J* = 2.2 Hz, 1H), 7.67 (d, *J* = 7.9 Hz, 1H), 7.21 (d, *J* = 7.9 Hz, 1H), 6.90 (d, *J* = 7.8 Hz, 2H), 5.65 (dd, *J* = 7.9, 2.2 Hz, 1H), 3.23 (d, *J* = 12.7 Hz, 2H), 3.11 (d, *J* = 11.4 Hz, 2H), 2.80 (t, *J* = 6.7 Hz, 2H), 2.03 - 1.78 (m, 7H).

LCMS (ESI) [M+H]⁺ = 314.30.

### Example 2: Synthesis of Compound A2

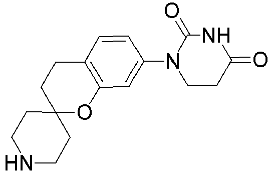

### 1-(spiro[chroman-2,4'-piperidin]-7-yl)dihydropyrimidine-2,4(1H,3H)-dione

### Synthetic Scheme

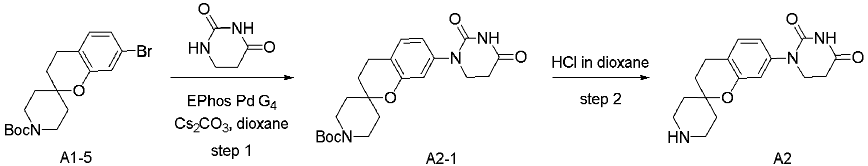

### Step 1: tert-Butyl 7-(2,4-dioxotetrahydropyrimidin-1(2H)-yl)spiro[chroman-2,4'-piperidine]-1'-carboxylate (Compound A2-1)

Compound A1-5 (5 g, 13.1 mmol, 1.0 eq), dihydropyrimidine-2,4(1H,3H)-dione (4.48 g, 39.2 mmol, 3.0 eq), (methanesulfonato {dicyclohexyl(3-isopropoxy-2',4',6'-triisopropyl-[1,1'-biphenyl]-2-yl)phosphane}(2'-methylamino-1,1'-biphenyl-2-yl)palladium (601 mg, 0.65 mmol, 0.05 eq), and cesium carbonate (12.78 g, 39.2 mmol, 3.0 eq) were dissolved in dioxane (50 mL). The mixture was stirred at 80°C overnight. The reaction solution was concentrated under reduced pressure, and the resulting crude product was purified by column chromatography to obtain a white solid Compound A1-6 (3 g, 55.21%).

LCMS (ESI) [M-t-Bu+H]⁺ = 360.30.

### Step 2: 1-(spiro[chroman-2,4'-piperidin]-7-yl)dihydropyrimidine-2,4(1H,3H)-dione (Compound A2)

Compound A2-1 (3 g, 7.2 mmol, 1.0 eq) was dissolved in dichloromethane (5 mL), and a solution of hydrogen chloride in dioxane (5 mL) was added. The mixture was stirred at room temperature for 12 hours. The reaction mixture was filtered to obtain a white solid Compound A2 (277 mg, 69.4%).

¹H NMR (600 MHz, DMSO-d₆) δ 10.35 (s, 1H), 7.10 (d, *J* = 8.8 Hz, 1H), 6.87 - 6.79 (m, 2H), 3.74 (t, *J =* 6.6 Hz, 3H), 3.19 (dt, *J* = 13.0, 3.4 Hz, 2H), 3.07 (h, *J* = 9.9 Hz, 2H), 2.75 (t, *J* = 6.8 Hz, 2H), 2.68 (t, *J =* 6.6 Hz, 2H), 1.93 - 1.78 (m, 6H).

LCMS (ESI) [M+H]⁺ = 316.30.

### Example 3: Synthesis of Compound A3

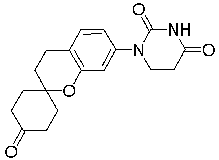

### 1-(4'-oxospiro[chroman-2,1'-cyclohexan]-7-yl)dihydropyrimidine-2,4(1H,3H)-dione

### Synthetic Scheme

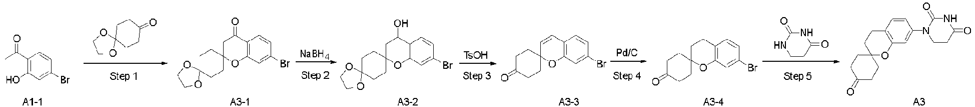

### Step 1: 7-Bromodipyrano[chroman-2,1'-cyclohexane-4',2"-[1,3]dioxolan]-4-one (CompoundA3-1)

Compound A1-1 (1.0 g, 4.7 mmol, 1.0 eq) was dissolved in methanol (10 mL). 1,4-cyclohexanedione monoethylene ketal (726 mg, 4.7 mmol, 1.0 eq) and pyrrolidine (331 mg, 4.7 mmol, 1.0 eq) were added, and the mixture was stirred at reflux under nitrogen protection for 12 hours. The reaction mixture was cooled to room temperature, and the precipitated solid was filtered off to obtain a yellow solid Compound A3-1 (1.4 g, 83.4%).

¹H NMR (600 MHz, Chloroform-d) δ 7.73 (d, *J =* 8.4 Hz, 1H), 7.21 (d, *J =* 1.8 Hz, 1H), 7.15 (dd, *J =* 8.4, 1.8 Hz, 1H), 4.03 - 3.95 (m, 4H), 2.72 (s, 2H), 2.15 - 2.09 (m, 2H), 1.98 (td, *J =* 13.3, 4.1 Hz, 2H), 1.76 (td, *J =* 13.6, 4.2 Hz, 2H), 1.67 - 1.61 (m, 2H).

LCMS (ESI): [M+H]⁺ = 353.19.

### Step 2: 7-Bromodipyrano[chroman-2,1'-cyclohexane-4',2"-[1,3]dioxolan]-4-ol (Compound A3-2)

To Compound A3-1 (1.0 g, 2.8 mmol, 1.0 eq) was added methanol (10 mL). Sodium borohydride (160 mg, 4.3 mmol, 1.5 eq) was added at 0°C, and the mixture was stirred at room temperature for 2 hours. The reaction mixture was concentrated under reduced pressure, then water was added. The mixture was extracted with ethyl acetate, washed with saturated brine, and the combined organic layers were dried over anhydrous sodium sulfate. Concentration under reduced pressure gave crude Compound A3-2 (1 g).

LCMS (ESI): [M-OH+H]⁺ = 337.19.

### Step 3: 7-Bromospiro[chroman-2,1'-cyclohexan]-4'-one (Compound A3-3)

Compound A3-2 (1.0 g, 2.8 mmol, 1.0 eq) was dissolved in toluene (10 mL). p-Toluenesulfonic acid (533 mg, 3.1 mmol, 1.1 eq) was added, and the mixture was stirred at 110°C for 12 hours. After cooling the reaction mixture to room temperature, it was concentrated under reduced pressure. The resulting crude product was purified by column chromatography to obtain a yellow oily Compound A3-3 (300 mg, 36.5%).

¹H NMR (400 MHz, Chloroform-d) δ 7.11 - 7.03 (m, 2H), 6.91 (d, *J =* 7.9 Hz, 1H), 6.43 (d, *J =* 9.8 Hz, 1H), 5.64 (d, *J* = 9.8 Hz, 1H), 2.84 (td, *J =* 14.2, 6.1 Hz, 2H), 2.51 - 2.27 (m, 4H), 1.89 (td, *J* = 13.8, 5.0 Hz, 2H).

LCMS (ESI): [M+H]⁺ = 294.98.

### Step 4: 7-Bromospiro[chroman-2,1'-cyclohexan]-4'-one (Compound A3-4)

Compound A3-3 (300 mg, 1.0 mmol, 1.0 eq) was dissolved in ethyl acetate (5 mL). Palladium on carbon (60 mg) was added. The mixture was stirred at room temperature under hydrogen pressure for 12 hours. The reaction mixture was filtered through Celite, and the filtrate was concentrated under reduced pressure. The resulting crude product was purified by column chromatography to obtain a white solid Compound A3-4 (150 mg, 49.7%).

¹H NMR (600 MHz, Chloroform-d) δ 7.08 (d, *J =* 1.8 Hz, 1H), 7.02 (dt, *J =* 8.1, 1.4 Hz, 1H), 6.97 (d, *J =* 8.1 Hz, 1H), 2.83 - 2.75 (m, 4H), 2.34 - 2.28 (m, 2H), 2.26 - 2.20 (m, 2H), 1.91 (t, *J =* 6.8 Hz, 2H), 1.83 (td, *J =* 13.7, 4.9 Hz, 2H).

LCMS (ESI) [M+H]⁺ = 296.40.

### Step 5: 1-(4'-Oxospiro[chroman-2,1'-cyclohexan]-7-yl)dihydropyrimidine-2,4(1H,3H)-dione (Compound A3)

To Compound A3-4 (100 mg, 0.34 mmol, 1.0 eq), dihydrouracil (116 mg, 1.0 mmol, 3.0 eq), (methanesulfonato{dicyclohexyl(3-isopropoxy-2',4',6'-triisopropyl-[1,1'-biphenyl]-2-yl)phosphane}(2'-methylamino-1,1'-biphenyl-2-yl)palladium (16 mg, 0.02 mmol, 0.05 eq) and cesium carbonate (221 mg, 0.68 mmol, 2.0 eq) was added super-dry 1,4-dioxane (2 mL). The mixture was stirred at 100°C under nitrogen protection for 12 hours. After cooling the reaction mixture to room temperature, it was concentrated under reduced pressure. The resulting crude product was purified by column chromatography to obtain a white solid Compound A3 (70 mg, 62.9%).

¹H NMR (600 MHz, DMSO-d₆) δ 10.34 (s, 1H), 7.13 - 7.09 (m, 1H), 6.85 - 6.80 (m, 2H), 3.75 (t, *J =* 6.6 Hz, 2H), 2.76 (t, *J* = 6.8 Hz, 2H), 2.68 (t, *J* = 6.6 Hz, 2H), 2.63 - 2.53 (m, 2H), 2.22 - 2.16 (m, 2H), 2.10 - 2.02 (m, 2H), 1.94 - 1.86 (m, 4H).

LCMS (ESI) [M+H]⁺ = 329.28.

### Example 4: Synthesis of Compound A4

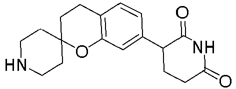

### 3-(spiro[chromene-2,4'-piperidin]-7-yl)piperidine-2,6-dione

### Synthetic Scheme

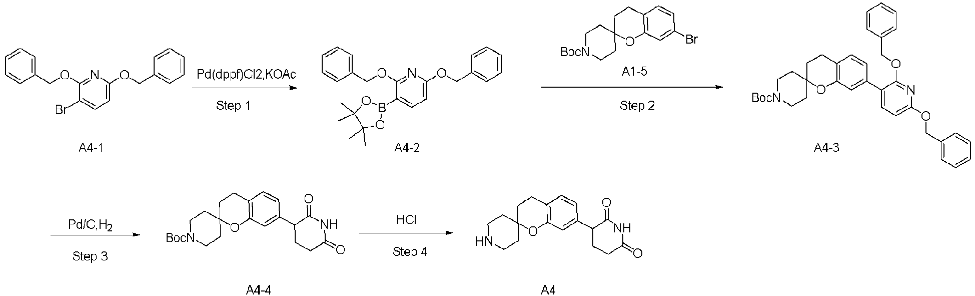

### Step 1: 2,6-Bis(benzyloxy)-3-(4,4,5,5-tetramethyl-1,3,2-dioxaborolan-2-yl)pyridine (Compound A4-2)

Compound A4-1 (5.2 g, 14.04 mmol, 1.0 eq) was dissolved in N,N-dimethylformamide (60 mL). 1,1'-Bis(diphenylphosphino)ferrocene-palladium(II) dichloride (2.06 g, 2.81 mmol, 0.2 eq), potassium acetate (4.14 g, 42.13 mmol, 3 eq) and bis(pinacolato)diboron (10.7 g, 42.13 mmol, 3 eq) were added to the reaction mixture. The mixture was stirred at 100°C under nitrogen protection for 12 hours. The resulting reaction mixture was concentrated under reduced pressure, and the crude product obtained by concentration was purified by normal phase column chromatography to obtain a crude light yellow solid A4-2 (8.2 g, 139.9%).

LCMS (ESI): [M+H]⁺ = 418.31.

### Step 2: tert-Butyl 7-(2,6-bis(benzyloxy)pyridin-3-yl)spiro[chromene-2,4'-piperidine]-1'-carboxylate (Compound A4-3)

Crude Compound A4-2 (5 g, 9.59 mmol, 1.5 eq) was dissolved in a mixed solvent of 1,4-dioxane and water (60 mL, 5:1). 1,1'-Bis(diphenylphosphino)ferrocene-palladium(II) dichloride (0.83 g, 1.28 mmol, 0.2 eq), sodium carbonate (2.03 g, 19.17 mmol, 3 eq) and Compound A1-5 (2.44 g, 6.39 mmol, 1 eq) were added to the reaction mixture. The mixture was stirred at 90°C under nitrogen protection for 12 hours. The resulting reaction mixture was concentrated under reduced pressure, and the crude product obtained by concentration was purified by normal phase column chromatography to obtain a red oily Compound A4-3 (2.7 g, 71.3%).

LCMS (ESI): [M+H]⁺ = 593.29.

### Step 3: tert-Butyl 7-(2,6-dioxopiperidin-3-yl)spiro[chromene-2,4'-piperidine]-1'-carboxylate (Compound A4-4)

Compound A4-3 (2.7 g, 4.56 mmol, 1.0 eq) was dissolved in methanol (30 mL). Palladium (20%) on carbon (1.35 g, 50% w/w) was added to the mixture. After three cycles of hydrogen replacement, the mixture was stirred at room temperature for 12 hours. The reaction mixture was filtered, and the filter cake was washed three times with methanol (5 mL each). The resulting filtrate was concentrated under reduced pressure, and the crude product obtained by concentration was purified by normal phase column chromatography to obtain a light yellow solid Compound A4-4 (689 mg, 36.5%).

LCMS (ESI): [M+H]⁺ = 415.22.

### Step 4: 3-(spiro[chromene-2,4'-piperidin]-7-yl)piperidine-2,6-dione (Compound A4)

Compound A4-4 (680 mg, 1.64 mmol, 1.0 eq) was dissolved in dichloromethane (12 mL). A solution of hydrogen chloride in 1,4-dioxane (120 mg, 3.28 mmol, 2 eq) was added. The mixture was stirred at room temperature for 2 hours, then separated by preparative HPLC to obtain a white solid Compound A4 (486 mg, 94.2%).

¹H NMR (600 MHz, DMSO-d₆) δ 10.83 (s, 1H), 7.06 (d, *J =* 7.8 Hz, 1H), 6.72 (dd, *J =* 7.8, 1.7 Hz, 1H), 6.69 (d, *J =* 1.7 Hz, 1H), 3.77 (dd, *J =* 11.2, 5.0 Hz, 1H), 3.27 - 3.17 (m, 2H), 3.15 - 3.05 (m, 2H), 2.74 (t, *J =* 6.7 Hz, 2H), 2.67 - 2.58 (m, 1H), 2.47 (dt, *J =* 17.2, 4.5 Hz, 1H), 2.20 - 2.10 (m, 1H), 2.06 - 1.96 (m, 1H), 1.93 - 1.87 (m, 2H), 1.83 (t, *J* = 6.8 Hz, 2H), 1.81 - 1.73 (m, 2H).

LCMS (ESI): [M+H]⁺ = 315.16.

### Example 5: Synthesis of Compound A5

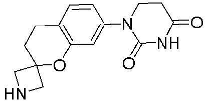

### 1-(spiro[azetidine-3,2'-chroman]-7'-yl)dihydropyrimidine-2,4(1H,3H)-dione

### Synthetic Scheme

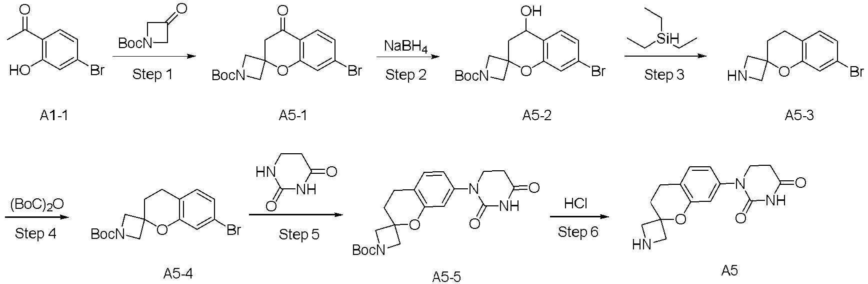

### Step 1: tert-Butyl 7'-bromo-4'-oxospiro[azetidine-3,2'-chroman]-1-carboxylate (Compound A5-1)

Compound A1-1 (10.0 g, 46.5 mmol, 1.0 eq) was dissolved in ethanol (100 mL). tert-Butyl 3-oxoazetidine-1-carboxylate (8.8 g, 51.2 mmol, 1.1 eq) and pyrrolidine (3.6 g, 51.2 mmol, 1.1 eq) were added, and the mixture was stirred at 50°C under nitrogen protection for 12 hours. After cooling the reaction mixture to room temperature, it was extracted with ethyl acetate, washed with saturated brine, and the combined organic layers were dried over anhydrous sodium sulfate. The organic layer was concentrated, and the resulting crude product was purified by column chromatography to obtain a yellow solid Compound A5-1 (5.5 g, 32.1%).

¹H NMR (600 MHz, DMSO-d₆) δ 7.67 (d, *J =* 8.4 Hz, 1H), 7.48 (d, *J =* 1.8 Hz, 1H), 7.33 (dd, *J =* 8.4, 1.8 Hz, 1H), 3.99 (d, *J* = 9.6 Hz, 2H), 3.90 (d, *J* = 9.6 Hz, 2H), 3.19 (s, 2H), 1.38 (s, 9H).

LCMS (ESI): [M-Boc+H]⁺ = 268.15.

### Step 2: tert-Butyl 7'-bromo-4'-hydroxyspiro[azetidine-3,2'-chroman]-1-carboxylate (Compound A5-2)

To Compound A5-1 (5.5 g, 14.9 mmol, 1.0 eq) was added methanol (50 mL). Sodium borohydride (969 mg, 25.6 mmol, 1.5 eq) was added at 0°C, and the mixture was stirred at room temperature for 12 hours. The reaction mixture was concentrated under reduced pressure, then water was added. The mixture was extracted with ethyl acetate, washed with saturated brine, and the combined organic layers were dried over anhydrous sodium sulfate. It was concentrated under reduced pressure to obtain a crude Compound A5-2 (5.5 g).

¹H NMR (400 MHz, DMSO-d₆) δ 7.31 - 7.25 (m, 1H), 7.12 (dd, *J* = 8.2, 2.0 Hz, 1H), 7.07 (d, *J* = 2.0 Hz, 1H), 5.56 (d, *J* = 5.1 Hz, 1H), 4.75 - 4.62 (m, 1H), 4.15 (d, *J* = 9.6 Hz, 1H), 4.00 (d, *J* = 9.7 Hz, 1H), 3.82 (dd, *J* = 19.0, 9.5 Hz, 2H), 2.30 - 2.10 (m, 2H), 1.38 (s, 9H).

LCMS (ESI): [M-Boc+H]⁺ = 272.17.

### Step 3: 7'-Bromospiro[azetidine-3,2'-chroman] (Compound A5-3)

Compound A5-2 (5.5 g, 14.8 mmol, 1.0 eq) was dissolved in trifluoroacetic acid (20 mL), and triethylsilane (3.5 g, 30.0 mmol, 2.0 eq) was added. The mixture was stirred at 50°C for 1 hour. After cooling the reaction mixture to room temperature, it was concentrated under reduced pressure to obtain a crude Compound A5-3 (3.8 g).

LCMS (ESI) [M+H]⁺ = 254.17.

### Step 4: tert-Butyl 7'-bromospiro[azetidine-3,2'-chroman]-1-carboxylate (Compound A5-4)

Compound A5-3 (3.8 g, 14.8 mmol, 1.0 eq) was dissolved in dichloromethane (40 mL). Triethylamine (4.5 g, 44.5 mmol, 3.0 eq) and di-tert-butyl dicarbonate (4.9 g, 22.3 mmol, 1.5 eq) were added at 0°C. The mixture was stirred at room temperature for 12 hours. Water was added to the reaction mixture, which was then extracted with dichloromethane, washed with saturated brine, and the combined organic layers were dried over anhydrous sodium sulfate. The organic layer was concentrated, and the resulting crude product was purified by column chromatography to obtain a white solid Compound A5-4 (2.3 g, 43.2%).

¹H NMR (400 MHz, DMSO-d₆) δ 7.09 - 7.02 (m, 3H), 3.93 (d, *J =* 9.3 Hz, 2H), 3.82 (d, *J =* 9.3 Hz, 2H), 2.74 (t, *J =* 6.5 Hz, 2H), 2.06 (t, *J =* 6.5 Hz, 2H), 1.39 (s, 9H).

LCMS (ESI) [M-Boc+H]⁺ = 254.17.

### Step 5: tert-Butyl 7'-(2,4-dioxotetrahydropyrimidin-1(2H)-yl)spiro[azetidine-3,2'-chroman]-1-carboxylate (Compound A5-5)

To Compound A5-4 (1.0 g, 2.8 mmol, 1.0 eq), dihydrouracil (966 mg, 8.5 mmol, 3.0 eq), (methanesulfonato{dicyclohexyl(3-isopropoxy-2',4',6'-triisopropyl-[1,1'-biphenyl]-2-yl)phosphane}(2'-methylamino-1,1'-biphenyl-2-yl)palladium (130 mg, 0.14 mmol, 0.05 eq) and cesium carbonate (2.8 g, 8.5 mmol, 3.0 eq) was added super-dry 1,4-dioxane (15 mL). The mixture was stirred at 100°C under nitrogen protection for 12 hours. After cooling the reaction mixture to room temperature, it was concentrated under reduced pressure. The resulting crude product was purified by column chromatography to obtain a white solid Compound A5-5 (655 mg, 59.9%).

¹H NMR (600 MHz, DMSO-d₆) δ 10.36 (s, 1H), 7.09 (d, *J* = 8.2 Hz, 1H), 6.84 (dd, *J* = 8.1, 2.2 Hz, 1H), 6.82 (d, *J* = 2.1 Hz, 1H), 3.93 (s, 2H), 3.83 (s, 2H), 3.74 (t, *J =* 6.7 Hz, 2H), 2.77 (t, *J* = 6.5 Hz, 2H), 2.68 (t, *J* = *6*.7 Hz, 2H), 2.07 *(t, J* = 6.5 Hz, 2H), 1.40 (s, 9H).

LCMS (ESI): [M-Boc+H]⁺ = 288.38.

### Step 6: 1-(spiro[azetidine-3,2'-chroman]-7'-yl)dihydropyrimidine-2,4(1H,3H)-dione (Compound A5)

Compound A5-5 (355 mg, 0.92 mmol, 1.0 eq) was dissolved in dichloromethane (5 mL), and a solution of hydrogen chloride in 1,4-dioxane (5 mL) was added. The mixture was stirred at room temperature for 2 hours. The reaction mixture was concentrated, and the resulting crude product was purified by reverse phase column chromatography to obtain a white solid Compound A5 (180 mg, 68.4%).

¹H NMR (600 MHz, DMSO-d₆) δ 10.37 (s, 1H), 9.32 (s, 1H), 7.12 (d, *J* = 8.2 Hz, 1H), 6.87 (dd, *J* = 8.1, 2.2 Hz, 1H), 6.84 (d, *J =* 2.1 Hz, 1H), 4.10 - 4.01 (m, 4H), 3.74 (t, *J =* 6.7 Hz, 2H), 2.78 (t, *J* = 6.5 Hz, 2H), 2.69 (t, *J* = 6.6 Hz, 2H), 2.18 (t, *J* = 6.5 Hz, 2H).

LCMS (ESI) [M+H]⁺ = 288.30.

### Example 6: Synthesis of Compound A6

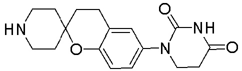

### 1-(spiro[chromate-2,4'-piperidin]-6-yl)dihydropyrimidine-2,4(1H,3H)-dione

### Synthetic Scheme

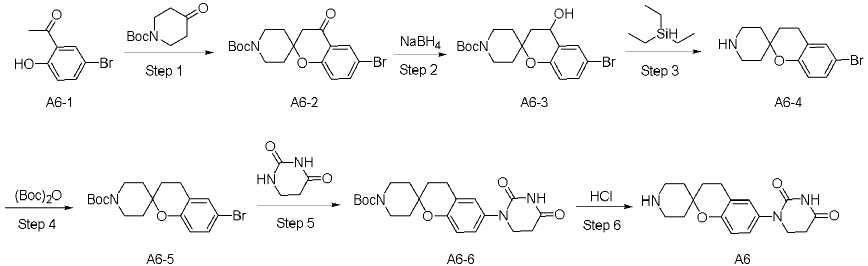

### Step 1: tert-Butyl 6-bromo-4-oxospiro[chroman-2,4'-piperidine]-1'-carboxylate (Compound A6-2)

Compound A6-1 (10.0 g, 46.5 mmol, 1.0 eq) was dissolved in methanol (100 mL). N-tert-Butoxycarbonyl-4-piperidone (10.2 g, 51.2 mmol, 1.1 eq) and pyrrolidine (3.6 g, 51.2 mmol, 1.1 eq) were added, and the mixture was stirred at 80°C under nitrogen protection for 12 hours. After cooling the reaction mixture to room temperature, it was extracted with ethyl acetate, washed with saturated brine, and the combined organic layers were dried over anhydrous sodium sulfate. The organic layer was concentrated, and the resulting crude product was purified by column chromatography to obtain a white solid Compound A6-2 (17.5 g, 95.0%).

¹H NMR (400 MHz, DMSO-d₆) δ 7.78 (d, *J* = 2.6 Hz, 1H), 7.74 (dd, *J* = 8.8, 2.6 Hz, 1H), 7.09 (d, *J* = 8.8 Hz, 1H), 3.71 (d, *J* = 13.4 Hz, 2H), 3.12 (s, 2H), 2.88 (s, 2H), 1.88 (d, *J* = 13.6 Hz, 2H), 1.70 - 1.58 (m, 2H), 1.40 (s, 9H).

LCMS (ESI) [M-Boc+H]⁺ = 298.18.

### Step 2: tert-Butyl 6-bromo-4-hydroxyspiro[chroman-2,4'-piperidine]-1'-carboxylate (Compound A6-3)

To Compound A6-2 (17.5 g, 44.2 mmol, 1.0 eq) was added methanol (200 mL). Sodium borohydride (2.5 g, 66.3 mmol, 1.5 eq) was added at 0°C, and the mixture was stirred at room temperature for 12 hours. The reaction mixture was concentrated under reduced pressure, then water was added. The mixture was extracted with ethyl acetate, washed with saturated brine, and the combined organic layers were dried over anhydrous sodium sulfate. It was concentrated under reduced pressure to obtain a crude Compound A6-3 (17.2 g).

¹H NMR (600 MHz, DMSO-d₆) δ 7.53 (dd, *J =* 2.6, 0.8 Hz, 1H), 7.32 - 7.27 (m, 1H), 6.76 (dd, *J =* 8.7, 0.7 Hz, 1H), 5.55 (d, *J* = 6.2 Hz, 1H), 4.75 - 4.64 (m, 1H), 3.73 - 3.60 (m, 2H), 3.06 (s, 2H), 2.11 (dd, *J* = 13.5, 6.1 Hz, 1H), 1.81 - 1.70 (m, 2H), 1.70 - 1.63 (m, 2H), 1.64 - 1.46 (m, 1H), 1.40 (s, 9H).

LCMS (ESI): [M-Boc+H]⁺ = 300.18.

### Step 3: 6-Bromospiro[chromium-2,4'-piperidine] (Compound A6-4)

Compound A6-3 (1.7 g, 4.2 mmol, 1.0 eq) was dissolved in trifluoroacetic acid (10 mL), and triethylsilane (981 mg, 8.4 mmol, 1.1 eq) was added. The mixture was stirred at 50°C for 1 hour. After cooling the reaction mixture to room temperature, it was concentrated under reduced pressure to obtain a crude Compound A6-4 (1.2 g).

LCMS (ESI) [M+H]⁺ = 282.28.

### Step 4: tert-Butyl 6-bromospiro[chroman-2,4'-piperidine]-1'-carboxylate (Compound A6-5)

Compound A6-4 (1.2 g, 4.2 mmol, 1.0 eq) was dissolved in dichloromethane (15 mL). Triethylamine (4.5 g, 44.5 mmol, 3.0 eq) and di-tert-butyl dicarbonate (1.4 g, 6.3 mmol, 1.5 eq) were added at 0°C. The mixture was stirred at room temperature for 12 hours. Water was added to the reaction mixture, which was then extracted with dichloromethane, washed with saturated brine, and the combined organic layers were dried over anhydrous sodium sulfate. The organic layer was concentrated, and the resulting crude product was purified by column chromatography to obtain a white solid Compound A6-5 (1.2 g, 75.1%).

¹H NMR (600 MHz, DMSO-d₆) δ 7.30 - 7.27 (m, 1H), 7.22 (dd, *J =* 8.7, 2.5 Hz, 1H), 6.75 (d, *J =* 8.7 Hz, 1H), 3.69 (d, *J* = 12.9 Hz, 2H), 2.74 (t, *J =* 6.8 Hz, 2H), 1.78 (t, *J =* 6.8 Hz, 2H), 1.66 (d, *J* = 13.3 Hz, 2H), 1.52 (ddd, *J* = 13.6, 11.3, 4.7 Hz, 2H), 1.41 (s, 9H), 0.90 (t, *J* = 7.9 Hz, 1H), 0.51 - 0.36 (m, 1H).

LCMS (ESI) [M-Boc+H]⁺ = 282.18.

### Step 5: tert-Butyl 6-(2,4-dioxotetrahydropyrimidin-1(2H)-yl)spiro[chroman-2,4'-piperidine]-1'-carboxylate (Compound A6-6)

To Compound A6-5 (750 mg, 2.0 mmol, 1.0 eq), dihydrouracil (672 mg, 5.9 mmol, 3 eq), (methanesulfonato{dicyclohexyl(3-isopropoxy-2',4',6'-triisopropyl-[1,1 '-biphenyl]-2-yl)phosphane}(2'-methylamino-1,1'-biphenyl-2-yl)palladium (90 mg, 0.98 mmol, 0.05 eq) and cesium carbonate (1.9 g, 5.9 mmol, 3.0 eq) was added super-dry 1,4-dioxane (10 mL). The mixture was stirred at 100°C under nitrogen protection for 12 hours. After cooling the reaction mixture to room temperature, it was concentrated under reduced pressure. The resulting crude product was purified by column chromatography to obtain a white solid Compound A6-6 (282 mg, 34.6%).

¹H NMR (600 MHz, DMSO-d₆) δ 10.29 (s, 1H), 7.04 (d, *J* = 2.5 Hz, 1H), 6.79 (d, *J* = 8.5 Hz, 1H), 5.76 (s, 1H), 3.69 (t, *J* = 6.7 Hz, 4H), 3.17 (s, 2H), 2.73 (t, *J* = 6.8 Hz, 2H), 2.67 (t, *J* = 6.6 Hz, 2H), 1.79 (t, *J* = 6.8 Hz, 2H), 1.68 (d, *J =* 13.5 Hz, 2H), 1.59 - 1.48 (m, 2H), 1.41 (s, 9H).

LCMS (ESI) [M-Boc+H]⁺ = 316.38.

### Step 6: 1-(spiro[chromate-2,4'-piperidin]-6-yl)dihydropyrimidine-2,4(1H,3H)-dione (Compound A6)

Compound A6-6 (1.2 g, 2.9 mmol, 1.0 eq) was dissolved in dichloromethane (10 mL), and a solution of hydrogen chloride in 1,4-dioxane (10 mL) was added. The mixture was stirred at room temperature for 2 hours. The reaction mixture was concentrated, and the resulting crude product was purified by reverse phase column chromatography to obtain a white solid Compound A6 (1.0 g, 70%).

¹H NMR (600 MHz, DMSO-d₆) δ 10.31 (s, 1H), 8.69 (s, 1H), 7.06 (d, *J* = 9.0 Hz, 2H), 6.84 (d, *J* = 8.4 Hz, 1H), 3.69 (t, *J* = 6.7 Hz, 2H), 3.22 (d, *J =* 12.8 Hz, 2H), 3.11 (s, 2H), 2.76 (t, *J =* 6.8 Hz, 2H), 2.68 (t, *J =* 6.6 Hz, 2H), 1.90 (d, *J* = 14.3 Hz, 2H), 1.84 (t, *J* = 6.8 Hz, 2H), 1.81 - 1.74 (m, 2H).

LCMS (ESI) [M+H]⁺ = 316.30.

### Example 7: Synthesis of Compound A7

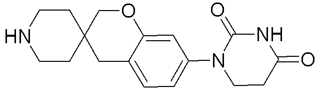

### 1-(spiro[benzopyran-3,4'-piperidin]-7-yl)dihydropyrimidine-2,4(1H,3H)-dione

### Synthetic Scheme

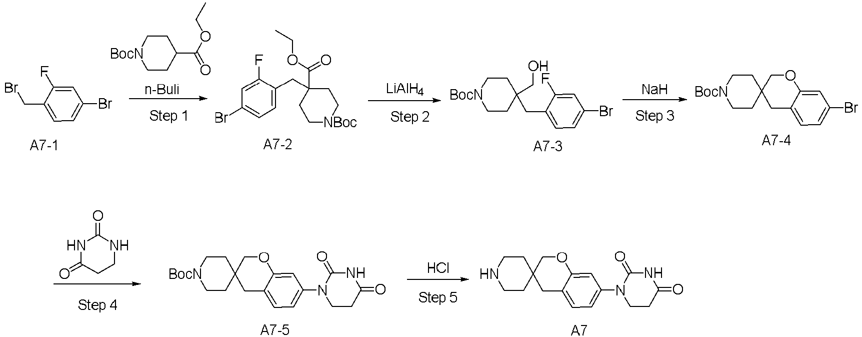

### Step 1: 1-tert-Butyl 4-ethyl 4-(4-bromo-2-fluorobenzyl)piperidine-1,4-dicarboxylate (Compound A7-2)

Diisopropylamine (2.91 g, 28.74 mmol, 1.1 eq) was dissolved in super-dry tetrahydrofuran (50 mL). n-Butyllithium (1.84 g, 11.0 mmol, 1.1 eq) was added to the reaction mixture, and the mixture was stirred at -30°C under nitrogen protection for 30 minutes. The temperature was lowered to -70°C, and a solution of 1-tert-butyl 4-ethyl piperidine-1,4-dicarboxylate (6.72 g, 26.13 mmol, 1.0 eq) in tetrahydrofuran was added dropwise. After the addition was complete, the mixture was stirred at -70°C for 1 hour. A solution of A7-1 (7.0 g, 26.13 mmol, 1.0 eq) in tetrahydrofuran was added to the reaction mixture. After the addition was complete, the mixture was stirred at - 70°C for 2 hours. Saturated aqueous ammonium chloride solution was added to the reaction mixture, and the mixture was extracted with ethyl acetate. The organic layers were washed with saturated brine, combined and dried over anhydrous sodium sulfate. The organic layer was concentrated, and the resulting crude product was purified by column chromatography to obtain a yellow oily Compound A7-2 (8.5 g, 75.9%).

¹H NMR (600 MHz, DMSO-d₆) δ 7.50 - 7.45 (m, 1H), 7.36 - 7.31 (m, 1H), 7.17 - 7.11 (m, 1H), 4.10 - 4.00 (m, 3H), 3.83 - 3.79 (m, 2H), 2.81 (s, 2H), 2.71 - 2.68 (m, 1H), 2.65 - 2.62 (m, 1H), 1.93 - 1.87 (m, 2H), 1.38 (s, 9H), 1.19 - 1.15 (m, 2H), 1.14 - 1.11 (m, 2H).

LCMS (ESI): [M-Boc+H]⁺ = 344.29.

### Step 2: tert-Butyl 4-(4-bromo-2-fluorobenzyl)-4-(hydroxymethyl)piperidine-1-carboxylate (Compound A7-3)

Compound A7-2 (8.5 g, 19.13 mmol, 1.0 eq) was dissolved in tetrahydrofuran (200 mL). The temperature was cooled to 0°C, and lithium aluminum hydride (798 mg, 11.04 mmol, 1.1 eq) was added portionwise. The mixture was allowed to warm to room temperature and stirred for 8 hours. The reaction was quenched by adding water, extracted with ethyl acetate, washed with saturated brine, and the combined organic layers were dried over anhydrous sodium sulfate. The organic layer was concentrated, and the resulting crude product was purified by column chromatography to obtain a white solid Compound A7-3 (4.26 g, 55.35%).

¹H NMR (600 MHz, DMSO-d₆) δ 7.49 - 7.44 (m, 1H), 7.37 - 7.31 (m, 1H), 7.29 - 7.24 (m, 1H), 4.75 - 4.71 (m, 1H), 3.45 - 3.40 (m, 1H), 3.42 - 3.38 (m, 1H), 3.27 - 3.17 (m, 4H), 2.67 - 2.60 (m, 2H), 1.38 - 1.32 (m, 11H), 1.21 - 1.13 (m, 2H).

LCMS (ESI): [M-Boc+H]⁺ = 302.18.

### Step 3: tert-Butyl 7-bromospiro[benzopyran-3,4'-piperidine]-1'-carboxylate (Compound A7-4)

Compound A7-3 (4.25 g, 10.59 mmol, 1.0 eq) was dissolved in N,N-dimethylformamide (20 mL). Sodium hydride (508 mg, 12.71 mmol, 1.2 eq) was added, and the mixture was stirred at 100°C for 8 hours. After cooling the reaction mixture to room temperature, water was added to quench the reaction, and the mixture was extracted with ethyl acetate. The organic layers were washed with saturated brine, combined and dried over anhydrous sodium sulfate. The organic layer was concentrated, and the resulting crude product was purified by column chromatography to obtain a yellow solid Compound A7-4 (2.2 g, 54.3%).

¹H NMR (400 MHz, DMSO-d₆) δ 7.01 - 6.95 (m, 3H), 3.91 (s, 2H), 3.50 - 3.39 (m, 2H), 3.30 - 3.21 (m, 2H), 2.64 (s, 2H), 1.40 (s, 9H), 1.38 - 1.32 (m, 2H), 1.35 - 1.29 (m, 1H), 1.32 - 1.14 (m, 1H).

LCMS (ESI): [M-Boc+H]⁺ = 282.38.

### Step 4: tert-Butyl 7-(2,4-dioxotetrahydropyrimidin-1(2H)-yl)spiro[chromodane-3,4'-piperidine]-1'-carboxylate (Compound A7-5)

Compound A7-4 (1.0 g, 2.62 mmol, 1.0 eq) was dissolved in 1,4-dioxane (10 mL). Dihydrouracil (895 mg, 7.85 mmol, 3.0 eq), cesium carbonate (3.43 g, 10.52 mmol, 3.0 eq), and (methanesulfonato{dicyclohexyl(3-isopropoxy-2',4',6'-triisopropyl-[1,1'-biphenyl]-2-yl)phosphane}(2'-methylamino-1,1'-biphenyl-2-yl)palladium (120 mg, 0.13 mmol, 0.05 eq) were added to the reaction mixture. The mixture was stirred at 100°C under nitrogen protection for 16 hours. The reaction solution was concentrated under reduced pressure, then water was added. The mixture was extracted with ethyl acetate, washed with saturated brine, and the combined organic layers were dried over anhydrous sodium sulfate. The organic layer was concentrated, and the resulting crude product was purified by column chromatography to obtain a yellow solid Compound A7-5 (160 mg, 14.7%).

¹H NMR (600 MHz, DMSO-d₆) δ 10.33 (s, 1H), 7.07 - 7.03 (m, 1H), 6.82 - 6.78 (m, 1H), 6.75 - 6.72 (m, 1H), 3.90 (s, 2H), 3.73 (t, *J* = 6.7 Hz, 2H), 3.51 - 3.43 (m, 2H), 3.29 - 3.26 (m, 2H), 2.70 - 2.65 (m, 4H), 1.40 (s, 9H), 1.40 - 1.29 (m, 3H), 1.29 - 1.22 (m, 1H).

LCMS (ESI) [M-Boc+H]⁺ = 316.38.

### Step 5: 1-(spiro[benzopyran-3,4'-piperidin]-7-yl)dihydropyrimidine-2,4(1H,3H)-dione (Compound A7)

Compound A7-5 (90 mg, 0.22 mmol, 1.0 eq) was dissolved in a solution of hydrogen chloride in 1,4-dioxane (3 mL). The reaction solution was stirred at room temperature overnight. The reaction mixture was filtered, and the filter cake was washed three times with acetonitrile (10 mL each) to obtain a white solid Compound A7 (30 mg, 43.9%).

¹H NMR (600 MHz, DMSO-d₆) δ 10.34 (s, 1H), 7.09 - 7.05 (m, 1H), 6.86 - 6.81 (m, 1H), 6.78 - 6.75 (m, 1H), 3.96 (s, 2H), 3.73 (t, *J* = 6.6 Hz, 2H), 3.15 - 3.12 (m, 2H), 3.09 (s, 2H), 2.71 (s, 2H), 2.69 - 2.64 (m, 2H), 1.67 - 1.54 (m, 4H).

LCMS (ESI) [M+H]⁺ = 316.35.

### Example 8: Synthesis of Compound A8

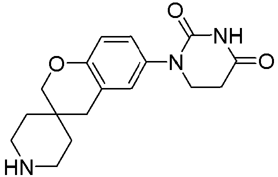

### 1-(spiro[benzopyran-3,4'-piperidin]-6-yl)dihydropyrimidine-2,4(1H,3H)-dione

### Synthetic Scheme

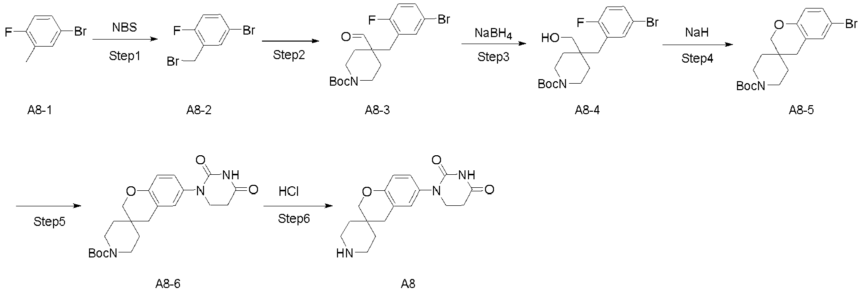

### Step 1: 4-Bromo-2-(bromomethyl)-1-fluorobenzene (Compound A8-2)

Compound A8-1 (10.0 g, 52.9 mmol, 1.0 eq) was dissolved in carbon tetrachloride (100 mL). N-Bromosuccinimide (11.30 g, 63.5 mmol, 1.2 eq) and azobisisobutyronitrile (868.72 mg, 5.3 mmol, 0.1 eq) were added to the reaction mixture. The reaction solution was heated to 80°C under nitrogen protection and stirred at this temperature for 12 hours. The resulting reaction mixture was filtered, and the filtrate was concentrated under reduced pressure. The crude product obtained by concentration was purified by column chromatography to obtain a colorless oily Compound A8-2 (13.89 g, 98.00%).

¹H NMR (400 MHz, DMSO-d₆) δ 7.79 (dd, *J =* 6.7, 2.6 Hz, 1H), 7.58 (ddd, *J =* 8.7, 4.6, 2.6 Hz, 1H), 7.23 (dd, *J* = 9.7, 8.8 Hz, 1H), 4.66 (d, *J* = 1.0 Hz, 2H).

### Step 2: tert-Butyl 4-(5-bromo-2-fluorobenzyl)-4-formylpiperidine-1-carboxylate (Compound A8-3)

Compound 1-tert-butoxycarbonylpiperidine-4-carbaldehyde (12.42 g, 58.23 mmol, 1.3 eq) was dissolved in tetrahydrofuran (120 mL). Potassium tert-butoxide (7.54 g, 67.18 mmol, 1.5 eq) was added at -30°C under nitrogen protection, and the mixture was stirred at -30°C for 30 minutes. Then, Compound A8-2 (12 g, 44.79 mmol, 1.0 eq) dissolved in tetrahydrofuran (120 mL) was added dropwise to the solution. The solution was stirred at -30°C for 4 hours. The resulting reaction mixture was concentrated under reduced pressure, and the crude product obtained by concentration was purified by column chromatography to obtain a yellow oily Compound A8-3 (8.97 g, 50.03%).

¹H NMR (600 MHz, DMSO-d₆) δ 8.35 (d, *J =* 2.7 Hz, 1H), 8.15 (dd, *J* = 9.0, 2.7 Hz, 1H), 7.36 - 7.22 (m, 1H), 4.65 (t, *J* = 5.2 Hz, 1H), 3.97 - 3.73 (m, 3H), 3.43 (d, *J =* 14.2, 14.8 Hz, 2H), 2.98 (dd, *J =* 12.4, 3.3 Hz, 1H), 2.08 (s, 4H), 1.42 (s, 9H).

LCMS (ESI): [M-Boc+H]⁺ = 301.17.

### Step 3: tert-Butyl 4-(5-bromo-2-fluorobenzyl)-4-(hydroxymethyl)piperidine-1-carboxylate (Compound A8-4)

Compound A8-3 (8.95 g, 22.36 mmol, 1.0 eq) was dissolved in methanol (100 mL). Sodium borohydride (1.26 g, 44.72 mmol, 2.0 eq) was slowly added at 0°C, and the mixture was stirred at room temperature for 12 hours after the addition. The resulting reaction mixture was concentrated under reduced pressure, then water was added. The mixture was extracted with ethyl acetate, washed with saturated brine, and the combined organic layers were dried over anhydrous sodium sulfate. The organic layer was concentrated to obtain a crude white oily Compound A8-4 (8.9 g, 98.94%). This crude product was used directly in the next step without further purification.

LCMS (ESI): [M-Boc+H]⁺ = 304.28.

### Step 4: tert-Butyl 6-bromospiro[chromodane-3,4'-piperidine]-1'-carboxylate (Compound A8-5)

Compound A8-4 (8.9 g, 22.37 mmol, 1 eq) was dissolved in N,N-dimethylformamide (100 mL). Sodium hydride (1.16 g, 29.08 mmol, 1.30 eq) was added, and the reaction mixture was heated to 110°C under nitrogen protection and stirred for 2 hours. The reaction mixture was cooled to room temperature, then saturated aqueous ammonium chloride solution was added. The mixture was extracted with ethyl acetate, washed with saturated brine, and the combined organic layers were dried over anhydrous sodium sulfate. The organic layer was concentrated, and the resulting crude product was purified by column chromatography to obtain a yellow oily Compound A8-5 (5.80 g, 67.82%).

¹H NMR (600 MHz, DMSO-d₆) δ 7.27 - 7.17 (m, 2H), 6.73 (d, *J =* 8.5 Hz, 1H), 3.90 (s, 2H), 3.46 (ddd, *J* = 13.8, 6.7, 4.0 Hz, 2H), 3.27 (s, 2H), 2.68 (s, 2H), 1.40 (s, 9H), 1.35 (dd, *J =* 8.6, 4.2 Hz, 2H), 1.30 (dddd, *J =* 13.6, 6.8, 4.0 Hz, 2H).

LCMS (ESI): [M-Boc+H]⁺ = 282.28.

### Step 5: tert-Butyl 6-(2,4-dioxotetrahydropyrimidin-1(2H)-yl)spiro[chromodane-3,4'-piperidine]-1'-carboxylate (CompoundA8-6)

Compound A8-5 (2.0 g, 5.23 mmol, 1.0 eq) was dissolved in 1,4-dioxane (20 mL). Dihydropyrimidine-2,4(1H,3H)-dione (1.79 g, 15.69 mmol, 3.0 eq), (methanesulfonato{dicyclohexyl(3-isopropoxy-2',4',6'-triisopropyl-[1,1'-biphenyl]-2-yl)phosphane}(2'-methylamino-1,1'-biphenyl-2-yl)palladium (240.28 mg, 0.26 mmol, 0.05 eq), and cesium carbonate (5.11 g, 15.69 mmol, 3.0 eq) were added. The mixture was stirred at 100°C under nitrogen protection for 12 hours. The reaction mixture was cooled to room temperature, concentrated under reduced pressure, and the resulting solid was dissolved in water. The mixture was extracted with ethyl acetate, washed with saturated brine, and the combined organic layers were dried over anhydrous sodium sulfate. The organic layer was concentrated, and the resulting crude product was purified by column chromatography to obtain Compound A8-6 as a white solid (463 mg, 21.3%).

¹H NMR (600 MHz, DMSO-d₆) δ 10.30 (s, 1H), 7.87 (dd, *J* = 6.6, 2.6 Hz, 1H), 7.68 (ddd, *J =* 8.9, 4.5, 2.6 Hz, 1H), 7.40 (s, 1H), 7.33 (dd, *J =* 10.4, 8.8 Hz, 1H), 7.02 (d, *J =* 8.4 Hz, 2H), 6.77 (d, *J =* 8.3 Hz, 1H), 3.90 (s, 2H), 3.70 (t, *J* = 6.7 Hz, 2H), 3.50 - 3.41 (m, 2H), 2.67 (d, *J =* 6.1 Hz, 4H), 1.40 (s, 9H), 1.36 - 1.32 (m, 2H).

LCMS (ESI): [M-Boc+H]⁺ = 316.38.

### Step 6: 1-(spiro[benzopyran-3,4'-piperidin]-6-yl)dihydropyrimidine-2,4(1H,3H)-dione (Compound A8)

Compound A8-6 (460 mg, 1.11 mmol, 1.0 eq) was dissolved in dichloromethane (2.5 mL). A solution of hydrogen chloride in dioxane (2.5 mL) was added, and the mixture was stirred at room temperature for 12 hours. The reaction solution was filtered, and the filter cake was triturated with n-hexane. It was filterred to obtain a white solid Compound A8 (349.16 mg, 28.64%).

¹H NMR (400 MHz, DMSO-d₆) δ 10.31 (s, 1H), 7.04 (d, *J =* 7.5 Hz, 2H), 6.79 (dd, *J =* 8.7, 1.2 Hz, 1H), 3.95 (s, 2H), 3.70 (t, *J =* 6.7 Hz, 2H), 3.10 (dd, *J =* 11.2, 9.7 Hz, 4H), 2.69 (ddd, *J =* 13.6, 6.9 Hz, 4H), 1.75 - 1.49 (m, 4H).

LCMS (ESI): [M+H]⁺ = 316.48.

### Example 9: Synthesis of Compound A9

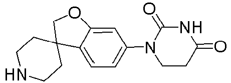

### 1-(2H-spiro[benzofuran-3,4'-piperidin]-6-yl)dihydropyrimidine-2,4(1H,3H)-dione

### Synthetic Scheme

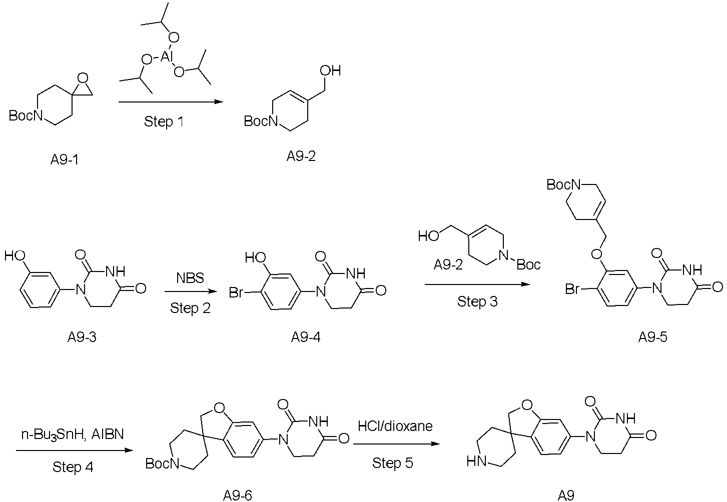

### Step 1: tert-Butyl 4-(hydroxymethyl)-3,6-dihydropyridine-1(2H)-carboxylate (Compound A9-2)

Compound A9-1 (10.0 g, 46.9 mmol) was dissolved in toluene (100 mL). Aluminum isopropoxide (12.5 g, 61.0 mmol) was added, and the mixture was stirred at 120°C for 24 hours. The reaction mixture was cooled to room temperature. Diluted hydrochloric acid (1.0 mol/L, 200 mL) was added, and the mixture was extracted three times with ethyl acetate. The organic layer was concentrated, and the resulting crude product was purified by column chromatography to obtain a colorless oily Compound A9-2 (4.7 g, 47%).

¹H NMR (600 MHz, CDCl₃) δ 5.67 (s, 1H), 4.07 (s, 2H), 3.98 - 3.92 (m, 2H), 3.54 (dd, *J =* 8.2, 4.0 Hz, 2H), 2.15 (d, *J* = 6.8 Hz, 2H), 1.49 (s, 9H).

LC-MS (ESI): [M-t-Bu+H]⁺ = 158.20.

### Step 2: 1-(4-Bromo-3-hydroxyphenyl)dihydropyrimidine-2,4(1H,3H)-dione (Compound A9-4)

Compound A9-3 (3.0 g, 14.55 mmol, 1.0 eq) was dissolved in acetonitrile (35 mL). N-Bromosuccinimide (3.11 g, 17.46 mmol, 1.2 eq) was added to the reaction mixture. The reaction solution was stirred at room temperature for 16 hours. The resulting reaction mixture was concentrated under reduced pressure, and the crude product obtained by concentration was purified by reverse phase column chromatography to obtain a white solid Compound A9-2 (3.4 g, 81.97%).

¹H NMR (400 MHz, DMSO-d₆) δ 10.44 (s, 1H), 10.40 (s, 1H), 7.47 (d, *J* = 8.5 Hz, 1H), 6.95 (d, *J =* 2.4 Hz, 1H), 6.75 - 6.72 (m, 1H), 3.74 (td, *J* = 6.7, 2.8 Hz, 2H), 2.72 - 2.67 (m, 2H).

LCMS (ESI): [M+H]⁺ = 285.10.

### Step 3: tert-Butyl 4-((2-bromo-5-(2,4-dioxotetrahydropyrimidin-1(2H)-yl)phenoxy)methyl)-3,6-dihydropyridine-1(2H)-carboxylate (Compound A9-5)

Compound A9-4 (3.4 g, 11.93 mmol, 1.0 eq), Compound A9-2 (3.82 g, 17.89 mmol, 1.5 eq) and triphenylphosphine (8.7 g, 23.85 mmol, 2 eq) were dissolved in tetrahydrofuran (50 mL). The mixture was cooled to 0°C, and diethyl azodicarboxylate (5.78 g, 23.85 mmol, 2.0 eq) was added. After the addition was complete, the mixture was allowed to warm to room temperature and stirred for 12 hours. The reaction solution was concentrated under reduced pressure, then water was added. The mixture was extracted with ethyl acetate, washed with saturated brine, and the combined organic layers were dried over anhydrous sodium sulfate. The organic layer was concentrated, and the resulting crude product was purified by reverse phase column chromatography to obtain a white solid Compound A9-5 (2.9 g, 50.62%).

LCMS (ESI): [M+H]⁺ = 481.99.

### Step 3: tert-Butyl 6-(2,4-dioxotetrahydropyrimidin-1(2H)-yl)-2H-spiro[benzofuran-3,4'-piperidine]-1'-carboxylate (Compound A9-6)

Compound A9-5 (2.9 g, 6.04 mmol, 1.0 eq), tributyltin hydride (7 g, 18.12 mmol, 3.0 eq) and azobisisobutyronitrile (2.2 g, 12.08 mmol, 2 eq) were dissolved in toluene (50 mL). The mixture was stirred at 110°C under nitrogen protection for 16 hours. After cooling the reaction mixture to room temperature, it was concentrated under reduced pressure, then water was added. The mixture was extracted with ethyl acetate, washed with saturated brine, and the combined organic layers were dried over anhydrous sodium sulfate. The organic layer was concentrated, and the resulting crude product was purified by column chromatography to obtain a yellow solid Compound A9-6 (400 mg, 16.50%).

LCMS (ESI): [M-t-Bu+H]⁺ = 346.30.

### Step 4: 1-(2H-spiro[benzofuran-3,4'-piperidin]-6-yl)dihydropyrimidine-2,4(1H,3H)-dione (Compound A9)

Compound A9-6 (400 mg, 1.0 mmol, 1.0 eq) was dissolved in dichloromethane (5 mL). A solution of hydrogen chloride in dioxane (5 mL) was added dropwise to the reaction solution. The mixture was stirred at room temperature for 1 hour. The organic layer was concentrated to obtain a crude Compound A9 (203 mg, 67.61%).

¹H NMR (600 MHz, DMSO-d₆) δ 10.38 (s, 1H), 7.16 (d, *J =* 8.0 Hz, 1H), 6.90 - 6.86 (m, 1H), 6.82 (d, *J* = 1.9 Hz, 1H), 4.52 (s, 2H), 3.74 (t, *J* = 6.7 Hz, 2H), 3.23 (s, 2H), 3.01 (q, *J* = 12.0 Hz, 2H), 2.69 (t*, J* = 6.6 Hz, 2H), 1.98 (td, *J* = 13.7, 4.3 Hz, 2H), 1.85 (d, *J* = 14.0 Hz, 2H), 1.60 - 0.68 (m, 1H).

LCMS (ESI): [M+H]⁺ = 302.30.

### Example 10: Synthesis of Compound A10

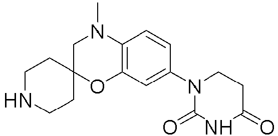

### 1-(4-methyl-3,4-dihydrospiro[benzo[b][1,4]oxazine-2,4'-piperidin]-7-yl)dihydropyrimidine-2,4(1H,3H)-dione

### Synthetic Scheme

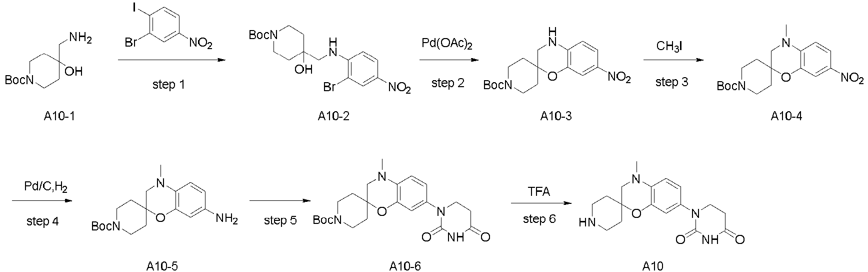

### Step 1: tert-Butyl 4-(((2-bromo-4-nitrophenyl)amino)methyl)-4-hydroxypiperidine-1-carboxylate (Compound A 10-2)

Compound A10-1 (5.0 g, 21.71 mmol, 1.0 eq), 2-bromo-1-iodo-4-nitrobenzene (5.0 g, 15.20 mmol, 0.7 eq), ethylene glycol (2.7 g, 43.42 mmol, 2.0 eq), copper(I) iodide (1.03 g, 5.43 mmol, 0.25 eq), and potassium phosphate (9.2 g, 43.42 mmol, 2 eq) were dissolved in isopropanol (50 mL). The mixture was stirred at 100°C under nitrogen protection for 12 hours. The reaction solution was filtered and concentrated, and the resulting crude product was purified by column chromatography to obtain a yellow oily Compound A10-2 (3.2 g, 34.2%).

¹H NMR (600 MHz, DMSO-d₆) δ 7.96 - 7.90 (m, 2H), 7.02 (t, *J =* 8.9 Hz, 1H), 6.77 (q, *J =* 5.8 Hz, 1H), 4.75 (s, 1H), 3.68 (s, 2H), 3.25 (d, *J* = 6.3 Hz, 2H), 3.04 (d, *J* = 41.0 Hz, 2H), 1.50 (d, *J* = 13.3 Hz, 2H), 1.43 (td, *J* = 13.0, 12.5, 4.7 Hz, 2H), 1.39 (s, 9H).

LCMS (ESI): [M-Boc+H]⁺ = 332.19.

### Step 2: tert-Butyl 7-nitro-3,4-dihydrospiro[benzo[b][1,4]oxazine-2,4'-piperidine]-1'-carboxylate (Compound A10-3)

Compound A10-2 (3.0 g, 7.0 mmol, 1.0 eq), 2-(di-tert-butylphosphino)-1,1'-binaphthyl (833 mg, 2.1 mmol, 0.3 eq), palladium(II) acetate (313 mg, 1.4 mmol, 0.2 eq), and cesium carbonate (2.5 g, 13.9 mmol, 2 eq) were dissolved in toluene. The mixture was stirred at 100°C under nitrogen protection for 12 hours. The reaction solution was concentrated, and the resulting crude product was purified by column chromatography to obtain a yellow solid Compound A10-3 (1.2 g, 49.3%).

¹H NMR (400 MHz, Chloroform-d) δ 7.77 (d, *J =* 7.3 Hz, 2H), 6.59 - 6.54 (m, 1H), 4.04 - 3.84 (m, 2H), 3.31 - 3.19 (m, 4H), 1.79 (d, *J =* 13.7 Hz, 2H), 1.66 - 1.54 (m, 2H), 1.49 (s, 9H).

LCMS (ESI): [M-Boc+H]⁺ = 250.37.

### Step 3: tert-Butyl 4-methyl-7-nitro-3,4-dihydrospiro[benzo[b][1,4]oxazine-2,4'-piperidine]-1'-carboxylate (Compound A10-4)

Compound A10-3 (1.0 g, 2.9 mmol, 1.0 eq) was dissolved in N,N-dimethylformamide. Sodium hydride (229 mg, 5.7 mmol, 2.0 eq, 60%) and iodomethane (609 mg, 4.3 mmol, 1.5 eq) were added at 0°C, and the mixture was stirred at 0°C for 30 minutes. The reaction solution was poured into ice water, and the precipitated solid was filtered off to obtain a crude Compound A10-4 (1.0 g).

LCMS (ESI): [M+H]⁺ = 364.35.

### Step 4: tert-Butyl 7-amino-4-methyl-3,4-dihydrospiro[benzo[b][1,4]oxazine-2,4'-piperidine]-1'-carboxylate (Compound A10-5)

Compound A10-4 (1.0 g, 2.75 mmol, 1.0 eq) was dissolved in methanol (15 mL). Palladium on carbon (100 mg, 10%) was added. After three cycles of hydrogen replacement, the mixture was stirred at room temperature for 12 hours. The mixture was filtered, and the organic layer was concentrated to obtain a purple solid Compound A10-5 (1.0 g). This crude product was used directly in the next step without further purification.

LCMS (ESI): [M+H]⁺ = 334.39.

### Step 5: tert-Butyl 7-(2,4-dioxotetrahydropyrimidin-1(2H)-yl)-4-methyl-3,4-dihydrospiro[benzo[b][1,4]oxazine-2,4'-piperidine]-1'-carboxylate (Compound A10-6)

Compound A10-5 (1.0 g, 3.0 mmol, 1.0 eq) was dissolved in toluene (10 mL). Acrylic acid (324 mg, 4.5 mmol, 1.5 eq) was added at room temperature, and the mixture was stirred at 90°C for 3 hours. After cooling to room temperature, acetic acid (5 mL) was added, followed by urea (900 mg, 15.0 mmol, 5.0 eq). The mixture was stirred at 100°C for 12 hours. The reaction solution was concentrated, and the resulting crude product was purified by column chromatography to obtain a yellow oily Compound A10-6 (300 mg, 23.2%).

¹H NMR (600 MHz, DMSO-d₆) δ 10.25 (s, 1H), 6.74 (d, *J =* 8.3 Hz, 2H), 6.70 (d, *J* = 8.2 Hz, 1H), 3.73 - 3.65 (m, 4H), 3.26 - 3.10 (m, 2H), 3.04 (s, 2H), 2.85 (s, 3H), 2.66 (t, *J =* 6.7 Hz, 2H), 1.68 - 1.63 (m, 2H), 1.61 - 1.54 (m, 2H), 1.41 (s, 9H).

LCMS (ESI) [M-t-Bu+H]⁺ = 375.39.

### Step 6: 1-(4-methyl-3,4-dihydrospiro[benzo[b][1,4]oxazine-2,4'-piperidin]-7-yl)dihydropyrimidine-2,4(1H,3H)-dione (Compound A10)

Compound A10-6 (200 mg, 464 µmol, 1.0 eq) was dissolved in dichloromethane (5 mL). Trifluoroacetic acid (1 mL) was added, and the mixture was stirred at room temperature for 1 hour. The reaction mixture was concentrated under reduced pressure and purified by preparative HPLC to obtain a white solid Compound A10 (100 mg, 65.15%).

¹H NMR (600 MHz, DMSO-d₆) δ 10.27 (s, 1H), 6.80 - 6.76 (m, 2H), 6.73 (d, *J =* 8.4 Hz, 1H), 3.68 (t, *J =* 6.7 Hz, 2H), 3.22 (dt, *J* = 13.2, 3.6 Hz, 2H), 3.12 - 3.05 (m, 4H), 2.88 (s, 3H), 2.66 (t,*J* = 6.7 Hz, 2H), 1.86 (dd, *J* = 7.8, 4.3 Hz, 4H).

LCMS (ESI) [M+H]⁺ = 331.25.

### Example 11: Synthesis of Compound A11

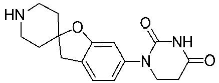

### 1-(3H-spiro[benzofuran-2,4'-piperidin]-6-yl)dihydropyrimidine-2,4(1H,3H)-dione

### Synthetic Scheme:

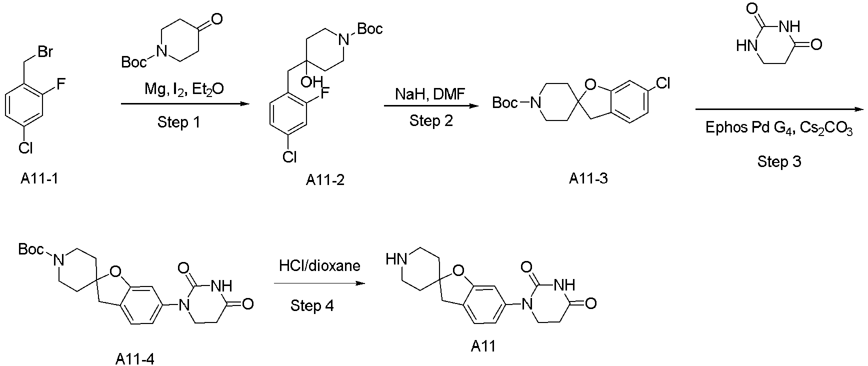

### Step 1: tert-Butyl 4-(4-chloro-2-fluorobenzyl)-4-hydroxypiperidine-1-carboxylate (Compound A11-2)

A11-1 (2.0 g, 8.95 mmol, 1.0 eq), magnesium (330 mg, 13.42 mmol, 1.5 eq) and iodine (45 mg, 0.18 mmol, 0.02 eq) were dissolved in diethyl ether (30 mL). The mixture was stirred at 35°C under nitrogen protection for 1 hour to obtain the Grignard reagent. tert-Butyl 4-oxopiperidine-1-carboxylate (1.8 g, 8.95 mmol, 1.0 eq) was dissolved in diethyl ether (10 mL) under nitrogen protection, and the temperature was lowered to -78°C. The obtained Grignard reagent was added to the reaction solution, and the mixture was stirred at room temperature overnight. The reaction was quenched with saturated ammonium chloride, and the reaction mixture was washed with water, extracted with ethyl acetate, washed with saturated brine, and concentrated under reduced pressure. The crude product was purified by column chromatography to obtain a yellow oily Compound A11-2 (1.8 g, 58.5%).

¹H NMR (600 MHz, Chloroform-d) δ 7.19 (t, *J* = 8.1 Hz, 1H), 7.14 - 7.09 (m, 2H), 3.98 - 3.78 (m, 2H), 3.11 (s, 2H), 2.80 (s, 2H), 1.65-1.61 (m, 2H), 1.51 (s, 2H), 1.47 (s, 9H).

LCMS (ESI): [M-t-Bu-OH+H]⁺ = 270.37.

### Step 2: tert-Butyl 6-chloro-3H-spiro[benzofuran-2,4'-piperidine]-1'-carboxylate (Compound A11-3)

Compound A11-2 (1.8 g, 5.24 mmol, 1.0 eq) was dissolved in N,N-dimethylformamide (10 mL). Sodium hydride (255 mg, 10.47 mmol, 2.0 eq) was added to the reaction mixture under nitrogen protection, and the mixture was stirred at 110°C for 2 hours. The reaction was quenched with saturated ammonium chloride, and the reaction mixture was washed with water, extracted with ethyl acetate, washed with saturated brine, and concentrated under reduced pressure. The crude product was purified by column chromatography to obtain a colorless liquid Compound A11-3 (600 mg, 35.4%).

¹H NMR (600 MHz, Chloroform-d) δ 7.06 (d, *J =* 7.9 Hz, 1H), 6.83 (dd, *J =* 7.9, 1.8 Hz, 1H), 6.78 (d, *J =* 1.8 Hz, 1H), 3.76 (s, 2H), 3.41 (t, *J* = 11.8 Hz, 2H), 2.96 (s, 2H), 1.91 (d, *J* = 13.3 Hz, 2H), 1.76 - 1.68 (m, 2H), 1.50 (d, *J =* 1.2 Hz, 9H).

LC-MS (ESI) [M-t-Bu+H]⁺ = 268.17.

### Step 3: tert-Butyl 6-(2,4-dioxotetrahydropyrimidin-1(2H)-yl)-3H-spiro[benzofuran-2,4'-piperidine]-1'-carboxylate (Compound A11-4)

Crude Compound A11-3 (600 mg, 1.85 mmol, 1.0 eq) was dissolved in 1,4-dioxane (15 mL). Dihydropyrimidine-2,4(1H,3H)-dione (260 mg, 2.22 mmol, 1.2 eq), (methanesulfonato{dicyclohexyl(3-isopropoxy-2',4',6'-triisopropyl-[1,1'-biphenyl]-2-yl)phosphane}(2'-methylamino-1,1'-biphenyl-2-yl)palladium (170 mg, 0.18 mmol, 0.1 eq) and cesium carbonate (1.8 g, 5.55 mmol, 3.0 eq) were added. The mixture was stirred at 100°C under nitrogen protection overnight. The reaction mixture was concentrated under reduced pressure and purified by column chromatography to obtain a yellow solid Compound A11-4 (190 mg, 25.5%).

¹H NMR (400 MHz, DMSO-d₆) δ 10.33 (s, 1H), 7.18 (d, *J =* 7.8 Hz, 1H), 6.80 - 6.70 (m, 2H), 3.72 (t, *J =* 6.7 Hz, 2H), 3.58 - 3.48 (m, 2H), 3.38 (s, 2H), 3.01 (s, 2H), 2.67 (t, *J* = 6.6 Hz, 2H), 1.79-1.67 (m, 4H), 1.42 (s, 9H).

LC-MS (ESI) [M-t-Bu+H]⁺ = 346.39.

### Step 4: 1-(3H-spiro[benzofuran-2,4'-piperidin]-6-yl)dihydropyrimidine-2,4(1H,3H)-dione (CompoundA11)

Compound A11-4 (1.3 g, 3.25 mmol, 1.0 eq) was dissolved in dichloromethane (5 mL). A solution of hydrogen chloride in dioxane (5 mL) was added, and the mixture was stirred at room temperature for 4 hours. The mixture was filtered, and the filter cake was washed with dichloromethane to obtain a white solid Compound A11 (1.12 g, 99.9%).

¹H NMR (600 MHz, DMSO-d₆) δ 10.35 (s, 1H), 7.21 (d, *J =* 7.9 Hz, 1H), 6.87 - 6.67 (m, 2H), 3.73 (t, *J =* 6.6 Hz, 2H), 3.26 - 3.13 (m, 4H), 3.10 (s, 2H), 2.68 (t, *J* = 6.6 Hz, 2H), 2.04-1.96 (m, 4H).

LC-MS (ESI) [M+H]⁺= 302.3.

### Example 12: Synthesis of Compound A12

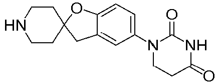

### 1-(3H-spiro[benzofuran-2,4'-piperidin]-5-yl)dihydropyrimidine-2,4(1H,3H)-dione

### Synthetic Scheme:

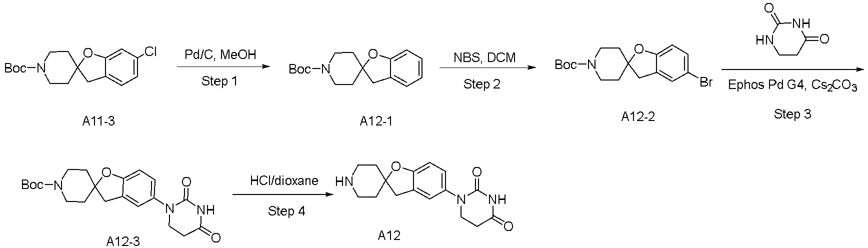

### Step 1: tert-Butyl 3H-spiro[benzofuran-2,4'-piperidine]-1'-carboxylate (Compound A12-1)

A11-3 (500 mg, 1.54 mmol, 1.0 eq) was dissolved in methanol (10 mL). Palladium on carbon (100 mg) was added to the reaction solution, and the mixture was purged with hydrogen and stirred at room temperature for 1 hour. The mixture was filtered through Celite, and concentrated under reduced pressure to obtain a yellow solid Compound A12-1 (450 mg, 100.7%).

¹H NMR (600 MHz, Chloroform-d) δ 7.17 - 7.12 (m, 2H), 6.86 (t, *J =* 6.0 Hz, 1H), 6.78 (d, *J* = 6.0 Hz, 1H), 3.75 (s, 2H), 3.47 - 3.43 (m, 2H), 3.02 (s, 2H), 1.96 - 1.87 (m, 2H), 1.74 - 1.70 (m, 2H), 1.50 (s, 9H).

LCMS (ESI): [M-Boc+H]⁺ = 190.34.

### Step 2: tert-Butyl 5-bromo-3H-spiro[benzofuran-2,4'-piperidine]-1'-carboxylate (Compound A12-2)

Compound A12-1 (450 mg, 1.56 mmol, 1.0 eq) was dissolved in dichloromethane (10 mL). N-Bromosuccinimide (340 mg, 1.72 mmol, 1.1 eq) was added to the reaction mixture, and the mixture was stirred at room temperature for 16 hours. The mixture was concentrated under reduced pressure, and the crude product was purified by column chromatography to obtain a white solid Compound A12-2 (420 mg, 73.3%).

¹H NMR (600 MHz, Chloroform-d) δ 7.27 - 7.26 (m, 1H), 7.24 - 7.20 (m, 1H), 6.66 (d, *J =* 8.4 Hz, 1H), 3.77 (s, 2H), 3.41 (t, *J* = 11.9 Hz, 2H), 3.00 (s, 2H), 1.91 (d, *J* = 13.3 Hz, 2H), 1.72 (t, *J* = 10.9 Hz, 2H), 1.50 (s, 9H).

LC-MS (ESI) [M-t-Bu+H]⁺ = 312.18.

### Step 3: tert-Butyl 5-(2,4-dioxotetrahydropyrimidin-1(2H)-yl)-3H-spiro[benzofuran-2,4'-piperidine]-1'-carboxylate (Compound A12-3)

Crude Compound A12-2 (420 mg, 1.14 mmol, 1.0 eq) was dissolved in 1,4-dioxane (15 mL). Dihydropyrimidine-2,4(1H,3H)-dione (260 mg, 2.28 mmol, 2.0 eq), (methanesulfonato{dicyclohexyl(3-isopropoxy-2',4',6'-triisopropyl-[1,1'-biphenyl]-2-yl)phosphane}(2'-methylamino-1,1'-biphenyl-2-yl)palladium (104 mg, 0.11 mmol, 0.1 eq) and cesium carbonate (1.11 g, 3.42 mmol, 3.0 eq) were added. The mixture was stirred at 100°C under nitrogen protection overnight. The reaction mixture was concentrated under reduced pressure, and the crude product was purified by column chromatography to obtain a yellow solid Compound A12-3 (125 mg, 27.3%).

LC-MS (ESI) [M-t-Bu+H]⁺ = 346.39.

### Step 4: 1-(3H-spiro[benzofuran-2,4'-piperidin]-5-yl)dihydropyrimidine-2,4(1H,3H)-dione (Compound A12)

Compound A12-3 (125 mg, 0.31 mmol, 1.0 eq) was dissolved in dichloromethane (5 mL). A solution of hydrogen chloride in dioxane (2 mL) was added, and the mixture was stirred at room temperature for 4 hours. The mixture was filtered, and the filter cake was washed with dichloromethane to obtain a white solid Compound A12 (100 mg, 99.6%).

¹H NMR (400 MHz, DMSO-d₆) δ 10.30 (s, 1H), 7.17 (d, *J =* 2.2 Hz, 1H), 7.05 (dd, *J =* 8.5, 2.3 Hz, 1H), 6.79 (d, *J =* 8.5 Hz, 1H), 3.68 (t, *J* = 6.6 Hz, 2H), 3.28 - 3.16 (m, 4H), 3.12 (s, 2H), 2.68 (t, *J =* 6.6 Hz, 2H), 2.04-1.90 (m, 4H).

LCMS (ESI): [M+H]⁺ = 302.3.

### Example 13: Synthesis of Compound A13

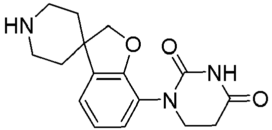

### 1-(2H-spiro[benzofuran-3,4'-piperidin]-7-yl)dihydropyrimidine-2,4(1H,3H)-dione

### Synthetic Scheme

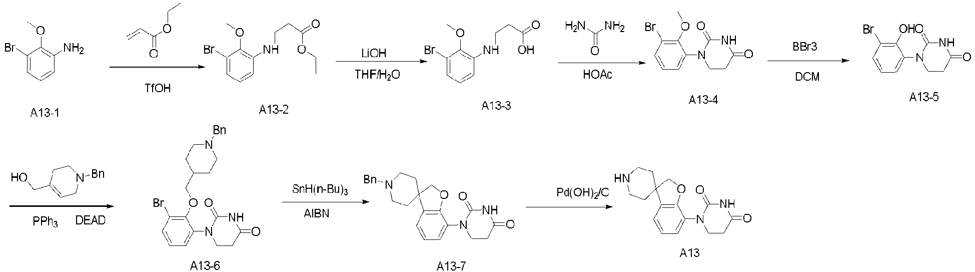

### Step 1: Ethyl 3-((3-bromo-2-methoxyphenyl)amino)propanoate (Compound A13-2)

Compound A13-1 (2.0 g, 9.90 mmol, 1.0 eq), ethyl acrylate (1.00 g, 9.99 mmol, 1.0 eq) and trifluoromethanesulfonic acid (150 mg, 999.53 µmol, 0.10 eq) were mixed. The reaction solution was heated to 100°C and stirred at this temperature for 12 hours. Crude A13-2 was obtained (3.15 g, 105%).

LCMS (ESI): [M+H]⁺ = 302.18.

### Step 2: 3-((3-Bromo-2-methoxyphenyl)amino)propanoic acid (Compound A13-3)

Compound A13-1 (3.15 g, 9.93 mmol, 1.0 eq) was dissolved in a mixed solvent of tetrahydrofuran and water (15/3 mL). Lithium hydroxide (2.38 g, 99.28 mmol, 10.0 eq) was added under ice-water bath conditions, and the mixture was allowed to warm to room temperature and stirred for 12 hours. Water (50 mL) was added to the mixture, and the pH of the reaction solution was adjusted to acidic with dilute hydrochloric acid. The mixture was extracted with ethyl acetate (100 mL × 3), and the combined organic layers were dried over anhydrous sodium sulfate. The organic layer was concentrated, and the resulting crude product was purified by normal phase silica gel column chromatography to obtain Compound A13-3 as a yellow solid (1.77 g, 65.04%).

¹H NMR (600 MHz, CDCl₃) δ 6.89 (m, 2H), 6.61 (m, 1H), 3.82 (s, 3H), 3.51 (t, *J* = 6.4 Hz, 2H), 2.71 (t, *J* = 6.4 Hz, 2H).

LCMS (ESI): [M+H]⁺ = 276.15.

### Step 3: 1-(3-Bromo-2-methoxyphenyl)dihydropyrimidine-2,4(1H,3H)-dione (Compound A13-4)

Compound A13-3 (1.40 g, 5.11 mmol, 1.0 eq) was dissolved in acetic acid (40 mL), then urea (1.84 g, 30.64 mmol, 6.0 eq) was added. The mixture was stirred at 120°C for 12 hours. After cooling the reaction mixture to room temperature, water (100 mL) was added, and the mixture was extracted with ethyl acetate (100 mL × 3). The combined organic layers were dried over anhydrous sodium sulfate. The organic layer was concentrated, and the resulting crude product was purified by column chromatography to obtain a white solid Compound A13-4 (1.10 g, 72.00%).

¹H NMR (600 MHz, DMSO) δ 10.48 (s, 1H), 7.62 (d, *J =* 8.0 Hz, 1H), 7.37 (d, *J* = 7.9 Hz, 1H), 7.14 (t, *J* = 8.0 Hz, 1H), 3.75 (s, 3H), 3.67 (t, *J* = 6.7 Hz, 2H), 2.72 (t, *J* = 6.7 Hz, 2H).

LCMS (ESI): [M+H]⁺ = 299.10.

### Step 4: 1-(3-Bromo-2-hydroxyphenyl)dihydropyrimidine-2,4(1H,3H)-dione (Compound A13-5)

Compound A13-4 (1.00 g, 3.34 mmol, 1.0 eq) was dissolved in dichloromethane (10 mL). The reaction mixture was cooled to 0°C, and a solution of boron tribromide (13 mL, 13.0 mmol, 4.0 eq) was slowly added dropwise. After the addition was complete, the mixture was stirred at room temperature for 1 hour. The reaction solution was concentrated under reduced pressure, dissolved in dichloromethane, and quenched with methanol under ice-water bath conditions. The mixture was then concentrated under reduced pressure, and the resulting crude product was purified by column chromatography to obtain a yellow solid Compound A12-5 (590 mg, 61.90%).

¹H NMR (400 MHz, DMSO) δ 10.40 (s, 1H), 9.75 (s, 1H), 7.48 (dd, *J =* 8.0, 1.9 Hz, 1H), 7.23 (dd, *J* = 7.9, 1.6 Hz, 1H), 6.80 (t, *J* = 7.9 Hz, 1H), 3.61 (s, 2H), 2.72 (s, 2H).

LCMS (ESI) [M+H]⁺ = 285.10.

### Step 5: 1-(2-((1-Benzyl-1,2,3,6-tetrahydropyridin-4-yl)methoxy)-3-bromophenyl)dihydropyrimidine-2,4(1H,3H)-dione (Compound A13-6)

CompoundA13-5 (380 mg, 1.33 mmol, 1.0 eq), triphenylphosphine (525 mg, 2.00 mmol, 1.5 eq) and (1-benzyl-1,2,3,6-tetrahydropyridin-4-yl)methanol (410 mg, 2.02 mmol, 1.5 eq) were dissolved in super-dry tetrahydrofuran (10 mL) under nitrogen protection. Diethyl azodicarboxylate (465 mg, 2.67 mmol, 2.0 eq) was added dropwise under ice-water bath conditions, and the mixture was stirred at room temperature for 2 hours. The reaction mixture was concentrated under reduced pressure, and the resulting crude product was purified by column chromatography to obtain crude A13-6 (580 mg, 92.51%).

LCMS (ESI) [M+H]⁺ = 470.25.

### Step 6: 1-(1'-Benzyl-2H-spiro[benzofuran-3,4'-piperidin]-7-yl)dihydropyrimidine-2,4(1H,3H)-dione (Compound A13-7)

Compound A13-6 (380 mg, 807.88 µmol, 1.0 eq) was dissolved in toluene (30 mL), then azobisisobutyronitrile (270 mg, 1.64 mmol, 2.0 eq) and tributyltin hydride (470 mg, 1.61 mmol, 2.0 eq) were added successively. The resulting reaction solution was stirred at 110°C for 4 hours. The reaction solution was concentrated under reduced pressure, and the resulting crude product was purified by reverse phase chromatography to obtain a white solid Compound A13-7 (210 mg, 66.40%).

¹H NMR (400 MHz, DMSO) δ 10.40 (s, 1H), 7.51 (m, 5H), 7.15 (d, *J* = 7.2 Hz, 1H), 7.04 (d, *J* = 7.3 Hz, 1H), 6.94 *(t, J* = 7.6 Hz, 1H), 4.56 (s, 2H), 4.35 (d, *J =* 4.0 Hz, 2H), 3.65 (t, *J =* 6.7 Hz, 2H), 3.37 (d, *J =* 12.3 Hz, 2H), 3.21 - 3.07 (m, 2H), 2.67 (t, *J =* 6.7 Hz, 2H), 2.16 - 2.03 (m, 2H), 1.93 (d, *J =* 14.0 Hz, 2H).

LCMS (ESI) [M+H]⁺ = 392.35.

### Step 7: 1-(2H-spiro[benzofuran-3,4'-piperidin]-7-yl)dihydropyrimidine-2,4(1H,3H)-dione (Compound A13)

Compound A12-7 (150 mg, 383.17 µmol, 1.0 eq) was dissolved in tetrahydrofuran (20 mL). Palladium(II) hydroxide (20%) on carbon (30 mg, 50.66 µmol, 0.13 eq) and palladium (10%) on carbon (30 mg, 19.68 mmol, 0.05 eq) were added. After three cycles of hydrogen replacement, the mixture was stirred at 30°C for 12 hours. The reaction mixture was filtered, and the filter cake was washed three times with dichloromethane (15 mL each). The filtrate was concentrated under reduced pressure, and the resulting crude product was purified by reverse phase column chromatography to obtain a white solid Compound A13 (43 mg, 37.24%).

¹H NMR (600 MHz, DMSO) δ 10.39 (s, 1H), 8.51 (s, 1H), 7.15 (d, *J* = 7.8 Hz, 1H), 7.11 (d*, J =* 7.3 Hz, 1H), 6.94 (t, *J =* 7.7 Hz, 1H), 4.54 (s, 2H), 3.66 (t, *J =* 6.6 Hz, 2H), 3.29 (d, *J =* 14.8 Hz, 2H), 2.98 (t, *J =* 12.1 Hz, 2H), 2.68 (t, *J* = 6.6 Hz, 2H), 1.99 (dt, *J =* 13.5, 6.9 Hz, 2H), 1.84 (d, *J =* 13.8 Hz, 2H).

LCMS (ESI) [M+H]⁺ = 302.38.

### Example 14: Synthesis of Compound A14

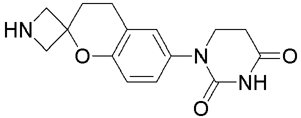

### 1-(spiro(azetidine-3,2'- benzodihydropyran)-6'-yl)dihydropyrimidine-2,4(1H,3H)-dione

### Synthetic Scheme

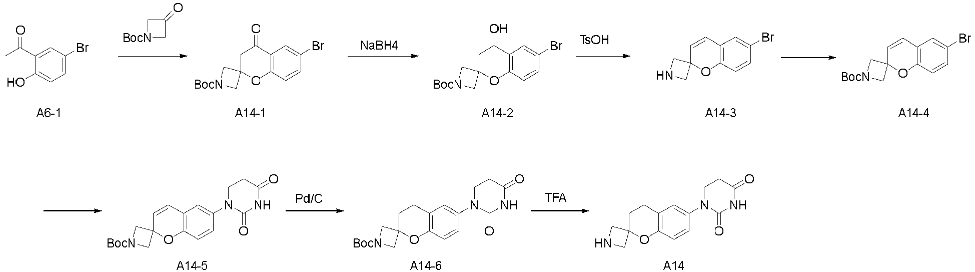

### Step 1: tert-Butyl 6'-bromo-4'-oxospiro(azetidine-3,2'-chroman)-1-carboxylate (Compound A14-1)

Compound A6-1 (10.0 g, 46.50 mmol, 1.0 eq) was dissolved in methanol (100 mL). tert-Butyl 3-oxoazetidine-1-carboxylate (8.76 g, 51.15 mmol, 1.1 eq) and pyrrolidine (11.39 g, 51.15 mmol, 1.1 eq) were added to the reaction mixture. The reaction solution was heated to 70°C and stirred at this temperature for 16 hours. The mixture was treated with water and extracted twice with ethyl acetate. The combined organic layers were washed with saturated sodium chloride solution, dried, and concentrated. The resulting crude product was purified by normal phase column chromatography to obtain a yellow solid Compound A14-1 (8 g, 46.42%).

LCMS (ESI): [M-t-Bu+H]⁺ = 314.15.

### Step 2: tert-Butyl 6'-bromo-4'-hydroxyspiro(azetidine-3,2'-chroman)-1-carboxylate (Compound A14-2)

Compound A14-1 (8 g, 10.86 mmol, 1.0 eq) was dissolved in methanol (100 mL). The temperature was cooled to 0°C, and sodium borohydride (1.64 g, 43.45 mmol, 2.0 eq) was added. After the addition was complete, the mixture was allowed to warm to room temperature and stirred for 1 hour. The reaction solution was quenched with saturated aqueous ammonium chloride solution and extracted twice with ethyl acetate. The combined organic layers were washed with saturated sodium chloride solution, dried, and concentrated to obtain a crude yellow solid Compound A14-2 as (8 g, 99.46%), which was used directly in the next step.

LCMS (ESI): [M-Boc+H]⁺ = 270.20.

### Step 3: 6'-Bromospiro(azetidine-3,2'-chromene) (Compound A14-3)

Compound A14-2 (8 g, 21.61 mmol, 1.0 eq) was dissolved in toluene (100 mL). p-Toluenesulfonic acid (7.44 g, 43.21 mmol, 2.0 eq) was added to the reaction mixture. The reaction mixture was heated to 110°C and stirred for 16 hours. After cooling the reaction mixture to room temperature, it was concentrated under reduced pressure to obtain crude Compound A 14-3, which was used directly in the next step without further treatment.

LCMS (ESI): [M+H]⁺ = 252.15.

### Step 4: tert-Butyl 6'-bromospiro(azetidine-3,2'-chromene)-1-carboxylate (Compound A14-4)

Compound A14-3 (5.4 g, 21.42 mmol, 1.0 eq) and triethylamine (10.84 g, 107.10 mmol, 5.0 eq) were dissolved in dichloromethane (60 mL). The reaction mixture was cooled to 0°C, and di-tert-butyl dicarbonate (14.02 g, 64.26 mmol, 3.0 eq) was added dropwise. After the addition was complete, the mixture was allowed to warm to room temperature and stirred for 1 hour. The reaction solution was concentrated, and the resulting crude product was purified by column chromatography to obtain a light yellow solid Compound A14-4 (4.5 g, 59.65%).

¹H NMR (400 MHz, DMSO-d₆) δ 7.39 - 7.28 (m, 2H), 6.85 (d, *J =* 8.6 Hz, 1H), 6.58 (d, *J =* 9.8 Hz, 1H), 6.22 (d, *J =* 9.8 Hz, 1H), 4.03 (s, 4H), 1.39 (s, 9H).

LCMS (ESI): [M-t-Bu+H]⁺ = 296.15.

### Step 5: tert-Butyl 6'-(2,4-dioxotetrahydropyrimidin-1(2H)-yl)spiro(azetidine-3,2'-chromene)-1-carboxylate (Compound A14-5)

Compound A14-4 (3 g, 8.52 mmol, 1.0 eq) was dissolved in 1,4-dioxane (50 mL). Dihydropyrimidine-2,4(1H,3H)-dione (2.92 g, 25.56 mmol, 3.0 eq), (methanesulfonato{dicyclohexyl(3-isopropoxy-2',4',6'-triisopropyl-[1,1'-biphenyl]-2-yl)phosphane}(2'-methylamino-1,1'-biphenyl-2-yl)palladium (391 mg, 0.43 mmol, 0.05 eq) and cesium carbonate (5.55 g, 17.03 mmol, 2.0 eq) were added successively. The reaction solution was heated to 100°C under nitrogen protection and stirred for 16 hours. The reaction solution was concentrated, and the resulting crude product was purified by column chromatography to obtain a white solid Compound A14-5 (1.5 g, 45.69%).

LCMS (ESI): [M-t-Bu+H]⁺ = 330.25.

### Step 6: tert-Butyl 6'-(2,4-dioxotetrahydropyrimidin-1(2H)-yl)spiro(azetidine-3,2'-chroman)-1-carboxylate (Compound A14-6)

Compound A14-5 (1.5 g, 3.89 mmol, 1.0 eq) was dissolved in methanol (10 mL) and tetrahydrofuran (50 mL). Palladium on carbon (750 mg) was added to the reaction mixture. After three cycles of hydrogen replacement, the mixture was stirred at room temperature for 4 days. The reaction mixture was filtered, and the filter cake was washed three times with methanol (20 mL) and dichloromethane (20 mL). The filtrate was concentrated under reduced pressure, and the resulting crude product was purified by column chromatography to obtain a white solid Compound A14-6 (1 g, 66.32%).

¹H NMR (600 MHz, DMSO-d₆) δ 10.31 (s, 1H), 7.08 - 7.03 (m, 2H), 6.86 - 6.81 (m, 1H), 3.93 (s, 2H), 3.83 (s, 2H), 3.70 (t, *J* = 6.7 Hz, 2H), 2.78 (t, *J* = 6.5 Hz, 2H), 2.68 (t, *J =* 6.7 Hz, 2H), 2.07 (t, *J* = 6.5 Hz, 2H), 1.40 (s, 9H).

LCMS (ESI): [M-t-Bu+H]⁺ = 332.30.

### Step 7: 1-(spiro(azetidine-3,2'-chroman)-6'-yl)dihydropyrimidine-2,4(1H,3H)-dione (Compound A14)

Compound A14-6 (1 g, 2.58 mmol, 1.0 eq) was dissolved in dichloromethane (15 mL). Trifluoroacetic acid (5 mL) was added dropwise. The mixture was stirred at room temperature for 3 hours. The reaction mixture was concentrated under reduced pressure, and the resulting crude product was triturated with acetonitrile to obtain Compound A14 as a white solid (450 mg, 60.68%).

¹H NMR (600 MHz, DMSO-d₆) δ 10.33 (s, 1H), 7.12 - 7.08 (m, 1H), 7.08 (s, 1H), 6.89 - 6.84 (m, 1H), 4.09 (d, *J* = 11.1 Hz, 2H), 4.05 (d, *J =* 11.3 Hz, 2H), 3.69 (t, *J =* 6.7 Hz, 2H), 2.80 (t, *J =* 6.5 Hz, 2H), 2.68 (t, *J =* 6.7 Hz, 2H), 2.16 (t*, J* = 6.5 Hz, 2H), 2.08 (s, 1H).

LCMS (ESI): [M+H]⁺ = 288.25.

### Example 15: Synthesis of Compound A15

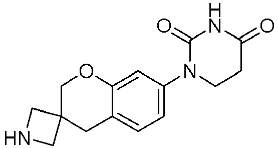

### 1-(spiro[azetidine-3,3'-indane]-7'-yl)dihydropyrimidine-2,4(1H,3H)-dione

### Synthetic Scheme

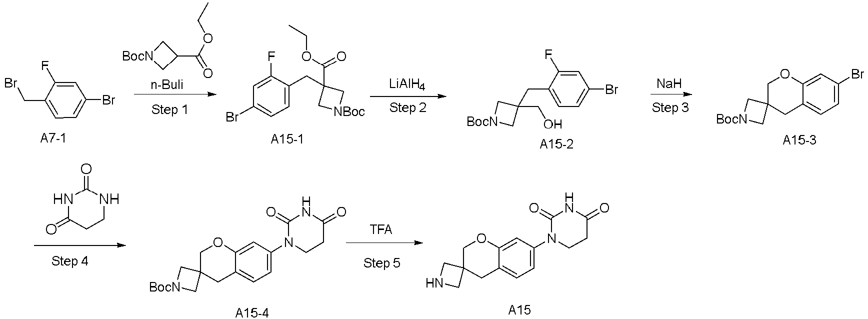

### Step 1: 1-tert-Butyl 3-ethyl 3-(4-bromo-2-fluorobenzyl)azetidine-1,3-dicarboxylate (Compound A15-1)

Diisopropylamine (415 mg, 4.11 mmol, 1.1 eq) was dissolved in super-dry tetrahydrofuran (10 mL). n-Butyllithium (1.6 mL, 4.11 mmol, 1.1 eq) was added to the reaction mixture, and the mixture was stirred at -30°C under nitrogen protection for 30 minutes. The temperature was lowered to -70°C, and a solution of 1-tert-butyl 3-ethyl azetidine-1,3-dicarboxylate (960 mg, 3.73 mmol, 1.0 eq) in tetrahydrofuran was added dropwise. After the addition was complete, the mixture was stirred at -70°C for 1 hour. A solution of A7-1 (1.0 g, 3.73 mmol, 1.0 eq) in tetrahydrofuran was added to the reaction mixture. After the addition was complete, the mixture was stirred at -70°C for 2 hours. Saturated aqueous ammonium chloride solution was added to the reaction mixture, and the mixture was extracted with ethyl acetate. The organic layers were washed with saturated brine, combined and dried over anhydrous sodium sulfate. The organic layer was concentrated, and the resulting crude product was purified by column chromatography to obtain a yellow oily Compound A15-2 (500 mg, 31.2%).

¹H NMR (600 MHz, DMSO-d₆) δ 7.54 - 7.50 (m, 1H), 7.39 - 7.34 (m, 1H), 7.27 - 7.22 (m, 1H), 4.10 - 4.05 (m, 2H), 4.02 - 3.98 (m, 2H), 3.87 - 3.82 (m, 2H), 3.19 (s, 2H), 1.36 (s, 9H), 1.65 - 1.12 (m, 3H).

LCMS (ESI): [M-Boc+H]⁺ = 316.08.

### Step 2: tert-Butyl 3-(4-bromo-2-fluorobenzyl)-3-(hydroxymethyl)azetidine-1-carboxylate (Compound A15-2)

Compound A15-1 (500 mg, 1.2 mmol, 1.0 eq) was dissolved in tetrahydrofuran (20 mL). The temperature was cooled to 0°C, and lithium aluminum hydride (59 mg, 1.56 mmol, 1.3 eq) was added portionwise. The mixture was allowed to warm to room temperature and stirred for 3 hours. The reaction was quenched by adding water, extracted with ethyl acetate, washed with saturated brine, and the combined organic layers were dried over anhydrous sodium sulfate. The organic layer was concentrated, and the resulting crude product was purified by column chromatography to obtain Compound A15-2 as a white solid (300 mg, 66.74%).

¹H NMR (600 MHz, DMSO-d₆) δ 7.55 - 7.49 (m, 1H), 7.39 - 7.35 (m, 1H), 7.35 - 7.27 (m, 1H), 5.07 - 5.01 (m, 1H), 3.56 (s, 4H), 3.36 (d, *J =* 5.3 Hz, 2H), 2.88 - 2.82 (m, 2H), 1.32 (s, 9H).

LCMS (ESI): [M-Boc+H]⁺ = 274.17.

### Step 3: tert-Butyl 7'-bromospiro[azetidine-3,3'-indane]-1-carboxylate (Compound A15-3)

Compound A15-1 (200 mg, 0.49 mmol, 1.0 eq) was dissolved in N,N-dimethylformamide (2 mL). Sodium hydride (39 mg, 1.0 mmol, 2.0 eq) was added, and the mixture was stirred at room temperature for 4 hours. After cooling the reaction mixture to room temperature, water was added to quench the reaction, and the mixture was extracted with ethyl acetate. The organic layers were washed with saturated brine, combined and dried over anhydrous sodium sulfate. The organic layer was concentrated, and the resulting crude product was purified by column chromatography to obtain a yellow solid Compound A15-3 (80 mg, 42.9%).

LCMS (ESI): [M-Boc+H]⁺ = 254.27.

### Step 4: tert-Butyl 7'-(2,4-dioxotetrahydropyrimidin-1(2H)-yl)spiro[azetidine-3,3'-indane]-1-carboxylate (Compound A15-4)

Compound A15-3 (80.0 mg, 0.21 mmol, 1.0 eq) was dissolved in 1,4-dioxane (1 mL). Dihydrouracil (71 mg, 0.63 mmol, 3.0 eq), cesium carbonate (204 mg, 0.63 mmol, 3.0 eq) and (methanesulfonato{dicyclohexyl(3-isopropoxy-2',4',6'-triisopropyl-[1,1'-biphenyl]-2-yl)phosphane}(2'-methylamino-1,1'-biphenyl-2-yl)palladium (9 mg, 0.01 mmol, 0.05 eq) were added to the reaction mixture. The mixture was stirred at 100°C under nitrogen protection for 16 hours. The reaction solution was concentrated under reduced pressure, then water was added. The mixture was extracted with ethyl acetate, washed with saturated brine, and the combined organic layers were dried over anhydrous sodium sulfate. The organic layer was concentrated, and the resulting crude product was purified by column chromatography to obtain a yellow solid Compound A15-4 (56 mg, 64.4%).

¹H NMR (400 MHz, DMSO-d₆) δ 7.08 (d, *J =* 8.2 Hz, 1H), 6.79 - 6.83 (m, 1H), 6.75 (d, *J =* 2.1 Hz, 1H), 4.15 (s, 2H), 3.75 - 3.66 (m, 5H), 3.62 - 3.58 (m, 1H), 2.95 (s, 2H), 2.70 - 2.63 (m, 2H), 1.38 (s, 9H).

LCMS (ESI): [M-Boc+H]⁺ = 288.38.

### Step 5: 1-(spiro[azetidine-3,3'-indane]-7'-yl)dihydropyrimidine-2,4(1H,3H)-dione (Compound A15)

Compound A15-4 (25 mg, 0.06 mmol, 1.0 eq) was dissolved in dichloromethane (2 mL). Trifluoroacetic acid (1 mL) was added. The reaction solution was stirred at room temperature overnight. The reaction mixture was concentrated, and the resulting crude product was purified by reverse phase column chromatography to obtain a white solid Compound A15 (18 mg, 53.9%).

¹H NMR (600 MHz, DMSO-d₆) δ 10.36 (s, 1H), 9.01 (s, 1H), 7.13 (d, *J* = 8.2 Hz, 1H), 6.88 - 6.83 (m, 1H), 6.78 (d, *J =* 2.1 Hz, 1H), 4.24 (s, 2H), 3.87 - 3.78 (m, 4H), 3.74 - 3.63 (m, 2H), 3.03 (s, 2H), 2.70 - 2.64 (m, 2H).

LCMS (ESI): [M+H]⁺ = 288.30.

### Example 16: Synthesis of Compound A16

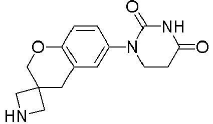

### 1-(spiro[azetidine-3,3'-chroman]-6'-yl)dihydropyrimidine-2,4(1H,3H)-dione

### Synthetic Scheme

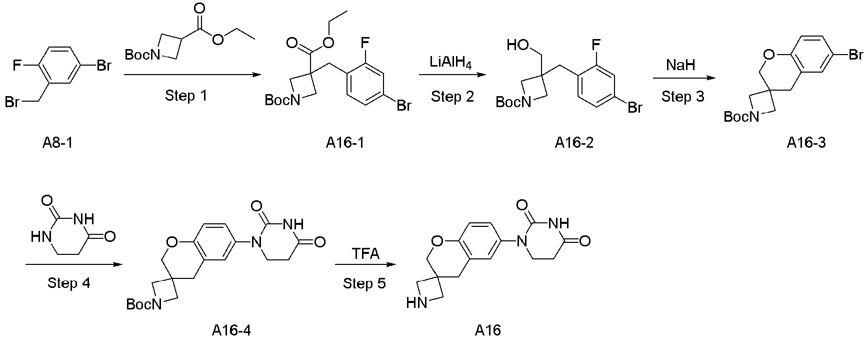

### Step 1: 1-tert-Butyl 3-ethyl 3-(4-bromo-2-fluorobenzyl)azetidine-1,3-dicarboxylate (Compound A16-1)

Diisopropylamine (755 mg, 7.5 mmol, 2.0 eq) was dissolved in super-dry tetrahydrofuran (10 mL) and cooled to -78°C. n-Butyllithium in n-hexane (3.0 mL, 7.5 mmol, 2.5 M, 2.0 eq) was added, and the mixture was stirred at this temperature for 30 minutes. Then, 1-tert-butyl 3-ethyl azetidine-1,3-dicarboxylate (1.3 g, 5.6 mmol, 1.5 eq) dissolved in super-dry tetrahydrofuran (10 mL) was slowly added dropwise to the reaction mixture at -78°C, and the mixture was stirred at this temperature for 1.5 hours. Then, Compound A8-1 (1.0 g, 3.7 mmol, 1.0 eq) dissolved in super-dry tetrahydrofuran (10 mL) was slowly added dropwise to the reaction mixture at -78°C, and the mixture was stirred at this temperature for 3 hours. Saturated aqueous ammonium chloride solution was added to the reaction mixture, which was then extracted with ethyl acetate, washed with saturated brine, and the combined organic layers were dried over anhydrous sodium sulfate. The organic layer was concentrated, and the resulting crude product was purified by column chromatography to obtain a colorless oily Compound A16-1 (480 mg, 30.9%).

¹H NMR (400 MHz, Chloroform-d) δ 7.36 (ddd, *J =* 8.7, 4.6, 2.5 Hz, 1H), 7.28 - 7.26 (m, 1H), 6.95 (t*, J* = 9.1 Hz, 1H), 4.24 - 4.16 (m, 4H), 3.90 (d, *J =* 8.9 Hz, 2H), 3.25 (s, 2H), 1.46 (s, 9H), 1.26 (t, *J =* 7.1 Hz, 3H).

LCMS (ESI): [M-Boc+H]⁺ = 318.28.

### Step 2: tert-Butyl 3-(4-bromo-2-fluorobenzyl)-3-(hydroxymethyl)azetidine-1-carboxylate (Compound A16-2)

Tetrahydrofuran (5 mL) was added to Compound A16-2(480 mg, 1.2 mmol, 1.0 eq). Lithium aluminum hydride (57 mg, 1.5 mmol, 1.2 eq) was added at 0°C, and the mixture was stirred at room temperature for 2 hours. The reaction mixture was filtered through Celite, and the filtrate was concentrated under reduced pressure. Water was added, and the mixture was extracted with ethyl acetate, washed with saturated brine, and the combined organic layers were dried over anhydrous sodium sulfate. The organic layer was concentrated, and the resulting crude product was purified by column chromatography to obtain a colorless oily Compound A16-2 (285 mg, 66.0%).

¹H NMR (600 MHz, Chloroform-d) δ 7.40 (dd, *J =* 6.6, 2.5 Hz, 1H), 7.37 (ddd, *J =* 8.6, 4.5, 2.6 Hz, 1H), 6.97 (t, *J* = 9.1 Hz, 1H), 3.78 (d, *J =* 8.7 Hz, 2H), 3.70 (d, *J =* 8.7 Hz, 2H), 3.64 (d, *J =* 4.9 Hz, 2H), 3.00 (s, 2H), 1.85 (dt, *J =* 6.6, 3.3 Hz, 1H), 1.46 (s, 9H).

LCMS (ESI): [M-Boc+H]⁺ = 274.27.

### Step 3: tert-Butyl 6'-bromospiro[azetidine-3,3'-chromane]-1-carboxylate (Compound A16-3)

Compound A16-2 (280 mg, 0.75 mmol, 1.0 eq) was dissolved in N,N-dimethylformamide (5 mL). Sodium hydride (60 mg, 1.5 mmol, 2.0 eq) was added at 0°C. The reaction mixture was stirred at 45°C for 2 hours. After cooling the reaction mixture to room temperature, ammonium chloride solution was added. The mixture was extracted with ethyl acetate, washed with saturated brine, and the combined organic layers were dried over anhydrous sodium sulfate. The organic layer was concentrated, and the resulting crude product was purified by column chromatography to obtain a white solid Compound A16-3 (150 mg, 56.6%).

¹H NMR (400 MHz, Chloroform-d) δ 7.24 - 7.18 (m, 2H), 6.71 (d, *J =* 8.5 Hz, 1H), 4.16 (s, 2H), 3.82 (d, *J* = 8.8 Hz, 2H), 3.71 (d, *J =* 8.8 Hz, 2H), 2.96 (s, 2H), 1.46 (s, 9H).

LCMS (ESI): [M-t-Bu+H]⁺ = 298.15.

### Step 4: tert-Butyl 6'-(2,4-dioxotetrahydropyrimidin-1(2H)-yl)spiro[azetidine-3,3'-chromane]-1-carboxylate (Compound A16-4)

Super-dry 1,4-dioxane (10 mL) was added to a mixture of compound A16-3 (150 mg, 0.42 mmol, 1.0 eq), dihydrouracil (145 mg, 1.3 mmol, 3.0 eq), (methanesulfonato{dicyclohexyl(3-isopropoxy-2',4',6'- triisopropyl- [1,1'-biphenyl]-2-yl)phosphane}(2'-methylamino-1,1'-biphenyl-2-yl)palladium (19 mg, 0.02 mmol, 0.05 eq) and cesium carbonate (276 mg, 0.85 mmol, 2.0 eq). The mixture was stirred at 100°C under nitrogen protection for 12 hours. After cooling the reaction mixture to room temperature, it was concentrated under reduced pressure. The resulting crude product was purified by column chromatography to obtain a white solid Compound A16-4 (100 mg, 61.0%).

¹H NMR (400 MHz, Chloroform-d) δ 7.06 - 7.00 (m, 2H), 6.87 (d, *J =* 8.3 Hz, 1H), 4.18 (s, 2H), 3.88 - 3.79 (m, 4H), 3.74 (d, *J =* 8.8 Hz, 2H), 2.99 (s, 2H), 2.84 (t, *J =* 6.7 Hz, 2H), 1.47 (s, 9H).

LCMS (ESI): [M-Boc+H]⁺ = 288.27.

### Step 5: 1-(spiro[azetidine-3,3'-chroman]-6'-yl)dihydropyrimidine-2,4(1H,3H)-dione (Compound A16)

Compound A16-4 (100 mg, 0.26 mmol, 1.0 eq) was dissolved in dichloromethane (1 mL). Trifluoroacetic acid (0.2 mL) was added, and the mixture was stirred at room temperature for 2 hours. The reaction mixture was concentrated, and the resulting crude product was purified by reverse phase column chromatography to obtain a white solid Compound A16 (50 mg, 67.4%).

¹H NMR (600 MHz, DMSO-d₆) δ 10.33 (s, 1H), 7.11 - 7.08 (m, 1H), 7.06 (dd, *J =* 8.7, 2.6 Hz, 1H), 6.81 (d, *J* = 8.6 Hz, 1H), 4.23 (d, *J =* 2.2 Hz, 2H), 3.87 - 3.81 (m, 4H), 3.70 (t, *J =* 6.7 Hz, 2H), 3.04 (s, 2H), 2.69 (t, *J =* 6.7 Hz, 2H).

LCMS (ESI): [M+H]⁺ = 288.25.

### Example 17: Synthesis of Compound A17

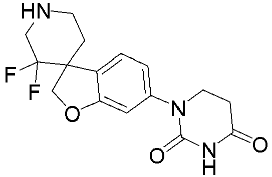

### 1-(3',3'-difluoro-2H-spiro[benzofuran-3,4'-piperidin]-6-yl)dihydropyrimidine-2,4(1H,3H)-dione

### Synthetic Scheme

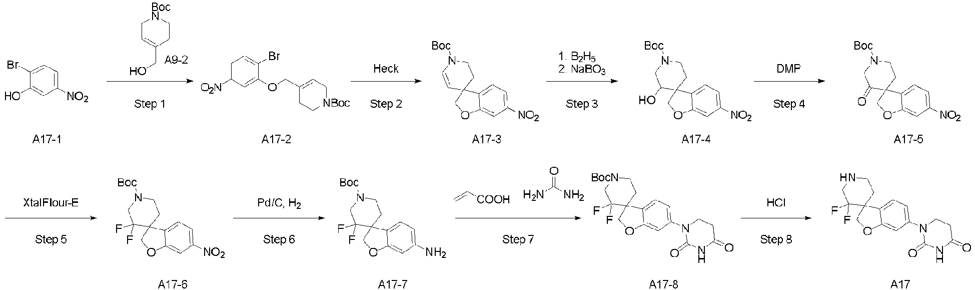

### Step 1: tert-Butyl 4-((2-bromo-5-nitrophenoxy)methyl)-3,6-dihydropyridine-1(2H)-carboxylate (Compound A17-2)

Compound A17-1 (5.0 g, 22.94 mmol, 1.0 eq), Compound A9-2 (5.38 g, 25.23 mmol, 1.1 eq) and triphenylphosphine (12.03 g, 45.87 mmol, 2.0 eq) were dissolved in tetrahydrofuran (50 mL). The mixture was cooled to 0°C, and diethyl azodicarboxylate (7.99 g, 45.87 mmol, 2.0 eq) was added. After the addition was complete, the mixture was allowed to warm to room temperature and stirred overnight. The reaction solution was concentrated under reduced pressure, then water (50 mL) was added. The mixture was extracted with ethyl acetate (100 mL × 3), washed with saturated brine (100 mL), and the combined organic layers were dried over anhydrous sodium sulfate. The organic layer was concentrated, and the resulting crude product was purified by column chromatography to obtain a light yellow oily Compound A17-2 (8.8 g, 92.84%).

LCMS (ESI): [M+H-100]⁺ = 313.08.

### Step 2: tert-Butyl 6-nitro-2',3'-dihydro-1'H,2H-spiro[benzofuran-3,4'-pyridine]-1'-carboxylate (Compound A17-3)

Compound A17-2 (8.8 g, 21.29 mmol, 1.0 eq), sodium formate (1.59 g, 23.42 mmol, 1.1 eq) and sodium acetate (4.37 g, 53.23 mmol, 2.5 eq) were dissolved in N,N-dimethylformamide (90 mL). Then palladium(II) acetate (478.06 mg, 2.13 mmol, 0.1 eq) and triethylamine (2.37 g, 23.42 mmol, 1.1 eq) were added. The mixture was stirred at 70°C under nitrogen protection overnight. After cooling the reaction mixture to room temperature, it was filtered and rinsed with ethyl acetate (100 mL). The organic layer was washed with saturated brine (60 mL × 3), and the combined organic layers were dried over anhydrous sodium sulfate. The organic layer was concentrated, and the resulting crude product was purified by column chromatography to obtain a yellow solid Compound A17-3 (4.3 g, 60.76%).

¹H NMR (400 MHz, DMSO-d₆) δ 7.84 - 7.75 (m, 1H), 7.65 - 7.58 (m, 1H), 7.38 (d, *J* = 8.1 Hz, 1H), 7.11 - 6.92 (m, 1H), 4.95 - 4.74 (m, 1H), 4.57 (d, *J* = 9.0 Hz, 1H), 4.37 (d, *J* = 9.0 Hz, 1H), 3.80 - 3.71 (m, 1H), 3.48 - 3.36 (m, 1H), 2.05 - 1.94 (m, 1H), 1.86 - 1.70 (m, 1H), 1.47 (s, 9H).

LCMS (ESI): [M+H-56]⁺ = 277.25.

### Step 3: tert-Butyl 3'-hydroxy-6-nitro-2H-spiro[benzofuran-3,4'-piperidine]-1'-carboxylate (Compound A17-4)

Compound A17-3 (2.0 g, 6.02 mmol, 1.0 eq) was dissolved in tetrahydrofuran (200 mL). The mixture was cooled to -78°C under nitrogen protection, and a solution of borane in tetrahydrofuran (15.04 mL, 15.04 mmol, 2.5 eq) was added dropwise. The mixture was then warmed to 0°C and stirred for 5 hours. After the reaction, water (2.5 mL) was slowly added dropwise, followed by sodium perborate tetrahydrate (4.63 g, 30.09 mmol, 5.0 eq). The reaction solution was warmed to room temperature and stirred overnight. The reaction mixture was concentrated under reduced pressure, then dichloromethane (300 mL) was added. The mixture was washed with saturated brine (150 mL × 2). The combined organic layers were dried over anhydrous sodium sulfate. The organic layer was concentrated, and the resulting crude product was purified by column chromatography to obtain a yellow solid Compound A17-4 (1.59 g, 59.1%).

¹H NMR (400 MHz, DMSO-d₆) δ 7.76 (dd, *J* = 8.2, 2.1 Hz, 1H), 7.58 - 7.54 (m, 1H), 7.51 (d, *J* = 2.1 Hz, 1H), 5.34 (d*, J* = 4.5 Hz, 1H), 4.81 (d, *J* = 9.0 Hz, 1H), 4.51 (d, *J* = 9.0 Hz, 1H), 4.06 - 3.81 (m, 2H), 3.78 - 3.70 (m, 1H), 2.85 - 2.57 (m, 2H), 1.87 - 1.75 (m, 2H), 1.43 (s, 9H).

LCMS (ESI): [M+H-100]⁺ = 251.27.

### Step 4: tert-Butyl 6-nitro-3'-oxo-2H-spirobenzofuran-3,4'-piperidine]-1'-carboxylate (Compound A17-5)

A17-4 (1.59 g, 4.54 mmol, 1.0 eq) was dissolved in dichloromethane (30 mL). Dess-Martin periodinane (4.81 g, 11.35 mmol, 2.5 eq) was added to the reaction mixture. The mixture was stirred at room temperature for 2.5 hours. The reaction mixture was diluted with dichloromethane (100 mL) and washed with saturated sodium bicarbonate (80 mL × 2). The organic layer was washed with saturated brine (60 mL × 2), and then dried over anhydrous sodium sulfate. The organic layer was concentrated, and the resulting crude product was purified by column chromatography to obtain a light yellow solid Compound A17-5 (1.33 g, 84.13%).

LCMS (ESI) [M+H-100]⁺ = 249.27.

### Step 5: tert-Butyl 3',3'-difluoro-6-nitro-2H-spiro[benzofuran-3,4'-piperidine]- 1'-carboxylate (Compound A17-6)

A17-5 (1.33 g, 3.82 mmol, 1.0 eq) was dissolved in dichloromethane (15 mL). Triethylamine trihydrofluoride (3.69 g, 22.91 mmol, 6.0 eq) and DAST fluoroborate (3.93 g, 17.18 mmol, 4.5 eq) were added to the reaction mixture, followed by triethylamine (965.87 mg, 9.54 mmol, 2.5 eq). The mixture was stirred at room temperature for 2 hours. After the reaction was complete, it was quenched with saturated sodium bicarbonate solution (50 mL) and extracted with dichloromethane (50 mL × 3). The organic layers were washed with saturated brine (50 mL), combined and dried over anhydrous sodium sulfate. The organic layer was concentrated, and the resulting crude product was purified by column chromatography to obtain a light yellow oily Compound A17-6 (530 mg, 37.48%).

¹H NMR (400 MHz, DMSO-d₆) δ 7.82 (dd, *J* = 8.3, 2.1 Hz, 1H), 7.67 - 7.63 (m, 2H), 4.97 (d, *J* = 10.1 Hz, 1H), 4.71 - 4.61 (m, 1H), 4.28 - 4.09 (m, 1H), 4.09 - 3.90 (m, 1H), 3.68 - 3.44 (m, 1H), 3.04 - 2.82 (m, 1H), 2.24 - 2.17 (m, 1H), 2.04 - 1.96 (m, 1H), 1.44 (s, 9H).

LCMS (ESI) [M+H-100]⁺ = 271.27.

### Step 6: tert-Butyl 6-amino-3',3'-difluoro-2H-spiro[benzofuran-3,4'-piperidine]-1'-carboxylate (Compound A17-7)

A17-6 (530 mg, 1.43 mmol, 1.0 eq) was dissolved in methanol (10 mL). 10% Palladium on carbon (250 mg, wet) was added to the reaction mixture. After three cycles of hydrogen replacement, the mixture was stirred at room temperature overnight. After the reaction was complete, the mixture was filtered and the filter cake was washed with methanol. The combined filtrates were concentrated to obtain crude Compound A17-7 as a light yellow solid (437 mg, 89.72%), which was used directly in the next step without further purification.

LCMS (ESI) [M+H]⁺ = 341.30.

### Step 7: tert-Butyl 6-(2,4-dioxotetrahydropyrimidin-1(2H)-yl)-3',3'-difluoro-2H-spiro[benzofuran-3,4'-piperidine]-1'-carboxylate (Compound A17-8)

A17-7 (500.0 mg, 1.47 mmol, 1.0 eq) was dissolved in toluene (5.0 mL). Acrylic acid (211.72 mg, 2.94 mmol, 2.0 eq) was added to the reaction mixture. The mixture was heated to 90°C and stirred for 48 hours. The reaction mixture was cooled to room temperature, and acetic acid (3.0 mL) and urea (529.33 mg, 8.81 mmol, 6 eq) were added. The mixture was heated to 100°C and stirred for 24 hours. The reaction mixture was concentrated under reduced pressure. The concentrate was diluted with saturated sodium bicarbonate (100 mL) and extracted with ethyl acetate (150 mL × 2). The organic layer was washed with saturated sodium bicarbonate (100 mL) and saturated brine (100 mL), and dried over anhydrous sodium sulfate. The organic layer was concentrated, and the resulting crude product was purified by column chromatography to obtain an off-white solid Compound A17-8 (210 mg, 32.68%).

LCMS (ESI) [M+H-56]⁺ = 382.39.

### Step 8: 1-(3',3'-difluoro-2H-spiro[benzofuran-3,4'-piperidin]-6-yl)dihydropyrimidine-2,4(1H,3H)-dione (Compound A17)

Compound A17-8 (100 mg, 228.60 µmol, 1.0 eq) was dissolved in dichloromethane (2.0 mL). A solution of hydrogen chloride in 1,4-dioxane (1.0 mL) was added. The mixture was stirred at room temperature for 2 hours. The reaction mixture was concentrated under reduced pressure, and the resulting crude product was purified by reverse phase column chromatography to obtain an off-white solid Compound A17 (50 mg, 62.35%).

¹H NMR (600 MHz, DMSO-d₆) δ 10.40 (s, 1H), 7.30 (d, *J =* 7.8 Hz, 1H), 6.92 (dd, *J =* 8.1, 1.8 Hz, 1H), 6.88 (d, *J =* 1.7 Hz, 1H), 4.92 - 4.85 (m, 1H), 4.56 (dd, *J =* 10.3, 2.7 Hz, 1H), 3.91 - 3.82 (m, 1H), 3.82 - 3.68 (m, 3H), 3.37 - 3.34 (m, 2H), 3.05 - 2.95 (m, 1H), 2.69 (t, *J =* 6.6 Hz, 2H), 2.40 - 2.30 (m, 1H), 2.21 - 2.12 (m, 1H).

LCMS (ESI) [M+H]⁺ = 338.25.

### Example 18: Synthesis of Compound B1

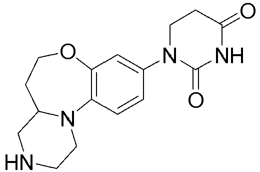

### 1-(2,3,4,4a,5,6-hexahydro-1H-benzo[b]pyrazino[1,2-d][1,4]oxazepin-9-yl)dihydropyrimidine-2,4(1H,3H)-dione

### Synthetic Scheme

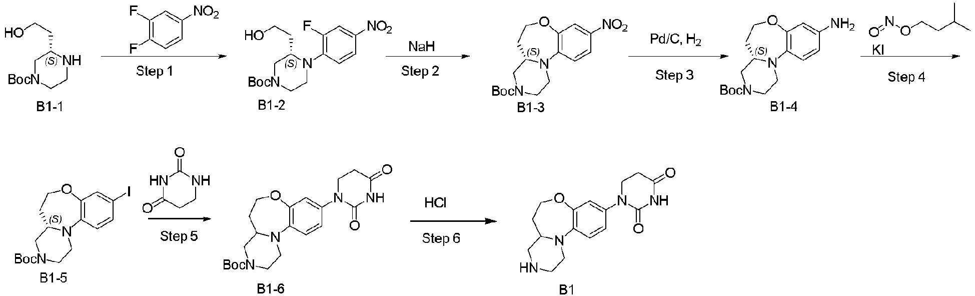

### Step 1: (S)-tert-Butyl 4-(2-fluoro-4-nitrophenyl)-3-(2-hydroxyethyl)piperazine-1-carboxylate (Compound B1-2)

Compound B1-1 (6.0 g, 37.71 mmol, 1.0 eq) was dissolved in dimethyl sulfoxide (20 mL). 3,4-Difluoronitrobenzene (8.69 g, 37.71 mmol, 1.0 eq) was added. The reaction solution was heated to 100°C and stirred at this temperature for 8 hours. After cooling the reaction mixture to room temperature, it was poured into water and extracted with ethyl acetate. The organic layers were washed with saturated brine, combined and dried over anhydrous sodium sulfate. The organic layer was concentrated, and the resulting crude product was purified by column chromatography to obtain a yellow solid Compound B1-2 (12.0 g, 86.1%).

LCMS (ESI): [M-Boc+H]⁺ = 270.27.

### Step 2: (S)-tert-Butyl 9-nitro-1,2,4,4a,5,6-hexahydro-3H-benzo[b]pyrazino[1,2-d][1,4]oxazepine-3-carboxylate (Compound B1-3)

Compound B1-2 (12.0 g, 32.49 mmol, 1.0 eq) was dissolved in N,N-dimethylformamide (50 mL). Sodium hydride (1.69 g, 42.23 mmol, 1.3 eq) was added, and the mixture was stirred at 100°C for 8 hours. After cooling the reaction mixture to room temperature, water was added to quench the reaction, and the mixture was extracted with ethyl acetate. The organic layers were washed with saturated brine, combined and dried over anhydrous sodium sulfate. The organic layer was concentrated, and the resulting crude product was purified by column chromatography to obtain a yellow solid Compound B1-3 (9.0 g, 79.2%).

LCMS (ESI): [M-Boc+H]⁺ = 250.37.

### Step 3: (S)-tert-Butyl 9-amino-1,2,4,4a,5,6-hexahydro-3H-benzo[b]pyrazino[1,2-d][1,4]oxazepine-3-carboxylate (Compound B1-4)

Compound B1-3 (9.0 g, 25.76 mmol, 1.0 eq) was dissolved in methanol (120 mL). Palladium on carbon (1.9 g) was added. After three cycles of hydrogen replacement, the mixture was stirred at room temperature for 12 hours. The reaction mixture was filtered, and the filter cake was washed three times with methanol. The filtrate was concentrated under reduced pressure to obtain crude Compound B1-4 (4.7 g).

LCMS (ESI): [M-Boc+H]⁺ = 320.38.

### Step 4: (S)-tert-Butyl 9-iodo-1,2,4,4a,5,6-hexahydro-3H-benzo[b]pyrazino[1,2-d][1,4]oxazepine-3-carboxylate (Compound B1-5)

Compound B1-4 (4.7 g, 14.71 mmol, 1.0 eq) was dissolved in diiodomethane (50 mL). Isoamyl nitrite (3.45 g, 29.43 mmol, 2.0 eq) and potassium iodide (7.33 g, 44.14 mmol, 3.0 eq) were added. The mixture was heated to 80°C and stirred under nitrogen atmosphere overnight. After cooling the reaction mixture to room temperature, it was concentrated, and the resulting crude product was purified by column chromatography to obtain a yellow oily Compound B1-5 (3.5 g, 55.2%).

¹H NMR (400 MHz, DMSO-d₆) δ 7.25 - 7.19 (m, 1H), 7.11 - 7.05 (m, 1H), 6.78 - 6.70 (m, 1H), 4.35 - 4.24 (m, 1H), 4.18 - 4.09 (m, 1H), 3.64 - 3.56 (m, 1H), 3.55 - 3.48 (m, 1H), 3.34 - 3.24 (m, 1H), 3.19 - 3.11 (m, 2H), 3.11 - 3.04 (m, 2H), 2.07 - 1.94 (m, 1H), 1.89 - 1.80 (m, 1H), 1.41 (s, 9H).

### Step 5: (S)-tert-Butyl 9-(2,4-dioxotetrahydropyrimidin-1(2H)-yl)-1,2,4,4a,5,6-hexahydro-3H-benzo[b]pyrazino[1,2-d][1,4]oxazepine-3-carboxylate (Compound B1-6)

Compound B1-5 (250 mg, 0.58 mmol, 1.0 eq) was dissolved in 1,4-dioxane (3 mL). Dihydrouracil (99 mg, 0.87 mmol, 1.5 eq), cesium carbonate (567 mg, 1.74 mmol, 3.0 eq), and (methanesulfonato{dicyclohexyl(3-isopropoxy-2',4',6'-triisopropyl-[1,1'-biphenyl]-2-yl)phosphane}(2'-methylamino-1,1'-biphenyl-2-yl)palladium(II) (26 mg, 0.029 mmol, 0.05 eq) were added to the reaction mixture. The mixture was stirred at 100°C under nitrogen protection for 16 hours. The reaction solution was concentrated under reduced pressure, then water was added. The mixture was extracted with ethyl acetate, washed with saturated brine, and the combined organic layers were dried over anhydrous sodium sulfate. The organic layer was concentrated, and the resulting crude product was purified by column chromatography to obtain a yellow oily Compound B1-6 (50 mg, 27.2%).

¹H NMR (400 MHz, DMSO-d₆) δ 10.31 (s, 1H), 6.96 - 6.90 (m, 1H), 6.88 - 6.84 (m, 1H), 6.76 (d, *J =* 2.5 Hz, 1H), 4.37 - 4.27 (m, 1H), 4.19 - 4.09 (m, 1H), 3.72 - 3.65 (m, 2H), 3.61 - 3.57 (m, 1H), 3.54 - 3.47 (m, 1H), 3.38 - 3.29 (m, 1H), 3.23 (s, 1H), 3.15 (s, 1H), 3.12 - 3.06 (m, 2H), 2.69 - 2.62 (m, 2H), 1.97 - 1.95 (m, 1H), 1.91 - 1.87 (m, 1H), 1.42 (s, 9H).

LCMS (ESI): [M+H]⁺ = 417.40.

### Step 6: 1-(2,3,4,4a,5,6-hexahydro-1H-benzo[b]pyrazino[1,2-d][1,4]oxazepin-9-yl)dihydropyrimidine-2,4(1H,3H)-dione (Compound B1)

Compound B1-6 (50 mg, 0.116 mmol, 1.0 eq) was dissolved in a solution of hydrogen chloride in 1,4-dioxane (0.5 mL) and dichloromethane (1 mL). The reaction solution was stirred at room temperature overnight. The reaction mixture was concentrated, and the resulting crude product was purified by reverse phase column chromatography to obtain Compound B1 as a white solid (15 mg, 39.1%).

¹H NMR (600 MHz, DMSO-d₆) δ 10.33 (s, 1H), 9.42 (s, 1H), 7.01 - 6.97 (m, 1H), 6.95 - 6.90 (m, 1H), 6.82 - 6.79 (m, 1H), 4.47 - 4.39 (m, 1H), 4.15 - 4.09 (m, 1H), 3.72 - 3.68 (m, 2H), 3.38 - 3.36 (m, 1H), 3.35 - 3.27 (m, 2H), 3.27 - 3.22 (m, 1H), 3.19 - 3.14 (m, 1H), 3.13 - 3.08 (m, 1H), 3.05 - 2.97 (m, 1H), 2.69 - 2.64 (m, 2H), 2.12 - 2.03 (m, 1H), 1.98 - 1.91 (m, 1H).

LCMS (ESI): [M+H]⁺ = 317.12.

### Example 19: Synthesis of Compound B2

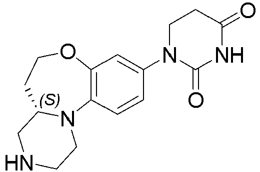

### (S)-1-(2,3,4,4a,5,6-hexahydro-1H-benzo[b]pyrazino[1,2-d][1,4]oxazepin-9-yl)dihydropyrimidine-2,4(1H,3H)-dione

### Synthetic Scheme

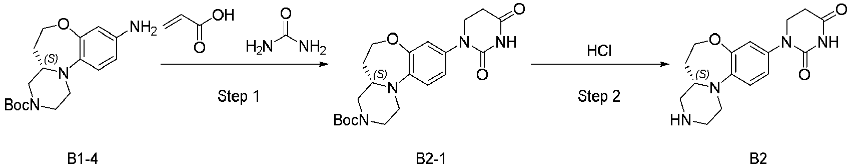

### Step 1: (S)-tert-Butyl 9-(2,4-dioxotetrahydropyrimidin-1(2H)-yl)-1,2,4,4a,5,6-hexahydro-3H-benzo[b]pyrazino[1,2-d][1,4]oxazepine-3-carboxylate (Compound B2-1)

B1-4 (4.0 g, 12.52 mmol, 1.0 eq) was dissolved in toluene (40 mL). Acrylic acid (1.35 g, 18.78 mmol, 1.5 eq) was added to the reaction mixture. The mixture was heated to 90°C and stirred for 24 hours. The reaction mixture was cooled to room temperature, and acetic acid (40 mL) and urea (3.76 g, 62.62 mmol, 5 eq) were added. The mixture was heated to 100°C and stirred for 24 hours. Water (200 mL) and sodium bicarbonate were added to the reaction mixture to neutralize it to weakly basic conditions. The mixture was extracted with ethyl acetate (250 mL × 3). The organic layers were washed with saturated brine (250 mL), combined and dried over anhydrous sodium sulfate. The organic layer was concentrated, and the resulting crude product was purified by column chromatography to obtain a light yellow solid Compound B2-1 (1.5 g, 28.76%).

¹H NMR (400 MHz, DMSO-d₆) δ 10.31 (s, 1H), 6.94 (d, *J =* 8.6 Hz, 1H), 6.87 (dd, *J =* 8.5, 2.4 Hz, 1H), 6.76 (d, *J* = 2.4 Hz, 1H), 4.32 (t, *J* = 9.6 Hz, 1H), 4.21 - 4.10 (m, 1H), 3.69 (t, *J* = 6.7 Hz, 2H), 3.66 - 3.55 (m, 1H), 3.51 (d, *J* = 11.1 Hz, 1H), 3.39 - 3.31 (m, 1H), 3.29 - 3.05 (m, 4H), 2.66 (t, *J =* 6.7 Hz, 2H), 2.07 - 1.95 (m, 1H), 1.95 - 1.81 (m, 1H), 1.42 (s, 9H).

LCMS (ESI) [M+H]⁺ = 417.79.

### Step 2: (S)-1-(2,3,4,4a,5,6-hexahydro-1H-benzo[b]pyrazino[1,2-d][1,4]oxazepin-9-yl)dihydropyrimidine-2,4(1H,3H)-dione (Compound B2)

Compound B2-1 (1.5 g, 3.60 mmol, 1.0 eq) was dissolved in dichloromethane (5 mL). A solution of hydrogen chloride in 1,4-dioxane (15 mL) was added. The mixture was stirred at room temperature for 2 hours. The reaction mixture was concentrated under reduced pressure, and the crude product was triturated with n-hexane to obtain a light yellow solid Compound B2 (1.4 g, overweight).

¹H NMR (600 MHz, CD₃OD) δ 7.07 (d, *J =* 8.6 Hz, 1H), 7.02 (dd, *J =* 8.5, 2.4 Hz, 1H), 6.91 (d, *J =* 2.4 Hz, 1H), 4.51 - 4.44 (m, 1H), 4.24 - 4.15 (m, 1H), 3.86 - 3.79 (m, 2H), 3.50 - 3.37 (m, 4H), 3.31 (d, *J =* 12.1 Hz, 1H), 3.25 - 3.16 (m, 1H), 2.81 (t, *J =* 6.7 Hz, 2H), 2.21 (ddd, *J* = 14.3, 9.2, 4.4 Hz, 1H), 2.02 - 1.94 (m, 1H).

LCMS (ESI) [M+H]⁺ = 317.30.

### Example 20: Synthesis of Compound B3

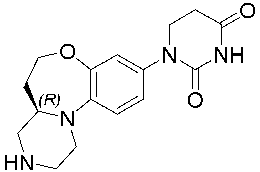

### (R)-1-(2,3,4,4a,5,6-hexahydro-1H-benzo[b]pyrazino[1,2-d][1,4]oxazepin-9-yl)dihydropyrimidine-2,4(1H,3H)-dione

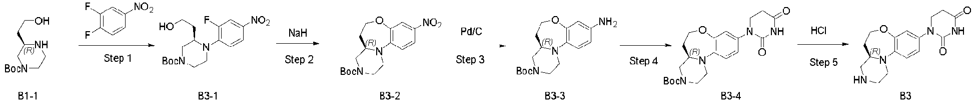

### Synthetic Scheme

Compound B3 was synthesized by the same method as Compound B2.

¹H NMR (600 MHz, DMSO-d₆) δ 10.33 (s, 1H), 7.00 (d, *J =* 8.6 Hz, 1H), 6.93 (dd, *J =* 8.5, 2.5 Hz, 1H), 6.81 (d, *J =* 2.5 Hz, 1H), 4.43 (td, *J =* 10.7, 2.8 Hz, 1H), 4.12 (dt, *J =* 11.3, 4.4 Hz, 1H), 3.71 (t, *J =* 6.7 Hz, 2H), 3.41 - 3.22 (m, 4H), 3.22 - 2.97 (m, 3H), 2.67 *(t, J* = 6.7 Hz, 2H), 2.08 (d, *J =* 6.1 Hz, 1H), 2.03 - 1.90 (m, 1H).

LCMS (ESI): [M+H]⁺ = 331.39.

### Example 21: Synthesis of Compound B4

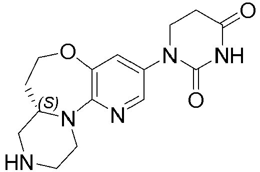

### (S)-1-(7,7a,8,9,10,11-hexahydro-6H-pyrazino[1,2-d]pyrido[3,2-b][1,4]oxazepin-3-yl)dihydropyrimidine-2,4(1H,3H)-dione

### Synthetic Scheme:

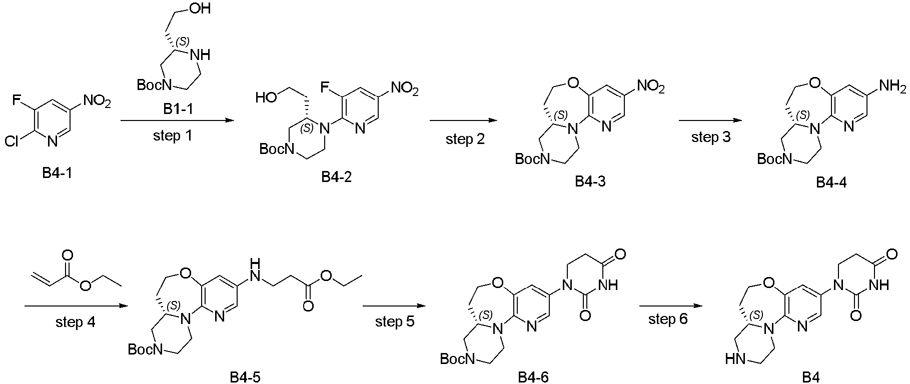

### Step 1: (S)-tert-Butyl 4-(3-fluoro-5-nitropyridin-2-yl)-3-(2-hydroxyethyl)piperazine-1-carboxylate (Compound B4-2)

Compound B4-1 (4.5 g, 25.5 mmol, 1.0 eq) and Compound B1-1 (5.9 g, 25.5 mmol, 1.0 eq) were dissolved in dimethyl sulfoxide (50 mL). The mixture was stirred at 110°C for 16 hours, washed with water, extracted with ethyl acetate, washed with saturated brine, and concentrated under reduced pressure. The crude product was purified by column chromatography to obtain a yellow solid Compound B4-2 (9.4 g, 95.3%).

LC-MS (ESI): [M+H]⁺ = 371.35.

### Step 2: (S)-tert-Butyl 3-nitro-6,7,7a,8,10,11-hexahydro-9H-pyrazino[1,2-d]pyrido[3,2-b][1,4]oxazepine-9-carboxylate (Compound B4-3)

Compound B4-2 (7.0 g, 18.9 mmol, 1.0 eq) was dissolved in N,N-dimethylformamide (100 mL). Sodium hydride (1.97 g, 49.2 mmol, 2.6 eq) was added at 0°C, and the mixture was stirred at room temperature for 2 hours. The reaction was quenched with ammonium chloride solution, and the reaction mixture was extracted with dichloromethane, and concentrated. The crude product was purified by column chromatography to obtain a brown solid Compound B4-3 (5.0 g, 75.51%).

LC-MS (ESI): [M+H]⁺= 315.30.

¹H NMR (600 MHz, DMSO-d₆) δ 8.73 (d, *J =* 2.4 Hz, 1H), 7.73 (d, *J =* 2.4 Hz, 1H), 4.38 - 4.34 (m, 1H), 4.25 - 4.21 (m, 2H), 3.99 - 3.97 (m, 1H), 3.81 - 3.68 (m, 2H), 3.58 (s, 1H), 3.48 (dd, *J =* 13.7, 6.0 Hz, 2H), 2.17 - 2.10 (m, 1H), 1.95 (s, 1H), 1.42 (s, 9H).

### Step 3: (S)-tert-Butyl 3-amino-6,7,7a,8,10,11-hexahydro-9H-pyrazino[1,2-d]pyrido[3,2-b][1,4]oxazepine-9-carboxylate (Compound B4-4)

Compound B4-3 (5.0 g, 14.3 mmol, 1.0 eq) was dissolved in methanol (50 mL). Palladium on carbon (500 mg) was added, and the mixture was purged with hydrogen and stirred at room temperature for 16 hours. The mixture was filtered through Celite, and the filtrate was concentrated to dryness to obtain a brown solid Compound B4-4 (4.0 g, 87.49%).

LC-MS (ESI): [M+H]⁺= 321.30.

### Step 4: (S)-tert-Butyl 3-((3-ethoxy-3-oxopropyl)amino)-6,7,7a,8,10,11-hexahydro-9H-pyrazino[1,2-d]pyrido[3,2-b][1,4]oxazepine-9-carboxylate (Compound B4-5)

Compound B4-4 (1.0 g, 3.1 mmol, 1.0 eq) was dissolved in ethyl acrylate (2 mL) and stirred at 80°C overnight. The mixture was concentrated under reduced pressure, and the crude product was purified by column chromatography to obtain a brown oily Compound B4-5 (140 mg, 10.67%).

LC-MS (ESI): [M+H]⁺ = 421.35.

### Step 5: (S)-tert-Butyl 3-(2,4-dioxotetrahydropyrimidin-1(2H)-yl)-6,7,7a,8,10,11-hexahydro-9H-pyrazino[1,2-d]pyrido[3,2-b][1,4]oxazepine-9-carboxylate (Compound B4-6)

Compound B4-5 (100 mg, 0.24 mmol, 1.0 eq) was dissolved in tetrahydrofuran (2 mL). Triphosgene (35 mg, 0.12 mmol, 0.5 eq) and N,N-diisopropylethylamine (61 mg, 0.48 mmol, 2.0 eq) were added, and the mixture was stirred at 20°C for 2 hours. A solution of ammonia in methanol (2 mL) was added at 0°C, and the mixture was slowly warmed to room temperature and then stirred at 100°C overnight. The mixture was concentrated under reduced pressure, and the crude product was purified by column chromatography to obtain a brown oily Compound B4-6 (12 mg, 12.09%).

LC-MS (ESI): [M+H]⁺ = 418.40.

### Step 6: (S)-1-(7,7a,8,9,10,11-hexahydro-6H-pyrazino[1,2-d]pyrido[3,2-b][1,4]oxazepin-3-yl)dihydropyrimidine-2,4(1H,3H)-dione (Compound B4)

Compound B4-6 (12 mg, 0.03 mmol, 1.0 eq) was dissolved in dichloromethane (0.5 mL). Trifluoroacetic acid (0.5 mL) was added, and the mixture was stirred at 20°C for 16 hours. The mixture was concentrated under reduced pressure, and the crude product was purified by HPLC to obtain a white solid Compound B4 (2 mg, 21.92%).

LC-MS (ESI): [M+H]⁺= 318.30.

¹H NMR (600 MHz, DMSO-d₆) δ 10.43 (s, 1H), 7.89 (s, 1H), 7.18 (s, 1H), 4.36 - 4.32 (m, 1H), 4.23 - 4.19 (m, 1H), 3.72 (t, *J =* 6.7 Hz, 2H), 3.61 - 3.58 (m, 2H), 3.27 - 3.25 (m, 2H), 3.06 (d, *J =* 12.1 Hz, 1H), 2.99 - 2.93 (m, 2H), 2.75 (t*, J* = 10.9 Hz, 1H), 2.69 (t, *J =* 6.6 Hz, 2H), 2.11 - 2.06 (m, 1H), 2.01 - 1.92 (m, 1H).

### Example 22: Synthesis of Compound B5

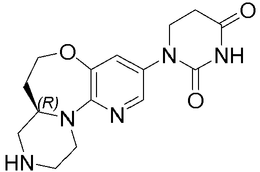

### (R)-1-(7,7a,8,9,10,11-hexahydro-6H-pyrazino[1,2-d]pyrido[3,2-b][1,4]oxirane-3-yl)dihydropyrimidine-2,4(1H,3H)-dione

### Synthetic Scheme:

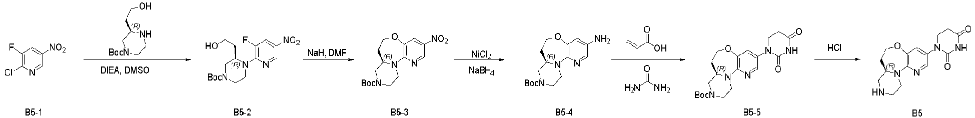

Step 1: (R)-tert-Butyl 4-(3-fluoro-5-nitropyridin-2-yl)-3-(2-hydroxyethyl)piperazine-1-carboxylate (Compound B5-2)

2-Chloro-3-fluoro-5-nitropyridine (2 g, 11.33 mmol, 1 eq) was dissolved in dimethyl sulfoxide. (R)-tert-Butyl 3-(2-hydroxyethyl)piperazine-1-carboxylate (2.61 g, 11.33 mmol, 1 eq) and N,N-diisopropylethylamine (4.39 g, 33.99 mmol, 3 eq) were added, and the mixture was stirred in an oil bath at 110°C overnight. The reaction solution was diluted with ethyl acetate and extracted three times with ethyl acetate. The combined organic layers were washed with brine, dried over anhydrous sodium sulfate, and concentrated under reduced pressure. The crude product obtaind by concentration was purified by normal phase column chromatography to obtain a yellow solid Compound B5-2 (3.6 g, 85%).

¹H NMR (600 MHz, DMSO-d₆) δ 8.87 (d, *J =* 1.4 Hz, 1H), 8.27 (dd, *J =* 13.8, 2.3 Hz, 1H), 4.72 (s, 1H), 4.49 (s, 1H), 4.22 (d, *J* = 13.2 Hz, 1H), 3.94 (d, *J* = 13.5 Hz, 2H), 3.42 (s, 2H), 3.18 (d, *J* = 5.3 Hz, 1H), 3.15 - 2.85 (m, 2H), 1.78 (s, 2H), 1.43 (s, 9H).

LCMS (ESI): [M-t-Bu+H]⁺ = 315.28.

### Step 2: (R)-tert-Butyl 3-nitro-6,7,7a,8,10,11-hexahydro-9H-pyrazino[1,2-d]pyrido[3,2-b][1,4]oxirane-9-carboxylate (Compound B5-3)

Compound B5-2 (3.55 g, 9.85 mmol, 1 eq) was dissolved in N,N-dimethylformamide (30 mL). Sodium hydride (996 mg, 24.9 mmol, 2.6 eq) was added portionwise at 0°C, and the mixture was stirred at room temperature for 2 hours. The reaction solution was diluted with ethyl acetate and extracted three times with ethyl acetate. The combined organic layers were washed with brine, dried over anhydrous sodium sulfate, and concentrated under reduced pressure. The crude product obtaind by concentration was purified by normal phase column chromatography to obtain a yellow solid Compound B5-3 (2.25 g, 67.04%).

¹H NMR (600 MHz, DMSO-d₆) δ 8.73 (d, *J =* 2.2 Hz, 1H), 7.73 (d, *J =* 2.3 Hz, 1H), 4.39 - 4.32 (m, 1H), 4.26 - 4.18 (m, 2H), 4.03 - 2.98 (m, 1H), 3.80 - 3.70 (m, 2H), 3.65 - 3.55 (m, 1H), 3.51 - 3.40 (m, 2H), 2.17 - 2.09 (m, 1H), 2.03 - 1.98 (m, 1H), 1.43 (s, 9H).

LCMS (ESI): [M+H]⁺ = 351.49.

### Step 3: (R)-tert-Butyl 3-amino-6,7,7a,8,10,11-hexahydro-9H-pyrazino[1,2-d]pyrido[3,2-b][1,4]oxirane-9-carboxylate (Compound B5-4)

Compound B5-3 (2.22 g, 6.34 mmol, 1 eq) was dissolved in a mixed solution of methanol and dichloromethane (v/v = 2:1). Nickel(II) chloride (1.64 g, 12.67 mmol, 2 eq) was added, followed by portionwise addition of sodium borohydride (0.958 g, 25.34 mmol, 4 eq) at 0°C. The mixture was stirred in an ice bath for 5 minutes. After the reaction was complete, the mixture was concentrated under reduced pressure. The crude product obtaind by concentration was purified by normal phase column chromatography to obtain a yellow solid Compound B5-4 (1.22 g, 61.10%).

¹H NMR (600 MHz, DMSO-d₆) δ 7.30 (d, *J =* 2.4 Hz, 1H), 6.43 (d, *J =* 2.4 Hz, 1H), 4.40 - 4.32 (m, 1H), 4.12 - 4.07 (m, 1H), 3.72 - 3.68 (m, 1H), 3.64 - 3.58 (m, 1H), 3.25 (dt, *J* = 6.8, 3.1 Hz, 1H), 3.20 - 3.10 (m, 1H), 3.10 - 2.95 (m, 3H), 2.06 - 1.98 (m, 1H), 1.82 - 1.76 (m, 1H), 1.42 (s, 9H).

LCMS (ESI): [M+H]⁺ = 321.38.

### Step 4: (R)-tert-Butyl 3-(2,4-dioxotetrahydropyrimidin-1(2H)-yl)-6,7,7a,8,10,11-hexahydro-9H-pyrazino[1,2-d]pyrido[3,2-b][1,4]oxirane-9-carboxylate (Compound B5-5)

Under nitrogen atmosphere, Compound B5-4 (1.11 g, 3.46 mmol, 1 eq) was dissolved in toluene (10 mL). Acrylic acid (374.49 mg, 5.20 mmol, 1.5 eq) was added at room temperature, and the reaction mixture was moved to an oil bath at 95°C and stirred for 13 hours. The reaction was terminated, and the mixture was allowed to cool to room temperature. Acetic acid (10 mL) and urea (1.04 g, 17.32 mmol, 1.5 eq) were added, and the mixture was further stirred at 100°C under nitrogen atmosphere for 24 hours. After the reaction, the mixture was quenched with aqueous sodium bicarbonate solution, diluted with ethyl acetate, and extracted three times with ethyl acetate. The combined organic layers were washed with brine, dried over anhydrous sodium sulfate, and concentrated under reduced pressure. The crude product obtaind by concentration was purified by normal phase column chromatography to obtain a yellow solid Compound B5-5 (206 mg, 14.24%).

¹H NMR (600 MHz, DMSO-d₆) δ 10.38 (s, 1H), 7.84 (d, *J =* 2.1 Hz, 1H), 7.15 (d, *J* = 2.2 Hz, 1H), 4.34 - 4.21 (m, 2H), 3.71 (t*, J* = 6.7 Hz, 2H), 3.68 - 3.58 (m, 4H), 3.47 - 3.38 (m, 1H), 3.32 - 3.20 (m, 2H), 2.69 (t, *J =* 6.7 Hz, 2H), 2.13 - 2.04 (m, 1H), 1.94 - 1.84 (m, 1H), 1.43 (s, 9H).

LCMS (ESI): [M+H]⁺ = 418.49.

### Step 5: (R)-1-(7,7a,8,9,10,11-hexahydro-6H-pyrazino[1,2-d]pyrido[3,2-b][1,4]oxirane-3-yl)dihydropyrimidine-2,4(1H,3H)-dione (Compound B5)

To a solution of Compound B5-5 (128 mg, 306.61 µmol, 1.0 eq) in dichloromethane (5 mL) was added a solution of hydrogen chloride in 1,4-dioxane (2 mL). The mixture was stirred at room temperature for 1 hour, then concentrated. The residue was lyophilized to obtain a white solid Compound B5 (81.90 mg, 84.17%).

¹H NMR (600 MHz, DMSO-d₆) δ 10.43 (s, 1H), 7.94 (d, *J =* 2.3 Hz, 1H), 7.26 (d, *J* = 2.2 Hz, 1H), 4.42 - 4.35 (m, 1H), 4.28 - 4.23 (m, 1H), 3.79 - 3.72 (m, 3H), 3.72 - 3.66 (m, 1H), 3.48 - 3.42 (m, 1H), 3.32 - 3.27 (m, 1H), 3.23 - 3.12 (m, 2H), 2.99 - 2.90 (m, 1H), 2.70 (t, *J =* 6.7 Hz, 2H), 2.22 - 2.14 (m, 1H), 2.03 - 1.96 (m, 1H).

LCMS (ESI): [M+H]⁺ = 318.48.

### Example 23: Synthesis of Compound B6

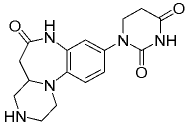

### 1-(6-oxo-1,2,3,4,4a,5,6,7-octahydrobenzo[b]pyrazino[1,2-d][1,4]diazepin-9-yl)dihydropyrimidine-2,4(1H,3H)-dione

### Synthetic Scheme:

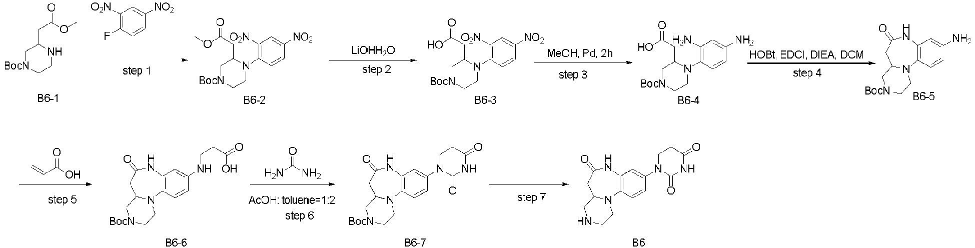

### Step 1: tert-Butyl 4-(2,4-dinitrophenyl)-3-(2-methoxy-2-oxoethyl)piperazine-1-carboxylate (Compound B6-2)

B6-1 (5.0 g, 19.3 mmol, 1.0 eq) and 2,4-dinitrofluorobenzene (4.0 g, 21.5 mmol, 1.1 eq) were dissolved in dimethyl sulfoxide (50 mL). The mixture was stirred at 100°C for 16 hours, washed with water, extracted with ethyl acetate, washed with saturated brine, and concentrated under reduced pressure. The crude product was purified by column chromatography to obtain a yellow solid Compound B6-2 (7.2 g, 78.9%).

¹H NMR (400 MHz, Chloroform-d) δ 8.72 (d, *J* = 2.7 Hz, 1H), 8.29 (dd, *J* = 9.3, 2.8 Hz, 1H), 7.18 (d, *J* = 9.3 Hz, 1H), 4.33-4.01 (m, 3H), 3.56 (s, 3H), 3.52-3.44 (m, 1H), 3.35 (s, 1H), 3.09-3.02 (m, 2H), 2.69 (s, 2H), 1.50 (s, 9H).

LC-MS (ESI): [M-t-Bu+H]⁺ = 369.29.

### Step 2: 2-(4-(tert-Butoxycarbonyl)-1-(2,4-dinitrophenyl)piperazin-2-yl)acetic acid (Compound B6-3)

Compound B6-2 (7.2 g, 16.9 mmol, 1.0 eq) was dissolved in a mixed solvent of tetrahydrofuran:methanol:water = 2:2:1 (30 mL). Lithium hydroxide (2.2 g, 50.9 mmol, 3.0 eq) was added, and the mixture was stirred at room temperature for 2 hours. The pH of the solution was adjusted to 4-5 with 2M hydrochloric acid. The mixture was washed with water, extracted with ethyl acetate, washed with saturated brine, and concentrated under reduced pressure. The crude product was purified by column chromatography to obtain a brown solid Compound B6-3 (2.3 g, 33.0%).

LC-MS (ESI): [M-t-Bu+H]⁺ = 355.39.

### Step 3: 2-(4-(tert-Butoxycarbonyl)-1-(2,4-diaminophenyl)piperazin-2-yl)acetic acid (Compound B6-4)

Compound B6-3 (1.2 g, 2.9 mmol, 1.0 eq) was dissolved in methanol (20 mL). Palladium on carbon (300 mg) was added, and the mixture was purged with hydrogen and stirred at room temperature for 2 hours. The mixture was filtered through Celite, and the filtrate was concentrated to dryness to obtain a brown solid Compound B6-4 (900 mg, 97.8%).

LC-MS (ESI): [M+H]⁺ = 351.39.

### Step 4: tert-Butyl 9-amino-6-oxo-1,2,4a,5,6,7-hexahydrobenzo[b]pyrazino[1,2-d][1,4]diazepine-3(4H)-carboxylate (Compound B6-5)

Compound B6-4 (900 mg, 2.6 mmol, 1.0 eq) was dissolved in dichloromethane (20 mL). 1-Hydroxybenzotriazole (500 mg, 3.7 mmol, 1.4 eq), 1-(3-dimethylaminopropyl)-3-ethylcarbodiimide hydrochloride (1.4 g, 7.3 mmol, 2.8 eq) and N,N-diisopropylethylamine (920 mg, 7.3 mmol, 2.8 eq) were added, and the mixture was stirred at room temperature overnight. The mixture was concentrated under reduced pressure, and the crude product was purified by column chromatography to obtain a brown oily Compound B6-5 (720 mg, 84.3%).

LC-MS (ESI): [M+H]⁺ = 333.39.

### Step 5: 3-((3-(tert-Butoxycarbonyl)-6-oxo-1,2,3,4,4a,5,6,7-octahydrobenzo[b]pyrazino[1,2-d][1,4]diazepin-9-yl)amino)propanoic acid (Compound B6-6)

Compound B6-5 (720 mg, 2.2 mmol, 1.0 eq) was dissolved in toluene (20 mL). Acrylic acid (315 mg, 4.4 mmol, 2.0 eq) was added, and the mixture was stirred at 110°C for 16 hours (overnight). The reaction solution was used directly in the next step without workup after cooling to room temperature.

LC-MS (ESI): [M+H]⁺ = 404.49.

### Step 6: tert-Butyl 9-(2,4-dioxotetrahydropyrimidin-1(2H)-yl)-6-oxo-1,2,4a,5,6,7-hexahydrobenzo[b]pyrazino[1,2-d][1,4]diazepine-3(4H)-carboxylate (Compound B6-7)

To the reaction solution from the previous step was added acetic acid (10 mL) and urea (1.3 g, 21.6 mmol, 10.0 eq). The mixture was stirred at 110°C for 16 hours (overnight). The mixture was concentrated under reduced pressure, and the crude product was purified by column chromatography to obtain a yellow solid Compound B6-7 (350 mg, 37.7%).

LC-MS (ESI): [M-t-Bu+H]⁺ = 374.49.

¹H NMR (600 MHz, DMSO-d₆) δ 10.35 (s, 1H), 9.65 (d, *J =* 1.7 Hz, 1H), 7.10 (d, *J* = 1.4 Hz, 2H), 6.93 (t, *J* = 1.4 Hz, 1H), 4.03 - 3.87 (m, 2H), 3.75-3.71 (m, 2H), 3.18-3.15 (m, 2H), 3.09 - 2.95 (m, 2H), 2.87 (s, 1H), 2.70 (t, *J* = 6.7 Hz, 2H), 2.59-2.55 (m, 1H), 2.10 - 1.97 (m, 1H), 1.43 (s, 9H).

### Step 7: 1-(6-oxo-1,2,3,4,4a,5,6,7-octahydrobenzo[b]pyrazino[1,2-d][1,4]diazepin-9-yl)dihydropyrimidine-2,4(1H,3H)-dione (Compound B6)

Compound B6-7 (350 mg, 0.81 mmol, 1.0 eq) was dissolved in dichloromethane (5 mL). A solution of hydrogen chloride in dioxane (5 mL) was added, and the mixture was stirred at room temperature for 1 hour. The mixture was filtered, and the crude product was triturated with methanol to obtain a yellow solid Compound B6 (267 mg, 99.5%).

¹H NMR (600 MHz, DMSO-d₆) δ 10.37 (s, 1H), 9.77 (d, *J* = 1.7 Hz, 1H), 7.20 - 7.09 (m, 2H), 6.95 (d, *J* = 2.4 Hz, 1H), 3.79 - 3.68 (m, 2H), 3.59 - 3.54 (m, 1H), 3.42-3.38 (m, 2H), 3.31 - 3.23 (m, 2H), 3.03 (d, *J* = 40.0 Hz, 2H), 2.71 (t, *J* = 6.7 Hz, 2H), 2.67-2.64 (m, 1H), 2.08 (d, *J* = 12.0 Hz, 1H).

LC-MS (ESI): [M+H]⁺ = 333.30.

### Example 24: Synthesis of Compound B7

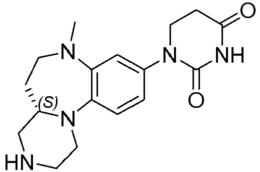

### (S)-1-(7-methyl-1,2,3,4,4a,5,6,7-octahydrobenzo[b]pyrazino[1,2-d][1,4]diazepin-9-yl)dihydropyrimidine-2,4(1H,3H)-dione

### Synthetic Scheme

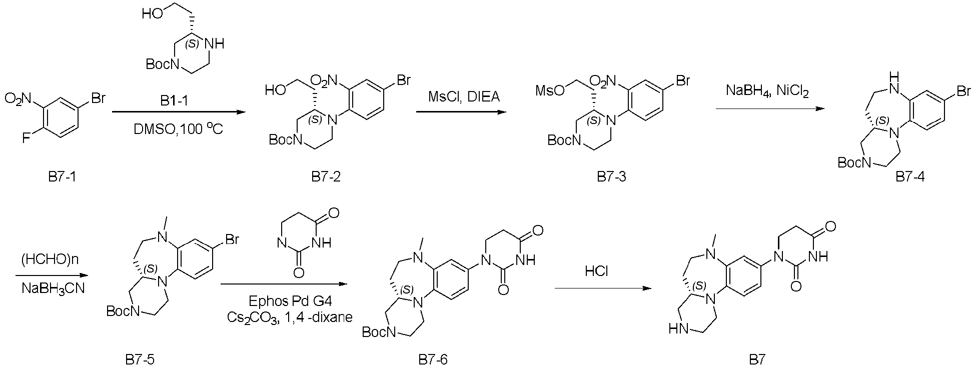

### Step 1: (S)-tert-Butyl 4-(4-bromo-2-nitrophenyl)-3-(2-hydroxyethyl)piperazine-1-carboxylate (Compound B7-2)

Compound B1-1 (5.0 g, 21.71 mmol, 1.0 eq) was dissolved in dimethyl sulfoxide (30 mL). Compound B7-1 (5.25 g, 23.88 mmol, 1.5 eq) and N,N-diisopropylethylamine (11.3 g, 47.76 mmol, 4.0 eq) were added to the reaction mixture. The reaction solution was heated to 100°C and stirred at this temperature for 12 hours. After cooling the reaction mixture to room temperature, it was concentrated under reduced pressure, then water (30 mL) was added. The mixture was extracted with ethyl acetate (30 mL × 3), washed with saturated brine (30 mL), and the combined organic layers were dried over anhydrous sodium sulfate. The organic layer was concentrated, and the crude product was purified by column chromatography to obtain a yellow solid Compound B7-2 (5.61 g, 60.05%).

¹H NMR (600 MHz, DMSO-d₆) δ 8.04 (d, *J* = 2.3 Hz, 1H), 7.75 (dd, *J* = 8.8, 2.2 Hz, 1H), 7.37 (s, 1H), 4.44 (t, *J* = 4.8 Hz, 1H), 3.75 - 3.35 (m, 4H), 3.33 - 3.28 (m, 2H), 3.21 - 3.05 (m, 1H), 2.82 - 2.72 (m, 1H), 1.54 - 1.35 (m, 11H).

LCMS (ESI): [M-t-Bu+H]⁺ = 374.29.

### Step 2: (S)-tert-Butyl 4-(4-bromo-2-nitrophenyl)-3-(2-((methylsulfonyl)oxy)ethyl)piperazine-1-carboxylate (Compound B7-3)

Compound B7-2 (6.55 g, 15.22 mmol, 1.0 eq) and N,N-diisopropylethylamine (5.9 g, 45.67 mmol, 3.0 eq) were dissolved in dichloromethane (30 mL). The mixture was cooled to 0°C, and methanesulfonyl chloride (5.23 g, 45.67 mmol, 3.0 eq) was added. After the addition was complete, the mixture was allowed to warm to room temperature and stirred for 2 hours. The reaction solution was concentrated, and the resulting crude product was purified by column chromatography to obtain a yellow solid Compound B7-3 (4.63 g, 59.83%).

¹H NMR (600 MHz, DMSO-d₆) δ 8.07 (d, *J* = 2.3 Hz, 1H), 7.77 (dd, *J* = 8.8, 2.4 Hz, 1H), 7.43 (d, *J* = 7.4 Hz, 1H), 4.23 - 4.08 (m, 2H), 3.65 - 3.40 (m, 4H), 3.30 - 3.10 (m, 2H), 3.07 (s, 3H), 2.80 - 2.72 (m, 1H), 1.79 - 1.63 (m, 2H), 1.42 (s, 9H).

LCMS (ESI): [M-t-Bu+H]⁺ = 452.19.

### Step 3: (S)-tert-Butyl 9-bromo-1,2,4a,5,6,7-hexahydrobenzo[b]pyrazino[1,2-d][1,4]diazepine-3(4H)-carboxylate (Compound B7-4)

Compound B7-3 (4.50 g, 8.85 mmol, 1.0 eq) and nickel(II) chloride (2.29 g, 17.70 mmol, 2.0 eq) were dissolved in methanol/dichloromethane (40 mL/20 mL). The mixture was cooled to 0°C, and sodium borohydride (1.34 g, 35.41 mmol, 4.0 eq) was added portionwise. The mixture was stirred at room temperature under nitrogen protection for 12 hours. The reaction mixture was filtered, and the filtrate was concentrated under reduced pressure. The resulting crude product was purified by column chromatography to obtain a white solid Compound B7-4 (2.5 g, 73.88%).

¹H NMR (600 MHz, DMSO-d₆) δ 6.73 - 6.68 (m, 2H), 6.65 (dd, *J* = 8.4, 2.3 Hz, 1H), 5.64 (s, 1H), 3.66 (d, *J* = 12.8 Hz, 1H), 3.55 - 3.38 (m, 2H), 3.27 - 2.91 (m, 5H), 2.90 - 2.80 (m, 1H), 1.92 - 1.83 (m, 1H), 1.70 - 1.62 (m, 1H), 1.41 (s, 9H).

LCMS (ESI): [M-t-Bu+H]⁺= 326.38.

### Step 4: (S)-tert-Butyl 9-bromo-7-methyl-1,2,4a,5,6,7-hexahydrobenzo[b]pyrazino[1,2-d][1,4]diazepine-3(4H)-carboxylate (Compound B7-5)

B7-4 (2.0 g, 5.23 mmol, 1.0 eq) and paraformaldehyde (4.71 g, 156.9 mmol, 30.0 eq) were dissolved in methanol (20 mL). Acetic acid (3.14 g, 52.31 mmol, 10.0 eq) was added dropwise. The mixture was stirred at room temperature for 3 hours. Sodium cyanoborohydride (986.26 mg, 15.69 mmol, 3.0 eq) was added, and the mixture was stirred at room temperature for 12 hours. The reaction mixture was filtered, and the filtrate was concentrated under reduced pressure. The resulting crude product was purified by column chromatography to obtain a white solid Compound B7-5 (1.7 g, 81.99%).

¹H NMR (600 MHz, DMSO-d₆) δ 6.89 (dd, *J* = 8.3, 2.3 Hz, 1H), 6.77 (d, *J* = 8.4 Hz, 1H), 6.75 (d, *J* = 2.3 Hz, 1H), 3.93 - 3.79 (m, 1H), 3.79 - 3.61 (m, 1H), 3.57 - 3.50 (m, 1H), 3.12 - 2.83 (m, 4H), 2.80 (dt, *J* = 11.7, 4.0 Hz, 1H), 2.75 (s, 3H), 2.69 - 2.60 (m, 1H), 1.81 - 1.71 (m, 1H), 1.68 - 1.57 (m, 1H), 1.42 (s, 9H).

LCMS (ESI): [M+H]⁺ = 396.39.

### Step 5: (S)-tert-Butyl 9-(2,4-dioxotetrahydropyrimidin-1(2H)-yl)-7-methyl-1,2,4a,5,6,7-hexahydrobenzo[b]pyrazino[1,2-d][1,4]diazepine-3(4H)-carboxylate (Compound B7-6)

B7-5 (2.6 g, 6.56 mmol, 1.0 eq) and dihydropyrimidine-2,4(1H,3H)-dione (898.2 mg, 7.87 mmol, 1.2 eq) were dissolved in 1,4-dioxane (25 mL). EPhos Pd G4 (603 mg, 0.656 mmol, 0.1 eq) and cesium carbonate (4.27 g, 13.12 mmol, 2 eq) were added. The mixture was stirred in an oil bath at 100°C overnight. The reaction mixture was concentrated under reduced pressure, and the resulting crude product was purified by column chromatography to obtain a white solid Compound B7-6 (1.06 g, 37.6%).

¹H NMR (600 MHz, CDCl₃) δ 7.61 (s, 1H), 6.89 (d, *J* = 8.3 Hz, 1H), 6.77 (dd, *J* = 8.3, 1.4 Hz, 1H), 6.67 (s, 1H), 4.15 - 3.95 (m, 1H), 3.95 - 3.71 (m, 3H), 3.66 (t, *J* = 11.5 Hz, 1H), 3.23 - 2.98 (m, 4H), 2.87 (s, 3H), 2.84 (t,*J* = 6.7 Hz, 2H), 2.81 - 2.75 (m, 1H), 1.98 (t, *J* = 13.5 Hz, 1H), 1.68 (s, 1H), 1.66 - 1.60 (m, 1H), 1.50 (s, 9H).

LCMS (ESI): [M+H]⁺ = 430.49.

### Step 6: (S)-1-(7-methyl-1,2,3,4,4a,5,6,7-octahydrobenzo[b]pyrazino[1,2-d][1,4]diazepin-9-yl)dihydropyrimidine-2,4(1H,3H)-dione (Compound B7)

B7-6 (1.1 g, 2.56 mmol, 1.0 eq) was dissolved in dichloromethane (10 mL). A solution of hydrogen chloride in 1,4-dioxane (10 mL) was added, and the mixture was stirred at room temperature for 6 hours. The reaction mixture was filtered, and the resulting filter cake was dried under vacuum to obtain a white solid Compound B7 (867 mg, 102.8%).

¹H NMR (600 MHz, MeOD) δ 7.71 (d, *J =* 17.1 Hz, 1H), 7.60 (dd, *J* = 8.5, 2.0 Hz, 1H), 7.47 - 7.40 (m, 1H), 3.99 - 3.91 (m, 2H), 3.64 - 3.56 (m, 2H), 3.55 - 3.45 (m, 2H), 3.41 - 3.34 (m, 4H), 3.33 - 3.20 (m, 4H), 2.87 (t, *J* = 6.7 Hz, 2H), 2.02 - 1.82 (m, 2H).

LCMS (ESI): [M+H]⁺ = 330.48.

### Example 25: Synthesis of Compound B8

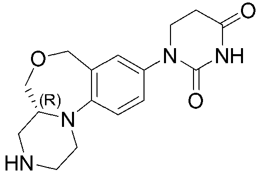

### (R)-1-(1,2,3,4,4a,5-hexahydro-7H-benzo[e]pyrazino[2,1-c][1,4]oxazepin-9-yl)dihydropyrimidine-2,4(1H,3H)-dione

### Synthetic Scheme

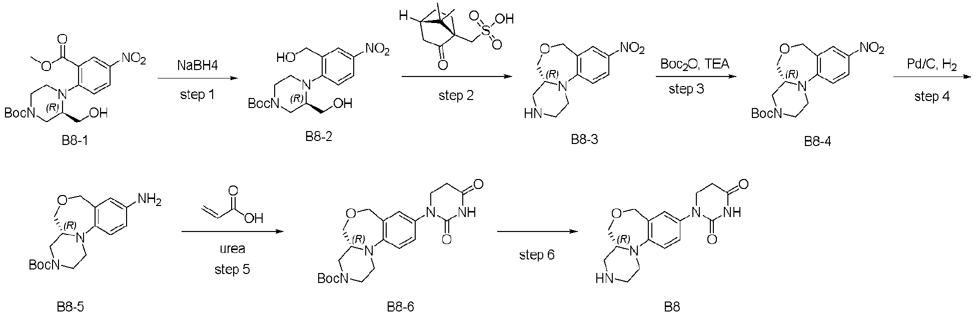

### Step 1: (R)-tert-Butyl 3-(hydroxymethyl)-4-(2-(hydroxymethyl)-4-nitrophenyl)piperazine-1-carboxylate (Compound B8-2)

Compound B8-1 (2 g, 5.06 mmol, 1.0 eq.) was dissolved in methanol (40 mL). The reaction mixture was cooled to 0°C, and sodium borohydride (957 mg, 25.29 mmol, 5.0 eq.) was added. The mixture was stirred at room temperature overnight, concentrated under reduced pressure, then water was added. The mixture was extracted three times with ethyl acetate, washed with saturated brine, and the combined organic layers were dried over anhydrous sodium sulfate. The organic layer was concentrated to obtain a crude white solid Compound B8-2 (1.76 g, 94.71%).

LCMS (ESI): [M+H]⁺ = 368.30.

### Step 2: (R)-9-Nitro-1,2,3,4,4a,5-hexahydro-7H-benzo[e]pyrazino[2,1-c][1,4]oxazepine (Compound B8-3)

Compound B8-2 (1.76 g, 4.79 mmol, 1.0 eq) and (+)-10-camphorsulfonic acid (2.23 g, 9.58 mmol, 2.0 eq) were dissolved in toluene (30 mL). The mixture was stirred at 100°C under nitrogen protection for 5 hours. The reaction solution was concentrated under reduced pressure to obtain crude B8-3, which was used directly in the next step.

LCMS (ESI): [M+H]⁺ = 250.20.

### Step 3: (R)-tert-Butyl 9-nitro-1,2,4a,5-tetrahydro-7H-benzo[e]pyrazino[2,1-c][1,4]oxazepine-3(4H)-carboxylate (Compound B8-4)

Compound B8-3 (1.2 g, 4.81 mmol, 1.0 eq) was dissolved in dichloromethane (15 mL). The reaction mixture was cooled to 0°C, and triethylamine (1.46 g, 14.44 mmol, 3.0 eq) and di-tert-butyl dicarbonate (1.58 g, 7.22 mmol, 1.5 eq) were added. The mixture was stirred at room temperature overnight. After concentration under reduced pressure, the crude product obtained by concentration was purified by column chromatography to obtain a yellow oily Compound B8-4 (1.5 g, 89.18%).

¹H NMR (600 MHz, DMSO-d₆) δ 8.15 - 8.02 (m, 2H), 7.05 (d, *J* = 9.0 Hz, 1H), 4.91 (d, *J* = 13.3 Hz, 1H), 4.64 (d, *J* = 13.3 Hz, 1H), 3.82 (d, *J* = 9.9 Hz, 1H), 3.74 - 3.61 (m, 4H), 3.50 (dt, *J* = 8.4, 4.0 Hz, 2H), 3.43 - 3.38 (m, 2H), 1.43 (s, 9H).

LCMS (ESI): [M+H]⁺ = 350.35.

### Step 4: (R)-tert-Butyl 9-amino-1,2,4a,5-tetrahydro-7H-benzo[e]pyrazino[2,1-c][1,4]oxazepine-3(4H)-carboxylate (Compound B8-5)

Compound B8-4 (1.5 g, 4.29 mmol, 1.0 eq) was dissolved in methanol (20 mL). Palladium on carbon (300 mg) was added. After three cycles of hydrogen replacement, the mixture was stirred at room temperature for 12 hours. The reaction mixture was filtered, and the filter cake was washed with methanol. The filtrate was concentrated under reduced pressure, and the crude product was purified by reverse phase column chromatography to obtain a white solid Compound B8-5 (620 mg, 45.21%).

LCMS (ESI): [M+H]⁺ = 320.36.

### Step 5: (R)-tert-Butyl 9-(2,4-dioxotetrahydropyrimidin-1(2H)-yl)-1,2,4a,5-tetrahydro-7H-benzo[e]pyrazino[2,1-c][1,4]oxazepine-3(4H)-carboxylate (Compound B8-6)

Compound B8-5 (100 mg, 0.31 mmol, 1.0 eq) and acrylic acid (34 mg, 0.47 mmol, 1.5 eq) were dissolved in toluene (4 mL). The mixture was stirred at 90°C for 16 hours. Urea (94 mg, 1.57 mmol, 5.0 eq) and acetic acid (2 mL) were added, and the mixture was stirred at 110°C for 12 hours. The reaction solution was concentrated under reduced pressure, and the resulting crude product was purified by column chromatography to obtain a yellow solid Compound B8-6 (40 mg, 30.68%).

### Step 6: (R)-1-(1,2,3,4,4a,5-hexahydro-7H-benzo[e]pyrazino[2,1-c][1,4]oxazepin-9-yl)dihydropyrimidine-2,4(1H,3H)-dione (Compound B8)

Compound B8-6 (40 mg, 0.10 mmol, 1.0 eq) was dissolved in dichloromethane (1 mL). Trifluoroacetic acid (1 mL) was added, and the mixture was stirred at room temperature for 12 hours. The reaction mixture was concentrated under reduced pressure, and the crude product was purified by HPLC to obtain a white solid Compound B8 (5 mg, 16.46%).

¹H NMR (600 MHz, DMSO-d₆) δ 10.36 (s, 1H), 7.29 (dd, *J* = 8.5, 2.6 Hz, 1H), 7.22 (d, *J* = 2.5 Hz, 1H), 7.01 (d, *J* = 8.5 Hz, 1H), 4.87 (d, *J* = 11.6 Hz, 1H), 4.59 (d, *J* = 11.6 Hz, 1H), 3.74 (t, *J* = 6.7 Hz, 2H), 3.66 (dd, *J* = 13.5, 3.1 Hz, 1H), 3.59 (dd, *J* = 13.5, 3.0 Hz, 1H), 3.42 - 3.38 (m, 1H), 3.31 - 3.21 (m, 3H), 3.17 - 3.02 (m, 3H), 2.70 (t, *J* = 6.7 Hz, 2H).

LCMS (ESI): [M+H]⁺ = 317.30.

### Example 26: Synthesis of Compound B9

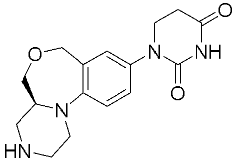

### (S)-1-(1,2,3,4,4a,5-hexahydro-7H-benzo[e]pyrazino[2,1-c][1,4]oxazepin-9-yl)dihydropyrimidine-2,4(1H,3H)-dione

### Synthetic Scheme

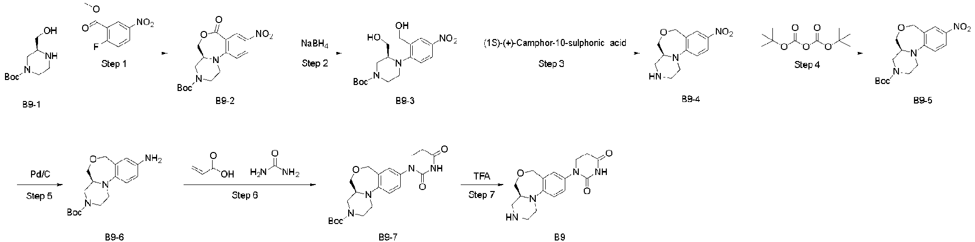

### Step 1: 4-Bromo-5-hydroxyisobenzofuran-1(3H)-one (Compound B9-2)

Compound B9-1 (10.0 g, 46.24 mmol, 1.0 eq) was dissolved in dimethyl sulfoxide (100 mL). Methyl 2-fluoro-5-nitrobenzoate (9.21 g, 46.24 mmol, 1.0 eq) was added to the reaction mixture. The reaction solution was heated to 100°C and stirred at this temperature for 12 hours. The reaction solution was concentrated under reduced pressure, then water (50 mL) was added. The mixture was extracted with ethyl acetate (100 mL × 3), washed with saturated brine (100 mL), and the combined organic layers were dried over anhydrous sodium sulfate. The organic layer was concentrated, and the resulting crude product was purified by reverse phase column chromatography to obtain a yellow solid Compound B9-2 (12.0 g, 71.62%).

LCMS (ESI): [M+H]⁺ = 364.37.

### Step 2: (S)-tert-Butyl 3-(hydroxymethyl)-4-(2-(hydroxymethyl)-4-nitrophenyl)piperazine-1-carboxylate (Compound B9-3)

Compound B9-2 (12.0 g, 33.02 mmol, 1.0 eq) was dissolved in methanol (120 mL). The mixture was cooled to 0°C, and sodium borohydride (6.25 g, 165 mmol) was slowly added. After the addition was complete, the mixture was allowed to warm to room temperature and stirred for 12 hours. The reaction solution was concentrated under reduced pressure, then water (50 mL) was added. The mixture was extracted with ethyl acetate (100 mL × 3), washed with saturated brine (100 mL), and the combined organic layers were dried over anhydrous sodium sulfate. The organic layer was concentrated, and the resulting crude product was purified by reverse phase column chromatography to obtain a yellow solid Compound B9-3 (8.0 g, 65.49%).

LCMS (ESI): [M+H]⁺ = 368.40.

### Step 3: (S)-9-Nitro-1,2,3,4,4a,5-hexahydro-7H-benzo[e]pyrazino[2,1-c][1,4]oxazepine (Compound B9-4)

Compound B9-3 (2.0 g, 5.44 mmol, 1.0 eq) and camphorsulfonic acid (2.53 g, 10.89 mmol, 2 eq) were dissolved in toluene (20 mL). The mixture was stirred at 100°C under nitrogen protection for 12 hours. After cooling the reaction mixture to room temperature, it was concentrated under reduced pressure, then water (100 mL) was added. The mixture was extracted with ethyl acetate (100 mL × 3), washed with saturated brine (100 mL), and the combined organic layers were dried over anhydrous sodium sulfate. The organic layer was concentrated, and the resulting crude product was purified by column chromatography to obtain a crude yellow solid Compound B9-4 (3.50 g).

LCMS (ESI): [M+H]⁺ = 250.27.

### Step 4: (S)-tert-Butyl 9-nitro-1,2,4a,5-tetrahydro-7H-benzo[e]pyrazino[2,1-c][1,4]oxazepine-3(4H)-carboxylate (Compound B9-5)

Compound B9-4 (3.50 g, 14.05 mmol, 1.0 eq) was dissolved in dichloromethane (35 mL). The reaction mixture was cooled to 0°C, and N,N-diisopropylethylamine (4 mL) and di-tert-butyl dicarbonate (4.59 g, 21.05 mmol, 1.5 eq) were added. After the addition was complete, the mixture was allowed to warm to room temperature and stirred for 12 hours. Saturated aqueous ammonium chloride solution (50 mL) was added, and the mixture was extracted with dichloromethane (50 mL × 3). The organic layers were washed with saturated brine, combined (100 mL) and dried over anhydrous sodium sulfate. The organic layer was concentrated, and the resulting crude product was purified by column chromatography to obtain a yellow oily Compound B9-5 (4.5 g, 91.83%).

LCMS (ESI): [M+H]⁺ = 350.39.

### Step 5: (S)-tert-Butyl 9-amino-1,2,4a,5-tetrahydro-7H-benzo[e]pyrazino[2,1-c][1,4]oxazepine-3(4H)-carboxylate (Compound B9-6)

Compound B9-5 (4.5 g, 12.89 mmol, 1.0 eq) was dissolved in methanol (50 mL). 10% Palladium on carbon (900 mg) was added, and the mixture was stirred at room temperature under hydrogen atmosphere for 12 hours. The reaction mixture was filtered, and the filtrate was concentrated to obtain a crude yellow solid Compound B9-6 (5 g).

LCMS (ESI): [M+H]⁺ = 320.41.

### Step 6: (S)-tert-Butyl 9-(2,4-dioxotetrahydropyrimidin-1(2H)-yl)-1,2,4a,5-tetrahydro-7H-benzo[e]pyrazino[2,1-c][1,4]oxazepine-3(4H)-carboxylate (Compound B9-7)

B9-6 (5.0 g, 15.67 mmol, 1.0 eq) was dissolved in toluene (30 mL). Acrylic acid (1.35 g, 18.80 mmol, 1.2 eq) was added to the reaction mixture. The reaction mixture was heated to 90°C and stirred for 12 hours. Then urea (4.7 g, 78.35 mmol, 5.0 eq) and acetic acid (30 mL) were added. The mixture was stirred at 120°C for 12 hours, then allowed to cool to room temperature. The pH of the reaction mixture was adjusted to 8 with saturated aqueous sodium bicarbonate solution, and the mixture was extracted with ethyl acetate (50 mL × 3). The combined organic layers were dried over anhydrous sodium sulfate. The organic layer was concentrated, and the resulting crude product was purified by column chromatography to obtain a white solid Compound B9-7 (3 g, 46.1%).

LCMS (ESI): [M+H]⁺ = 417.48.

### Step 7: (S)-1-(1,2,3,4,4a,5-hexahydro-7H-benzo[e]pyrazino[2,1-c][1,4]oxazepin-9-yl)dihydropyrimidine-2,4(1H,3H)-dione (Compound B9)

Compound B9-7 (3 g, 7.21 mmol, 1.0 eq) was dissolved in dichloromethane (30 mL). Trifluoroacetic acid (10 mL) was added, and the mixture was stirred at room temperature for 2 hours. The mixture was concentrated under reduced pressure, and the resulting crude product was purified by reverse phase column chromatography to obtain a white solid Compound B9 (2 g, 87.79%).

¹H NMR (600 MHz, DMSO) δ 10.37 (s, 1H), 7.31 (dd, *J* = 8.5, 2.5 Hz, 1H), 7.24 (d, *J* = 2.4 Hz, 1H), 7.02 (d, *J* = 8.5 Hz, 1H), 4.90 (d, *J* = 11.5 Hz, 1H), 4.59 (d, *J* = 11.5 Hz, 2H), 3.74 (t, *J =* 6.7 Hz, 2H), 3.67 (dd, *J* = 13.5, 3.1 Hz, 1H), 3.58 (dd, *J* = 13.5, 2.6 Hz, 1H), 3.45 - 3.27 (m, 4H), 3.24 - 3.11 (m, 2H), 3.07 (dd, *J* = 10.6, 2.5 Hz, 1H), 2.70 (t, *J* = 6.7 Hz, 2H).

LCMS (ESI): [M+H]⁺ = 317.36.

### Example 27: Synthesis of Compound B10

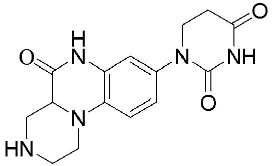

### 1-(5-oxo-2,3,4,4a,5,6-hexahydro-1H-pyrazino[1,2-a]quinoxalin-8-yl)dihydropyrimidine-2,4(1H,3H)-dione

### Synthetic Scheme

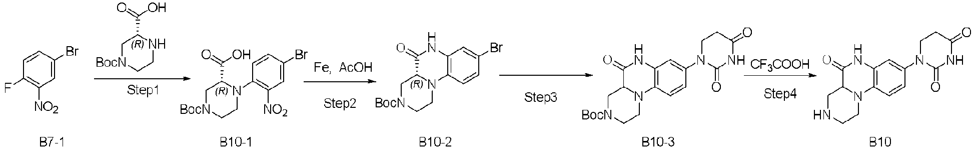

### Step 1: (R)-1-(4-Bromo-2-nitrophenyl)-4-(tert-butoxycarbonyl)piperazine-2-carboxylic acid (Compound B10-1)

Compound B7-1 (1 g, 4.55 mmol, 1.0 eq) was dissolved in N,N-dimethylformamide (10 mL). 1-(tert-Butoxycarbonyl)piperazine-1,3-dicarboxylic acid (1.05 g, 4.55 mmol, 1.0 eq) and cesium carbonate (2.81 g, 8.64 mmol, 1.9 eq) were added to the reaction mixture. The reaction solution was heated to 60°C and stirred at this temperature for 12 hours. The reaction mixture was cooled to room temperature, water was added, and the mixture was extracted with ethyl acetate, washed with saturated brine, and the combined organic layers were dried over anhydrous sodium sulfate. The organic layer was concentrated to obtain a crude yellow oily Compound B10-1 (1.85 g, 94.59%). This crude product was used directly in the next step without further purification.

LCMS (ESI): [M-Boc+H]⁺ = 332.19.

### Step 2: (R)-tert-Butyl 8-bromo-5-oxo-1,2,4,4a,5,6-hexahydro-3H-pyrazino[1,2-a]quinoxaline-3-carboxylate (Compound B10-2)

Compound B10-1 (1.55 g, 3.60 mmol, 1.0 eq) was dissolved in acetic acid (16 mL). Iron powder (1.01 g, 18.01 mmol, 5.0 eq) was added to the reaction mixture. The reaction solution was heated to 60°C and stirred at this temperature for 1 hour. The reaction solution was filtered, and the filtrate was concentrated under reduced pressure. Water was added to the residue, and the mixture was extracted with ethyl acetate, washed with saturated brine, and the combined organic layers were dried over anhydrous sodium sulfate. The organic layer was concentrated, and the resulting crude product was purified by column chromatography to obtain a brown solid Compound B10-2 (732 mg, 53.04%).

¹H NMR (600 MHz, Chloroform-d) δ 8.28 (s, 1H), 7.14 (dd, *J* = 8.6, 2.2 Hz, 1H), 6.93 (d, *J* = 2.2 Hz, 1H), 6.67 (d, *J* = 8.7 Hz, 1H), 4.63 (s, 1H), 4.28 (s, 1H), 3.62 - 3.45 (m, 2H), 3.12 - 2.69 (m, 3H), 1.42 (s, 9H).

LCMS (ESI): [M-Boc+H]⁺ = 284.28.

### Step 3: (R)-tert-Butyl 8-(2,4-dioxotetrahydropyrimidin-1(2H)-yl)-5-oxo-1,2,4,4a,5,6-hexahydro-3H-pyrazino[1,2-a]quinoxaline-3-carboxylate (Compound B10-3)

Compound B10-2 (100 mg, 0.36 mmol, 1.0 eq) was dissolved in 1,4-dioxane (2 mL). Dihydropyrimidine-2,4(1H,3H)-dione (90 mg, 0.78 mmol, 3.0 eq), (methanesulfonato{dicyclohexyl(3-isopropoxy-2',4',6'-triisopropyl-[1,1'-biphenyl]-2-yl)phosphane}(2'-methylamino-1,1'-biphenyl-2-yl)palladium(II) (24 mg, 0.03 mmol, 0.1 eq), and cesium carbonate (256 mg, 0.78 mmol, 3.0 eq) were added. The mixture was stirred at 100°C under nitrogen protection for 12 hours. The reaction mixture was cooled to room temperature, concentrated under reduced pressure, and the resulting solid was dissolved in water. The mixture was extracted with ethyl acetate, washed with saturated brine, and the combined organic layers were dried over anhydrous sodium sulfate. The organic layer was concentrated, and the resulting crude product was purified by column chromatography to obtain a white solid Compound B10-3 (70 mg, 64.41%).

¹H NMR (400 MHz, DMSO-d₆) δ 10.71 (s, 1H), 10.32 (s, 1H), 6.92 - 6.83 (m, 2H), 6.81 (d, *J =* 2.0 Hz, 1H), 4.34 (d, *J* = 13.2 Hz, 1H), 4.03 (d, *J =* 7.2 Hz, 1H), 3.69 (t, *J =* 6.7 Hz, 2H), 3.20 (td, *J =* 6.8, 2.6 Hz, 2H), 2.89 (s, 2H), 2.58 (td, *J* = 12.2, 3.6 Hz, 1H), 2.44 (t, *J* = 6.8 Hz, 2H), 1.43 (s, 9H).

LCMS (ESI): [M-Boc+H]⁺ = 316.28.

### Step 4: 1-(5-oxo-2,3,4,4a,5,6-hexahydro-1H-pyrazino[1,2-a]quinoxalin-8-yl)dihydropyrimidine-2,4(1H,3H)-dione (Compound B10)

Compound B10-3 (65 mg, 0.156 mmol, 1.0 eq) was dissolved in dichloromethane (1.5 mL). Trifluoroacetic acid (0.5 mL) was added, and the mixture was stirred at room temperature for 12 hours. The resulting reaction mixture was concentrated under reduced pressure, and the crude product obtained by concentration was purified by reverse phase column chromatography and lyophilized to obtain a white solid Compound B10 (20.0 mg, 40.54%).

¹H NMR (400 MHz, DMSO-d₆) δ 10.88 (s, 1H), 10.34 (s, 1H), 6.92 (d, *J* = 1.5 Hz, 2H), 6.86 (d, *J* = 1.5 Hz, 1H), 3.90 - 3.76 (m, 2H), 3.70 (t, *J* = 6.7 Hz, 3H), 3.16 - 3.09 (m, 2H), 2.96 - 2.82 (m, 1H), 2.69 (t, *J* = 6.7 Hz, 2H), 1.27 - 1.21 (m, 2H).

LCMS (ESI): [M+H]⁺ = 316.16.

### Example 28: Synthesis of Compound B11

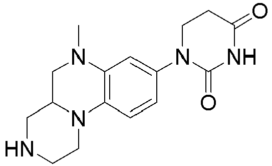

### 1-(6-methyl-2,3,4,4a,5,6-hexahydro-1H-pyrazino[1,2-a]quinoxalin-8-yl)dihydropyrimidine-2,4(1H,3H)-dione

### Synthetic Scheme

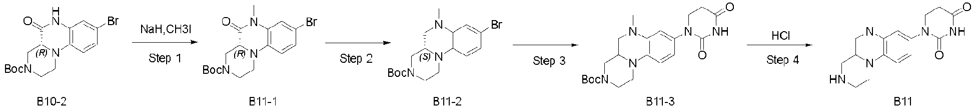

### Step 1: (R)-tert-Butyl 8-bromo-6-methyl-5-oxo-1,2,4,4a,5,6-hexahydro-3H-pyrazino[1,2-a]quinoxaline-3-carboxylate (Compound B11-1)

Compound B10-2 (3 g, 7.85 mmol, 1.0 eq) was dissolved in N,N-dimethylformamide (30 mL). Sodium hydride (470.8 mg, 11.77 mmol, 1.5 eq) was added at 0°C under nitrogen protection. The mixture was stirred for 30 minutes, then allowed to warm to room temperature. Iodomethane (1.67 g, 11.77 mmol, 1.5 eq) was added dropwise, and the mixture was stirred at room temperature for 2 hours. Saturated aqueous ammonium chloride solution was added to the reaction mixture, which was then extracted with ethyl acetate, washed with saturated brine, and the combined organic layers were dried over anhydrous sodium sulfate. The organic layer was concentrated, and the resulting crude product was purified by column chromatography to obtain a yellow solid Compound B11-1 (2.96 g, 95.17%).

¹H NMR (400 MHz, DMSO-d₆) δ 7.24 - 7.17 (m, 2H), 6.85 (d, *J* = 8.6 Hz, 1H), 4.36 (d, *J* = 13.1 Hz, 1H), 4.03 (dd, *J* = 7.2, 4.1 Hz, 1H), 3.68 - 3.56 (m, 2H), 3.47 (dd, *J* = 10.9, 4.0 Hz, 3H), 2.90 (s, 2H), 2.58 (td, *J* = 12.1, 3.7 Hz, 1H), 1.42 (s, 9H).

LCMS (ESI): [M-Boc+H]⁺ = 296.18.

### Step 2: (S)-tert-Butyl 8-bromo-6-methyl-1,2,4,4a,5,6-hexahydro-3H-pyrazino[1,2-a]quinoxaline-3-carboxylate (Compound B11-2)

Compound B11-1 (2.96 g, 7.47 mmol, 1.0 eq) was dissolved in tetrahydrofuran (30 mL). Borane dimethyl sulfide (1.70 g, 22.41 mmol, 3.0 eq) was added, and the reaction mixture was heated to 50°C and stirred for 20 hours. The reaction mixture was cooled to room temperature, poured into water, and stirred for 30 minutes. The mixture was extracted with ethyl acetate, washed with saturated brine, and the combined organic layers were dried over anhydrous sodium sulfate. The organic layer was concentrated, and the resulting crude product was purified by reverse phase column chromatography to obtain a white solid Compound B11-2 (2.68 g, 93.85%).

¹H NMR (400 MHz, DMSO-d₆) δ 6.71 - 6.49 (m, 3H), 3.96 (d, *J* = 14.7 Hz, 2H), 3.68 (d, *J* = 12.5 Hz, 1H), 3.29 (dd, *J* = 11.1, 3.2 Hz, 1H), 3.08 - 2.83 (m, 3H), 2.78 (s, 3H), 2.54 (d, *J* = 3.4 Hz, 1H), 2.48 (d, *J* = 3.5 Hz, 1H), 1.41 (s, 9H).

LCMS (ESI): [M+H]⁺ = 383.39.

### Step 3: tert-Butyl 8-(2,4-dioxotetrahydropyrimidin-1(2H)-yl)-6-methyl-1,2,4,4a,5,6-hexahydro-3H-pyrazino[1,2-a]quinoxaline-3-carboxylate (Compound B11-3)

Compound B11-2 (1 g, 2.62 mmol, 1.0 eq) was dissolved in 1,4-dioxane (15 mL). Dihydropyrimidine-2,4(1H,3H)-dione (895 mg, 7.85 mmol, 3.0 eq), (methanesulfonato{dicyclohexyl(3-isopropoxy-2',4',6'-triisopropyl-[1,1'-biphenyl]-2-yl)phosphane}(2'-methylamino-1,1'-biphenyl-2-yl)palladium(II) (120 mg, 0.13 mmol, 0.05 eq), and cesium carbonate (1.7 g, 5.23 mmol, 2.0 eq) were added. The mixture was stirred at 100°C under nitrogen protection for 12 hours. The reaction mixture was cooled to room temperature, concentrated under reduced pressure, and the resulting solid was dissolved in water. The mixture was extracted with ethyl acetate, washed with saturated brine, and the combined organic layers were dried over anhydrous sodium sulfate. The organic layer was concentrated, and the resulting crude product was purified by column chromatography to obtain a white solid Compound B11-3 (300 mg, 27.60%).

¹H NMR (400 MHz, DMSO-d₆) δ 10.33 (s, 1H), 7.07 (d, *J* = 8.1 Hz, 1H), 6.91 - 6.68 (m, 2H), 3.73 (t, *J* = 6.6 Hz, 4H), 3.14 (s, 2H), 2.69 (ddd, *J =* 17.7, 6.7 Hz, 4H), 1.79 (t, *J =* 6.7 Hz, 2H), 1.68 (d, *J* = 13.6 Hz, 2H), 1.57 - 1.47 (m, 2H), 1.40 (s, 9H).

LCMS (ESI): [M-Boc+H]⁺ = 316.90.

### Step 4: 1-(6-methyl-2,3,4,4a,5,6-hexahydro-1H-pyrazino[1,2-a]quinoxalin-8-yl)dihydropyrimidine-2,4(1H,3H)-dione (Compound B11)

Compound B11-3 (300 mg, 0.72 mmol, 1.0 eq) was dissolved in dichloromethane (1.5 mL). A solution of hydrogen chloride in dioxane (2 mL) was added, and the mixture was stirred at room temperature for 12 hours. The reaction solution was filtered, and the filter cake was triturated with n-hexane. It was filtered to obtain a white solid Compound B11 (127 mg, 44.03%).

¹H NMR (400 MHz, DMSO-d₆) δ 10.33 (s, 1H), 7.07 (d, *J* = 8.1 Hz, 1H), 6.91 - 6.68 (m, 2H), 3.73 (t, *J* = 6.6 Hz, 4H), 3.14 (s, 2H), 2.69 (ddd, *J =* 17.7, 6.7 Hz, 4H), 1.79 (t, *J =* 6.7 Hz, 2H), 1.68 (d, *J* = 13.6 Hz, 2H), 1.57 - 1.47 (m, 2H).

LCMS (ESI): [M+H]⁺ = 316.40.

### Example 29: Synthesis of Compound B12

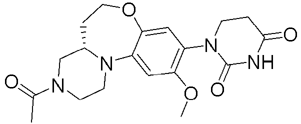

### (S)-1-(3-acetyl-10-methoxy-2,3,4,4a,5,6-hexahydro-1H-benzo[b]pyrazino[1,2-d][1,4]oxirane-9-yl)dihydropyrimidine-2,4(1H,3H)-dione

### Synthetic Scheme

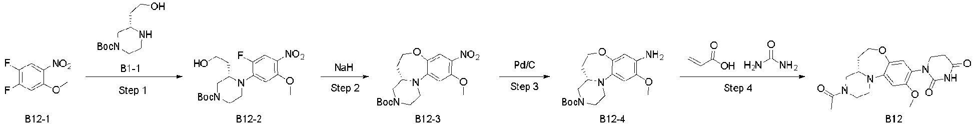

### Step 1: (S)-tert-Butyl 4-(2-fluoro-5-methoxy-4-nitrophenyl)-3-(2-hydroxyethyl)piperazine-1-carboxylate (Compound B12-2)

Compound B12-1 (328 mg, 1.74 mmol, 1.0 eq) was dissolved in dimethyl sulfoxide (2 mL). Compound B1-1 (400 mg, 1.74 mmol, 1.0 eq) was added to the reaction mixture. The reaction solution was heated to 120°C and stirred at this temperature for 12 hours. The reaction solution was concentrated under reduced pressure, then water (50 mL) was added. The mixture was extracted with ethyl acetate (100 mL × 3), washed with saturated brine (100 mL), and the combined organic layers were dried over anhydrous sodium sulfate. The organic layer was concentrated, and the resulting crude product was purified by reverse phase column chromatography to obtain a yellow solid Compound B12-2 (289 mg, 41.66%).

LCMS (ESI): [M+H]⁺ = 400.42.

### Step 2: (S)-tert-Butyl 10-methoxy-9-nitro-1,2,4,4a,5,6-hexahydro-3H-benzo[b]pyrazino[1,2-d][1,4]oxirane-3-carboxylate (Compound B12-3)

Compound B12-2 (249 mg, 0.623 mmol, 1.0 eq) was dissolved in N,N-dimethylformamide (5 mL). The mixture was cooled to 0°C, and sodium hydride (44.88 mg, 1.87 mmol, 3 eq) was added. The reaction solution was heated to 120°C and stirred at this temperature for 12 hours. The reaction solution was concentrated under reduced pressure, then water (50 mL) was added. The mixture was extracted with ethyl acetate (100 mL × 3), washed with saturated brine (100 mL), and the combined organic layers were dried over anhydrous sodium sulfate. The organic layer was concentrated, and the resulting crude product was purified by reverse phase column chromatography to obtain a yellow solid Compound B12-3 (120 mg, 50.73%).

LCMS (ESI): [M+H]⁺ = 380.41.

### Step 3: (S)-tert-Butyl 9-amino-10-methoxy-1,2,4,4a,5,6-hexahydro-3H-benzo[b]pyrazino[1,2-d][1,4]oxirane-3-carboxylate (Compound B12-4)

Compound B12-3 (120 mg, 0.316 mmol, 1.0 eq) was dissolved in methanol (5 mL). 10% Palladium on carbon (24 mg) was added, and the mixture was stirred at room temperature under hydrogen atmosphere for 12 hours. The reaction mixture was filtered, and the filtrate was concentrated to obtain a crude gray oily Compound B12-4 (136 mg).

LCMS (ESI): [M+H]⁺ = 350.43.

### Step 4: (S)-1-(3-acetyl-10-methoxy-2,3,4,4a,5,6-hexahydro-1H-benzo[b]pyrazino[1,2-d][1,4] oxirane-9-yl)dihydropyrimidine-2,4(1H,3H)-dione (Compound B12)

B12-4 (110 mg, 0.315 mmol, 1.0 eq) was dissolved in toluene (5 mL). Acrylic acid (27.23 mg, 0.378 mmol, 1.2 eq) was added to the reaction mixture. The reaction mixture was heated to 90°C and stirred for 12 hours. Then urea (78.37 mg, 1.30 mmol, 5.0 eq) and acetic acid (5 mL) were added. The mixture was stirred at 120°C for 12 hours, then allowed to cool to room temperature. The pH of the reaction mixture was adjusted to 8 with saturated aqueous sodium bicarbonate solution, and the mixture was extracted with ethyl acetate (50 mL × 3). The combined organic layers were dried over anhydrous sodium sulfate. The organic layer was concentrated, and the resulting crude product was purified by column chromatography to obtain a white solid Compound B12 (20 mg, 16.4%).

¹H NMR (400 MHz, DMSO) δ 10.23 (s, 1H), 6.69 (s, 1H), 6.63 (d, *J* = 1.2 Hz, 1H), 4.18 - 4.05 (m, 3H), 3.74 (t, *J* = 5.7 Hz, 3H), 3.63 (dd, *J* = 15.8, 6.6 Hz, 2H), 3.50 (t, *J =* 6.7 Hz, 3H), 3.30 - 3.24 (m, 2H), 2.68 - 2.58 (m, 2H), 2.07 - 1.86 (m, 6H).

LCMS (ESI): [M+H]⁺ = 389.42.

### Example 30: Synthesis of Compound B13

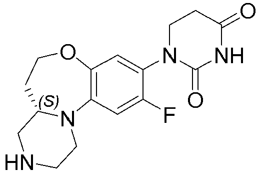

### (S)-1-(10-fluoro-2,3,4,4a,5,6-hexahydro-1H-benzo[b]pyrazino[1,2-d][1,4]oxirane-9-yl)dihydropyrimidine-2,4(1H,3H)-dione

### Synthetic Scheme

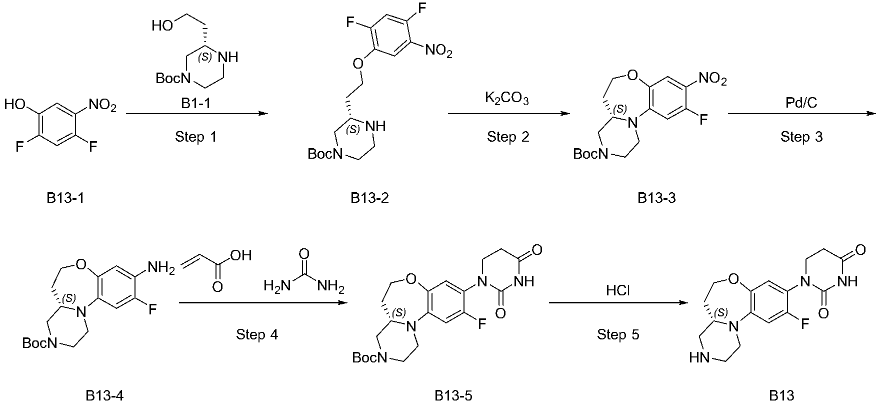

### Step 1: (S)-tert-Butyl 3-(2-(2,4-difluoro-5-nitrophenoxy)ethyl)piperazine-1-carboxylate (Compound B13-2)

Compound B13-1 (5.00 g, 21.71 mmol, 1.0 eq), Compound B1-1 (4.94 g, 28.22 mmol, 1.3 eq) and triphenylphosphine (11.39 g, 43.42 mmol, 2.0 eq) were dissolved in tetrahydrofuran (50 mL) under nitrogen protection. The mixture was cooled to 0°C, and diethyl azodicarboxylate (11.34 g, 65.13 mmol, 3.0 eq) was slowly added. The mixture was allowed to warm to room temperature and stirred overnight. Saturated aqueous ammonium chloride solution (200 mL) was added, and the mixture was extracted with ethyl acetate (250 mL × 3). The organic layers were washed with saturated brine (250 mL), combined and dried over anhydrous sodium sulfate. The organic layer was concentrated, and the resulting crude product was purified by column chromatography to obtain a light yellow solid Compound B13-2 (2.70 g, 32.10%).

LCMS (ESI): [M+H]⁺ = 388.49.

### Step 2: (S)-tert-Butyl 10-fluoro-9-nitro-1,2,4,4a,5,6-hexahydro-3H-benzo[b]pyrazino[1,2-d][1,4] oxirane-3-carboxylate (Compound B13-3)

Compound B13-2 (2.70 g, 6.97 mmol, 1.0 eq) was dissolved in N,N-dimethylformamide (30 mL). Potassium carbonate (1.06 g, 7.67 mmol, 1.1 eq) was added, and the mixture was stirred at room temperature overnight. Saturated aqueous ammonium chloride solution (200 mL) was added, and the mixture was extracted with ethyl acetate (250 mL × 3). The organic layers were washed with saturated brine (250 mL), combined and dried over anhydrous sodium sulfate. The organic layer was concentrated, and the resulting crude product was purified by column chromatography to obtain a light yellow solid Compound B13-3 (1.78 g, 69.52%).

¹H NMR (400 MHz, DMSO-d₆) δ 7.51 (d, *J* = 7.8 Hz, 1H), 6.91 (d, *J* = 14.3 Hz, 1H), 4.30 - 4.14 (m, 2H), 3.91 (dq, *J* = 6.7, 3.5 Hz, 1H), 3.64 (dd, *J* = 13.4, 3.7 Hz, 2H), 3.53 - 3.29 (m, 4H), 2.11 - 1.87 (m, 2H), 1.42 (s, 9H).

LCMS (ESI): [M+H-Boc]⁺ = 268.27.

### Step 3: (S)-tert-Butyl 9-amino-10-fluoro-1,2,4,4a,5,6-hexahydro-3H-benzo[b]pyrazino[1,2-d][1,4] oxirane-3-carboxylate (Compound B13-4)

The synthetic method was the same as in Step 3 of Compound B1 synthesis.

LCMS (ESI): [M+H]⁺ = 338.39.

### Step 4: (S)-tert-Butyl 9-(2,4-dioxotetrahydropyrimidin-1(2H)-yl)-10-fluoro-1,2,4,4a,5,6-hexahydro-3H-benzo[b]pyrazino[1,2-d][1,4]oxirane-3-carboxylate (Compound B13-5)

The synthetic method was the same as in Step 1 of Compound B2 synthesis.

¹H NMR (600 MHz, DMSO-d₆) δ 10.40 (s, 1H), 6.86 (dd, *J* = 13.3, 9.9 Hz, 2H), 4.25 (dq, *J* = 10.7, 3.6 Hz, 1H), 4.15 (ddt, *J* = 16.2, 10.8, 5.5 Hz, 1H), 3.63 (t, *J* = 6.7 Hz, 3H), 3.56 (d, *J* = 13.0 Hz, 1H), 3.32 - 3.25 (m, 2H), 3.16 (q, *J* = 4.2 Hz, 2H), 2.68 (t, *J* = 6.7 Hz, 2H), 2.05 - 1.97 (m, 2H), 1.91 (d, *J* = 10.6 Hz, 1H), 1.43 (s, 9H).

LCMS (ESI): [M+H-t-Bu]⁺ = 379.49.

### Step 5: (S)-1-(10-fluoro-2,3,4,4a,5,6-hexahydro-1H-benzo[b]pyrazino[1,2-d][1,4]oxirane-9-yl)dihydropyrimidine-2,4(1H,3H)-dione (Compound B2)

The synthetic method was the same as in Step 2 of Compound B2 synthesis.

¹H NMR (600 MHz, CD₃OD) δ 6.97 - 6.92 (m, 2H), 4.38 (ddd, *J* = 12.0, 8.7, 3.8 Hz, 1H), 4.19 (dt, *J* = 11.0, 5.3 Hz, 1H), 3.76 (t, *J* = 6.7 Hz, 2H), 3.59 - 3.50 (m, 2H), 3.47 - 3.39 (m, 2H), 3.34 (d, *J =* 12.8 Hz, 2H), 3.31 (dd, *J* = 11.5, 3.9 Hz, 1H), 2.82 (t, *J* = 6.7 Hz, 2H), 2.19 (ddt, *J* = 15.4, 8.9, 4.5 Hz, 1H), 2.00 (dtd, *J* = 15.8, 5.9, 3.9 Hz, 1H).

LCMS (ESI): [M+H]⁺ = 335.30.

### Example 30-1: Synthesis of Compound B14

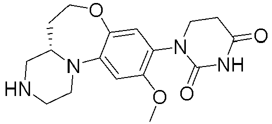

### (S)-1-(10-methoxy-2,3,4,4a,5,6-hexahydro-1H-benzo[b]pyrazino[1,2-d][1,4]oxazepin-9-yl)dihydropyrimidine-2,4(1H,3H)-dione

### Synthetic Scheme

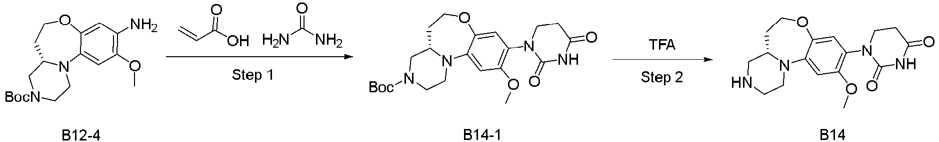

### Step 1: (S)-1-(3-acetyl-10-methoxy-2,3,4,4a,5,6-hexahydro-1H-benzo[b]pyrazino[1,2-d][1,4]oxazepin-9-yl)dihydropyrimidine-2,4(1H,3H)-dione (Compound B14-1)

B12-4 (110 mg, 0.315 mmol, 1.0 eq) was dissolved in toluene (5 mL). Acrylic acid (27.23 mg, 0.378 mmol, 1.2 eq) was added to the reaction mixture. The reaction mixture was heated to 90°C and stirred for 12 hours. Then urea (78.37 mg, 1.30 mmol, 5.0 eq) and acetic acid (5 mL) were added. The mixture was stirred at 90°C for 12 hours, then allowed to cool to room temperature. The pH of the reaction mixture was adjusted to 8 with saturated aqueous sodium bicarbonate solution, and the mixture was extracted with ethyl acetate (50 mL × 3). The combined organic layers were dried over anhydrous sodium sulfate. The organic layer was concentrated, and the resultling crude product was purified by column chromatography to obtain a white solid Compound B14-1 (20 mg, 16.4%).

### Step 2: (S)-1-(10-methoxy-2,3,4,4a,5,6-hexahydro-1H-benzo[b]pyrazino[1,2-d][1,4]oxazepin-9-yl)dihydropyrimidine-2,4(1H,3H)-dione (Compound B14)

B14-1 (20 mg, 0.045 mmol, 1.0 eq) was dissolved in dichloromethane (2 mL). Trifluoroacetic acid (1 mL) was added, and the mixture was stirred at room temperature for 2 hours. The reaction solution was concentrated, and the resulting crude product was purified by reverse phase column chromatography to obtain a white solid Compound B14 (8 mg, 51.6%).

¹H NMR (400 MHz, DMSO-d₆) δ 10.25 (s, 1H), 6.75 (s, 1H), 6.69 (s, 1H), 4.20 (ddd, *J* = 12.1, 7.9, 4.4 Hz, 1H), 4.07 (dt, *J* = 11.0, 5.4 Hz, 1H), 3.75 (s, 3H), 3.37-3.31 (m, 4H), 3.26 - 3.11 (m, 3H), 2.99-3.07 (m, 2H), 2.63 (t, *J* = 6.7 Hz, 2H), 2.06-1.91 (m, 2H).

LCMS (ESI): [M+H]⁺ = 346.39.

### Example 30-2: Synthesis of Compound B15

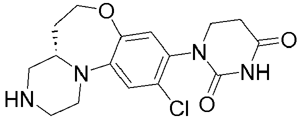

### (S)-1-(10-chloro-2,3,4,4a,5,6-hexahydro-1H-benzo[b]pyrazino[1,2-d][1,4]oxazepin-9-yl)dihydropyrimidine-2,4(1H,3H)-dione

### Synthetic Scheme

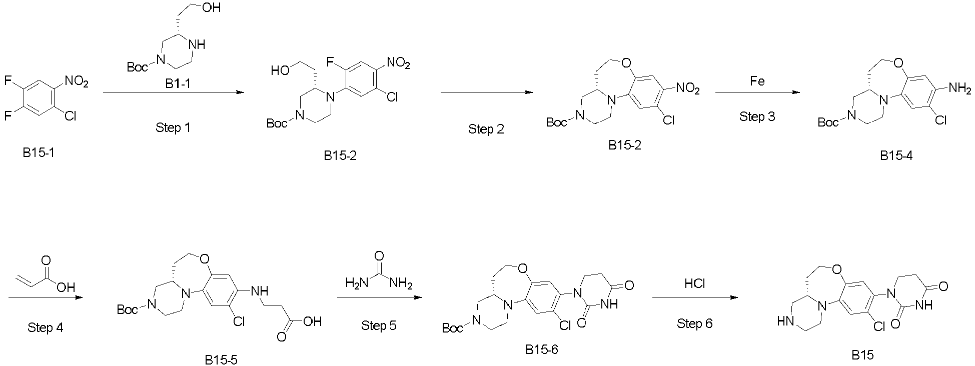

### Step 1: (S)-tert-Butyl 4-(5-chloro-2-fluoro-4-nitrophenyl)-3-(2-hydroxyethyl)piperazine-1-carboxylate (Compound B15-2)

Compound B1-1 (500.0 mg, 2.17 mmol, 1.0 eq) and Compound B15-1 (420.16 mg, 2.17 mmol, 1.0 eq) were dissolved in dimethyl sulfoxide (5.0 mL). The mixture was heated to 120°C and stirred overnight. The reaction solution was purified by reverse phase column chromatography to obtain a light yellow solid Compound B15-2 (277 mg, 31.59%).

¹H NMR (400 MHz, DMSO-d₆) δ 8.09 - 7.98 (m, 1H), 7.33 - 7.21 (m, 1H), 4.13 - 3.85 (m, 3H), 3.45 - 2.87 (m, 7H), 1.77 - 1.52 (m, 2H), 1.42 (s, 9H).

LCMS (ESI): [M+H]⁺ = 404.35.

### Step 2: (S)-tert-Butyl 10-chloro-9-nitro-1,2,4,4a,5,6-hexahydro-3H-benzo[b]pyrazino[1,2-d][1,4]oxazepine-3-carboxylate (Compound B15-3)

Compound B15-2 (500.0 mg, 1.24 mmol, 1.0 eq) was dissolved in N,N-dimethylformamide (5.0 mL). The mixture was cooled to 0°C, and sodium hydride (148.56 mg, 3.71 mmol, 3.0 eq) was added. The mixture was stirred at room temperature under nitrogen protection for 2 hours. The reaction mixture was cooled to 0°C, quenched with saturated ammonium chloride solution, and diluted with ethyl acetate (100 mL). The organic layer was washed with saturated brine (60 mL × 3), and the combined organic layers were dried over anhydrous sodium sulfate. The organic layer was concentrated, and the resulting crude product was purified by column chromatography to obtain a yellow solid Compound B15-3 (430.0 mg, 90.48%).

LCMS (ESI): [M+H-56]⁺= 328.35.

### Step 3: (S)-tert-Butyl 9-amino-10-chloro-1,2,4,4a,5,6-hexahydro-3H-benzo[b]pyrazino[1,2-d][1,4]oxazepine-3-carboxylate (Compound B15-4)

Compound B15-3 (300.0 mg, 781.60 µmol, 1.0 eq) was dissolved in ethanol (8.0 mL). A solution of ammonium chloride (418.07 mg, 7.82 mmol, 10.0 eq) in water (2.0 mL) and iron powder (218.24 mg, 3.91 mmol, 5.0 eq) were added. The mixture was heated to 80°C under nitrogen protection and stirred overnight. The mixture was quickly filtered, and the filtrate was diluted with water (100 mL) and extracted with ethyl acetate (100 mL × 2). The organic layers were washed with saturated brine (80 mL), combined and dried over anhydrous sodium sulfate. The organic layer was concentrated to obtain a crude yellow solid Compound B15-4 (275 mg, 99.43%), which was used directly in the next step.

LCMS (ESI): [M+H]⁺ = 353.39.

### Step 4: (S)-3-((3-(tert-Butoxycarbonyl)-10-chloro-2,3,4,4a,5,6-hexahydro-1H-benzo[b]pyrazino[1,2-d][1,4]oxazepin-9-yl)amino)propanoic acid (Compound B15-5)

B15-4 (275 mg, 777.17 µmol, 1.0 eq) was dissolved in toluene (5.0 mL). Acrylic acid (168.02 mg, 2.33 mmol, 3.0 eq) was added to the reaction mixture. The mixture was heated to 90°C and stirred for 24 hours. The reaction mixture was cooled to room temperature, and the organic layer was concentrated to obtain a crude black oily Compound B15-5 (331.0 mg, 100%), which was used directly in the next step.

LCMS (ESI): [M+H]⁺ = 426.35.

### Step 5: (S)-tert-Butyl 10-chloro-9-(2,4-dioxotetrahydropyrimidin-1(2H)-yl)-1,2,4,4a,5,6-hexahydro-3H-benzo [b]pyrazino[1,2-d] [1,4]oxazepine-3-carboxylate (Compound B15-6)

B15-5 (331 mg, 777.16 µmol, 1.0 eq) was dissolved in toluene (5.0 mL). Urea (466.73 mg, 7.77 mmol, 10.0 eq) and formic acid (3.0 mL) were added. The mixture was heated to 110°C and stirred for 24 hours. The reaction mixture was cooled to room temperature. Water (100 mL) and sodium bicarbonate were added to neutralize the mixture to weakly basic conditions. The mixture was extracted with ethyl acetate (100 mL × 2). The organic layers were washed with saturated brine (100 mL), combined and dried over anhydrous sodium sulfate. The organic layer was concentrated, and the resulting crude product was purified by column chromatography to obtain an off-white solid Compound B15-6 (48 mg, 13.70%).

LCMS (ESI): [M+H]⁺ = 451.29.

### Step 6: (S)-1-(10-chloro-2,3,4,4a,5,6-hexahydro-1H-benzo[b]pyrazino[1,2-d][1,4]oxazepin-9-yl)dihydropyrimidine-2,4(1H,3H)-dione (Compound B15)

Compound B15-6 (48 mg, 106.45 µmol, 1.0 eq) was dissolved in dichloromethane (2.0 mL). A 4 M solution of hydrogen chloride in 1,4-dioxane (1.0 mL) was added. The mixture was stirred at room temperature for 3 hours. The reaction mixture was concentrated under reduced pressure to obtain an off-white solid Compound B15 (35 mg, 93.73%).

¹H NMR (400 MHz, DMSO-d₆) δ 10.41 (s, 1H), 9.27 (s, 1H), 7.16 (s, 1H), 6.97 (s, 1H), 4.46 - 4.31 (m, 1H), 4.22 - 4.07 (m, 1H), 3.76 - 3.42 (m, 4H), 3.34 - 3.25 (m, 2H), 3.23 - 2.96 (m, 3H), 2.78 - 2.61 (m, 2H), 2.15 - 1.90 (m, 2H).

LCMS (ESI): [M+H]⁺ = 351.25.

### Test Example 1: Binding activity of the E3 ligase inhibitor disclosed in the present invention to E3 ubiquitin ligase CRBN

The binding activity of the E3 ligase inhibitors disclosed in the present invention and the positive control samples lenalidomide and pomalidomide with the E3 ubiquitin ligase CRBN were determined by the competitive binding of the E3 ligase inhibitor disclosed in the present invention, lenalidomide and pomalidomide with thalidomide labeled with XL665 to the CRBN protein, thereby preventing the occurrence of fluorescence resonance energy transfer (FRET), and thus determining the binding affinity of the compound to the CRBN protein.

CRBN binding activity test kit (CEREBLON BINDING KITS (PE, 64BD CRBN PEG)).

The detection is based on homogeneous time-resolved fluorescence (HTRF) technology. When the donor (Donor) and acceptor (Acceptor) are close to each other, the donor can transfer energy to the acceptor, exciting it to emit light at 665nm.

This kit uses a europium-labeled GST antibody (GST Eu Cryptate Antibody) as the donor and XL665-labeled thalidomide (Thalidomide-Red reagent) as the receptor. The donor binds to the GST-tagged CRBN protein. The E3 ligase inhibitors disclosed herein, as well as lenalidomide and pomalidomide, compete with thalidomide for binding to the CRBN protein. The binding activity of the compound is determined by the fluorescence emitted by the receptor at 665 nm. The stronger the binding affinity of the compounds, the weaker the signal.

The donor and acceptor were each diluted 50-fold using the buffer (PROTAC binding buffer 1) in the kit. The CRBN protein was diluted 45-fold. The 8 mM compound solution disclosed in the present invention was diluted 10-fold using the diluent (1 X diluent) in the kit, and then the dilutd solution was subjected to 5-fold gradient dilution of the diluent, diluted 7 times sequentially. 5 µl of compound, 5 µl of diluted CRBN protein, and 10 µl of an equal volume mixture of donor and acceptor were added to the wells of a white 384-well plate (PE, Part number: 6008280). The mixture was incubated at room temperature for 3 hours. The ratio of the acceptor and donor emission signals for each individual well was calculated. $Ratio = 665 nm signal value / 620 nm signal {value * 10}^{4}$ $Calculate the coefficient of variation \left(CV\right) = standard deviation \left(SD\right) / Ratio mean * 100 ;$

IC₅₀ value was calculated based on compound concentration, Ratio, and coefficient of variation using GraphPad Prism.

**Table 1: shows the IC₅₀ values of compounds of Examples 1 to 32 of the present disclosure.**

| Compounds | IC₅₀ (µM) | Compounds | IC₅₀ (µM) |
|---|---|---|---|
| A1 | 25.66 | B1 | 5.84 |
| A2 | 1.53 | B2 | 6.11 |
| A3 | 0.59 | B3 | 5.67 |
| A4 | 0.55 | B4 | 5.49 |
| A5 | 3.06 | B5 | 10.18 |
| A6 | 7.47 | B6 | 24.30 |
| A7 | 12.65 | B7 | 3.81 |
| A8 | 9.50 | B8 | 10.00 |
| A9 | 11.45 | B9 | 8.59 |
| A10 | 0.91 | B10 | 3.34 |
| A11 | 6.15 | B11 | 3.76 |
| A12 | 13.34 | B12 | 2.26 |
| A13 | 28.84 | B13 | 4.45 |
| A14 | 8.36 | B14 | 5.40 |
| A15 | 9.08 | B15 | 3.70 |
| A16 | 12.58 | Lenalidomide | 3.10 |
| A17 | 1.73 | Pomalidomide | 1.30 |

Conclusion: The E3 ligase inhibitors disclosed in the present invention can bind well to the CRBN protein, and the binding ability of some E3 ligase inhibitors to CRBN is comparable to or even or superior to that of the positive controls lenalidomide and pomalidomide.

The present invention uses the above-mentioned compounds to exemplify the synthesis of protein-targeted chimera (PROTAC) compounds targeting androgen receptor, estrogen receptor, and IRAK4 receptor to verify the excellent effects of the compounds provided by the present invention.
**The first part is PROTAC compounds targeting androgen receptor (AR);**
AR-PROTAC compounds were synthesized by the following general synthetic formulas:
PROTAC general synthetic formula AR-T-1: 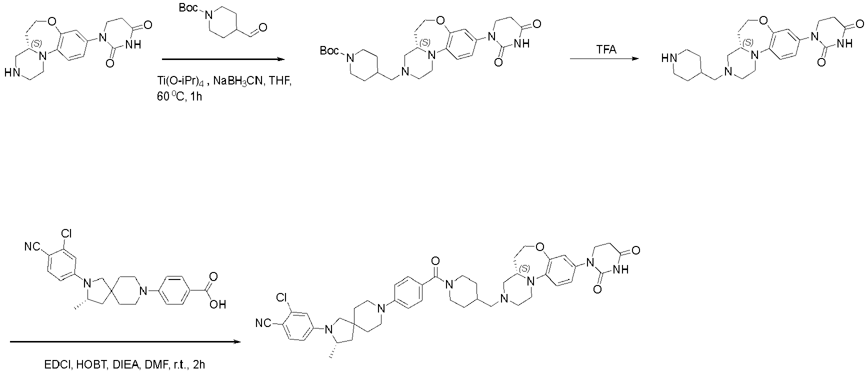
PROTAC general synthetic formula AR-T-2 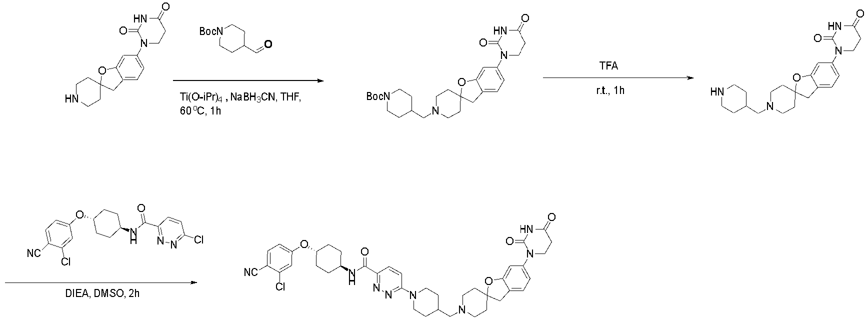
PROTAC general synthetic formula AR-T-3 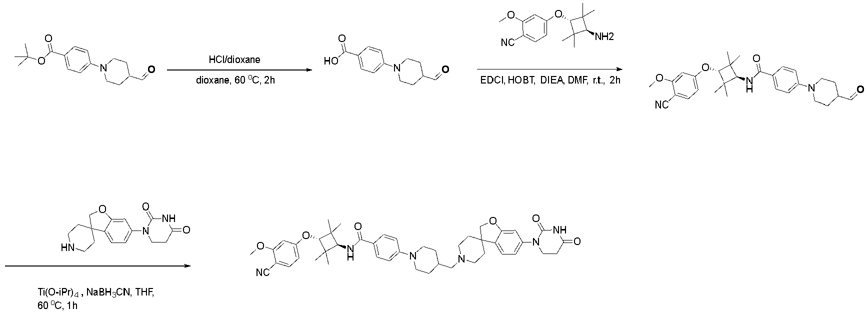
PROTAC general synthetic formula AR-T-4

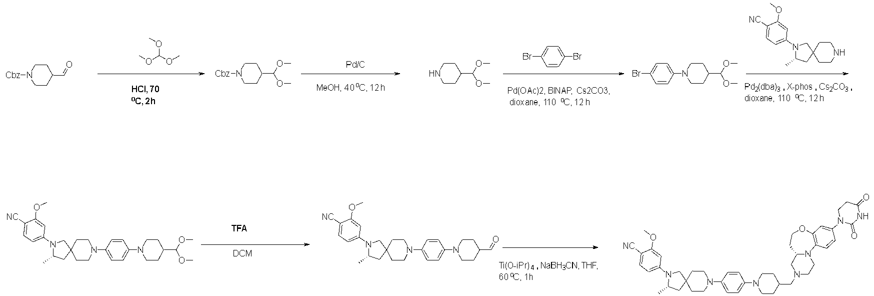

**Table A: The following compounds of Examples 31 to 43 were synthesized according to the general synthetic formulas above:**

| Examples | Compounds | Structure and Name | ¹H-NMR | LC-MS | Synthetic method |
|---|---|---|---|---|---|
| 31 | A-P-77 | 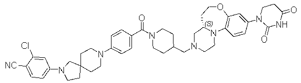 4-((S)-8-(4-(4-(S)-9-(2,4-dioxotetrahydropyrimidin-1(2H)-yl)-1,2,4,4a,5,6-hexahydro-3H-benzo[b]pyrazino[1,2-d][1,4]oxazepin-3-yl)methyl)piperidine-1-carbonyl)phenyl)-3-methyl-2,8-diazaspiro[4.5]decane-2-yl)-2-chloro-benzonitrile | ¹H NMR (600 MHz, DMSO) δ 10.34 (s, 1H), 7.61 (d, *J* = 8.9 Hz, 1H), 7.27 (d, *J* = 8.7 Hz, 2H), 6.97 (ddd, *J* = 12.8, 11.0, 5.5 Hz, 4H), 6.82 (dd, *J* = 10.4, 2.3 Hz, 2H), 6.67 (dd, *J* = 9.0, 2.3 Hz, 1H), 4.52 (t, *J* = 9.8 Hz, 1H), 4.15 - 4.08 (m, 2H), 4.05 (dd, *J* = 13.4, 6.6 Hz, 2H), 3.74 - 3.68 (m, 4H), 3.60 (d, *J* = 11.2 Hz, 1H), 3.51 (d, *J* = 10.8 Hz, 1H), 3.45 (d, *J* = 10.8 Hz, 1H), 3.41 - 3.29 (m, 7H), 3.22 (ddd, *J* = 20.7, 8.1, 4.5 Hz, 3H), 3.18 - 3.05 (m, 4H), 2.68 (t, *J* = 6.7 Hz, 2H), 2.25 (dd, *J* = 12.7, 7.7 Hz, 1H), 2.14 (d, *J* = 7.2 Hz, 2H), 1.85 - 1.69 (m, 5H), 1.59 (dd, *J* = 12.8, 6.5 Hz, 1H), 1.51 (dd, *J* = 10.3, 6.3 Hz, 2H), 1.21 (d, *J* = 6.1 Hz, 3H). | 805.56 | ART-1 |
| 32 | A-P-83 | 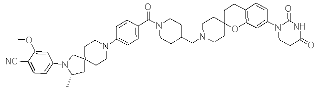 (S)-4-(8-(4-(4-((7-(2,4-dioxotetrahydropyrimidin-1(2H)-yl)spiro[chroman-2,4'-piperidine]-1'-yl)methyl)piperidine-1-carbonyl)phenyl)-3-methyl-2,8-diazaspiro[4.5]dec-2-yl)-2-methoxybenzonitrile | ¹H NMR (600 MHz, DMSO-*d₆*) δ 10.34 (s, 1H), 7.37 (d, *J* = 8.7 Hz, 1H), 7.25 (d, *J* = 8.5 Hz, 2H), 7.09 (d, *J* = 7.4 Hz, 1H), 6.96 (d, *J* = 8.7 Hz, 2H), 6.80 (d, *J* = 6.4 Hz, 1H), 6.27 (dd, *J* = 8.8, 1.6 Hz, 1H), 6.19 (s, 1H), 4.66 - 4.08 (m, 3H), 4.08 - 4.01 (m, 1H), 3.87 (s, 3H), 3.79 - 3.67 (m, 2H), 3.43 (d, *J* = 10.6 Hz, 2H), 3.37 (s, 1H), 3.32 (s, 1H), 3.24 (ddd, *J* = 20.3, 10.5, 5.7 Hz, 2H), 2.90 (s, 4H), 2.69 (dd, *J* = 22.5, 15.9 Hz, 5H), 2.44 - 2.11 (m, 4H), 2.07 - 1.61 (m, 11H), 1.58 (dd, *J* = 12.8, 6.5 Hz, 1H), 1.55 - 1.43 (m, 2H), 1.23 (d, *J* = 6.0 Hz, 3H), 1.12 (s, 2H). | 800.76 | ART-1 |
| 33 | A-P-195 | 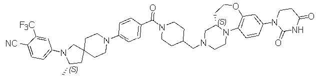 4-((S)-8-(4-(4-(S)-9-(2,4-dioxotetrahydropyrimidin-1(2H)-yl)-1,2,4,4a,5,6-hexahydro-3H-benzo[b]pyrazino[1,2-d][1,4]oxazepin-3-yl)methyl)piperidine-1-carbonyl)phenyl)-3-methyl-2,8-diazaspiro[4.5]decane-2-yl)-2-(trifluoromethyl)benzonitrile | ¹H NMR (600 MHz, CD₃OD) δ 7.68 (d, *J* = 8.7 Hz, 1H), 7.38 (d, *J* = 8.7 Hz, 2H), 7.09 (d, *J* = 8.7 Hz, 2H), 7.04 (q, *J* = 8.6 Hz, 2H), 6.98 (d, *J* = 2.5 Hz, 1H), 6.91 (dd, *J* = 8.8, 2.4 Hz, 2H), 4.57 (t, *J* = 10.0 Hz, 1H), 4.20 (d, *J* = 8.5 Hz, 1H), 4.15 (q, *J* = 6.7 Hz, 1H), 3.83 (t, *J* = 6.7 Hz, 2H), 3.72 (d, *J* = 12.2 Hz, 1H), 3.57 (d, *J* = 11.9 Hz, 1H), 3.53 - 3.47 (m, 3H), 3.47 - 3.42 (m, 3H), 3.41 - 3.34 (m, 3H), 3.31 - 3.26 (m, 1H), 3.23 - 3.19 (m, 2H), 3.17 (d, *J* = 11.8 Hz, 1H), 2.81 (t, *J* = 6.7 Hz, 2H), 2.41 (dd, *J* = 12.9, 7.6 Hz, 1H), 2.33 - 2.19 (m, 3H), 1.97 - 1.84 (m, 5H), 1.75 - 1.61 (m, 4H), 1.39 - 1.35 (m, 4H), 1.33 (d, *J* = 6.1 Hz, 3H). | 839.76 | ART-1 |
| 34 | A-P-196 | 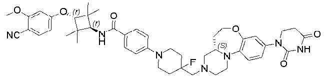 N-((1r,3r)-3-(4-cyano-3-methoxyphenoxy)-2,2,4,4-tetramethylcyclobutyl)-4-(4-((S)-9-(2,4-dioxotetrahydropyrimidin-1(2H)-yl)-1,2,4,4a,5,6-hexahydro-3H-benzo[b]pyrazino[1,2-d][1,4]oxirane-3-yl)methyl)-4-fluoropiperidin-1-yl)benzamide | ¹H NMR (400 MHz,CD₃OD) δ 7.80 (d, J = 8.7 Hz, 2H), 7.55 (d, J = 8.6 Hz, 1H), 7.13 - 7.00 (m, 4H), 6.92 (d, J = 2.3 Hz, 1H), 6.68 - 6.55 (m, 2H), 4.55 (d, J = 10.5 Hz, 1H), 4.32 - 4.13 (m, 3H), 3.95 (s, 3H), 3.93 - 3.74 (m, 5H), 3.71 - 3.43 (m, 7H), 3.25 (t, J = 12.4 Hz, 2H), 2.81 (t, J = 6.7 Hz, 2H), 2.21 (dt, J = 23.2, 11.6 Hz, 3H), 2.12 - 1.81 (m, 3H), 1.31 (s, 7H), 1.26 (s, 6H). | 808.55 | ART-3 |
| 35 | A-P-197 | 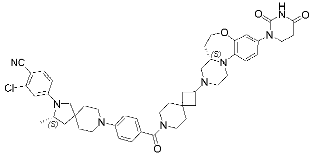 2-Chloro-4-((S)-8-(4-(2-(S)-9-(2,4-dioxotetrahydropyrimidin-1(2H)-yl)-1,2,4,4a,5,6-hexahydro-3H-benzo[b]pyrazino[1,2-d][1,4]oxirane-3-yl)-7-azaspiro[3.5]non-7-enoyl)phenyl)-3-methyl-2,8-diazaspiro[4.5]dec-2-yl)benzonitrile | ¹H NMR (600 MHz, CD₃OD) δ 7.51 (d, J = 8.8 Hz, 1H), 7.44 - 7.36 (m, 2H), 7.17 (d, J = 8.5 Hz, 2H), 7.10 - 7.01 (m, 2H), 6.92 (d, J = 2.2 Hz, 1H), 6.79 (d, J = 2.3 Hz, 1H), 6.68 (dd, J = 8.8, 2.4 Hz, 1H), 4.54 (t, J = 10.0 Hz, 1H), 4.25 - 4.01 (m, 2H), 3.83 (t, J = 6.7 Hz, 3H), 3.72 - 3.57 (m, 3H), 3.56 - 3.43 (m, 6H), 3.42 - 3.36 (m, 5H), 3.04 (dt, J = 54.1, 11.6 Hz, 2H), 2.81 (t, J = 6.7 Hz, 2H), 2.51 - 2.35 (m, 3H), 2.30 - 2.19 (m, 1H), 2.09 (d, J = 38.1 Hz, 2H), 1.99 - 1.85 (m, 3H), 1.70 (dt, J = 12.8, 8.2 Hz, 7H), 1.31 (d, J = 5.9 Hz, 5H). | 831.56 | ART-1 |
| 36 | A-P-198 | 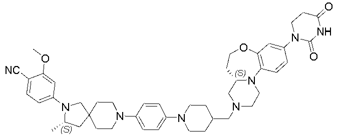 4-(S)-8-(4-((S)-9-(2,4-dioxotetrahydropyrimidin-1(2H)-yl)-1,2,4-,4a,5,6-hexahydro-3H-benzo[b]pyrazino[1,2-d][1,4]oxirane-3-yl)methyl)piperidin-1-yl)phenyl)-3-methyl-2,8-diazaspiro[4.5]dec-2-yl)-2-methoxybenzonitrile | ¹H NMR (400 MHz, CD₃OD) δ 7.46 (d, *J* = 8.8 Hz, 2H), 7.35 (d, *J* = 8.7 Hz, 1H), 7.23 (d, *J* = 9.1 Hz, 2H), 7.09 - 6.99 (m, 2H), 6.92 (d, *J* = 2.4 Hz, 1H), 6.34 (dd, *J* = 8.8, 2.1 Hz, 1H), 6.25 (d, *J* = 2.1 Hz, 1H), 4.57 (s, 1H), 4.25 - 4.09 (m, 2H), 3.94 (s, 3H), 3.90 - 3.79 (m, 4H), 3.59 (dd, *J* = 10.6, 6.0 Hz, 6H), 3.50 (d,*J* = 10.5 Hz, 4H), 3.24 (d, *J* = 7.0 Hz, 2H), 3.06 (q, *J* = 12.2 Hz, 2H), 2.81 (t, *J* = 6.7 Hz, 2H), 2.46 (t, *J* = 10.5 Hz, 1H), 2.32 - 1.99 (m, 7H), 1.97 - 1.83 (m, 3H), 1.77 (dd, *J* = 13.0, 6.9 Hz, 1H), 1.59 (q, *J* = 11.6 Hz, 2H), 1.39 - 1.29 (m, 5H). | 773.66 | ART-4 |
| 37 | A-P-208 | 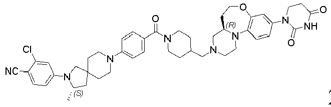 2-Chloro-4-((S)-8-(4-((R)-9-(2,4-dioxotetrahydropyrimidin-1(2H)-yl)-1,2,4,4a,5,6-hexahydro-3H-benzo[b]pyrazino[1,2-d][1,4]oxazin-3-yl)methyl)piperidine-1-carbonyl)phenyl)-3-methyl-2,8-diazaspiro[4.5]decane-2-yl)benzonitrile | ¹H NMR (600 MHz, DMSO-*d*₆) δ 10.32 (s, 1H), 7.61 (d, *J* = 8.9 Hz, 1H), 7.25 (d, *J* = 8.6 Hz, 2H), 6.96 (d, *J* = 8.7 Hz, 3H), 6.90 (s, 1H), 6.81 (d, *J* = 2.2 Hz, 1H), 6.78 (s, 1H), 6.66 (dd, *J* = 9.0, 2.2 Hz, 1H), 4.53 - 4.18 (m, 2H), 4.17 - 4.00 (m, 3H), 3.75 - 3.64 (m, 2H), 3.44 (d*, J* = 10.8 Hz, 1H), 3.40 - 3.36 (m, 1H), 3.32 (d, *J* = 10.8 Hz, 2H), 3.26 - 3.04 (m, 5H), 2.87 (d, *J* = 68.7 Hz, 3H), 2.67 (t, *J* = 6.7 Hz, 2H), 2.43 - 2.15 (m, 3H), 2.09 (d, *J* = 28.8 Hz, 2H), 1.98 - 1.84 (m, 2H), 1.84 - 1.66 (m, 4H), 1.58 (dd, *J* = 12.8, 6.5 Hz, 1H), 1.54 - 1.44 (m, 2H), 1.45 - 1.31 (m, 1H), 1.29 - 1.02 (m, 6H). | 805.66 | ART-1 |
| 38 | A-P-221 | 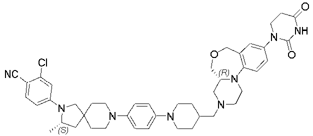 2-Chloro-4-((S)-8-(4-((R)-9-(2,4-dioxotetrahydropyrimidin-1(2H)-yl)-1,2,4a,5-tetrahydro-7H-benzo[e]pyrazino[2,1-c][1,4]oxirane-3(4H)-yl)methyl)piperidin-1-yl)phenyl)-3-methyl-2,8-diazaspiro[4.5]dec-2-yl)benzonitrile | ¹H NMR (600 MHz, DMSO-*d*₆) δ 10.38 (s, 1H), 7.63 (d, *J* = 8.8 Hz, 1H), 7.58 - 7.09 (m, 6H), 7.04 (d*, J* = 8.5 Hz, 1H), 6.82 (d, *J* = 2.3 Hz, 1H), 6.68 (dd, *J* = 8.9, 2.3 Hz, 1H), 4.95 (d, *J* = 11.3 Hz, 1H), 4.60 (d, *J* = 11.3 Hz, 1H), 4.06 (q, *J* = 6.6 Hz, 1H), 3.76 - 3.73 (m, 5H), 3.55 *(d, J* = 13.2 Hz, 3H), 3.50 (d, *J* = 12.5 Hz, 3H), 3.46 - 3.34 (m, 7H), 3.30 (d, *J* = 11.1 Hz, 2H), 3.20 (s, 3H), 3.13 (d, *J* = 11.2 Hz, 1H), 2.71 (t, *J* = 6.7 Hz, 2H), 2.15 (s, 2H), 2.04 - 1.80 (m, 4H), 1.65 (s, 2H), 1.43 (s, 2H), 1.22 (d, *J* = 6.0 Hz, 3H). | 777.56 | ART-4 |
| 39 | A-P-225 | 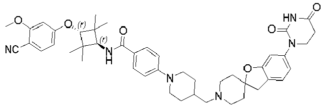 N-((1r,3r)-3-(4-cyano-3-methoxyphenoxy)-2,2,4,4-tetramethylcyclobutyl)-4-(4-((6-(2,4-dioxotetrahydropyrimidin-1(2H)-yl)-3H-spiro[benzofuran-2,4'-piperidine]-1'-yl)methyl)piperidin-1-yl)benzamide | ¹H NMR (600 MHz, DMSO-*d*₆) δ 10.37 (s, 1H), 7.77 (d, *J* = 8.6 Hz, 2H), 7.66 (d, *J* = 8.6 Hz, 1H), 7.55 (d, *J* = 9.2 Hz, 1H), 7.23 (d, *J* = 7.9 Hz, 1H), 7.00 (d, *J* = 8.6 Hz, 2H), 6.81 (d, *J* = 7.9 Hz, 1H), 6.78 - 6.75 (m, 1H), 6.64 (d, *J* = 1.9 Hz, 1H), 6.54 (dd, *J* = 8.6, 1.9 Hz, 1H), 4.28 (s, 1H), 4.06 (d, *J* = 9.2 Hz, 1H), 3.91 (s, 3H), 3.90 (s, 1H), 3.75 - 3.71 (m, 2H), 3.53 (dd, *J* = 28.8, 11.1 Hz, 2H), 3.18 (s, 3H), 3.13 (s, 2H), 3.10 (s, 2H), 2.82 (t, *J* = 11.7 Hz, 2H), 2.69 (t, *J* = 6.6 Hz, 2H), 2.11 (s, 5H), 1.84 (d, *J* = 11.7 Hz, 2H), 1.34 - 1.27 (m, 2H), 1.23 (s, 6H), 1.15 (s, 6H). | 775.66 | ART-3 |
| 40 | A-P-230 | 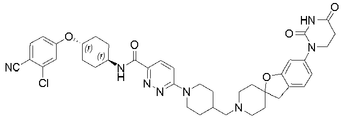 N-((1r,4r)-4-(3-chloro-4-cyanophenoxy)cyclohexyl)-6-(4-((6-(2,4-dioxotetrahydro-pyrimidin- 1(2H)-yl)-3H-spiro[benzofuran-2,4'-piperidine]-1'-yl)methyl)piperidin-1-yl)pyridazin-3-carboxamide | ¹H NMR (600 MHz, DMSO-*d*₆) δ 10.37 (s, 1H), 8.62 (d, J = 8.1 Hz, 1H), 7.91 - 7.80 (m, 2H), 7.46 - 7.36 (m, 2H), 7.23 (dd, J = 8.1, 4.3 Hz, 1H), 7.14 (dd, J = 8.8, 2.4 Hz, 1H), 6.84 - 6.73 (m, 2H), 4.54 (tt, J = 10.6, 4.1 Hz, 3H), 3.89 - 3.84 (m, 1H), 3.76 - 3.71 (m, 2H), 3.24 - 3.02 (m, 8H), 2.69 (t, J = 6.7 Hz, 2H), 2.27 - 2.18 (m, 1H), 2.15 - 2.06 (m, 5H), 2.07 - 1.96 (m, 1H), 1.89 (ddd, J = 22.3, 11.2, 7.2 Hz, 4H), 1.64 (qd, J = 13.2, 3.1 Hz, 2H), 1.56 - 1.47 (m, 2H), 1.24 (dtd, J = 16.6, 12.1, 11.6, 7.1 Hz, 4H). | 753.47 | ART-2 |
| 41 | A-P-233 | 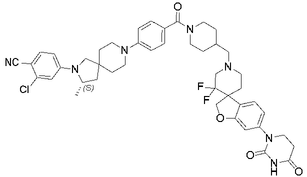 2-Chloro-4-((3S)-8-(4-((6-(2,4-dioxotetrahydropyrimidin-1(2H)-yl)-3',3'-difluoro-2H-spiro[benzofuran-3,4'-piperidine]-1'-yl)methyl)piperidine-1-carbonyl)phenyl)-3-methyl-2,8-diazaspiro[4.5]dec-2-yl)benzonitrile | ¹H NMR (600 MHz, DMSO-*d*₆) δ 10.39 (s, 1H), 7.61 (d, *J* = 8.9 Hz, 1H), 7.35 - 7.24 (m, 3H), 6.99 (d,*J* = 8.5 Hz, 2H), 6.90 (d, *J* = 8.2 Hz, 1H), 6.87 (s, 1H), 6.81 (d, *J* = 2.3 Hz, 1H), 6.67 (dd, *J* = 9.0, 2.3 Hz, 1H), 4.80 (d, *J* = 9.9 Hz, 1H), 4.52 (d, *J* = 8.3 Hz, 1H), 4.28 - 3.86 (m, 4H), 3.77 (t, *J* = 6.7 Hz, 2H), 3.45 (d, *J* = 10.8 Hz, 1H), 3.42 - 3.15 (m, 7H), 2.92 (s, 4H), 2.77 - 2.63 (m, 3H), 2.44 - 2.31 (m, 1H), 2.25 (dd, *J* = 12.7, 7.7 Hz, 1H), 2.07 (d, *J* = 16.3 Hz, 2H), 1.82 - 1.65 (m, 4H), 1.64 - 1.56 (m, 1H), 1.56 - 1.46 (m, 2H), 1.25 - 1.08 (m, 5H). | 826.56 | ART-1 |
| 42 | A-P-235 | 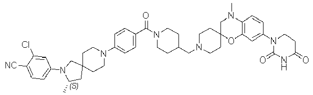 (S)-2-chloro-4-(8-(4-(4-((7-(2,4-dioxotetrahydropyrimidin-1(2H)-yl)-4-methyl-3,4-dihydrospiro[benzo[b][1,4]oxazin-2,4'-piperidine]-1'-yl)methyl)piperidine-1-carbonyl)phenyl)-3-methyl-2,8-diazaspiro[4.5]decane-2-yl)benzonitrile | ¹H NMR (600 MHz, CD₃OD) δ 7.51 (d, *J* = 8.8 Hz, 1H), 7.38 (d,*J* = 8.7 Hz, 2H), 7.10 (d, *J* = 8.5 Hz, 2H), 6.90 - 6.86 (m, 2H), 6.81 (d, *J* = 8.5 Hz, 1H), 6.78 (d, *J* = 2.3 Hz, 1H), 6.67 (dd, *J* = 8.9, 2.4 Hz, 1H), 4.08 (h, *J* = 6.4 Hz, 1H), 3.80 (t, *J* = 6.8 Hz, 2H), 3.59 (d, *J* = 12.4 Hz, 2H), 3.47 (d, *J* = 10.4 Hz, 1H), 3.44 (dd, *J* = 5.0, 2.2 Hz, 2H), 3.39 (d, *J* = 10.5 Hz, 1H), 3.38 - 3.34 (m, 2H), 3.31 (s, 2H), 3.28 - 3.21 (m, 1H), 3.18 (d, *J* = 7.0 Hz, 2H), 3.14 (s, 2H), 2.96 (s, 3H), 2.80 (t, *J* = 6.7 Hz, 2H), 2.38 (dd, *J* = 12.9, 7.6 Hz, 1H), 2.31 - 2.24 (m, 1H), 2.23 - 2.18 (m, 1H), 2.13 (d, *J* = 14.8 Hz, 2H), 2.00 (td, *J* = 14.4, 4.4 Hz, 2H), 1.93 - 1.83 (m, 4H), 1.72 - 1.61 (m, 4H), 1.39 - 1.33 (m, 3H), 1.31 *(d, J* = 6.0 Hz, 3H). | 819.56 | ART-1 |
| 43 | A-P-265 | 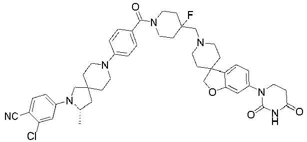 (S)-2-chloro-4-(8-(4-(4-((6-(2,4-dioxotetrahydropyrimidin-1(2H)-yl)-2H-spiro[benzofuran-3,4'-piperidine]-1'-yl)methyl)-4-fluoropiperidine-1-carbonyl)phenyl)-3-methyl-2,8-diazaspiro[4.5]dec-2-yl)benzonitrile | ¹H NMR (400 MHz, DMSO-*d*₆) δ 10.37 (s, 1H), 7.61 (d, *J* = 8.8 Hz, 1H), 7.30 (d, *J* = 8.5 Hz, 2H), 7.11 (d, *J* = 7.9 Hz, 1H), 6.98 (d, *J* = 8.8 Hz, 2H), 6.88 (dd, *J* = 8.0, 1.9 Hz, 1H), 6.81 (dd, *J* = 5.9, 2.1 Hz, 2H), 6.67 (dd, *J* = 8.9, 2.4 Hz, 1H), 4.54 (s, 2H), 4.05 (q, *J* = 6.5 Hz, 2H), 3.74 (t, *J* = 6.7 Hz, 2H), 3.35 (t, *J* = 15.0 Hz, 5H), 3.30 - 3.13 (m, 6H), 2.69 (t, *J* = 6.7 Hz, 2H), 2.25 (dt, *J* = 12.9, 7.1 Hz, 4H), 2.01 (dq, *J* = 14.1, 7.2, 6.3 Hz, 3H), 1.90 (d,*J* = 14.8 Hz, 3H), 1.75 (ddd, *J* = 12.8, 8.5, 4.8 Hz, 3H), 1.59 (dd, *J* = 12.8, 6.5 Hz, 1H), 1.54 - 1.46 (m, 2H), 1.32 - 1.23 (m, 2H), 1.21 (d, *J* = 6.0 Hz, 3H). | 808.56 | ART-1 |

### Test Example 2: Degradation activity of the compounds of the present disclosure on AR protein in LNCAP cells

AR-positive human prostate cancer cells (LNCaP, cell source: ATCC) were cultured in RPMI 1640 (ATCC catalog number 30-2001) containing 10% FBS (Hyclone catalog number SH30406.05), and the cells were inoculated into a six-well microplate (Thermo catalog number 140675) at a density of 1.5 × 10⁵ cells/well. After the cells were cultured overnight in a carbon dioxide incubator (ESCO), the prepared compound of the present invention and the positive control sample ARV-766 (source: energy chemical) solution of different concentrations were added at a volume of 1 µL/well to make the final concentrations of the compound and the positive control sample reach 100nM and 10nM, respectively. At the same time, the corresponding solvent control was set and cultured for 8 hours. After 8 hours of culture, the culture medium was removed, the cells were washed with PBS, and RIPA lysis buffer containing 1% Protease Inhibitor Cocktail was added. After lysis and centrifugation, the total protein extract was obtained, and the protein concentration in the extract was detected by BCA method. Protein electrophoresis was performed using SDS-PAGE, and then the protein was transferred to a PVDF (Millipore Sigma catalog number PSRP010R5) membrane at a constant voltage of 100V for 60 minutes. The PVDF membrane was placed in a 5% milk powder 1×TBST solution and blocked at room temperature for 1 hour. The primary antibody solution (Anti-Androgen receptor, Cell Signaling Technology catalog number: 5153S; 1:2000 dilution) was prepared and incubated at 4°C overnight. The primary antibody solution was discarded and the PVDF membrane was washed with 1×TBST for 5 minutes each time, three times. The secondary antibody solution (Thermo, catalog number SA5-35571; 1:7500 dilution) was prepared and incubated at room temperature for 1 hour. The secondary antibody solution was discarded and the PVDF membrane was washed with 1×TBST for 5 minutes each time, three times. A dual-color infrared laser imaging system (Licor catalog number Odyssey CLX) was used for Western membrane imaging. The Image Studio software was used to analyze the grayscale of the bands. GAPDH protein bands were also detected for each sample as an internal reference. The AR protein degradation rate of the compound and the positive control sample was calculated based on the grayscale of the protein bands. Among them, "A" means degradation rate ≥ 50%, "B" means 50%> degradation rate ≥ 20%, "C" means 20% degradation rate ≥ 10%, and "D" means degradation rate less than 10%.

The positive reference used in the present invention is ARV766, and its structure is as follows:

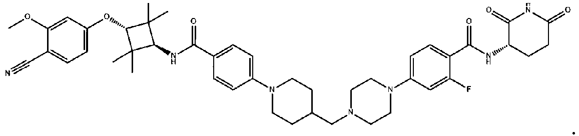

**Table 2: Degradation rate of AR protein in LNCAP cells by the compounds of the present disclosure**

| | 100 nM (%) | 10 nM (%) |
|---|---|---|
| ARV766 | A | B |
| A-P-77 | A | A |
| A-P-83 | A | A |
| A-P-195 | A | A |
| A-P-196 | A | B |
| A-P-233 | A | A |
| A-P-235 | A | A |

### Test Example 3: Degradation activity of the compounds of the present disclosure on AR protein in VCAP cells

AR-positive human prostate cancer cells VCaP (cell source: ATCC) were cultured in DMEM (GiBco catalog number 11995-065) containing 10% FBS (Hyclone catalog number SH30406.05), and the cells were inoculated in a six-well microplate (Thermo catalog number 140675) at a density of 3 × 10⁵ cells/well. After being cultured overnight in a carbon dioxide incubator (ESCO), the prepared compound of the present invention and the positive control sample ARV-766 (energy chemical) solution of different concentrations were added at a volume of 1 µL/well to make the final concentrations of the compound and the positive control sample reach 5nM and 0.5nM, respectively. At the same time, the corresponding solvent control was set and cultured for 8h. After 8h of culture, the culture medium was removed, and the cells were washed with PBS. RIPA lysis buffer containing 1% Protease Inhibitor Cocktail was added. After lysis and centrifugation, a total protein extract was obtained, and the protein concentration in the extract was detected by BCA method. Protein electrophoresis was performed using SDS-PAGE, and then the protein was transferred to a PVDF (Millipore Sigma catalog number PSRP010R5) membrane at a constant voltage of 100V for 60min. The PVDF membrane was placed in a 5% milk powder 1×TBST solution and blocked at room temperature for 1h. The primary antibody solution (Anti-Androgen receptor, Cell Signaling Technology catalog number: 5153S; 1:2000 dilution) was prepared and incubated at 4°C overnight. The primary antibody solution was discarded and the PVDF membrane was washed with 1×TBST for 5 minutes each time, three times. The secondary antibody solution (Thermo catalog number SA5-35571; 1:7500 dilution) was prepared and incubated at room temperature for 1 hour. The secondary antibody solution was discarded and the PVDF membrane was washed with 1×TBST for 5 minutes each time, three times. A dual-color infrared laser imaging system (Licor catalog number Odyssey CLX) was used for Western membrane imaging. The Image Studio software was used to analyze the grayscale of the bands. GAPDH protein bands were also detected for each sample as an internal reference. The AR protein degradation rate of the compound and the positive control sample was calculated based on the grayscale of the protein bands. Among them, "A" means degradation rate ≥ 50%, "B" means 50%> degradation rate ≥ 20%, "C" means 20% degradation rate ≥ 10%, and "D" means degradation rate less than 10%.

The degradation rates of AR protein in VCAP cells by the compounds in the present disclosure are shown in Table 3 below.

**Table 3: Degradation rate of AR protein in VCAP cells by the compounds disclosed in the present invention**

| | 5 nM | 0.5 nM |
|---|---|---|
| ARV766 | A | A |
| A-P-77 | A | B |
| A-P-195 | A | B |
| A-P-196 | A | A |
| A-P-233 | B | - |
| A-P-235 | A | A |
| A-P-265 | A | B |

| | | |
|---|---|---|
| "-" means not tested. | | |

### Test Example 4: Inhibitory activity of the compounds in the present disclosure on LNCAP cell proliferation

LNCaP FGC cells (ATCC, CLR-1740) were cultured in RPMI 1640 (ATCC, 30-2001) complete medium supplemented with 10% fetal bovine serum (Hyclone, SH30406.05). On the first day of the experiment, LNCaP FGC cells were seeded at a density of 5,000 cells/well in 96-well plates using RPMI 1640 medium supplemented with 10% fetal bovine serum, with 100 µL of cell suspension per well. The cells were cultured overnight in a 37°C, 5% CO₂ cell incubator. On the second day, 100 µL of different concentrations of the compound to be tested and the positive control sample ARV-766 (source: Energy Chemical) prepared in culture medium were added to each well. The final concentration of the compound was 9 concentration points of 5-fold serial dilution starting from 10 µM. The wells to which 100 µL of RPMI 1640 culture medium containing 10% fetal bovine serum was added were set as controls. The cells were cultured in a cell culture incubator at 37°C and 5% CO₂ for 96 hours. On the sixth day, the 96-well cell culture plate was removed, and 1/10 volume of WST-8 (Beytime, C0040) reagent was added to each well. The absorbance at 450 nm was detected using a microplate reader (TECAN, F50). The 50% proliferation inhibition concentration (IC₅₀ value) was calculated by logistic regression based on the concentration and absorbance values of the compound using GraphpadPrism software.

The inhibitory IC₅₀ values of the compounds of the present disclosure on LNCAP cell proliferation are shown in Table 4 below.

**Table 4: IC₅₀ values of the compounds of the present disclosure for inhibiting LNCAP cell proliferation**

| | IC₅₀ (µM) |
|---|---|
| ARV766 | 0.49 |
| A-P-77 | 0.3 |
| A-P-83 | 0.55 |
| A-P-195 | 0.10 |
| A-P-196 | 0.24 |
| A-P-233 | 0.077 |
| A-P-235 | >50 |

### Test Example 5: Inhibitory activity of the compounds of the present disclosure on VCaP cell proliferation

VCaP cells (ATCC, CRL-2876) were cultured in DMEM (Gibco, 11995-065) complete medium containing 10% fetal bovine serum (Hyclone, SH30406.05). On the first day of the experiment, VCaP cells were seeded at a density of 15,000 cells/well in a 96-well plate using DMEM medium containing 10% fetal bovine serum, with 100 µL of cell suspension per well, and cultured overnight in a cell culture incubator at 37°C and 5% CO₂. On the second day, 100 µL of the compound of the present invention and the positive control ARV-110 (source: Energy Chemical) prepared in culture medium at different concentrations were added to each well. The final concentration of the compound was 9 concentration points of 5-fold serial dilution starting from 10 µM. The wells to which 100 µL of DMEM medium containing 10% fetal bovine serum was added were set as blank controls and cultured in a cell culture incubator at 37°C and 5% CO₂ for 168 hours. On the ninth day, the 96-well cell culture plate was removed and 1/10 volume of WST-8 (Beytime, C0040) reagent was added to each well. The absorbance at 450 nm was measured using a microplate reader (TECAN, F50). The 50% proliferation inhibition concentration (IC₅₀ value) was calculated by logistic regression based on the concentration and absorbance of the compound using Graphpad Prism software.

The inhibitory IC₅₀ values of the compounds of the present disclosure on VCAP cell proliferation are shown in Table 5 below.

**Table 5: IC₅₀ values of the compounds of the present disclosure for inhibiting VCAP cell proliferation**

| | IC₅₀(µM) |
|---|---|
| ARV766 | 0.0025 |
| A-P-77 | 0.00017 |
| A-P-83 | <0.000026 |
| A-P-195 | 0.013 |
| A-P-196 | 0.001 |
| A-P-233 | 0.015 |
| A-P-235 | 0.001 |

| | |
|---|---|
| Conclusion: From the results in Tables 2 to 5 above, it can be seen that the PROTAC compounds synthesized from the E3 ligase inhibitors provided in this specification and the androgen receptor target protein showed significant AR protein degradation effects in prostate cancer cells LNCAP and VCaP, and also had significant prostate cancer cell proliferation inhibition effects. The target protein degradation effects and cell proliferation inhibition effects of some compounds were significantly superior to those of the control compound ARV766. | |

### The second part is PROTAC compounds targeting estrogen receptor (ER):

ER PROTAC general synthetic formula ER-T-1

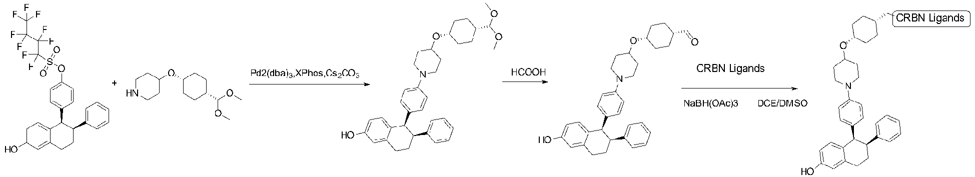

**Table B: The following compounds of Examples 44 to 49 were synthesized according to the above general formula:**

| Ex am ple s | Co mp oun ds | Structures and Names | ¹H-NMR | LC-MS | Synthetic Method |
|---|---|---|---|---|---|
| 44 | ER-P-1 | 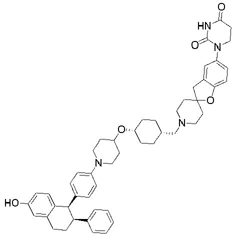 1-(1'-(((1S,4S)-4-((1-(4-((1R,2S)-6-hydroxy-2-phenyl-1,2,3,4-tetrahydro-naphthalen-1-yl)phenyl)piperidin-4-yl)oxy)cyclohexyl)methyl)-3H-spiro [benzofuran-2,4'-piperidine]-5-yl)dihydropyrimidine-2,4(1H,3H)-dione | ¹H NMR (600 MHz, DMSO-*d*₆) δ 10.31 (s, 1H), 7.20-7.18 (m, 1H), 7.16-7.11 (m, 3H), 7.07-7.04 (m, 1H), 6.84 (d, *J* = 7.3 Hz, 2H), 6.78 (t, *J* = 9.2 Hz, 1H), 6.70 - 6.58 (m, 4H), 6.49 (dd, *J* = 8.3, 2.5 Hz, 1H), 6.26 (s, 2H), 4.16 (d, *J* = 5.1 Hz, 1H), 3.72 - 3.64 (m, 3H), 3.52-3.50 (m, 2H), 3.39 - 3.27 (m, 4H), 3.22 - 3.09 (m, 5H), 3.07 (t, *J* = 6.2 Hz, 2H), 3.01-2.90 (m, 3H), 2.83 (s, 2H), 2.69 (t, *J* = 6.7 Hz, 2H), 2.10-2.06 (m, 5H), 1.85 (s, 2H), 1.77 - 1.68 (m, 3H), 1.55 - 1.49 (m, 3H), 1.45-1.44 (m, 2H), 1.36-1.33 (m, 2H) | 795.66 | PROTAC general synthetic formula ER-T-1 |
| 45 | ER-P-2 | 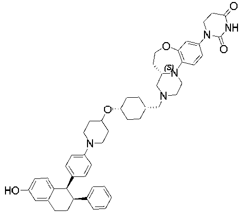 1-((S)-3-(((1S,4R)-4-((1-(4-((1R,2S)-6-hydroxy-2-phenyl-1,2,3,4-tetrahydronaphthalen-1-yl)phenyl)piperidin-4-yl)oxy)cyclohexyl)methyl)-2,3,4,4a,5,6-hexahydro-1H-benzo[b]pyrazino[1,2-d][1,4]oxazepin-9-yl)dihydropyrimidine-2,4(1H,3H)-dione | ¹H NMR (400 MHz, DMSO-*d*₆) δ 10.34 (s, 1H), 9.17 (s, 1H), 7.17-7.10 (m, 3H), 7.02 - 6.92 (m, 2H), 6.87 - 6.79 (m, 3H), 6.78 - 6.57 (m, 4H), 6.48 (dd, *J* = 8.3, 2.5 Hz, 1H), 6.28 (d, *J* = 8.1 Hz, 2H), 4.50 (t, *J* = 10.1 Hz, 1H), 4.22-4.18 (m, 1H), 4.14 - 4.06 (m, 1H), 3.73 - 3.70 (m, 2H), 3.46 (d, *J* = 11.9 Hz, 2H), 3.41 - 3.26 (m, 7H), 3.17 - 3.01 (m, 5H), 2.99 - 2.85 (m, 4H), 2.67 (t, *J* = 6.7 Hz, 2H), 2.14-2.06 (m, 2H), 1.90-1.82 (m, 3H), 1.81-1.78 (m, 1H), 1.77-1.71 (m, 3H), 1.59 - 1.41 (m, 6H), 1.36 (t, *J* = 11.1 Hz, 2H) | 810.65 | PROTAC general synthetic formula ER-T-1 |
| 46 | ER-P-3 | 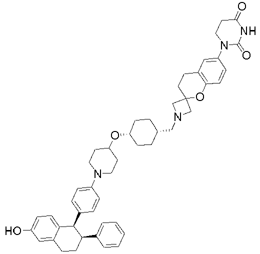 1-(1-(((1S,4S)-4-((1-(4-((1R,2S)-6-hydroxy-2-phenyl-1,2,3,4-tetrahydronaphthalenyl-1-yl)phenyl)piperidin-4-yl)oxy)cyclohexyl)methyl)spiro[aze tidin-3,2'-benzofuran]-6'-yl)dihydropyrimidine-2,4(1H,3H)-dione | ¹H NMR (400 MHz, DMSO-*d*₆) δ 10.33 (s, 1H), 9.15 (s, 1H), 7.19 - 7.05 (m, 5H), 6.90 - 6.79 (m, 3H), 6.73 - 6.57 (m, 4H), 6.48 (dd, *J* = 8.2, 2.5 Hz, 1H), 6.26 (d, *J* = 8.1 Hz, 2H), 4.44 - 4.35 (m, 1H), 4.31-4.26 (m, 2H), 4.23 - 4.14 (m, 2H), 3.69 (t, *J* = 6.6 Hz, 2H), 3.39 - 3.27 (m, 4H), 3.24 - 3.13 (m, 3H), 3.00-2.92 (m, 3H), 2.87-2.80 (m, 2H), 2.78 - 2.73 (m, 1H), 2.68 (t, *J* = 6.6 Hz, 2H), 2.19-2.14 (m, 2H), 2.11-2.05 (m, 1H), 1.99-1.90 (m, 1H), 1.86-1.83 (m, 2H), 1.74-1.68 (m, 4H), 1.56 - 1.39 (m, 5H), 1.37 - 1.27 (m, 2H) | 781.60 | PROTAC general synthetic formula ER-T-1 |
| 47 | ER-P-4 | 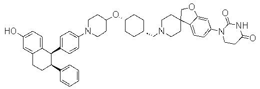 1-(1'-(((1S,4S)-4-((1-(4-((1R,2S)-6-hydroxy-2-phenyl-1,2,3,4-tetrahydronaphthalen-1-yl)phenyl)piperidin-4-yl)oxy)cyclohexyl)methyl)-2H-spiro [benzofuran-3,4'-piperidine]-6-yl)dihydropyrimidine-2,4(1H,3H)-dione | ¹H NMR (600MHz, DMSO-d*₆*) δ 10.38 (s, 1H), 8.93 (s, 1H), 7.28 - 7.08 (m, 4H), 6.92 - 6.79 (m, 4H), 6.77 - 6.57 (m, 4H), 6.49 (dd, J = 8.4, 2.5 Hz, 1H), 6.27 (d, J = 8.1 Hz, 2H), 5.33 (t, J = 5.0 Hz, 1H), 4.52 (s, 2H), 4.17 (d, J = 5.1 Hz, 1H), 3.74 (t, J = 6.6 Hz, 2H), 3.18 (d, J = 8.8 Hz, 1H), 3.07 - 2.90 (m, 6H), 2.83 (d, J = 15.4 Hz, 2H), 2.69 (t, J = 6.7 Hz, 2H), 2.21 - 2.04 (m, 3H), 2.04 - 1.92 (m, 4H), 1.93 - 1.83 (m, 4H), 1.83 - 1.68 (m, 4H), 1.47 (dd, J = 20.8, 13.6 Hz, 3H), 1.30 (s, 1H), 1.22 - 1.15 (m, 2H), 1.04 (t, J = 11.5 Hz, 2H), 0.86 (t, J = 6.9 Hz, 1H) | 795.04 | PROTAC general synthetic formula ER-T-1 |
| 48 | ER-P-5 | 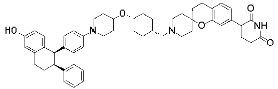 3-(1'-(((1S,4S)-4-((1-(4-((1R,2S)-6-hydroxy-2-phenyl-1,2,3,4-tetrahydronaphthyl-1-yl)phenyl)piperidin-4-yl)oxy)cyclohexyl)methyl)spiro[chr oman-2,4'-piperidine]-7-yl)piperidine-2,6-dione | ¹H NMR (600MHz, DMSO-d*₆*) δ10.85 (d, J = 9.5 Hz, 1H), 8.93 (s, 1H), 7.14 (dt, J = 13.2, 7.0 Hz, 3H), 7.07 (d, J = 7.7 Hz, 1H), 6.83 (d, J = 7.3 Hz, 2H), 6.76 - 6.60 (m, 6H), 6.49 (d, J = 8.2 Hz, 1H), 6.27 (d, J = 8.1 Hz, 2H), 4.17 (d, J = 5.1 Hz, 1H), 3.76 (dd, J = 11.1, 5.0 Hz, 2H), 3.69 - 3.63 (m, 2H), 3.31 (dd, J = 13.8, 5.1 Hz, 3H), 3.19 - 3.02 (m, 5H), 3.02 - 2.83 (m, 4H), 2.74 (t, J = 6.8 Hz, 2H), 2.64 (ddd, J = 16.9, 11.2, 5.2 Hz, 1H), 2.47 (dd, J = 17.5, 5.0 Hz, 1H), 2.15 - 2.06 (m, 2H), 2.06 - 1.97 (m, 2H), 1.95 - 1.82 (m, 6H), 1.80 (t, J = 6.8 Hz, 2H), 1.71 (dd, J = 11.8, 6.0 Hz, 3H), 1.56 - 1.41 (m, 6H), 1.33 (d, J = 11.2 Hz, 2H) | 808.08 | PROTAC general synthetic formula ER-T-1 |
| 49 | ER-P-6 | 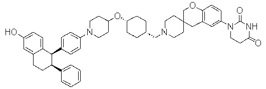 1-(1'-(((1S,4S)-4-((1-(4-((1R,2S)-6-hydroxy-2-phenyl-1,2,3,4-tetrahydronaphthalen-1-yl)phenyl)piperidin-4-yl)oxy)cyclohexyl)methyl)spiro[ben zo-dihydro-3,4'-piperidin]-6-yl)dihydropyrimidine-2,4(1H,3H)-dione | ¹H NMR (400 MHz, DMSO- d*₆*) δ 10.32 (s, 1H), 8.83 (d, J = 11.3 Hz, 1H), 7.14(q, J = 7.8, 7.0 Hz, 3H), 7.08 - 6.98 (m, 2H), 6.81 (t, J = 8.3 Hz, 3H), 6.74 - 6.58 (m, 4H), 6.48 (dd, J = 8.4, 2.5 Hz, 1H), 6.26 (d, J = 8.1 Hz, 2H), 4.23 -4.07 (m, 2H), 3.82 (s, 1H), 3.69 (td, J = 6.6, 3.8 Hz, 2H), 3.33 - 3.30 (m, 2H), 3.22 - 2.99 (m, 4H), 2.95 (q, J = 5.7 Hz, 4H), 2.86 (s, 3H), 2.68 (td, J = 6.7, 2.1 Hz, 3H), 2.57 (s, 1H), 2.08 (dd, J = 12.1, 6.6 Hz, 1H), 1.99 - 1.89 (m, 2H), 1.85 (d, J = 12.2 Hz, 2H), 1.82 - 1.64 (m, 8H), 1.54 (dd, J = 42.8, 13.0 Hz, 4H), 1.20 - 1.11 (m, 2H), 1.01 (q, J = 12.3 Hz, 2H) | 809.06 | PROTAC general synthetic formula ER-T-1 |

### Test Example 6 Inhibitory Effect of the PROTAC Compounds of the present disclosure on Cell Proliferation in T-47D Cells

The complete culture medium required for breast ductal carcinoma T-47D cells is 1640 (ATCC, Product number: A10491-01) basal medium containing 10% fetal bovine serum. T-47D cells were seeded in 96-well plates at a density of 3,000/well, and different concentrations of PROTAC compounds 1-55 provided in the embodiments of the present invention and the positive control sample ARV-471 (source: Inokai) were prepared to treat the cells and incubated at 37°C, 5% CO₂ in a cell culture incubator for 6 days. According to the manufacturer's instructions, the CCK-8 kit (Beyotime, Product number: C0040) was used to detect cell viability. The IC₅₀ of each compound for 6 days was calculated using GraphPadPrism software based on the inhibition rate. The results are shown in Table 6.

**Table 6: IC₅₀ value for the inhibition of T-47D cell proliferation by PROTAC compounds and positive control sample**

| Compounds | IC₅₀ (T-47D) [nM] |
|---|---|
| ARV-471 | 2.69 |
| ER-P-2 | 21.7 |
| ER-P-3 | 51.58 |
| ER-P-4 | 52.53 |
| ER-P-6 | 367 |

| | |
|---|---|
| Conclusion: The PROTAC compounds provided by the present invention can effectively inhibit the proliferation of T-47D cells, and the inhibitory effects of some compounds are comparable to or superior to those of the positive control sample. | |

### Test Example 7 Inhibitory effect of the disclosed PROTAC compounds on cell proliferation in MCF-7 cells

The complete culture medium required for breast cancer MCF-7 cells (source ATCC) is DMEM (gibco, Product number: 11995-065) basal medium containing 10% fetal bovine serum. MCF-7 cells were seeded in 96-well plates at a density of 800 cells/well, and different concentrations of PROTAC compounds 1-55 provided in the embodiments of the present invention and the positive control sample ARV-471 (source: Inokai) were prepared to treat cells. The cells were incubated at 37 °C and 5% CO₂ in a cell culture incubator for 6 days. Cell viability was detected using a CCK-8 kit (Beyotime, Product number: C0040) according to the manufacturer's instructions. The IC₅₀ value of each PROTAC compound for 6 days was calculated using GraphPad Prism software based on the inhibition rate. The results are shown in Table 7.

**Table 7: IC₅₀ value for the inhibition of MCF-7 cell proliferation by PROTAC compounds of the present disclosure and positive control sample**

| Compounds | IC₅₀ (MCF-7) [nM] |
|---|---|
| ARV-471 | 113 |
| ER-P-1 | 845 |
| ER-P-2 | / |
| ER-P-3 | / |
| ER-P-4 | 944 |
| ER-P-5 | 873 |
| ER-P-6 | 847 |

| | |
|---|---|
| Conclusion: The PROTAC compounds of the present invention can effectively inhibit the proliferation of MCF-7 cells. The inhibitory effects of some PROTAC compounds on MCF-7 cells are comparable to or even superior to those of the positive control sample. | |

### Test Example 8 Degradation of ERα by PROTAC Compounds of the Present Disclosure in MCF-7 Cells

MCF-7 (ATCC) cells were cultured in DMEM medium (11995-065, Gibco) containing 10% FBS (10099141C, Gibco) at 37°C and 5% CO₂ until they entered the logarithmic growth phase. MCF-7 cells were seeded at a density of 3×10⁵ cells/well in a 6-well plate and incubated at 37°C, 5% CO₂ for 24 hours. After cell attachment, 1 µL of compound in dimethyl sulfoxide solution (final concentration 0.15-1000 nM) was added and the cells were incubated for another 4 hours at 37°C, 5% CO₂. After 4 hours, the culture medium was discarded, and each well was washed once with 1 mL of pre-chilled 1× DPBS. The cells were then lysed with 50 µL of RIPA lysis buffer (P0013B, Beyotime) and 1× protease inhibitor cocktail (P1005, Beyotime). After incubation on ice for 20 minutes, the cells were centrifuged at 14,000 g at 4°C for 30 minutes. The supernatant was carefully collected and analyzed for ERα protein levels by immunoblotting.

Western blotting: The total protein concentration in the collected cell lysate supernatant was determined using the BCA protein concentration assay kit (enhanced) (P0009, Beyotime). Based on the total protein concentration determined by BCA, the protein concentration was adjusted to 1 µg/µL using RIPA lysis buffer and 5× SDS-PAGE protein loading buffer (MB01015, GenScript). The protein was denatured in a 95°C water bath for 5 minutes. After denaturation, the condensed water on the tube wall was collected by centrifugation and used as the loading sample. 20 µL of the loading sample (20 µg total protein) was added to the sample well of a 12% precast gel (M00669, GenScript). Electrophoresis was performed at 100 V for 20 minutes, followed by switching to 150 V for 40 minutes. After electrophoresis, the proteins from the SDS-PAGE gel were transferred to a PVDF membrane at a constant voltage of 100 V for 60 minutes. After transfer, the PVDF membrane corresponding to the ERα protein and internal reference protein was cut and incubated in 1× QµickBlock blocking buffer (P0252, Beyotime) for 30 minutes at room temperature on a shaker. After blocking, the ERα primary antibody (1:1000, 8644S, CST) was added and incubated overnight at 4°C on a shaker. After incubation, the PVDF membrane was washed three times with 1× TBST buffer for 5 minutes each. Rabbit IgG (H+L) secondary antibody (1:7500, SA5-35571, Invitrogen) was added and incubated for 1 hour at room temperature on a shaker. The membrane was then washed again three times with 1× TBST buffer for 5 minutes each. Finally, the membrane was imaged using a dual-color infrared laser imaging system (Odyssey DLx, LI-COR). Protein maps were analyzed for grayscale values using Image Studio Lite software. The grayscale correction value for each sample was calculated using the formula: grayscale correction value = grayscale value of the target protein / grayscale value of the corresponding internal reference. Then the grayscale correction values of the compound group and the control group were compared to calculate the degradation rate of the compound.

Some of the compounds of the embodiments of the present disclosure have a significant degradation effect on ERα in MCF-7 cells. For example, the degradation rate of ER-P-2 is greater than or equal to 30% at a concentration of 10 nM.

### Test Example 9 Degradation of ERα by PROTAC Compounds of the Present Disclosure in T-47D Cells

T-47D (ATCC) cells were cultured in PRMI-1640 medium (30-2001, ATCC) containing 10% FBS (10099141C, Gibco) at 37°C and 5% CO₂ until they entered the logarithmic growth phase. T-47D cells were seeded onto 6-well plates at a density of 3×10⁵ cells per well, and incubated at 37°C with 5% CO₂ for 24 hours. After cell attachment, 1 µL of compound in dimethyl sulfoxide solution (final concentration 0.15-1000 nM) was added and the cells were incubated for another 4 hours at 37°C, 5% CO₂. After 4 hours, the culture medium was discarded, and each well was washed once with 1 mL of pre-chilled 1× DPBS. The cells were then lysed with 50 µL of RIPA lysis buffer (P0013B, Beyotime) and 1× protease inhibitor cocktail (P1005, Beyotime). After incubation on ice for 20 minutes, the cells were centrifuged at 14,000 g at 4°C for 30 minutes. The supernatant was carefully collected and analyzed for ERα protein levels by immunoblotting.

Some of the compounds in the embodiments of the present disclosure have a significant degradation effect on ERα in T-47D cells. For example, the degradation rates of ER-P-1 and ER-P-3 are greater than or equal to 30% at a concentration of 10 nM.

**The third part is PROTAC compounds targeting IRAK4 receptor:**
PROTAC general synthetic formula IR-T-1 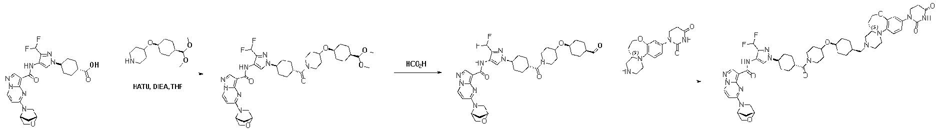
PROTAC general synthetic formula IR-T-2

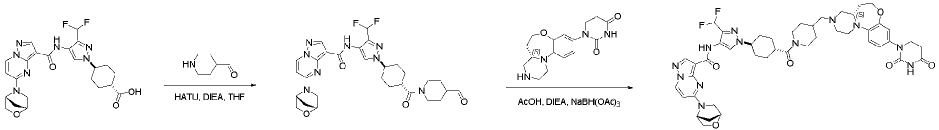

**Table C: The following compounds of Examples 50 to 56 were synthesized according to the above general formulas:**

| Exa mple s | compoun ds | Structures and Names | ¹H-NMR | LC-MS | Synthetic Method |
|---|---|---|---|---|---|
| 50 | IR-P-1 | 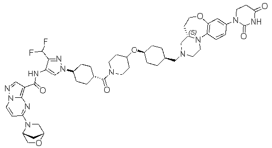 | ¹H NMR (400 MHz, DMSO-*d₆*) δ 10.32 (s, 1H), 9.51 (d, *J* = 6.6 Hz, 1H), 8.79 (d, *J* = 7.7 Hz, 1H), 8.40 (d, *J* = 4.8 Hz, 1H), 8.26 (d, *J* = 5.6 Hz, 1H), 7.27 - 6.90 (m, 3H), 6.90 - 6.42 (m, 2H), 5.30 - 5.05 (m, 1H), 4.77 (d, *J* = 19.9 Hz, 1H), 4.52 (t, *J* = 9.6 Hz, 1H), 4.33 - 4.20 (m, 1H), 4.16 - 4.06 (m, 1H), 3.95 - 3.86 (m, 1H), 3.84 - 3.55 (m, 8H), 3.36 - 3.22 (m, 6H), 3.20 - 2.97 (m, 6H), 2.80 - 2.63 (m, 3H), 2.19 - 1.67 (m, 15H), 1.63 - 1.21 (m, 10H). | 995. 85 | PROTAC general synthetic formula IR-T-1 |
| 51 | IR-P-2 | 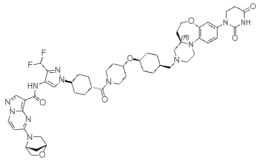 | ¹H NMR (400 MHz, DMSO-*d₆*) δ 10.32 (s, 1H), 9.51 (d,*J* = 6.7 Hz, 1H), 8.79 (d, *J* = 7.7 Hz, 1H), 8.40 (d, *J* = 4.8 Hz, 1H), 8.26 (d, *J* = 5.6 Hz, 1H), 7.27 - 6.91 (m, 3H), 6.90 - 6.43 (m, 2H), 5.30 - 5.05 (m, 1H), 4.77 (d, *J* = 20.1 Hz, 1H), 4.52 (t, *J* = 9.3 Hz, 1H), 4.32 - 4.20 (m, 1H), 4.15 - 4.03 (m, 1H), 3.95 - 3.85 (m, 1H), 3.85 - 3.59 (m, 8H), 3.40 - 3.22 (m, 6H), 3.21 - 2.97 (m, 6H), 2.80 - 2.60 (m, 3H), 2.19 - 1.68 (m, 15H), 1.65 - 1.21 (m, 10H). | 995. 75 | PROTAC general synthetic formula IR-T-1 |
| 52 | IR-P-3 | 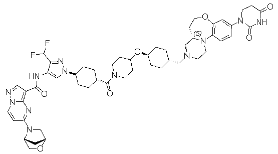 | ¹H NMR (400 MHz, DMSO-*d₆*) δ 10.32 (s, 1H), 9.51 (d, *J* = 6.9 Hz, 1H), 8.79 (d, *J* = 7.7 Hz, 1H), 8.40 (d, *J* = 4.8 Hz, 1H), 8.26 (d, *J* = 5.6 Hz, 1H), 7.27 - 6.91 (m, 3H), 6.90 - 6.40 (m, 2H), 5.30 - 5.05 (m, 1H), 4.77 (d, *J* = 20.9 Hz, 1H), 4.51 (t,*J* = 9.8 Hz, 1H), 4.32 - 4.20 (m, 1H), 4.16 - 4.08 (m, 1H), 3.96 - 3.87 (m, 1H), 3.86 - 3.53 (m, 8H), 3.50 - 3.42( m, 3H), 3.30 - 2.94 (m, 8H), 2.78 - 2.63 (m, 3H), 2.20 - 1.68 (m, 16H), 1.65 - 1.48 (m, 3H), 1.41 - 1.32 (m, 1H), 1.32 - 1.13 (m, 4H), 1.13 - 0.97 (m, 2H). | 995. 75 | PROTAC general synthetic formula IR-T-1 |
| 53 | IR-P-4 | 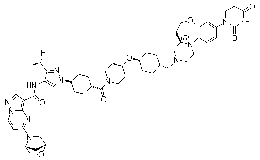 | ¹H NMR (400 MHz, DMSO-*d₆*) δ 10.32 (s, 1H), 9.51 (d, *J* = 6.7 Hz, 1H), 8.79 (d, *J* = 7.7 Hz, 1H), 8.40 (d, *J* = 4.8 Hz, 1H), 8.26 (d, *J* = 5.6 Hz, 1H), 7.28 - 6.91 (m, 3H), 6.90 - 6.43 (m, 2H), 5.30 - 5.05 (m, 1H), 4.77 (d*, J* = 20.3 Hz, 1H), 4.56 - 4.47 (m, 1H), 4.31 - 4.21 (m, 1H), 4.11 (dd, *J* = 12.3, 7.4 Hz, 1H), 3.97 - 3.88 (m, 1H), 3.85 - 3.55 (m, 8H), 3.53 - 3.40 (m, 6H), 3.33 - 2.98 (m, 8H), 2.79 - 2.65 (m, 3H), 2.40 - 1.70 (m, 14H), 1.65 - 1.48 (m, 2H), 1.43 - 1.13 (m, 5H), 1.12 - 0.95 (m, 2H). | 995. 65 | PROTAC general synthetic formula IR-T-1 |
| 54 | IR-P-5 | 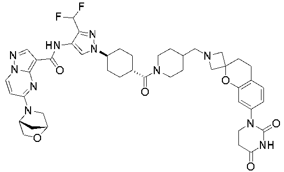 | ¹H NMR (600 MHz, DMSO-*d₆*) δ 10.36 (d, *J* = 5.2 Hz, 1H), 9.51 (d, *J* = 10.4 Hz, 1H), 8.79 (d, *J* = 7.7 Hz, 1H), 8.40 (d, *J* = 7.3 Hz, 1H), 8.26 (d, *J* = 8.5 Hz, 1H), 7.23 - 7.00 (m, 2H), 6.93 - 6.44 (m, 3H), 5.30 - 5.05 (m, 1H), 4.77 (d, *J* = 32.3 Hz, 1H), 4.51 - 4.20 (m, 5H), 4.08 - 3.98 (m, 1H), 3.88 - 3.71 (m, 3H), 3.67 - 3.44 (m, 4H), 3.29 - 3.18 (m, 2H), 3.01 (t, *J* = 12.0 Hz, 1H), 2.88 - 2.65 (m, 4H), 2.25 - 2.13 (m, 2H), 2.11 - 1.65 (m, 10H), 1.64 - 1.49(m, 2H), 1.36 - 0.96 (m, 4H). | 868. 86 | PROTAC general synthetic formula IR-T-2 |
| 55 | IR-P-6 | 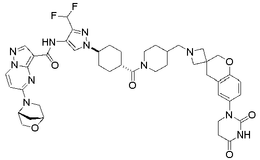 | ¹H NMR (600 MHz, DMSO-*d₆*) δ 10.32 (d, *J* = 6.1 Hz, 1H), 9.51 (d, *J* = 10.4 Hz, 1H), 8.79 (d, *J* = 7.4 Hz, 1H), 8.40 (d, *J* = 7.3 Hz, 1H), 8.26 (d, *J* = 8.5 Hz, 1H), 7.24 - 6.99 (m, 3H), 6.90 - 6.42 (m, 2H), 5.30 - 5.05 (m, 1H), 4.77 (d, *J* = 32.4 Hz, 1H), 4.40 (d, *J* = 12.5 Hz, 1H), 4.31 - 4.19 (m, 3H), 4.15 - 4.06 (m, 2H), 4.03 (d, *J* = 6.6 Hz, 3H), 3.87 - 3.43 (m, 6H), 3.26 - 2.94 (m, 5H), 2.77 - 2.66 (m, 3H), 2.12 - 1.49 (m, 12H), 1.35 - 0.96 (m, 4H). | 868. 86 | PROTAC general synthetic formula IR-T-2 |
| 56 | IR-P-7 | 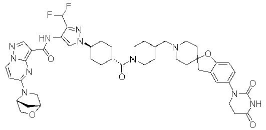 | ¹H NMR (600 MHz, DMSO-*d₆*) δ 10.30 (s, 1H), 9.52 (d, *J* = 10.1 Hz, 1H), 8.80 (d, *J* = 7.7 Hz, 1H), 8.41 (d, *J* = 7.2 Hz, 1H), 8.26 (d, *J* = 8.4 Hz, 1H), 7.23 - 7.00 (m, 3H), 6.92 - 6.43 (m, 2H), 5.30 - 5.05 (m, 1H), 4.77 (d, *J* = 32.7 Hz, 1H), 4.43 (d, *J* = 12.0 Hz, 1H), 4.27 (td, *J =* 12.0, 4.2 Hz, 1H), 4.06 (d, *J =* 12.5 Hz, 1H), 3.85 - 3.52 (m, 8H), 3.24 - 3.01 (m, 6H), 2.76 (t, *J* = 11.4 Hz, 1H), 2.70 (t, *J* = 6.6 Hz, 2H), 2.63 - 2.54 (m, 1H), 2.17 - 1.72 (m, 12H), 1.64 - 1.52 (m, 2H), 1.34 - 0.96 (m, 6H). | 882. 76 | PROTAC general synthetic formula IR-T-2 |

### Test Example 10: Degradation of IRAK4 in THP-1 cells by compounds

THP-1(TIB-202^{™}, ATCC) cells were cultured in RPMI 1640 medium (30-2001, ATCC) containing 10% FBS (10099141C, Gibco) and 0.05 mM β-mercaptoethanol (M3148, Sigma-Aldrich) at 37°C and 5% CO₂ until they entered the logarithmic growth phase. 2 mL of THP-1 cells were seeded into a 6-well cell culture plate at a density of 4 × 10⁵ cells/well. After adding 1 µL of compound in dimethyl sulfoxide solution (final concentration 10-10,000 nM) and continuing to incubate at 37°C, 5% CO₂ for 24 hours, cells were collected into 2 mL centrifuge tubes and centrifuged at 125 g at 4°C for 5 minutes. The cell pellet was washed once with 1 mL of pre-chilled 1× DPBS and resuspended and lysed in 50 µL of RIPA lysis buffer (P0013B, Beyotime) and 1× protease inhibitor cocktail (P1005, Beyotime). After incubation on ice for 20 minutes, the cells were centrifuged at 14,000 g at 4°C for 30 minutes. The supernatant was carefully collected and analyzed for IRAK4 protein levels by immunoblotting.

Western blotting: The total protein concentration in the collected cell lysate supernatant was determined using the BCA protein concentration assay kit (enhanced) (P0009, Beyotime). Based on the total protein concentration detected by BCA, the protein concentration was adjusted to 1 µg/µL using RIPA lysis buffer and 5× SDS-PAGE protein loading buffer (MB01015, GenScript). The protein was denatured in a 95°C water bath for 5 minutes. After denaturation, the condensed water on the centrifuge tube wall was collected by centrifugation and used as the loading sample. 20 µL of the loading sample (20 µg total protein) was added to the sample well of a 12% precast gel (M00669, GenScript). Electrophoresis was performed at 100 V for 20 minutes, then switched to 150 V for 40 minutes. After electrophoresis, the proteins in the SDS-PAGE gel were transferred to a PVDF membrane and transferred at a constant voltage of 100 V for 60 minutes. After transfer, the PVDF membrane corresponding to the IRAK4 protein and internal reference protein was cut and incubated in 1× QuickBlock blocking buffer (P0252, Beyotime) for 30 minutes at room temperature on a shaker. After blocking, the IRAK4 primary antibody (1:1000, 4363S, CST) was added and incubated overnight at 4°C on a shaker. After incubation, the PVDF membrane was washed three times with 1× TBST buffer for 5 minutes each. Rabbit IgG (H+L) secondary antibody (1:7500, SA5-35571, Invitrogen) was added and incubated for 1 hour at room temperature on a shaker. The membrane was then washed again three times with 1× TBST buffer for 5 minutes each. Finally, the membrane was imaged using a dual-color infrared laser imaging system (Odyssey DLx, LI-COR). Protein maps were analyzed for grayscale values using Image Studio Lite software. The grayscale correction value for each sample was calculated using the formula: grayscale correction value = grayscale value of the target protein / grayscale value of the corresponding internal reference. The grayscale correction values of the compound group and the control group were then compared to calculate the degradation rate of the compound. "A" indicates a degradation rate ≥ 50%, "B" indicates 50% > degradation rate ≥ 20%, "C" indicates 20% degradation rate ≥ 10%, and "D" indicates a degradation rate less than 10%.

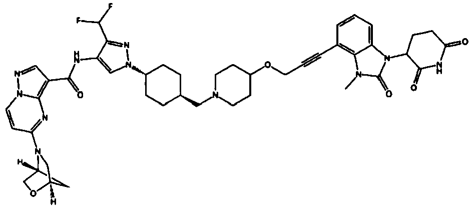

### (Structure of control compound KT-474)

**Table 8: Degradation effect of example compounds on IRAK4 in THP-1 cells**

| Compounds | IRAK4 degradation (100nM) | IRAK4 degradation (10nM) |
|---|---|---|
| KT-474 | A | B |
| IR-P-1 | B | B |
| IR-P-2 | B | B |
| IR-P-3 | A | A |
| IR-P-4 | A | B |
| IR-P-5 | - | B |

| | | |
|---|---|---|
| "-" means not tested. | | |

### Test Example 11: Determination of TNFα concentration secreted by PBMC induced by LPS

After thawing, cryopreserved hPBMC (FPB003F-C, Shanghai OriBiotech) cells were resuspended in RPMI 1640 medium (30-2001,ATCC) containing 10% inactivated FBS (10099141C, Gibco) and cultured overnight at 37°C and 5% CO₂. The next day, hPBMC cells were seeded in a 96-well cell culture plate at a density of 2 × 10⁵ cells/well/100 µL (2 × 10⁶ cells/mL), and 100 µL of compound in dimethyl sulfoxide solution (final concentration 0.0064-2500 nM) was added. After incubation at 37°C, 5% CO₂ for 20 hours, 20 µL of LPS (055:B5) (L5418, Sigma) was added to the drug-treated cell plate at a final concentration of 200 ng/mL. The cell plate was placed in a 37°C, 5% CO₂ incubator for 5 hours, and then centrifuged at 1000 rpm for 15 minutes. 100 µL of cell supernatant was collected from each well for cytokine concentration testing, which was tested immediately or frozen at -80°C for testing.

### HTRF detection of TNFα

The TNFα concentration in the supernatant of LPS-induced THP-1 cells was determined using the TNF ALPHAHTRF detection kit (62HTNFAPEG, PerkinElmer).

TNFα standard solutions of varying concentrations were prepared according to the product instructions, ranging from 0 to 2500 pg/mL. Equal volumes of Eu Cryptate antibody (1×) and d2 antibody (1×) were used to prepare the antibody mixture. 16 µL of standard solution or sample supernatant and 4 µL of the antibody mixture were added to each well of a 384-well white plate. The mixture was centrifuged at 800 rpm for 1 minute and incubated at room temperature for 2 hours. The absorbance at 620 nm and 655 nm was read using a microplate reader (SPARK m1000pro, TECAN). A standard curve was plotted using the signal values of each TNFα standard solution. The TNFα concentration in each sample was determined based on the corresponding concentration of the signal value on the standard curve. The inhibitory rate of the compound on LPS-induced TNFα expression in THP-1 cell supernatants at different drug concentrations was calculated based on the TNFα concentration. The inhibition rate data were plotted in GraphPad Prism 8.0 software, and the IC₅₀ value was calculated. $Inhibition Rate = \left[1-\frac{OD \left(Sample\right) - OD \left(Blank\right)}{OD \left(Control\right) - OD \left(Blank\right)}\right] \times 100 %$

**Table 9: Degradation effect of example compounds on IRAK4 in PBMC cells**

| Compounds | IC₅₀ (nM) |
|---|---|
| KT-474 | 52.27 |
| IR-P-1 | 78.57 |
| IR-P-2 | 184 |
| IR-P-3 | 64.28 |
| IR-P-4 | 142 |
| IR-P-5 | 69.57 |
| IR-P-6 | 95.62 |

### Test Example 12: Determination of IL-6 Concentration Secreted by hPBMC Induced by LPS

After thawing, cryopreserved hPBMC (FPB003F-C, Shanghai OriBiotech.) cells were resuspended in RPMI 1640 medium (30-2001, ATCC) containing 10% inactivated FBS (10099141C, Gibco) and cultured overnight at 37°C and 5% CO₂. The next day, hPBMC cells were seeded in a 96-well cell culture plate at a density of 2 × 10⁵ cells/well/100 µL (2 × 10⁶ cells/mL), and 100 µL of compound in dimethyl sulfoxide solution (final concentration 0.0064-2500 nM) was added. After incubation at 37°C, 5% CO₂ for 20 h, 20 µL of LPS (055:B5) (L5418, Sigma) was added to the drug-treated cell plate at a final concentration of 200 ng/mL. The cell plate was placed in a 37°C, 5% CO₂ incubator for 5 h, and then centrifuged at 1000 rpm for 15 min. 100 µL of cell supernatant was collected from each well for cytokine concentration testing, awhich was tested immediately or frozen at -80°C for testing.

### ELISA detection of IL-6

The IL-6 concentration in the supernatant of hPBMC cells induced by LPS was determined using a Human IL-6 ELISA kit (D6050, RD).

IL-6 standard solutions of varying concentrations were prepared according to the product instructions, ranging from 0 to 300 pg/mL. 100 µL of Assay Diluent RD1W and 100 µL of standard solution or sample supernatant were added to a pre-coated ELISA plate and incubated at room temperature for 2 hours. After washing the plate four times with 1× wash buffer, then 200 µL of Human IL-6 Conjugate was added and incubated at room temperature for 2 hours. After washing the plate four times with 1× wash buffer, then 200 µL of Substrate Solution was added and incubated at room temperature for 20 minutes. Finally, 50 µL of Stop Solution was added to terminate the reaction. The absorbance at 450 nm and 570 nm were read using a microplate reader (SPARK m1000pro, TECAN). The standard curve was plottd by the signal value of each concentration of IL-6 standard solution, and the IL-6 concentration in each sample was determined according to the corresponding concentration of the signal value of each well on the standard curve. The inhibition rate of the compound on LPS-induced IL-6 in the hPBMC cell supernatant at different drug concentrations was calculated according to the IL-6 concentration. The inhibition rate data were plotted in GraphPad Prism 8.0 software to calculate the IC₅₀ value. $Inhibition Rate = \left[1-\frac{OD \left(sample\right) - OD \left(blank\right)}{OD \left(control\right) - OD \left(blank\right)}\right] \times 100 %$

**Table 10: Inhibitory effect of Example compounds on LPS-induced IL-6 secretion by hPBMC**

| Compounds | Inhibition Rate of IL-6 (2500nM) | Inhibition Rate of IL-6 (20nM) | |
|---|---|---|---|
| KT-474 | 85% | 63% | |
| IR-P-1 | 87% | - | |
| IR-P-2 | 86% | - | |
| IR-P-3 | 89% | 63% | |
| IR-P-4 | 84% | - | |
| IR-P-5 | 78% | - | |
| IR-P-6 | 65% | - | |
| IR-P-7 | 46% | - | |

| | | | |
|---|---|---|---|
| "-" means not tested. | | | |

The foregoing descriptions of specific exemplary embodiments of the present invention are for purposes of illustration and description. These descriptions are not intended to limit the invention to the precise forms disclosed, and it is apparent that many modifications and variations are possible in light of the foregoing teachings. The exemplary embodiments have been selected and described for the purpose of explaining the specific principles of the invention and their practical application, thereby enabling those skilled in the art to make and utilize a variety of exemplary embodiments of the invention and various options and variations. The scope of the invention is intended to be defined by the claims and their equivalents.

## Claims

1. A compound of Formula I, or an isomer, an isotopic derivative, a polymorph, a prodrug, or a pharmaceutically acceptable salt or a solvate thereof, 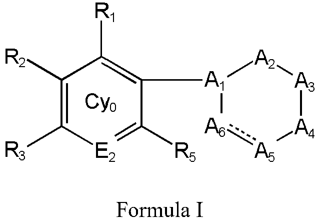
wherein, A₁ is selected from CRₐ and N;
A₂ and A₄ are each independently selected from C(O) and C(Rₐ)₂;
E₂ is selected from CR₄ and N;
A₃ is NRₐ; preferably, A₃ is NH;
the bond between A₅ and A₆ is a single bond or a double bond; when A₅ and A₆ are connected by a single bond, A₅ and A₆ are each independently selected from NRₐ and C(Rₐ)₂; when A₅ and A₆ are connected by a double bond, A₅ and A₆ are each independently selected from N and CRₐ; preferably, A₆ and A₅ are connected by a single bond and are both C(H)₂;
R₁ and R₂, together with the atoms to which they are attached, form a ring Cy₁ fused to Cy₀; R₃, R₄, and R₅, at each occurrence, are each independently selected from H, deuterium atom, halogen, C₁₋₆ alkyl, C₁₋₆ deuterated alkyl, carboxyl, C₁₋₆ heteroalkyl, alkenyl, alkynyl, C₁₋₆ alkoxy, C₁₋₆ haloalkyl, C₁₋₆ haloalkoxy, hydroxyl, C₁₋₆ hydroxyalkyl, nitro, cyano, amino, C₁₋₆ alkylamino, C₁₋₆ alkylcarbonyl, C₁₋₆ alkoxycarbonyl, C₁₋₆ alkylaminocarbonyl, cycloalkyl, heterocyclyl, aryl, and heteroaryl, wherein the C₁₋₆ alkyl, C₁₋₆ heteroalkyl, alkenyl, alkynyl, C₁₋₆ alkoxy, C₁₋₆ haloalkyl, C₁₋₆ haloalkoxy, C₁₋₆ hydroxyalkyl, C₁₋₆ alkylamino, C₁₋₆ alkylcarbonyl, C₁₋₆ alkoxycarbonyl, C₁₋₆ alkylaminocarbonyl, cycloalkyl, heterocyclyl, aryl, and heteroaryl are each independently optionally substituted with one or more substituents selected from halogen, alkyl, heteroalkyl, alkoxy, haloalkyl, hydroxyl, hydroxyalkyl, cyano, amino, nitro, cycloalkyl, heterocyclyl, alkenyl, alkynyl, alkylamino, alkylcarbonyl, alkoxycarbonyl, alkylaminocarbonyl, aryl, and heteroaryl; preferably, R₃, R₄, and R₅, at each occurrence, are each independently selected from H, deuterium atom, halogen, C₁₋₆ alkyl, C₁₋₆ deuterated alkyl, C₁₋₆ heteroalkyl, alkenyl, alkynyl, C₁₋₆ alkoxy, C₁₋₆ haloalkyl, C₁₋₆ haloalkoxy, hydroxyl, C₁₋₆ hydroxyalkyl, and amino; more preferably, R₃, R₄, and R₅, at each occurrence, are each independently selected from H, deuterium atom, F, Cl, Br, I, C₁₋₆ alkyl, C₁₋₆ deuterated alkyl, C₁₋₆ alkoxy, C₁₋₆ haloalkyl, C₁₋₆ haloalkoxy, and hydroxyl; or
R₂ and R₃, together with the atoms to which they are attached, form a ring Cy₂ fused to Cy₀; R₁, R₄, and R₅, at each occurrence, are each independently selected from H, deuterium atom, halogen, C₁₋₆ alkyl, C₁₋₆ deuterated alkyl, carboxyl, C₁₋₆ heteroalkyl, alkenyl, alkynyl, C₁₋₆ alkoxy, C₁₋₆ haloalkyl, C₁₋₆ haloalkoxy, hydroxyl, C₁₋₆ hydroxyalkyl, nitro, cyano, amino, C₁₋₆ alkylamino, C₁₋₆ alkylcarbonyl, C₁₋₆ alkoxycarbonyl, C₁₋₆ alkylaminocarbonyl, cycloalkyl, heterocyclyl, aryl, and heteroaryl, wherein the C₁₋₆ alkyl, C₁₋₆ heteroalkyl, alkenyl, alkynyl, C₁₋₆ alkoxy, C₁₋₆ haloalkyl, C₁₋₆ haloalkoxy, C₁₋₆ hydroxyalkyl, C₁₋₆ alkylamino, C₁₋₆ alkylcarbonyl, C₁₋₆ alkoxycarbonyl, C₁₋₆ alkylaminocarbonyl, cycloalkyl, heterocyclyl, aryl, and heteroaryl are each independently optionally substituted with one or more substituents selected from halogen, alkyl, heteroalkyl, alkoxy, haloalkyl, hydroxyl, hydroxyalkyl, cyano, amino, nitro, cycloalkyl, heterocyclyl, alkenyl, alkynyl, alkylamino, alkylcarbonyl, alkoxycarbonyl, alkylaminocarbonyl, aryl, and heteroaryl; preferably, R₁, R₄, and R₅, at each occurrence, are each independently selected from H, deuterium atom, halogen, C₁₋₆ alkyl, C₁₋₆ deuterated alkyl, C₁₋₆ heteroalkyl, alkenyl, alkynyl, C₁₋₆ alkoxy, C₁₋₆ haloalkyl, C₁₋₆ haloalkoxy, hydroxyl, C₁₋₆ hydroxyalkyl, and amino; more preferably, R₁, R₄, and R₅, at each occurrence, are each independently selected from H, deuterium atom, F, Cl, Br, I, C₁₋₆ alkyl, C₁₋₆ deuterated alkyl, C₁₋₆ alkoxy, C₁₋₆ haloalkyl, C₁₋₆ haloalkoxy, and hydroxyl; or
when R₄ is present, R₃ and R₄, together with the atoms to which they are attached, form a ring Cy₃ fused to Cy₀; R₁, R₂, and R₅, at each occurrence, are each independently selected from H, deuterium atom, halogen, C₁₋₆ alkyl, C₁₋₆ deuterated alkyl, carboxyl, C₁₋₆ heteroalkyl, alkenyl, alkynyl, C₁₋₆ alkoxy, C₁₋₆ haloalkyl, C₁₋₆ haloalkoxy, hydroxyl, C₁₋₆ hydroxyalkyl, nitro, cyano, amino, C₁₋₆ alkylamino, C₁₋₆ alkylcarbonyl, C₁₋₆ alkoxycarbonyl, C₁₋₆ alkylaminocarbonyl, cycloalkyl, heterocyclyl, aryl, and heteroaryl, wherein the C₁₋₆ alkyl, C₁₋₆ heteroalkyl, alkenyl, alkynyl, C₁₋₆ alkoxy, C₁₋₆ haloalkyl, C₁₋₆ haloalkoxy, C₁₋₆ hydroxyalkyl, C₁₋₆ alkylamino, C₁₋₆ alkylcarbonyl, C₁₋₆ alkoxycarbonyl, C₁₋₆ alkylaminocarbonyl, cycloalkyl, heterocyclyl, aryl, and heteroaryl are each independently optionally substituted with one or more substituents selected from halogen, alkyl, heteroalkyl, alkoxy, haloalkyl, hydroxyl, hydroxyalkyl, cyano, amino, nitro, cycloalkyl, heterocyclyl, alkenyl, alkynyl, alkylamino, alkylcarbonyl, alkoxycarbonyl, alkylaminocarbonyl, aryl, and heteroaryl; preferably, R₁, R₂, and R₅, at each occurrence, are each independently selected from H, deuterium atom, halogen, C₁₋₆ alkyl, C₁₋₆ deuterated alkyl, C₁₋₆ heteroalkyl, alkenyl, alkynyl, C₁₋₆ alkoxy, C₁₋₆ haloalkyl, C₁₋₆ haloalkoxy, hydroxyl, C₁₋₆ hydroxyalkyl, and amino; more preferably, R₁, R₂, and R₅, at each occurrence, are each independently selected from H, deuterium atom, F, Cl, Br, I, C₁₋₆ alkyl, C₁₋₆ deuterated alkyl, C₁₋₆ alkoxy, C₁₋₆ haloalkyl, C₁₋₆ haloalkoxy, and hydroxyl; or
when R₄ is present, R₄ and R₅, together with the atoms to which they are attached, form a ring Cy₄ fused to Cy₀; R₁, R₂, and R₃, at each occurrence, are each independently selected from H, deuterium atom, halogen, C₁₋₆ alkyl, C₁₋₆ deuterated alkyl, carboxyl, C₁₋₆ heteroalkyl, alkenyl, alkynyl, C₁₋₆ alkoxy, C₁₋₆ haloalkyl, C₁₋₆ haloalkoxy, hydroxyl, C₁₋₆ hydroxyalkyl, nitro, cyano, amino, C₁₋₆ alkylamino, C₁₋₆ alkylcarbonyl, C₁₋₆ alkoxycarbonyl, C₁₋₆ alkylaminocarbonyl, cycloalkyl, heterocyclyl, aryl, and heteroaryl, wherein the C₁₋₆ alkyl, C₁₋₆ heteroalkyl, alkenyl, alkynyl, C₁₋₆ alkoxy, C₁₋₆ haloalkyl, C₁₋₆ haloalkoxy, C₁₋₆ hydroxyalkyl, C₁₋₆ alkylamino, C₁₋₆ alkylcarbonyl, C₁₋₆ alkoxycarbonyl, C₁₋₆ alkylaminocarbonyl, cycloalkyl, heterocyclyl, aryl, and heteroaryl are each independently optionally substituted with one or more substituents selected from halogen, alkyl, heteroalkyl, alkoxy, haloalkyl, hydroxyl, hydroxyalkyl, cyano, amino, nitro, cycloalkyl, heterocyclyl, alkenyl, alkynyl, alkylamino, alkylcarbonyl, alkoxycarbonyl, alkylaminocarbonyl, aryl, and heteroaryl; preferably, R₁, R₂, and R₃, at each occurrence, are each independently selected from H, deuterium atom, halogen, C₁₋₆ alkyl, C₁₋₆ deuterated alkyl, C₁₋₆ heteroalkyl, C₂₋₆ alkenyl, C₂₋₆ alkynyl, C₁₋₆ alkoxy, C₁₋₆ haloalkyl, C₁₋₆ haloalkoxy, hydroxyl, C₁₋₆ hydroxyalkyl, and amino; more preferably, R₁, R₂, and R₃, at each occurrence, are each independently selected from H, deuterium atom, F, Cl, Br, I, C₁₋₆ alkyl, C₁₋₆ deuterated alkyl, C₁₋₆ alkoxy, C₁₋₆ haloalkyl, C₁₋₆ haloalkoxy, and hydroxyl;
exactly one of Cy₁, Cy₂, Cy₃, and Cy₄ is present, and is selected from structures 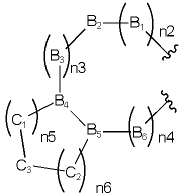 and 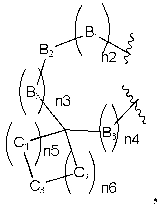 wherein,
B₂ is selected from C(Rₐ)₂, NRₐ, O, and S; and when exactly one of Cy₁, Cy₂, Cy₃, and Cy₄ is present as the structure 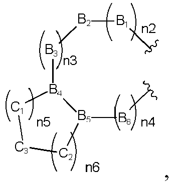 B₂ is selected from NRₐ, O, and S;
B₄ and B₅ are each independently selected from CRₐ and N;
B₁ and B₃, at each occurrence, are each independently selected from C(Rₐ)₂ and C(O);
B₆, at each occurrence, is selected from C(Rₐ)₂ and NRₐ;
C₃ is selected from C(O) and NRₛ₁;
Rₛ₁ is selected from H, deuterium atom, F, Cl, Br, I, C₁₋₆ alkyl, deuterated C₁₋₆ alkyl, carboxyl, C₁₋₆ heteroalkyl, C₂₋₆ alkenyl, C₂₋₆ alkynyl, -C(O)-C₁₋₆ alkyl, C₁₋₆ alkoxy, C₁₋₆ haloalkyl, C₁₋₆ haloalkoxy, hydroxyl, C₁₋₆ hydroxyalkyl, nitro, cyano, and amino; preferably, Rₛ₁ is selected from H, deuterium atom, F, Cl, Br, I, C₁₋₆ alkyl, deuterated C₁₋₆ alkyl, carboxyl, C₁₋₆ heteroalkyl, C₂₋₆ alkenyl, C₂₋₆ alkynyl, -C(O)-C₁₋₆ alkyl, C₁₋₆ alkoxy, C₁₋₆ haloalkyl, C₁₋₆ haloalkoxy, hydroxyl, and C₁₋₆ hydroxyalkyl; more preferably, Rₛ₁ is selected from H, deuterium atom, F, Cl, Br, I, C₁-₃ alkyl, deuterated C₁-₃ alkyl, carboxyl, -C(O)-C₁-₃ alkyl, C₁-₃ alkoxy, C₁-₃ haloalkyl, and C₁-₃ haloalkoxy;
n2, n3, n4, n5, and n6 are each independently selected from 0, 1, 2, and 3; and n5 and n6 are not both 0;
preferably, when exactly one of Cy₁, Cy₂, Cy₃, and Cy₄ is present as the structure 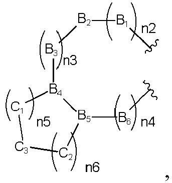 and when B₂ is selected from O and S, n2+n3+n4 is greater than or equal to 2;
C₁ and C₂, at each occurrence, are each independently C(Rₐ)₂;
Rₐ, at each occurrence, is each independently selected from H, deuterium atom, F, Cl, Br, I, C₁₋₆ alkyl, deuterated C₁₋₆ alkyl, carboxyl, C₁₋₆ heteroalkyl, C₂₋₆ alkenyl, C₂₋₆ alkynyl, -C(O)-C₁₋₆ alkyl, C₁₋₆ alkoxy, C₁₋₆ haloalkyl, C₁₋₆ haloalkoxy, hydroxyl, C₁₋₆ hydroxyalkyl, nitro, cyano, amino, C₁₋₆ alkylamino, C₁₋₆ alkylcarbonyl, C₁₋₆ alkoxycarbonyl, C₁₋₆ alkylaminocarbonyl, 3-10-membered cycloalkyl, 3-10-membered heterocyclyl, 6-10-membered aryl, and 5-10-membered heteroaryl, wherein the C₁₋₆ alkyl, C₁₋₆ heteroalkyl, C₂₋₆ alkenyl, C₂₋₆ alkynyl, -C(O)-C₁₋₆ alkyl, C₁₋₆ alkoxy, C₁₋₆ haloalkyl, C₁₋₆ haloalkoxy, C₁₋₆ hydroxyalkyl, C₁₋₆ alkylamino, C₁₋₆ alkylcarbonyl, C₁₋₆ alkoxycarbonyl, C₁₋₆ alkylaminocarbonyl, 3-10-membered cycloalkyl, 3-10-membered heterocyclyl, 6-10-membered aryl, and 5-10-membered heteroaryl are each independently optionally substituted with one or more substituents selected from halogen, alkyl, heteroalkyl, alkoxy, haloalkyl, hydroxyl, hydroxyalkyl, cyano, amino, nitro, cycloalkyl, heterocyclyl, alkenyl, alkynyl, alkylamino, alkylcarbonyl, alkoxycarbonyl, alkylaminocarbonyl, aryl, and heteroaryl; preferably, Rₐ, at each occurrence, is each independently selected from H, deuterium atom, F, Cl, Br, I, C₁₋₆ alkyl, C₁₋₆ deuterated alkyl, C₁₋₆ heteroalkyl, C₂₋₆ alkenyl, C₂₋₆ alkynyl, C₁₋₆ alkoxy, C₁₋₆ haloalkyl, C₁₋₆ haloalkoxy, hydroxyl, C₁₋₆ hydroxyalkyl, and amino; more preferably, Rₐ, at each occurrence, is each independently selected from H, deuterium atom, F, Cl, Br, I, C₁-₃ alkyl, C₁-₃ deuterated alkyl, C₁-₃ alkoxy, C₁-₃ haloalkyl, C₁-₃ haloalkoxy, and hydroxyl;
in the structures of Cy₁, Cy₂, Cy₃, or Cy₄, 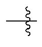 represents the point of attachment to Cy₀;
the compound of Formula I is not the following structures:

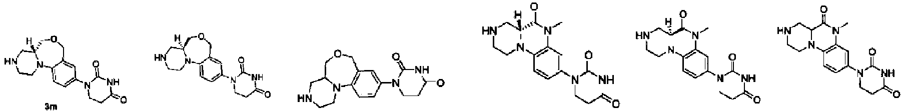

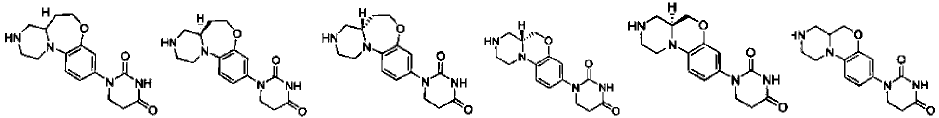

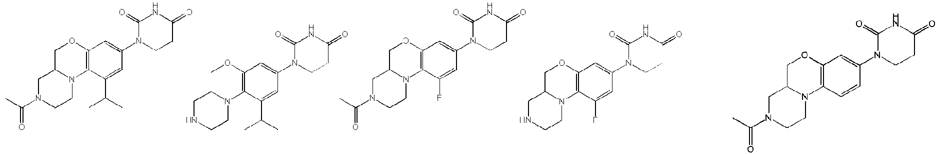

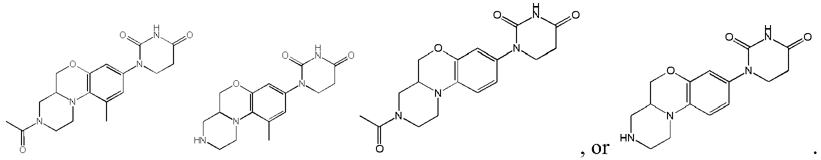

2. A compound of Formula 2, or an isomer, an isotopic derivative, a polymorph, a prodrug, or a pharmaceutically acceptable salt or a solvate thereof, 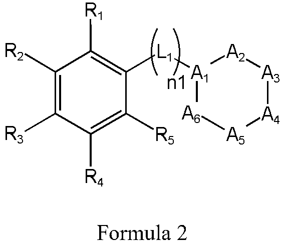 wherein,
A₁ is selected from CRₐ and N;
A₂ and A₄ are each independently selected from C(O) and C(Rₐ)₂;
A₃ is NRₐ;
A₅ and A₆ are each independently selected from a single bond, NRₐ, and C(Rₐ)₂; preferably, A₅ and A₆ are each independently C(Rₐ)₂;
L₁, at each occurrence, is each independently selected from a single bond, alkylene, haloalkylene, -O-, - N(R_{b})C(O)-, -C(O)-, -OC(O)-, -NR_{b}-, -S-, -SO-, -SO₂-, cycloalkylene, heterocyclylene, arylene, and heteroarylene, wherein the cycloalkylene, heterocyclylene, arylene, and heteroarylene are optionally substituted with 0-6 R_{b} groups; preferably, L₁ is a single bond;
n1 is selected from 1, 2, 3, 4, and 5;
R₁ and R₂, together with the atoms to which they are attached, form a 3-10-membered heterocycloalkyl, a 3-10-membered cycloalkyl, a 6-10-membered aryl, a 6-10-membered heteroaryl, a 5-15-membered fused ring group or heterofused ring group, or a 5-15-membered spiro ring or heterospiro ring group; the 3-10-membered heterocycloalkyl, 3-10-membered cycloalkyl, 6-10-membered aryl, 6-10-membered heteroaryl, 5-15-membered fused ring group or heterofused ring group, or 5-15-membered spiro ring or heterospiro ring group are optionally substituted with 1, 2, or more substituents selected from halogen, carboxyl, alkoxy, alkyl, hydroxyl, amino, haloalkyl, 3-10-membered heterocycloalkyl, 3-10-membered cycloalkyl, 6-10-membered aryl, and 6-10-membered heteroaryl; when R₅ is present, R₃, R₄, and R₅, at each occurrence, are each independently selected from H, deuterium atom, carboxyl, halogen, alkyl, deuterated alkyl, heteroalkyl, alkenyl, alkynyl, alkoxy, haloalkyl, haloalkoxy, hydroxyl, hydroxyalkyl, nitro, cyano, amino, alkylamino, alkylcarbonyl, alkoxycarbonyl, alkylaminocarbonyl, cycloalkyl, heterocyclyl, aryl, and heteroaryl; preferably, R₁ and R₂, together with the atoms to which they are attached, form a 5-15-membered heterofused ring group, or a 5-15-membered spiro ring or heterospiro ring group; the 5-15-membered heterofused ring group, or 5-15-membered spiro ring or heterospiro ring group are optionally substituted with 1, 2, or more substituents selected from halogen, carboxyl, alkoxy, alkyl, hydroxyl, amino, haloalkyl, 3-10-membered heterocycloalkyl, 3-10-membered cycloalkyl, 6-10-membered aryl, and 6-10-membered heteroaryl; R₃, R₄, and R₅, at each occurrence, are each independently selected from H, deuterium atom, carboxyl, halogen, alkyl, deuterated alkyl, heteroalkyl, alkenyl, alkynyl, alkoxy, haloalkyl, haloalkoxy, hydroxyl, hydroxyalkyl, nitro, cyano, amino, alkylamino, alkylcarbonyl, alkoxycarbonyl, alkylaminocarbonyl, cycloalkyl, heterocyclyl, aryl, and heteroaryl; or
R₂ and R₃, together with the atoms to which they are attached, form a 3-10-membered heterocycloalkyl, a 3-10-membered cycloalkyl, a 6-10-membered aryl, a 6-10-membered heteroaryl, a 5-15-membered fused ring group or heterofused ring group, or a 5-15-membered spiro ring or heterospiro ring group; the 3-10-membered heterocycloalkyl, 3-10-membered cycloalkyl, 6-10-membered aryl, 6-10-membered heteroaryl, 5-15-membered fused ring group or heterofused ring group, or 5-15-membered spiro ring or heterospiro ring group are optionally substituted with 1, 2, or more substituents selected from halogen, carboxyl, alkoxy, alkyl, hydroxyl, amino, haloalkyl, 3-10-membered heterocycloalkyl, 3-10-membered cycloalkyl, 6-10-membered aryl, and 6-10-membered heteroaryl; R₁, R₄, and R₅, at each occurrence, are each independently selected from H, deuterium atom, carboxyl, halogen, alkyl, deuterated alkyl, heteroalkyl, alkenyl, alkynyl, alkoxy, haloalkyl, haloalkoxy, hydroxyl, hydroxyalkyl, nitro, cyano, amino, alkylamino, alkylcarbonyl, alkoxycarbonyl, alkylaminocarbonyl, cycloalkyl, heterocyclyl, aryl, and heteroaryl; preferably, R₂ and R₃, together with the atoms to which they are attached, form a 5-15-membered fused ring group or heterofused ring group, or a 5-15-membered spiro ring or heterospiro ring group; the 5-15-membered heterofused ring group, or 5-15-membered spiro ring or heterospiro ring group are optionally substituted with 1, 2, or more substituents selected from halogen, carboxyl, alkoxy, alkyl, hydroxyl, amino, haloalkyl, 3-10-membered heterocycloalkyl, 3-10-membered cycloalkyl, 6-10-membered aryl, and 6-10-membered heteroaryl; R₁, R₄, and R₅, at each occurrence, are each independently selected from H, deuterium atom, carboxyl, halogen, alkyl, deuterated alkyl, heteroalkyl, alkenyl, alkynyl, alkoxy, haloalkyl, haloalkoxy, hydroxyl, hydroxyalkyl, nitro, cyano, amino, alkylamino, alkylcarbonyl, alkoxycarbonyl, alkylaminocarbonyl, cycloalkyl, heterocyclyl, aryl, and heteroaryl; or
R₃ and R₄, together with the atoms to which they are attached, form a 3-10-membered heterocycloalkyl, a 3-10-membered cycloalkyl, a 6-10-membered aryl, a 6-10-membered heteroaryl, a 5-15-membered fused ring group or heterofused ring group, or a 5-15-membered spiro ring or heterospiro ring group; the 3-10-membered heterocycloalkyl, 3-10-membered cycloalkyl, 6-10-membered aryl, 6-10-membered heteroaryl, 4-15-membered fused ring group or heterofused ring group, and 5-15-membered spiro ring or heterospiro ring group are optionally substituted with 1, 2, or more substituents selected from halogen, carboxyl, alkoxy, alkyl, hydroxyl, amino, haloalkyl, 3-10-membered heterocycloalkyl, 3-10-membered cycloalkyl, 6-10-membered aryl, and 6-10-membered heteroaryl; R₁, R₂, and R₅, at each occurrence, are each independently selected from H, deuterium atom, carboxyl, halogen, alkyl, deuterated alkyl, heteroalkyl, alkenyl, alkynyl, alkoxy, haloalkyl, haloalkoxy, hydroxyl, hydroxyalkyl, nitro, cyano, amino, alkylamino, alkylcarbonyl, alkoxycarbonyl, alkylaminocarbonyl, cycloalkyl, heterocyclyl, aryl, and heteroaryl; preferably, R₃ and R₄, together with the atoms to which they are attached, form a 5-15-membered heterofused ring group, or a 5-15-membered spiro ring or heterospiro ring group; the 5-15-membered heterofused ring group, or 5-15-membered spiro ring or heterospiro ring group are optionally substituted with 1, 2, or more substituents selected from halogen, carboxyl, alkoxy, alkyl, hydroxyl, amino, haloalkyl, 3-10-membered heterocycloalkyl, 3-10-membered cycloalkyl, 6-10-membered aryl, and 6-10-membered heteroaryl; R₁, R₄, and R₅, at each occurrence, are each independently selected from H, deuterium atom, carboxyl, halogen, alkyl, deuterated alkyl, heteroalkyl, alkenyl, alkynyl, alkoxy, haloalkyl, haloalkoxy, hydroxyl, hydroxyalkyl, nitro, cyano, amino, alkylamino, alkylcarbonyl, alkoxycarbonyl, alkylaminocarbonyl, cycloalkyl, heterocyclyl, aryl, and heteroaryl; or
R₄ and R₅, together with the atoms to which they are attached, form a 3-10-membered heterocycloalkyl, a 3-10-membered cycloalkyl, a 6-10-membered aryl, a 6-10-membered heteroaryl, a 5-15-membered fused ring group or heterofused ring group, or a 5-15-membered spiro ring or heterospiro ring group, the 3-10-membered heterocycloalkyl, 3-10-membered cycloalkyl, 6-10-membered aryl, 6-10-membered heteroaryl, 5-15-membered fused ring group or heterofused ring group, or 5-15-membered spiro ring or heterospiro ring group are optionally substituted with 1, 2, or more substituents selected from halogen, carboxyl, alkoxy, alkyl, hydroxyl, amino, haloalkyl, 3-10-membered heterocycloalkyl, 3-10-membered cycloalkyl, 6-10-membered aryl, and 6-10-membered heteroaryl; R₁, R₂, and R₃, at each occurrence, are each independently selected from H, deuterium atom, carboxyl, halogen, alkyl, deuterated alkyl, heteroalkyl, alkenyl, alkynyl, alkoxy, haloalkyl, haloalkoxy, hydroxyl, hydroxyalkyl, nitro, cyano, amino, alkylamino, alkylcarbonyl, alkoxycarbonyl, alkylaminocarbonyl, cycloalkyl, heterocyclyl, aryl, and heteroaryl; preferably, R₄ and R₅, together with the atoms to which they are attached, form a 5-15-membered heterofused ring group, or a 5-15-membered spiro ring or heterospiro ring group, the 5-15-membered heterofused ring group, or 5-15-membered spiro ring or heterospiro ring group are optionally substituted with 1, 2, or more substituents selected from halogen, carboxyl, alkoxy, alkyl, hydroxyl, amino, haloalkyl, 3-10-membered heterocycloalkyl, 3-10-membered cycloalkyl, 6-10-membered aryl, and 6-10-membered heteroaryl; R₁, R₂, and R₃, at each occurrence, are each independently selected from H, deuterium atom, carboxyl, halogen, alkyl, deuterated alkyl, heteroalkyl, alkenyl, alkynyl, alkoxy, haloalkyl, haloalkoxy, hydroxyl, hydroxyalkyl, nitro, cyano, amino, alkylamino, alkylcarbonyl, alkoxycarbonyl, alkylaminocarbonyl, cycloalkyl, heterocyclyl, aryl, and heteroaryl;
Rₐ and R_{b}, at each occurrence, are each independently selected from H, deuterium atom, halogen, alkyl, deuterated alkyl, carboxyl, heteroalkyl, alkenyl, alkynyl, alkoxy, haloalkyl, haloalkoxy, hydroxyl, hydroxyalkyl, nitro, cyano, amino, alkylamino, alkylcarbonyl, alkoxycarbonyl, alkylaminocarbonyl, cycloalkyl, heterocyclyl, aryl, and heteroaryl, wherein the alkyl, heteroalkyl, alkenyl, alkynyl, alkoxy, cycloalkyl, heterocyclyl, aryl, and heteroaryl are each independently optionally substituted with one or more substituents selected from halogen, alkyl, heteroalkyl, alkoxy, haloalkyl, hydroxyl, hydroxyalkyl, cyano, amino, nitro, cycloalkyl, heterocyclyl, alkenyl, alkynyl, alkylamino, alkylcarbonyl, alkoxycarbonyl, alkylaminocarbonyl, aryl, and heteroaryl; preferably, Rₐ and R_{b}, at each occurrence, are each independently selected from H, F, Cl, Br, I, C₁-₃ alkyl, C₁-₃ alkoxy, hydroxyl, amino, and -C(O)-C₁-₃ alkyl;
the compound is not the following structures:

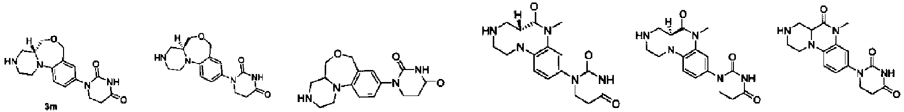

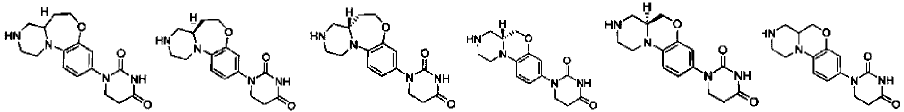

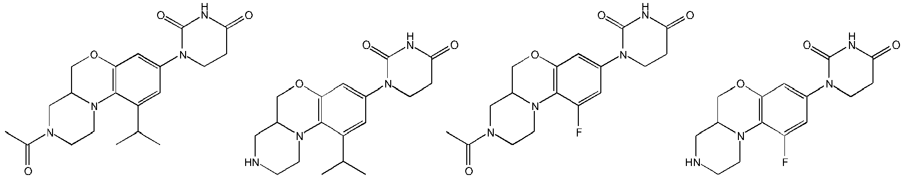

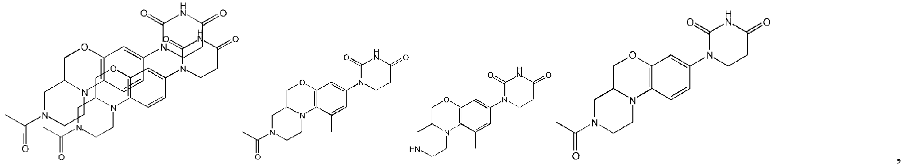

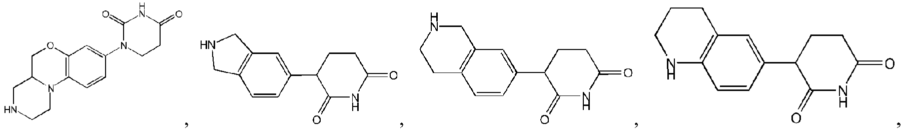

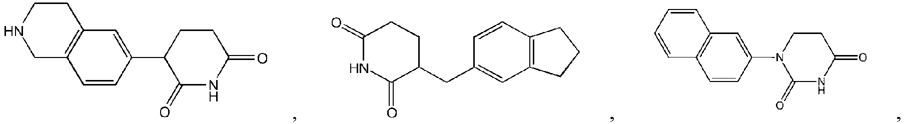

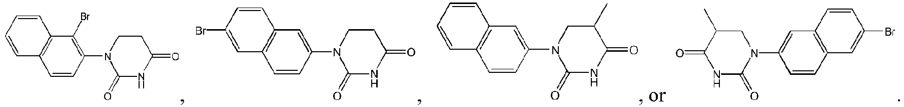

3. The compound according to claim 2, or an isomer, an isotopic derivative, a polymorph, a prodrug, or a pharmaceutically acceptable salt or a solvate thereof,
wherein,
at least one of A₂ and A₄ is C(O); preferably, both A and A₄ are C(O); and/or
A₃ is NH; and/or
A₅ and A₆ are each independently selected from a single bond and C(H)₂; preferably, A₅ and A₆ are C(H)₂; and/or
L₁, at each occurrence, is each independently selected from a single bond, C₁₋₆ alkylene, C₁₋₆ haloalkylene, -O-, -N(R_{b})C(O)-, -C(O)-, -OC(O)-, -NR_{b}-, -S-, -SO-, -SO₂-, C₃-₁₅ cycloalkylene, C₃-₁₅ heterocyclylene, C₆-₁₅ arylene, and C₅-₁₅ heteroarylene, wherein the cycloalkylene, heterocyclylene, arylene, and heteroarylene are optionally substituted with 0-6 R_{b} groups; preferably, L₁ is a single bond; and/or
n1 is selected from 1, 2, 3; and/or
R₁ and R₂, together with the atoms to which they are attached, form a 3-10-membered saturated or unsaturated heterocycloalkyl containing 1, 2, or 3 N, O, S atoms, a 6-10-membered aryl, 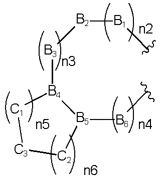 or 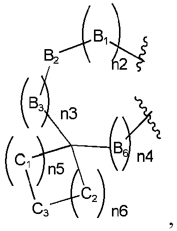 wherein the 3-10-membered saturated or unsaturated heterocycloalkyl containing 1, 2, or 3 N, O, S atoms, and 6-10-membered aryl are optionally substituted with 1, 2, or more substituents selected from halogen, carboxyl, C₁₋₆ alkoxy, C₁₋₆ alkyl, hydroxyl, amino, C₁₋₆ haloalkyl, 3-10-membered heterocycloalkyl, 3-10-membered cycloalkyl, 6-10-membered aryl, and 6-10-membered heteroaryl; when R₅ is present, R₃, R₄, and R₅, at each occurrence, are each independently selected from H, deuterium atom, carboxyl, halogen, C₁₋₆ alkyl, C₁₋₆ deuterated alkyl, C₁₋₆ heteroalkyl, alkenyl, alkynyl, C₁₋₆ alkoxy, C₁₋₆ haloalkyl, C₁₋₆ haloalkoxy, hydroxyl, C₁₋₆ hydroxyalkyl, nitro, cyano, amino, C₁₋₆ alkylamino, C₁₋₆ alkylcarbonyl, C₁₋₆ alkoxycarbonyl, C₁₋₆ alkylaminocarbonyl, cycloalkyl, heterocyclyl, aryl, and heteroaryl; preferably, R₁ and R₂, together with the atoms to which they are attached, form 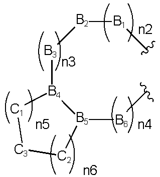 or 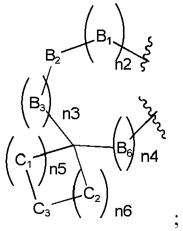
R₂ and R₃, together with the atoms to which they are attached, form a 3-10-membered saturated or unsaturated heterocycloalkyl containing 1, 2, or 3 N, O, S atoms, a 6-10-membered aryl, 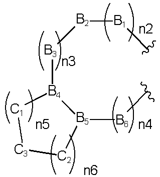 or 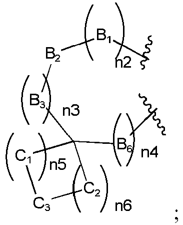 wherein the 3-10-membered saturated or unsaturated heterocycloalkyl containing 1, 2, or 3 N, O, S atoms, and 6-10-membered aryl are optionally substituted with 1, 2, or more substituents selected from halogen, carboxyl, alkoxy, alkyl, hydroxyl, amino, haloalkyl, 3-10-membered heterocycloalkyl, 3-10-membered cycloalkyl, 6-10-membered aryl, and 6-10-membered heteroaryl; when R₅ is present, R₁, R₄, and R₅, at each occurrence, are each independently selected from H, deuterium atom, carboxyl, halogen, C₁₋₆ alkyl, C₁₋₆ deuterated alkyl, C₁₋₆ heteroalkyl, alkenyl, alkynyl, C₁₋₆ alkoxy, C₁₋₆ haloalkyl, C₁₋₆ haloalkoxy, hydroxyl, C₁₋₆ hydroxyalkyl, nitro, cyano, amino, C₁₋₆ alkylamino, C₁₋₆ alkylcarbonyl, C₁₋₆ alkoxycarbonyl, C₁₋₆ alkylaminocarbonyl, cycloalkyl, heterocyclyl, aryl, and heteroaryl; preferably, R₂ and R₃, together with the atoms to which they are attached, form 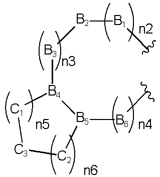 or 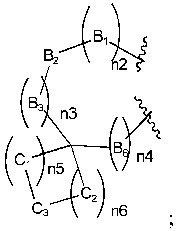 or
R₃ and R₄, together with the atoms to which they are attached, form a 3-10-membered saturated or unsaturated heterocycloalkyl containing 1, 2, or 3 N, O, S atoms, a 6-10-membered aryl ring, 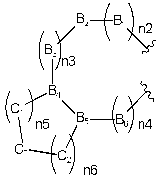 or 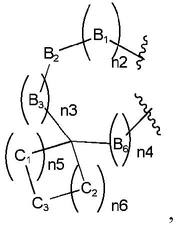 wherein the 3-10-membered saturated or unsaturated heterocycloalkyl containing 1, 2, or 3 N, O, S atoms, and 6-10-membered aryl are optionally substituted with 1, 2, or more substituents selected from halogen, carboxyl, alkoxy, alkyl, hydroxyl, amino, haloalkyl, 3-10-membered heterocycloalkyl, 3-10-membered cycloalkyl, 6-10-membered aryl, and 6-10-membered heteroaryl; when R₅ is present, R₁, R₂, and R₅, at each occurrence, are each independently selected from H, deuterium atom, carboxyl, halogen, C₁₋₆ alkyl, C₁₋₆ deuterated alkyl, C₁₋₆ heteroalkyl, alkenyl, alkynyl, C₁₋₆ alkoxy, C₁₋₆ haloalkyl, C₁₋₆ haloalkoxy, hydroxyl, C₁₋₆ hydroxyalkyl, nitro, cyano, amino, C₁₋₆ alkylamino, C₁₋₆ alkylcarbonyl, C₁₋₆ alkoxycarbonyl, C₁₋₆ alkylaminocarbonyl, cycloalkyl, heterocyclyl, aryl, and heteroaryl; preferably, R₃ and R₄, together with the atoms to which they are attached, form 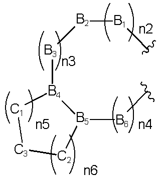 or 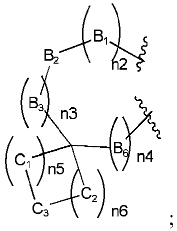 or
R₄ and R₅, together with the atoms to which they are attached, form a 3-10-membered saturated or unsaturated heterocycloalkyl containing 1, 2, or 3 N, O, S atoms, a 6-10-membered aryl, 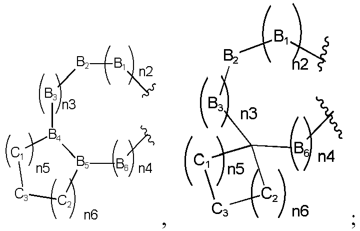 the 3-10-membered saturated or unsaturated heterocycloalkyl containing 1, 2, or 3 N, O, S atoms, and 6-10-membered aryl are optionally substituted with 1, 2, or more substituents selected from halogen, carboxyl, alkoxy, alkyl, hydroxyl, amino, haloalkyl, 3-10-membered heterocycloalkyl, 3-10-membered cycloalkyl, 6-10-membered aryl, and 6-10-membered heteroaryl; R₁, R₂, and R₃, at each occurrence, are each independently selected from H, deuterium atom, carboxyl, halogen, C₁₋₆ alkyl, C₁₋₆ deuterated alkyl, C₁₋₆ heteroalkyl, alkenyl, alkynyl, C₁₋₆ alkoxy, C₁₋₆ haloalkyl, C₁₋₆ haloalkoxy, hydroxyl, C₁₋₆ hydroxyalkyl, nitro, cyano, amino, C₁₋₆ alkylamino, C₁₋₆ alkylcarbonyl, C₁₋₆ alkoxycarbonyl, C₁₋₆ alkylaminocarbonyl, cycloalkyl, heterocyclyl, aryl, and heteroaryl; preferably, R₄ and R₅ together with the atoms to which they are attached form 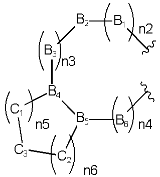 or 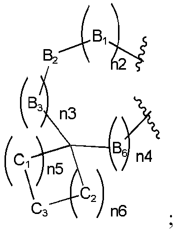
B₂, at each occurrence, is selected from C(Rₐ)₂, NRₐ, O, and S;
B₄ and B₅, at each occurrence, are each independently selected from CRₐ and N;
C₃, at each occurrence, is selected from C(O), CR_{c}R_{d}, and NRₐ;
R_{c} and R_{d}, at each occurrence, are each independently selected from H, deuterium atom, halogen, C₁₋₆ alkyl, C₁₋₆ deuterated alkyl, carboxyl, C₁₋₆ heteroalkyl, alkenyl, alkynyl, C₁₋₆ alkoxy, C₁₋₆ haloalkyl, C₁₋₆ haloalkoxy, hydroxyl, C₁₋₆ hydroxyalkyl, nitro, cyano, amino, C₁₋₆ alkylamino, C₁₋₆ alkylcarbonyl, C₁₋₆ alkoxycarbonyl, C₁₋₆ alkylaminocarbonyl, cycloalkyl, heterocyclyl, aryl, and heteroaryl, wherein the alkyl, heteroalkyl, alkenyl, alkynyl, alkoxy, cycloalkyl, heterocyclyl, aryl, and heteroaryl are each independently optionally substituted with one or more substituents selected from halogen, alkyl, heteroalkyl, alkoxy, haloalkyl, hydroxyl, hydroxyalkyl, cyano, amino, nitro, cycloalkyl, heterocyclyl, alkenyl, alkynyl, alkylamino, alkylcarbonyl, alkoxycarbonyl, alkylaminocarbonyl, aryl, and heteroaryl; or R_{c} and R_{d}, together with the carbon atom to which they are attached, form a 3-10-membered heterocycloalkyl, a 3-10-membered cycloalkyl, a 6-10-membered aryl, a 6-10-membered heteroaryl, wherein the 3-10-membered heterocycloalkyl, 3-10-membered cycloalkyl, 6-10-membered aryl, and 6-10-membered heteroaryl are optionally substituted with 1, 2, or more substituents selected from halogen, carboxyl, alkoxy, alkyl, hydroxyl, amino, haloalkyl, 3-10-membered heterocycloalkyl, 3-10-membered cycloalkyl, 6-10-membered aryl, and 6-10-membered heteroaryl;
n2, n3, n4, n5, and n6 are each independently selected from 0, 1, 2, and 3;
B₁, B₃, B₆, C₁, C₂, D₁, and D₂, at each occurrence, are each independently selected from C(Rₐ)₂;
D₃ is selected from C(O) and NRₐ; and/or
Rₐ and R_{b}, at each occurrence, are each independently selected from H, deuterium atom, F, Cl, Br, I, C₁₋₆ alkyl, deuterated C₁₋₆ alkyl, carboxyl, C₁₋₆ heteroalkyl, C₂₋₆ alkenyl, C₂₋₆ alkynyl, C₁₋₆ alkoxy, C₁₋₆ haloalkyl, C₁₋₆ haloalkoxy, hydroxyl, C₁₋₆ hydroxyalkyl, nitro, cyano, amino, C₁₋₆ alkylamino, C₁₋₆ alkylcarbonyl, C₁₋₆ alkoxycarbonyl, C₁₋₆ alkylaminocarbonyl, 3-10-membered cycloalkyl, 3-10-membered heterocyclyl, 6-10-membered aryl, and 5-10-membered heteroaryl, wherein the alkyl, heteroalkyl, alkenyl, alkynyl, alkoxy, cycloalkyl, heterocyclyl, aryl, and heteroaryl are each independently optionally substituted with one or more substituents selected from halogen, alkyl, heteroalkyl, alkoxy, haloalkyl, hydroxyl, hydroxyalkyl, cyano, amino, nitro, cycloalkyl, heterocyclyl, alkenyl, alkynyl, alkylamino, alkylcarbonyl, alkoxycarbonyl, alkylaminocarbonyl, aryl, and heteroaryl; 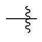 represents the point of attachment.

4. The compound according to any one of claims 1 to 3, wherein the compound is selected from the following structures: 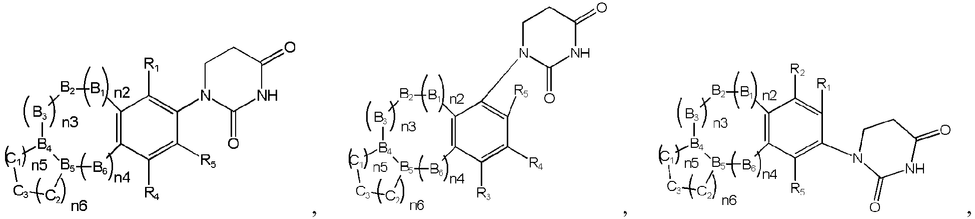 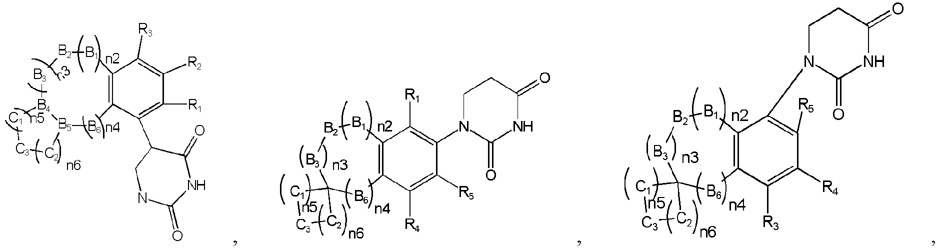 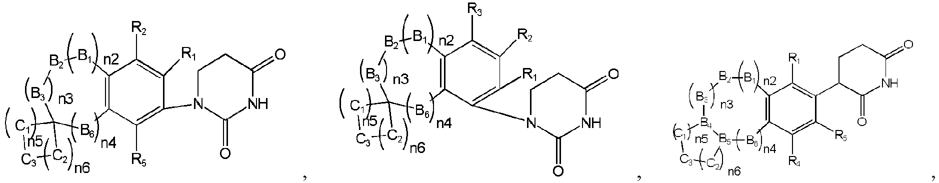 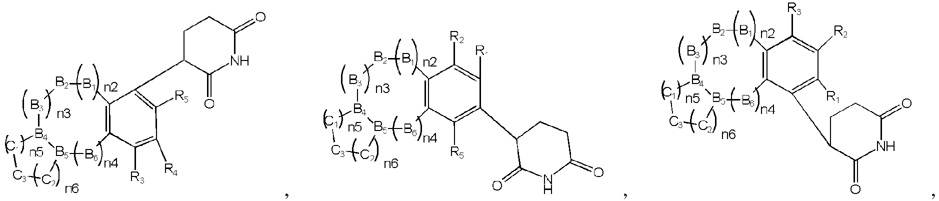 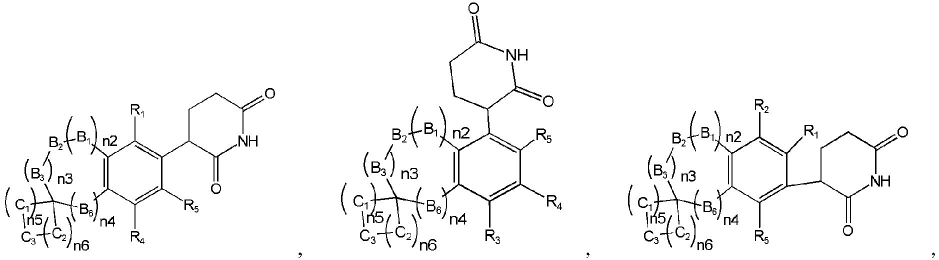 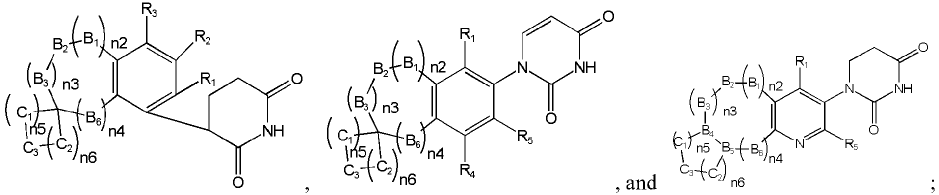
wherein, R₁, R₂, R₃, R₄, R₅, B₁, B₂, B₃, B₄, B₅, B₆, C₁, C₂, C₃, Rₐ, n2, n3, n4, n5, and n6 are as defined in any one of claims 1 to 3;
preferably:
R₁, R₂, R₃, R₄, and R₅ are each independently selected from H, F, Cl, Br, I, C₁-₃ alkyl, C₁-₃ alkoxy, hydroxyl, and amino;
B₂, at each occurrence, is selected from C(Rₐ)₂, NRₐ, O, and S;
B₁ and B₃, at each occurrence, are each independently selected from C(Rₐ)₂ and CO;
B₆, at each occurrence, is selected from C(Rₐ)₂ and NRₐ;
One of B₄ and B₅ is N, and the other is CRₐ;
C₁ and C₂, at each occurrence, are each independently C(Rₐ)₂;
C₃ is selected from C(O) and NRₐ;
Rₐ, at each occurrence, is each independently selected from H, F, Cl, Br, I, C₁-₃ alkyl, C₁-₃ alkoxy, hydroxyl, amino, and -C(O)-C₁-₃ alkyl; and
n2, n3, n4, n5, and n6 are each independently selected from 0, 1, 2, and 3.

5. The compound according to any one of claims 1 to 4, wherein the compound is selected from any one of the following structures: 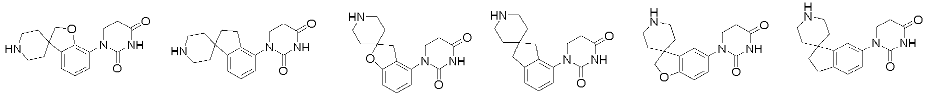 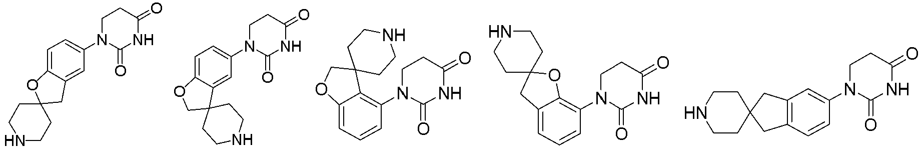 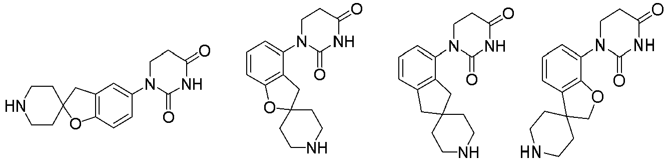 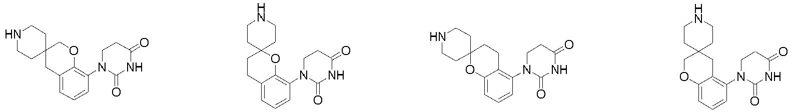 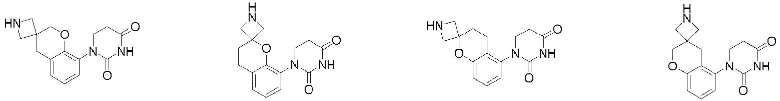 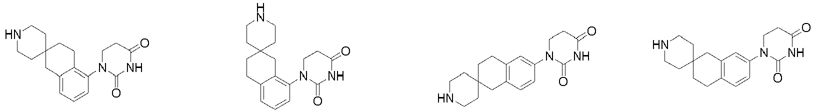 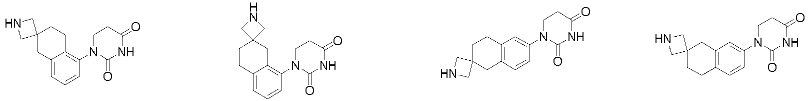 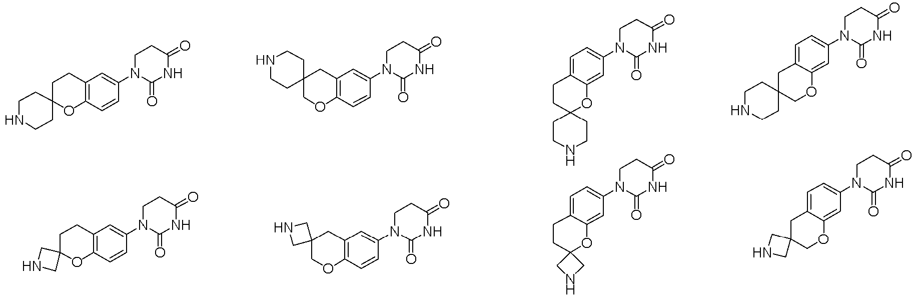 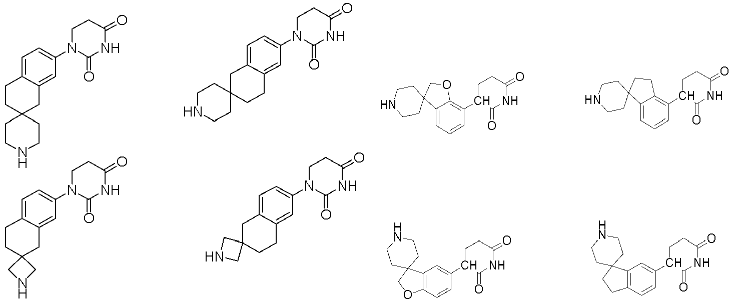 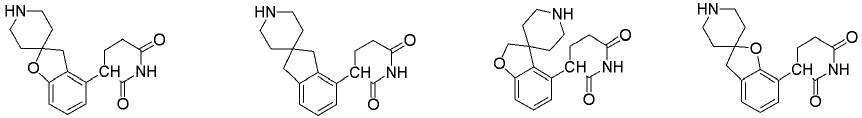 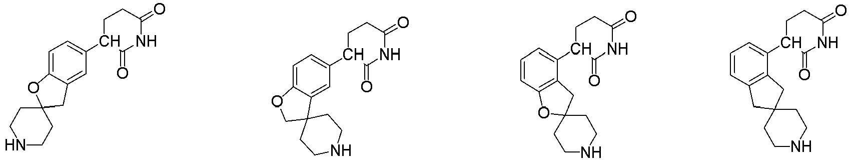 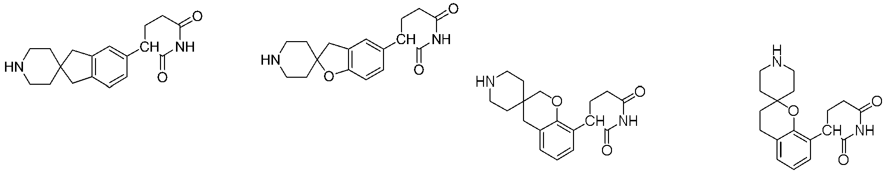 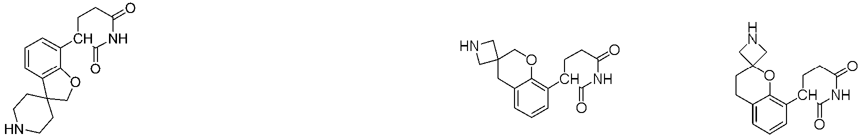 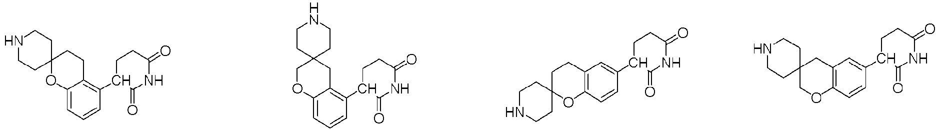 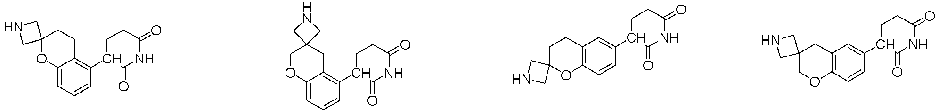 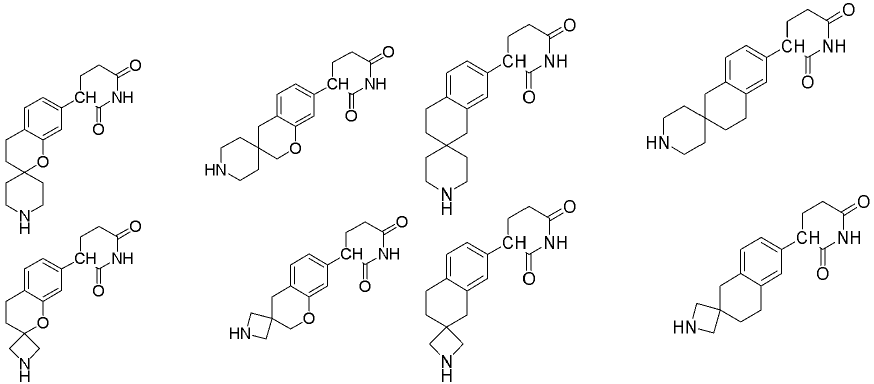 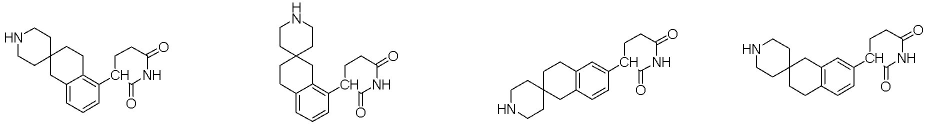 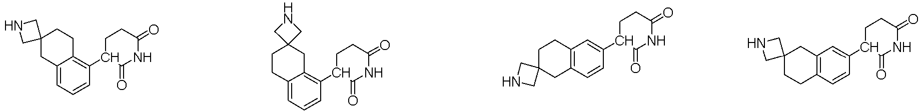 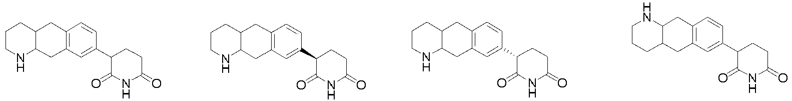 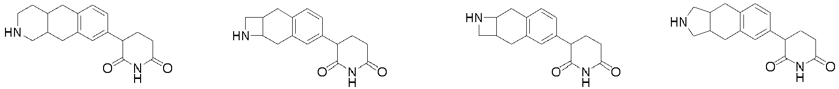 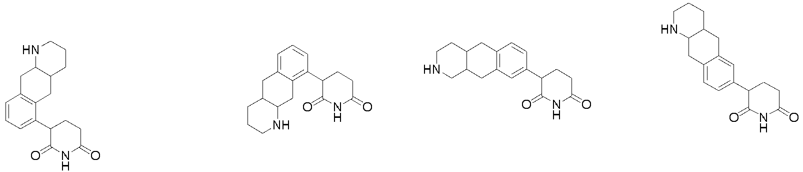 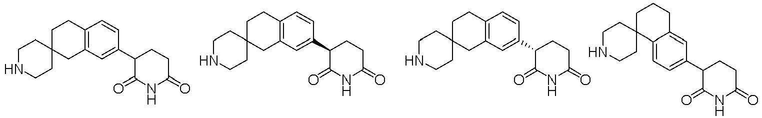 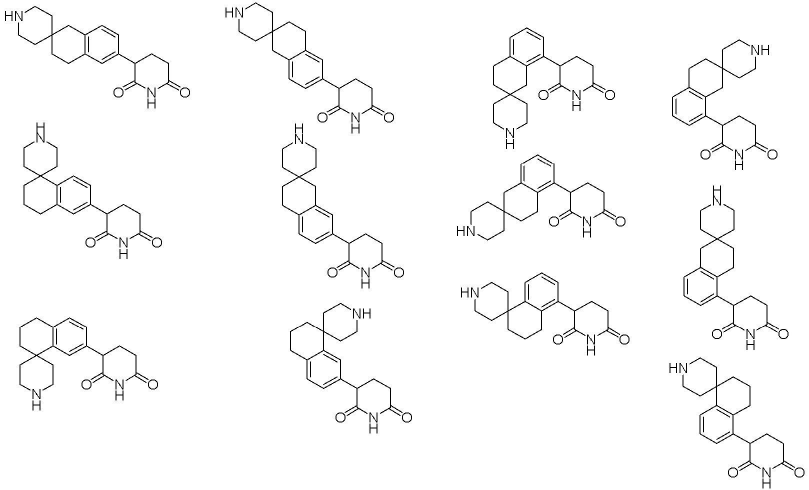 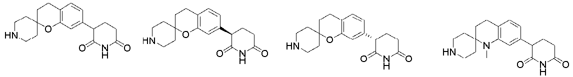 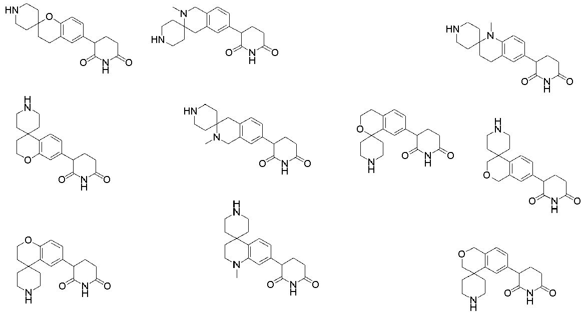 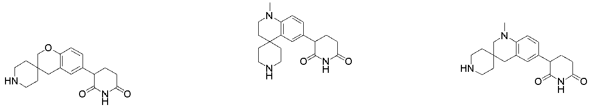 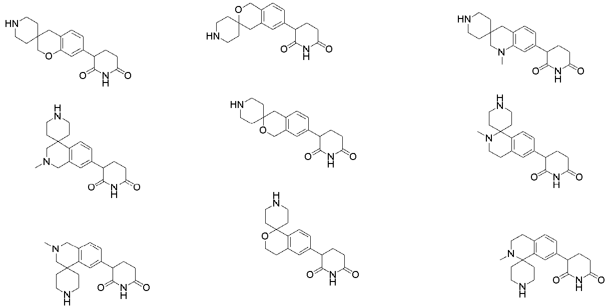 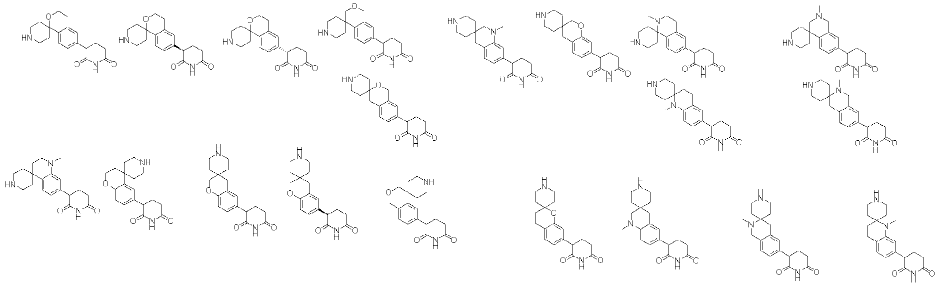 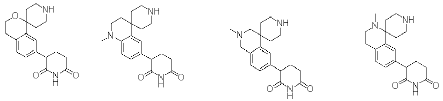 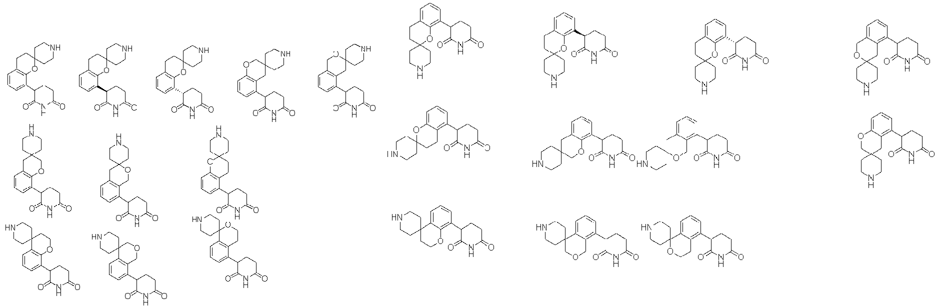 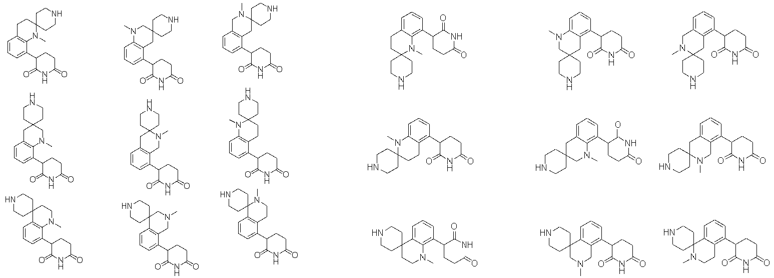 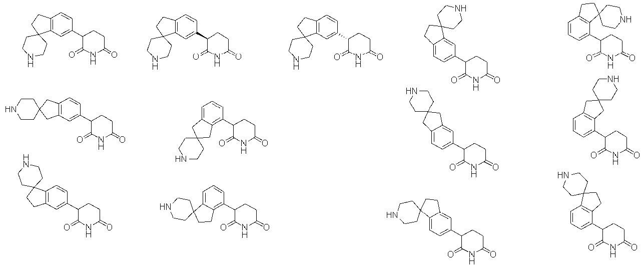 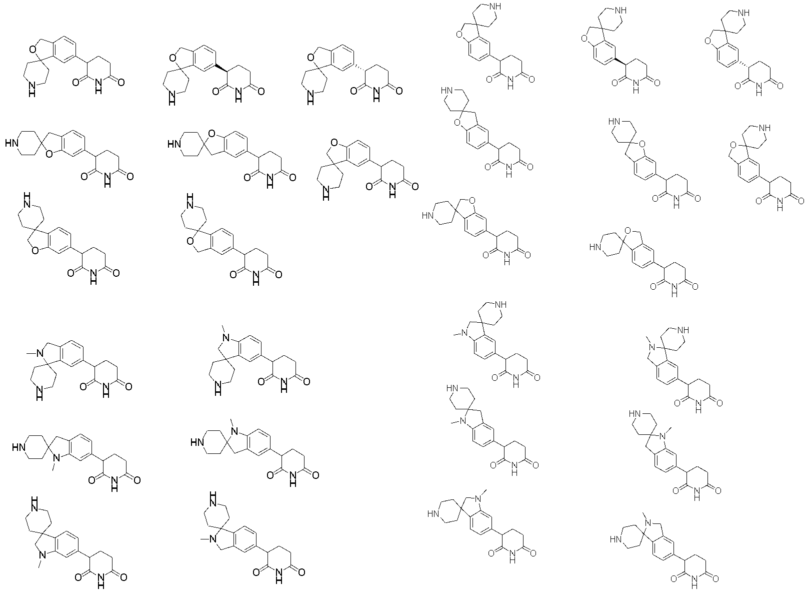 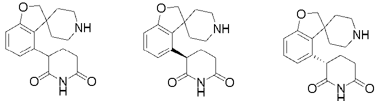 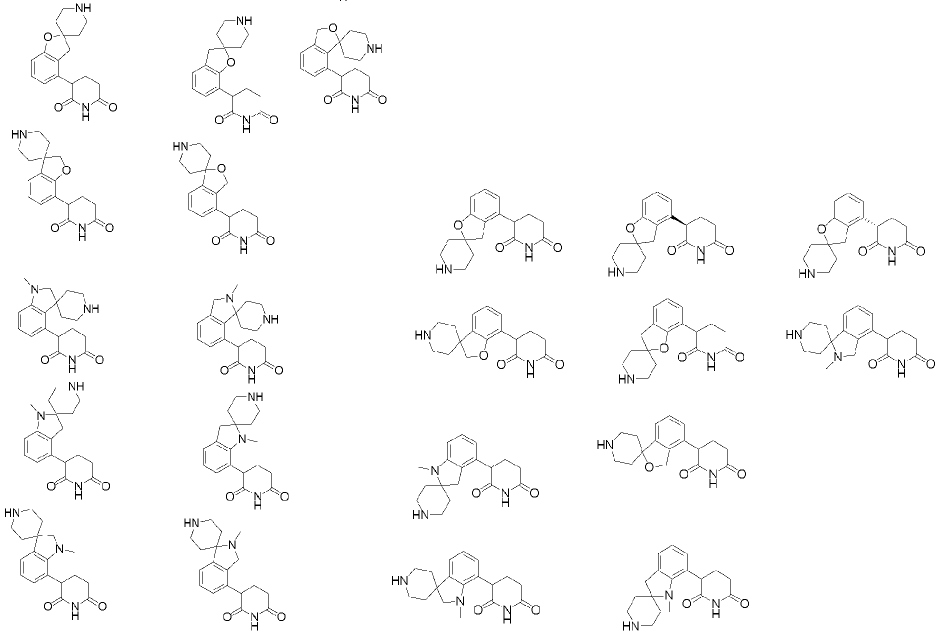 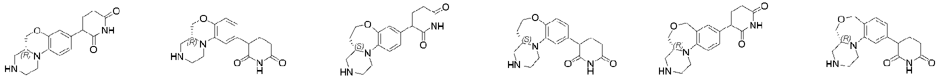 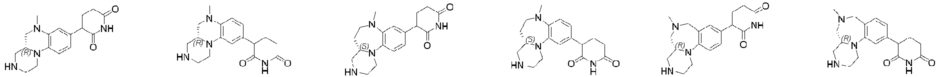 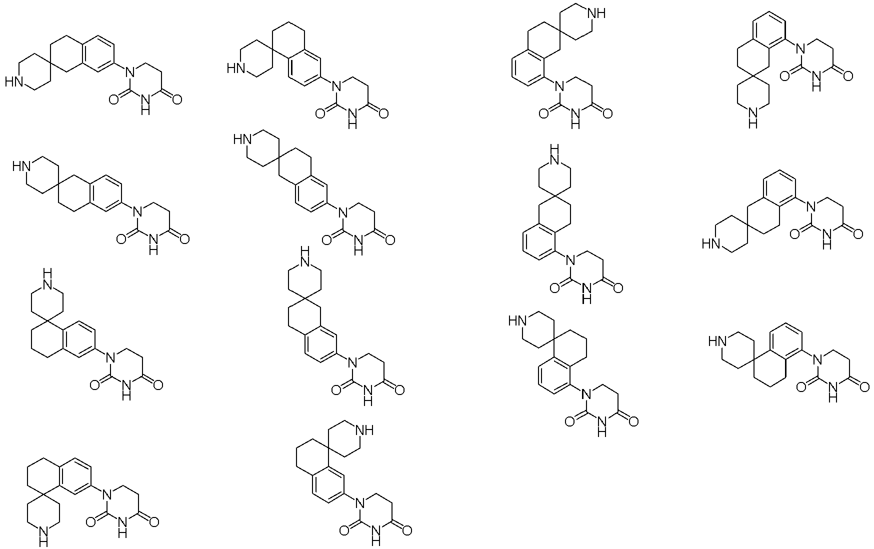 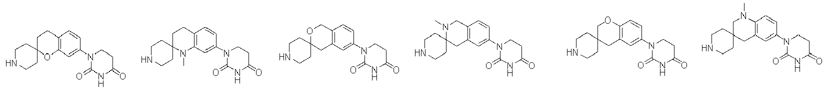 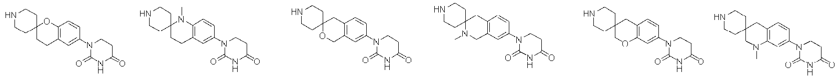 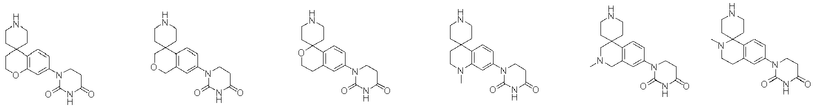 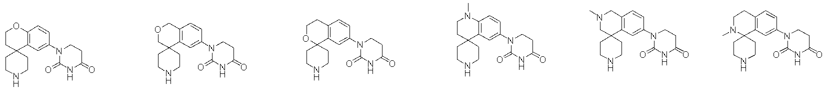 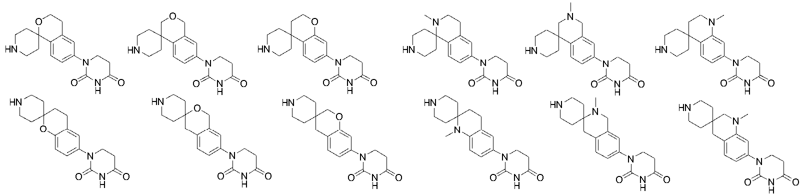 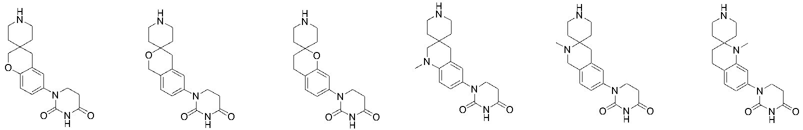 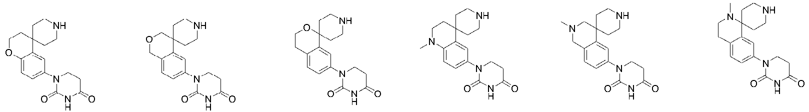 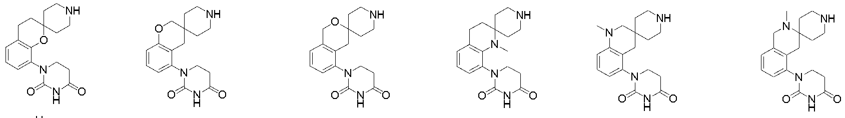 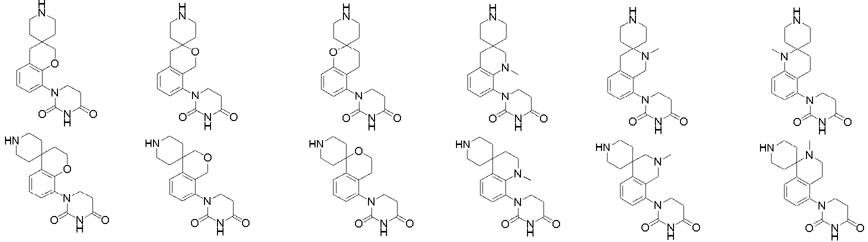 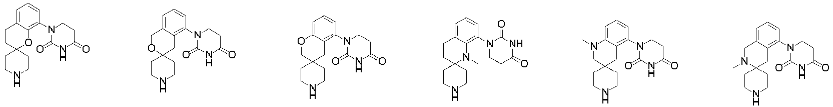 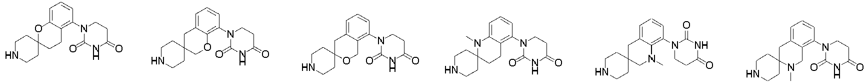 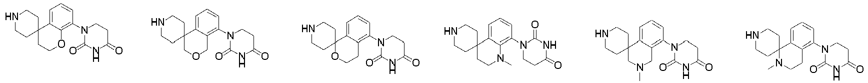 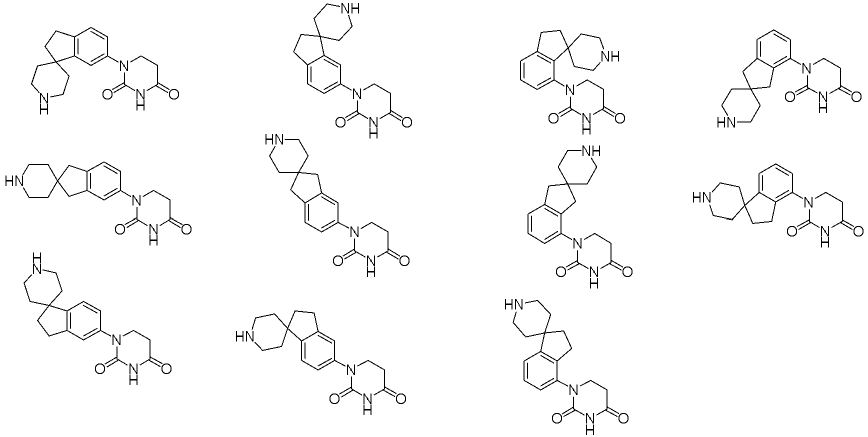 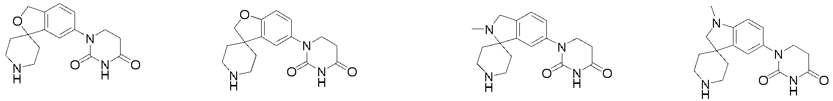 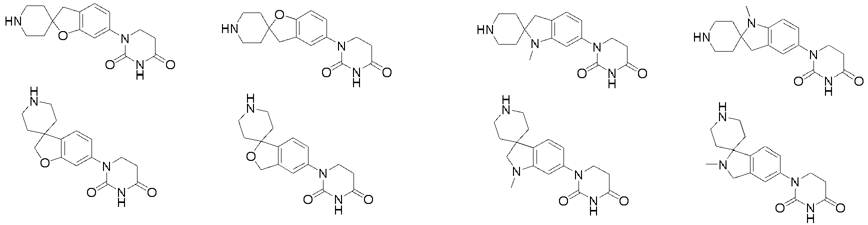 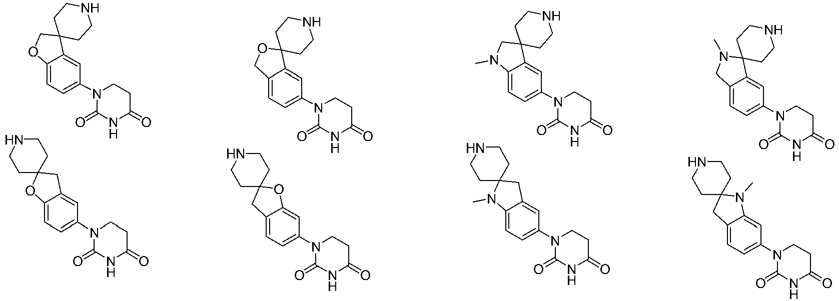 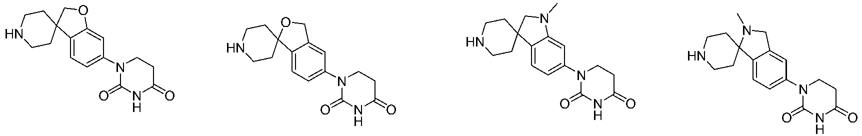 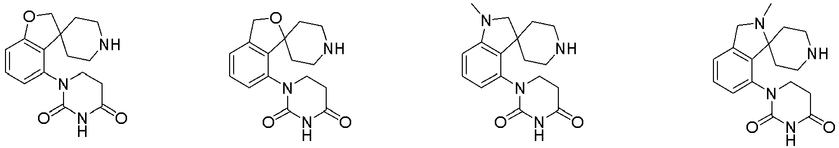 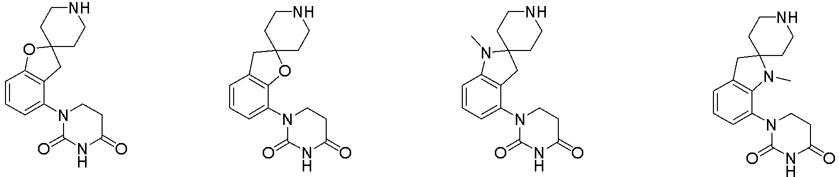 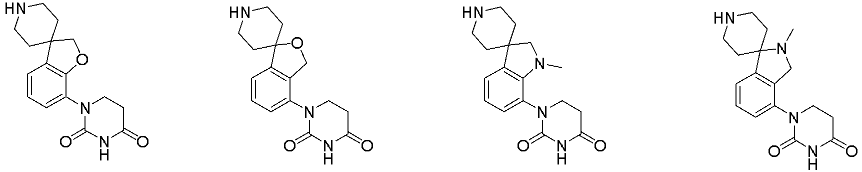 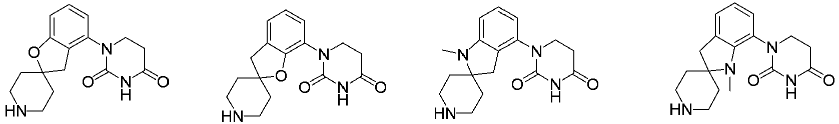 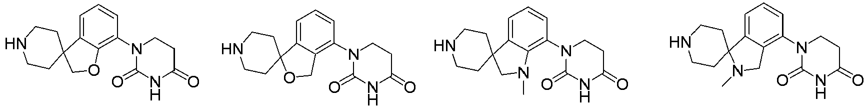 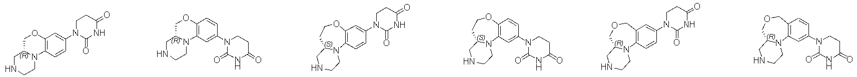 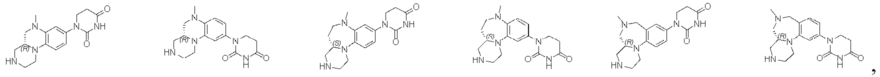 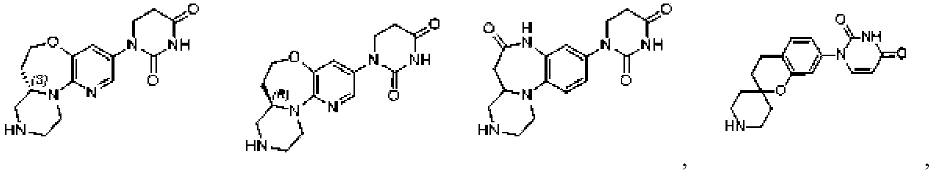 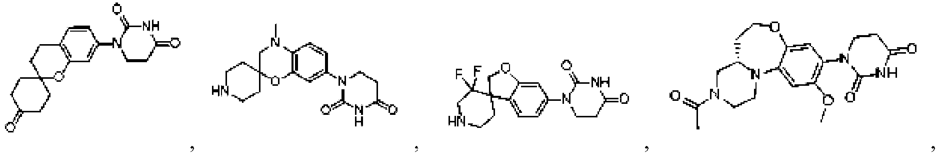 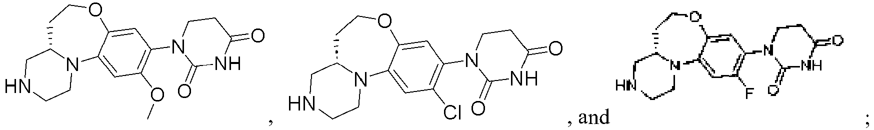 preferably, the compound is selected from:

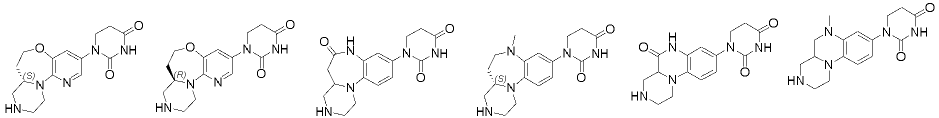

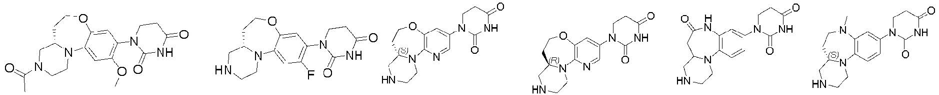

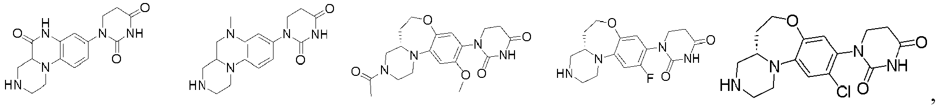

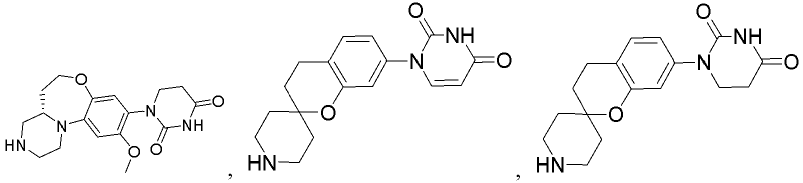

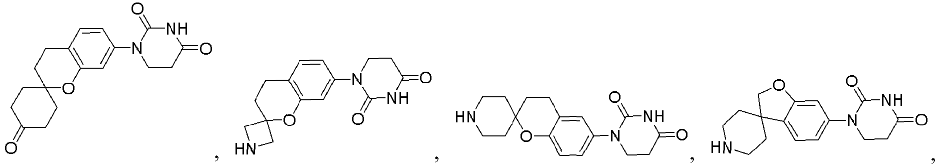

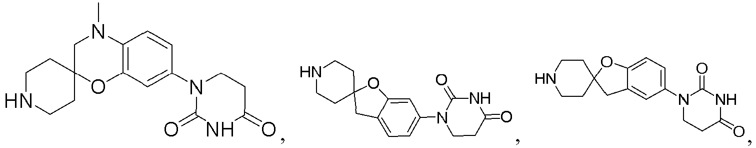

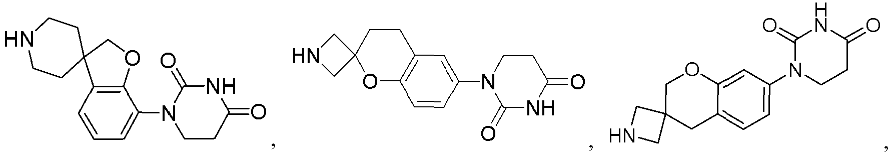

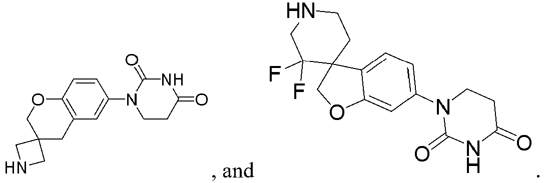

6. A compound of Formula II, or an isomer, an isotopic derivative, a polymorph, a prodrug, or a pharmaceutically acceptable salt or a solvate thereof,
PTM-L-CLM (Formula II)
wherein,
L is a bond or a chemical linking moiety that covalently connects the PTM and CLM moieties; and
PTM is a target protein binding moiety; preferably, the target protein is selected from B7.1, B7, TNFR2, NADPH oxidase, BclIBax and other partners in the apoptosis pathway, C5a receptor, HMG-CoA reductase, PDE V phosphodiesterase type, PDEIV phosphodiesterase type 4, PDEI I, PDEI II, PDE III, squalene epoxidase, CXCR1, CXCR2, nitric oxide (NO) synthase, cyclooxygenase 1, cyclooxygenase 2, 5HT receptor, dopamine receptor, G protein (i.e., Gq), histamine receptor, 5-lipoxygenase, tryptase serine protease, thymidylate synthase, purine nucleoside phosphorylase, GAPDH trypanosome, glycogen phosphorylase, carbonic anhydrase, chemokine receptor, JAW STAT, RXR and analogs thereof, HIV1 protease, HIV1 integrase, influenza neuraminidase, hepatitis B reverse transcriptase, sodium channel, multi-drug resistance bacteria, P-glycoprotein, tyrosine kinase, CD23, CD124, tyrosine kinase p56lck, CD4, CD5, IL-2 receptor, IL-1 receptor, TNF-αR, ICAM1, Ca²⁺ channel, VCAM, VLA-4 integrin, selectin, CD40/CD40L, inosine monophosphate dehydrogenase, p38 MAP kinase, Ras/Raf/MEWERK pathway, interleukin-1 converting enzyme, caspase, HCV NS3 protease, HCV NS3 RNA helicase, glycinamide ribonucleotide formyltransferase, rhinovirus 3C protease, herpes simplex virus-I (HSV-I) protease, cytomegalovirus (CMV) protease, poly(ADP-ribose) polymerase, cyclin-dependent kinase 4/6, vascular endothelial growth factor, oxytocin receptor, microsomal transfer protein inhibitors, bile acid transport inhibitors, 5α reductase inhibitors, angiotensin II, glycine receptor, norepinephrine reuptake receptor, endothelin receptor, neuropeptide Y and receptor thereof, adenosine receptor, adenosine kinase and AMP deaminase, purinergic receptor (P2Y1, P2Y2, P2Y4, P2Y6, P2X1-7), farnesyl transferase, geranylgeranyl transferase, TrkA receptor of NGF, β-amyloid, tyrosine kinase Flk-II/KDR, vitronectin receptor, integrin receptor, Her-2/neu, telomerase inhibitor, cytosolic phospholipase A2, ecdysone 20-monooxygenase, ion channel of GABA-gated chloride channel, acetylcholinesterase, voltage-sensitive sodium channel protein, calcium release channel and chloride channel, acetyl-CoA carboxylase, adenylosuccinate synthetase, protoporphyrinogen oxidase, enolpyruvylshikimate phosphate synthase, MYC protein, androgen receptor, estrogen receptor, Aurora kinase A, Bcr-Abl protein, epidermal growth factor receptor, anaplastic lymphoma kinase, Bruton's tyrosine kinase, bromodomain protein 4, and interleukin-1 receptor-associated kinase 4 (IRAK4); preferably, the target protein is selected from the androgen receptor, estrogen receptor, and interleukin-1 receptor-associated kinase 4 (IRAK4); and
CLM is a cereblon E3 ubiquitin ligase binding moiety selected from any one of the following structures: 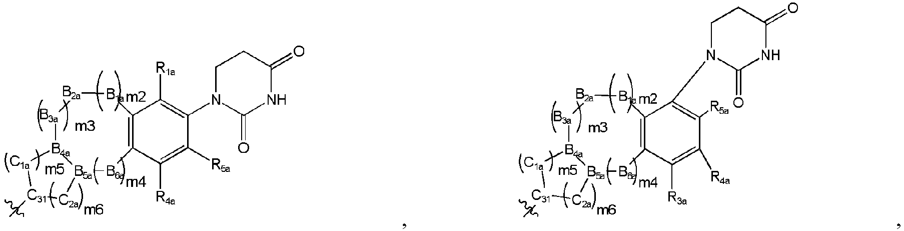 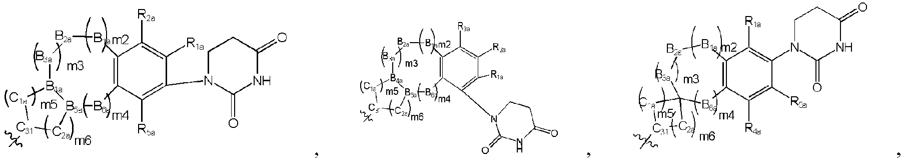 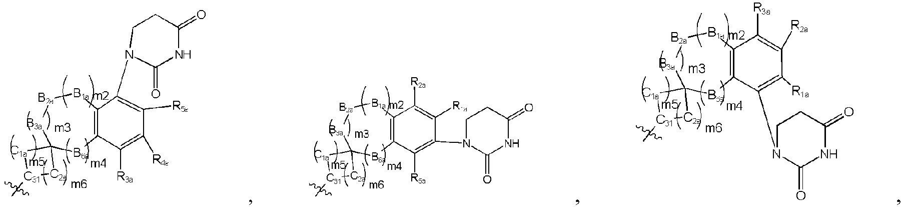 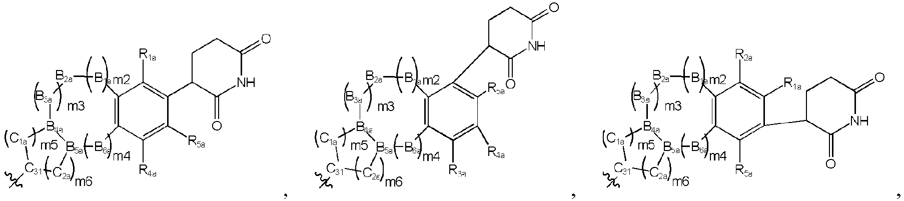 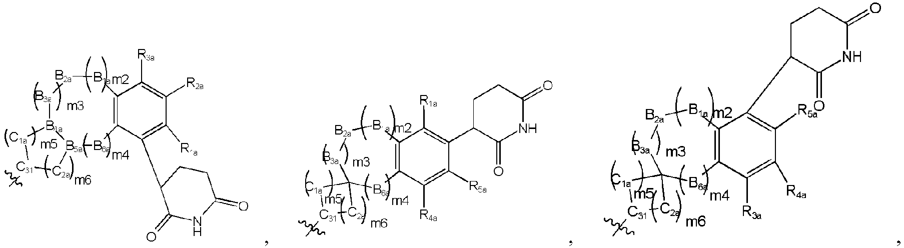 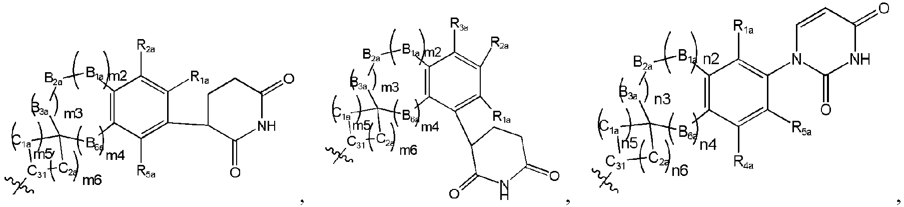 and 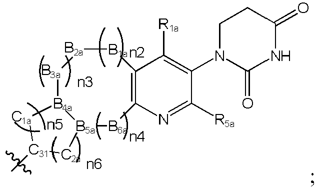
wherein, R₁ₐ, R₂ₐ, R₃ₐ, R₄ₐ, and R₅ₐ, at each occurrence, are each independently selected from H, deuterium atom, halogen, C₁₋₆ alkyl, C₁₋₆ deuterated alkyl, carboxyl, C₁₋₆ heteroalkyl, alkenyl, alkynyl, C₁₋₆ alkoxy, C₁₋₆ haloalkyl, C₁₋₆ haloalkoxy, hydroxyl, C₁₋₆ hydroxyalkyl, nitro, cyano, amino, C₁₋₆ alkylamino, C₁₋₆ alkylcarbonyl, C₁₋₆ alkoxycarbonyl, C₁₋₆ alkylaminocarbonyl, cycloalkyl, heterocyclyl, aryl, and heteroaryl, wherein the C₁₋₆ alkyl, C₁₋₆ heteroalkyl, alkenyl, alkynyl, C₁₋₆ alkoxy, C₁₋₆ haloalkyl, C₁₋₆ haloalkoxy, C₁₋₆ hydroxyalkyl, C₁₋₆ alkylamino, C₁₋₆ alkylcarbonyl, C₁₋₆ alkoxycarbonyl, C₁₋₆ alkylaminocarbonyl, cycloalkyl, heterocyclyl, aryl, and heteroaryl are each independently optionally substituted with one or more substituents selected from halogen, alkyl, heteroalkyl, alkoxy, haloalkyl, hydroxyl, hydroxyalkyl, cyano, amino, nitro, cycloalkyl, heterocyclyl, alkenyl, alkynyl, alkylamino, alkylcarbonyl, alkoxycarbonyl, alkylaminocarbonyl, aryl, and heteroaryl; preferably, R₁ₐ, R₂ₐ, R₃ₐ, R₄ₐ, and R₅ₐ, at each occurrence, are each independently selected from H, deuterium atom, halogen, C₁₋₆ alkyl, C₁₋₆ deuterated alkyl, C₁₋₆ heteroalkyl, C₂₋₆ alkenyl, C₂₋₆ alkynyl, C₁₋₆ alkoxy, C₁₋₆ haloalkyl, C₁₋₆ haloalkoxy, hydroxyl, C₁₋₆ hydroxyalkyl, and amino; more preferably, R₁ₐ, R₂ₐ, R₃ₐ, R₄ₐ, and R₅ₐ, at each occurrence, are each independently selected from H, deuterium atom, F, Cl, Br, I, C₁-₃ alkyl, C₁-₃ deuterated alkyl, C₁-₃ alkoxy, C₁-₃ haloalkyl, C₁-₃ haloalkoxy, amino, and hydroxyl; further preferably, R₁ₐ, R₂ₐ, R₃ₐ, R₄ₐ, and R₅ₐ, at each occurrence, are each independently selected from H, F, Cl, Br, I, C₁-₃ alkyl, C₁-₃ alkoxy, hydroxyl, and amino;
B₂ₐ is selected from C(Rₐₐ)₂, NRₐₐ, O, and S; and when B₄ₐ and B₅ₐ are present, B₂ₐ is selected from NRₐₐ, O, and S;
B₄ₐ and B₅ₐ are each independently selected from CRₐₐ and N; preferably, one of B₄ₐ and B₅ₐ is N, and the other is CRₐₐ;
B₁ₐ and B₃ₐ, at each occurrence, are each independently selected from C(Rₐₐ)₂ and C(O);
B₆ₐ, at each occurrence, is selected from C(Rₐₐ)₂ and NRₐₐ;
C₁ₐ and C₂ₐ, at each occurrence, are each independently C(Rₐₐ)₂;
m2, m3, m4, m5, and m6 are each independently selected from 0, 1, 2, and 3; and m5 and m6 are not both 0; C₃₁ is selected from N and CR_{d};
Rₐₐ and R_{d}, at each occurrence, are each independently selected from H, deuterium atom, F, Cl, Br, I, C₁₋₆ alkyl, deuterated C₁₋₆ alkyl, carboxyl, C₁₋₆ heteroalkyl, C₂₋₆ alkenyl, C₂₋₆ alkynyl, -CO-C₁₋₆ alkyl, C₁₋₆ alkoxy, C₁₋₆ haloalkyl, C₁₋₆ haloalkoxy, hydroxyl, C₁₋₆ hydroxyalkyl, nitro, cyano, amino, C₁₋₆ alkylamino, C₁₋₆ alkylcarbonyl, C₁₋₆ alkoxycarbonyl, C₁₋₆ alkylaminocarbonyl, 3-10-membered cycloalkyl, 3-10-membered heterocyclyl, 6-10-membered aryl, and 5-10-membered heteroaryl, wherein the C₁₋₆ alkyl, C₁₋₆ heteroalkyl, C₂₋₆ alkenyl, C₂₋₆ alkynyl, -CO-C₁₋₆ alkyl, C₁₋₆ alkoxy, C₁₋₆ haloalkyl, C₁₋₆ haloalkoxy, C₁₋₆ hydroxyalkyl, C₁₋₆ alkylamino, C₁₋₆ alkylcarbonyl, C₁₋₆ alkoxycarbonyl, C₁₋₆ alkylaminocarbonyl, 3-10-membered cycloalkyl, 3-10-membered heterocyclyl, 6-10-membered aryl, and 5-10-membered heteroaryl are each independently optionally substituted with one or more substituents selected from halogen, alkyl, heteroalkyl, alkoxy, haloalkyl, hydroxyl, hydroxyalkyl, cyano, amino, nitro, cycloalkyl, heterocyclyl, alkenyl, alkynyl, alkylamino, alkylcarbonyl, alkoxycarbonyl, alkylaminocarbonyl, aryl, and heteroaryl; preferably, Rₐₐ and R_{d}, at each occurrence, are each independently selected from H, deuterium atom, F, Cl, Br, I, C₁₋₆ alkyl, deuterated C₁₋₆ alkyl, carboxyl, C₁₋₆ heteroalkyl, C₂₋₆ alkenyl, C₂₋₆ alkynyl, -CO-C₁₋₆ alkyl, C₁₋₆ alkoxy, C₁₋₆ haloalkyl, C₁₋₆ haloalkoxy, hydroxyl, C₁₋₆ hydroxyalkyl, nitro, cyano, amino, C₁₋₆ alkylamino, C₁₋₆ alkylcarbonyl, C₁₋₆ alkoxycarbonyl, and C₁₋₆ alkylaminocarbonyl; more preferably, Rₐₐ and R_{d}, at each occurrence, are each independently selected from H, F, Cl, Br, I, C₁₋₆ alkyl, carboxyl, C₁₋₆ heteroalkyl, C₂₋₆ alkenyl, C₂₋₆ alkynyl, -CO-C₁₋₆ alkyl, C₁₋₆ alkoxy, C₁₋₆ haloalkyl, C₁₋₆ haloalkoxy, hydroxyl, C₁₋₆ hydroxyalkyl, nitro, cyano, and amino; further preferably, Rₐₐ and R_{d}, at each occurrence, are each independently selected from H, F, Cl, Br, I, C₁-₃ alkyl, C₁-₃ alkoxy, hydroxyl, amino, and -C(O)-C₁-₃ alkyl;
the PTM is not 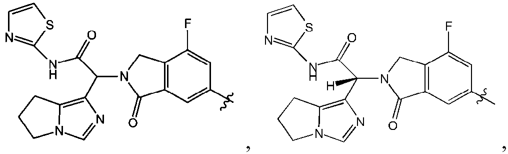
or 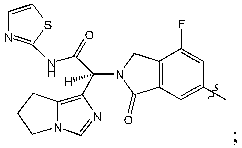
and when the PTM is an IRAK4 binding moiety, the CLM is 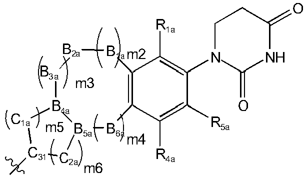 or 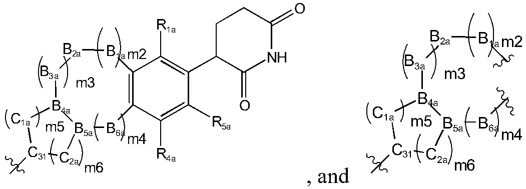 is not

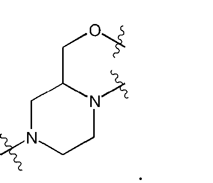

7. The compound according to claim 6, **characterized in that** the CLM is selected from any one of the following structures: 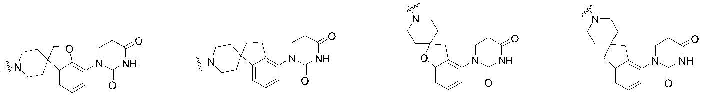 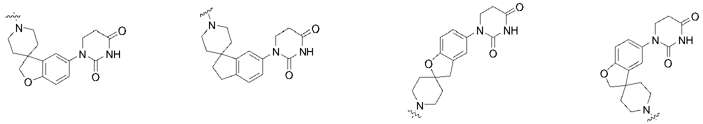 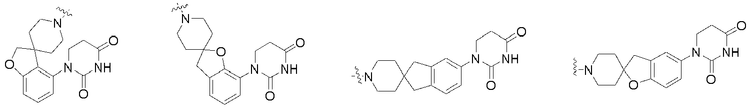 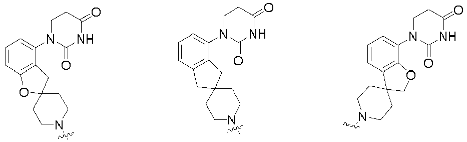 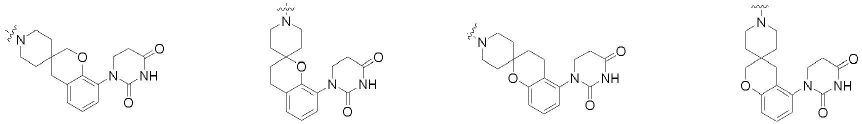 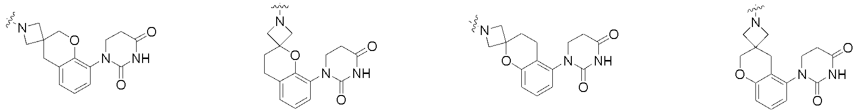 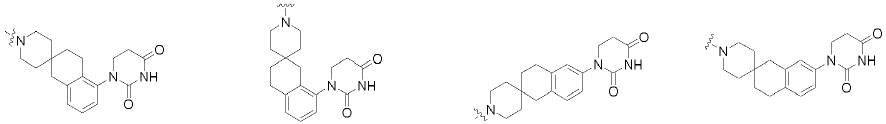 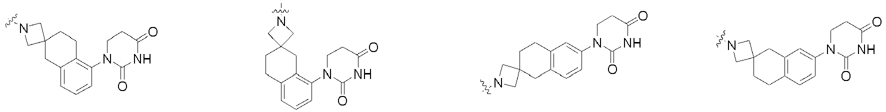 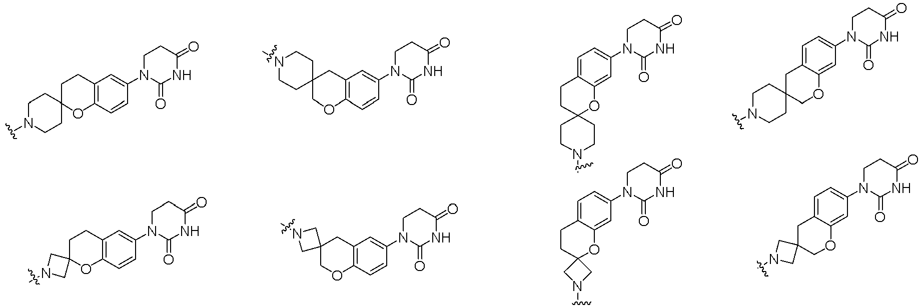 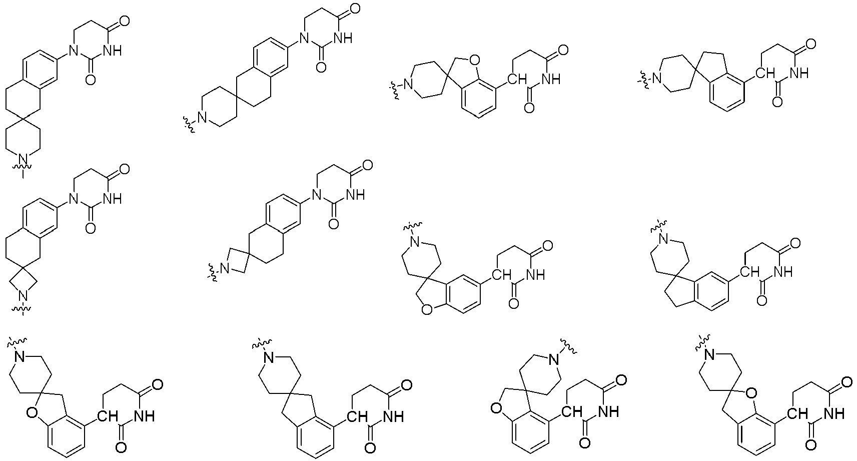 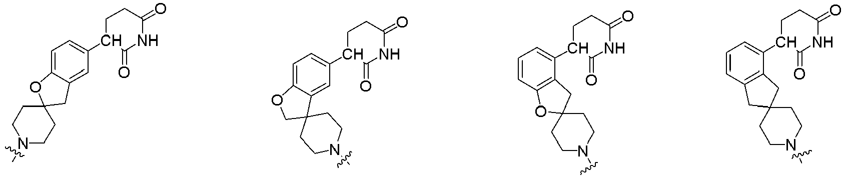 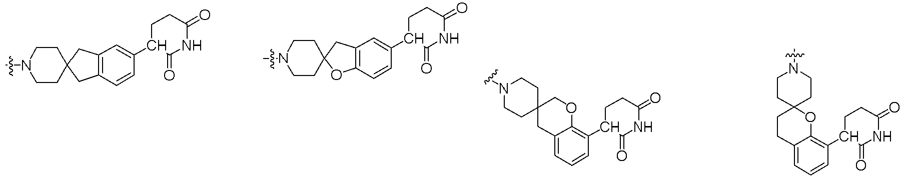 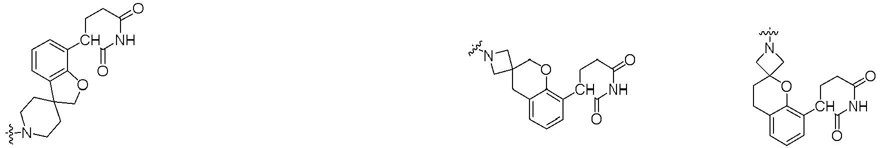 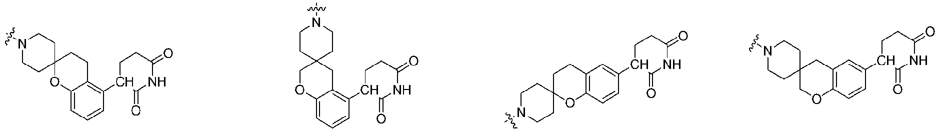 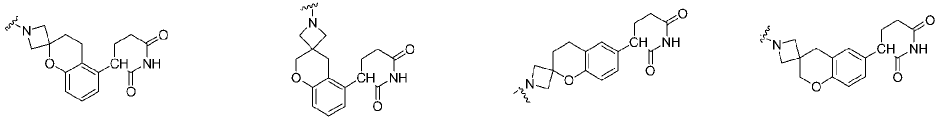 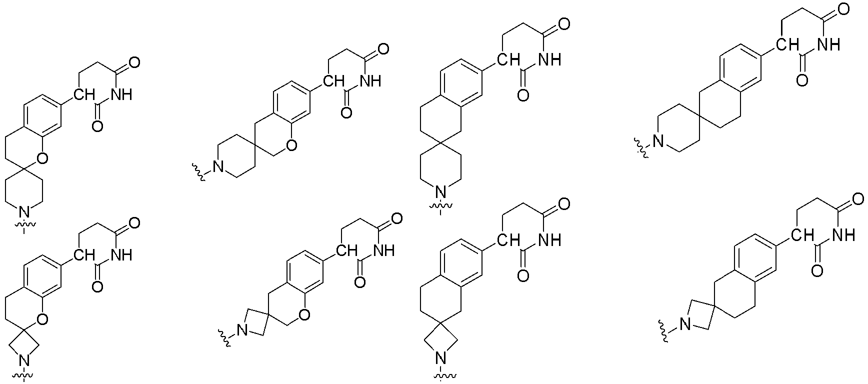 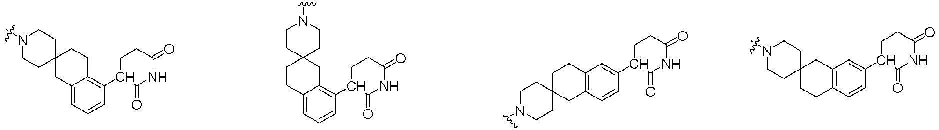 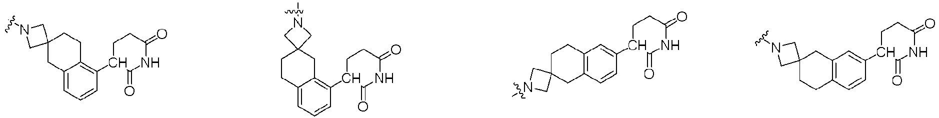 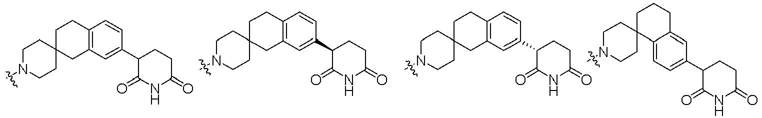 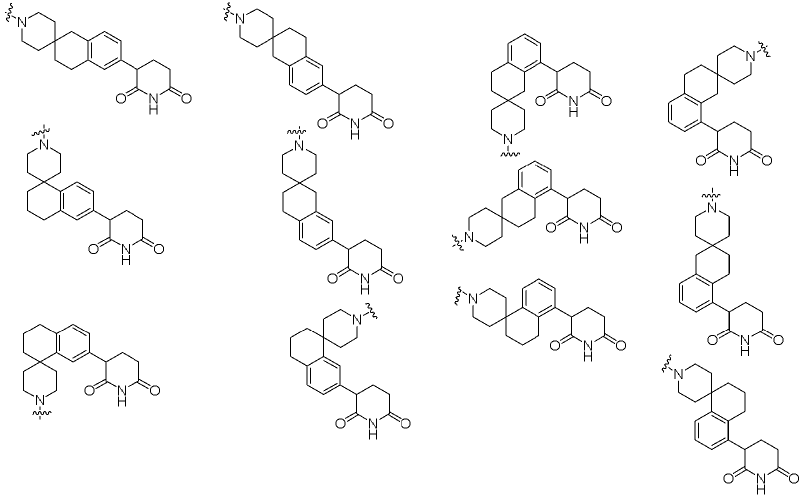 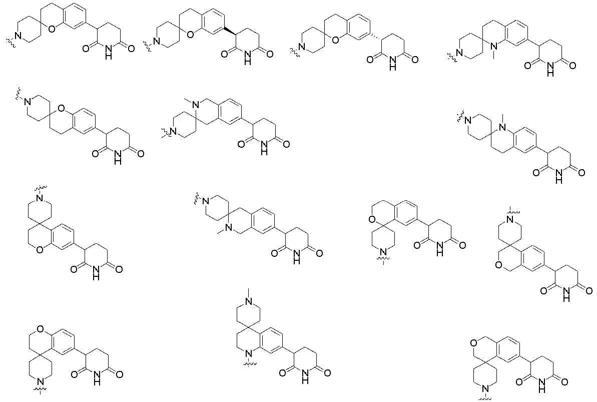 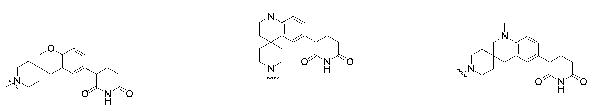 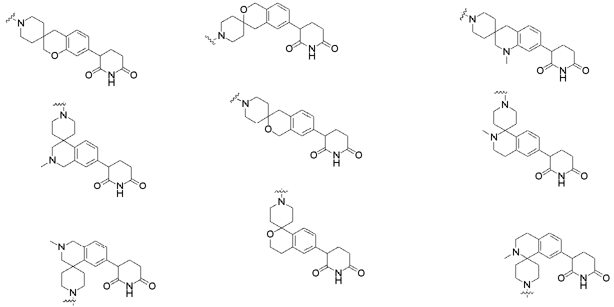 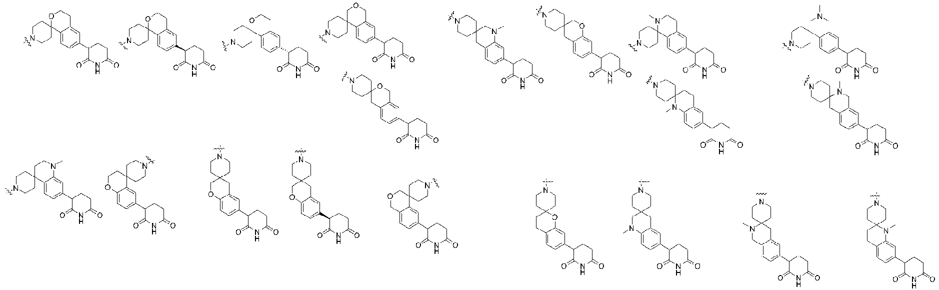 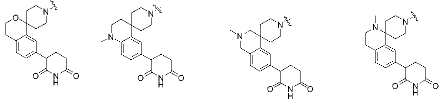 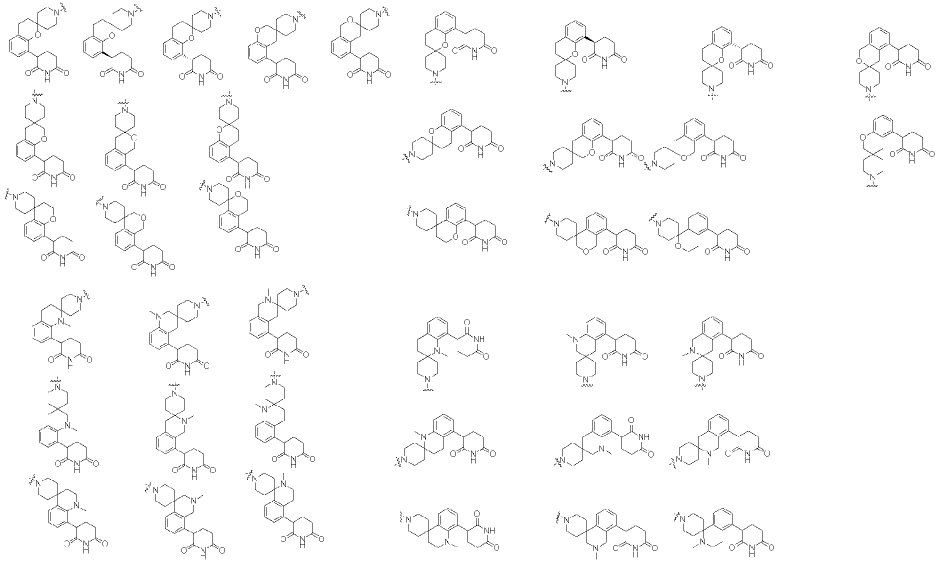 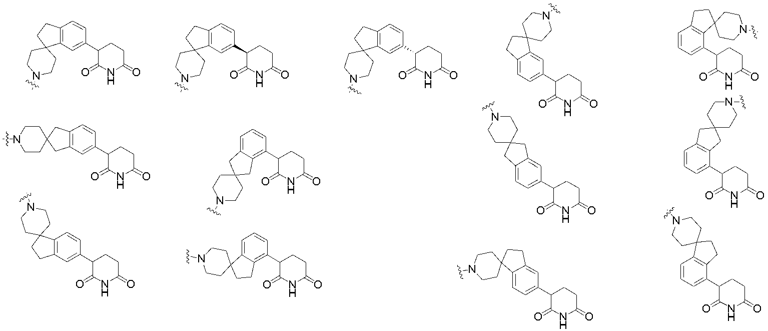 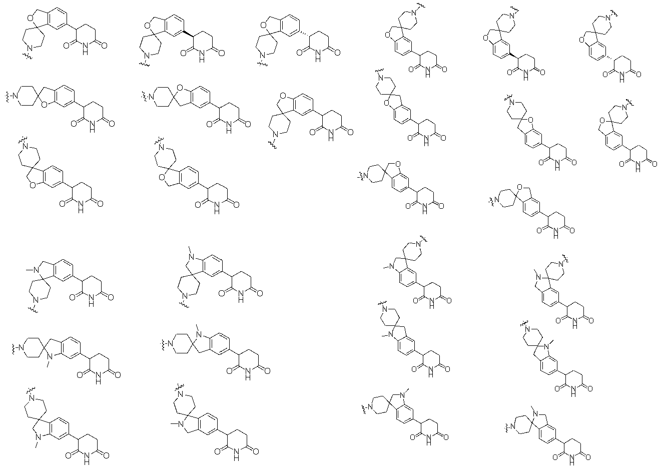 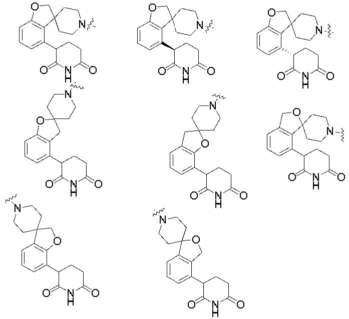 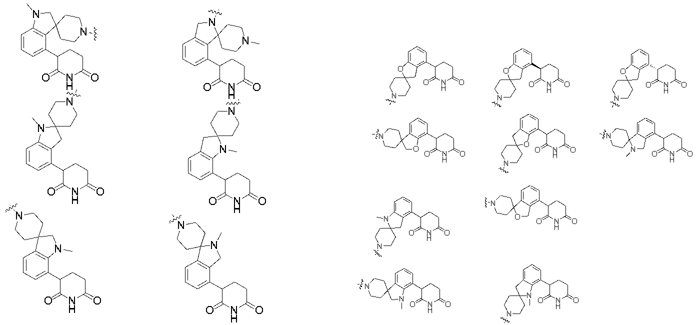 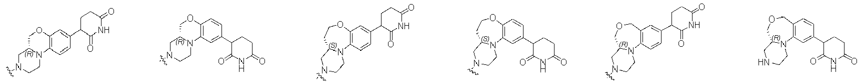 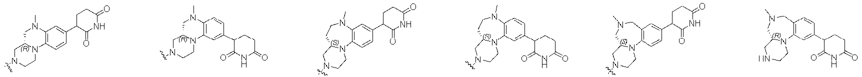 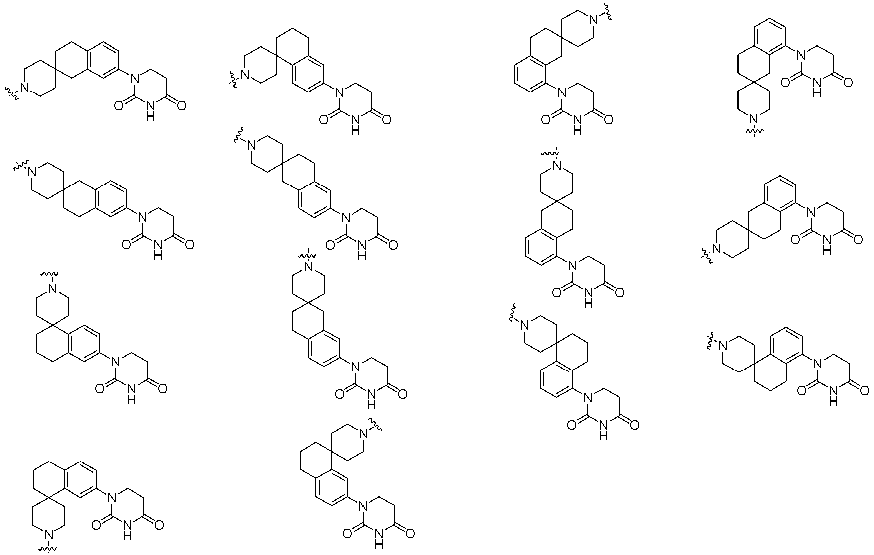 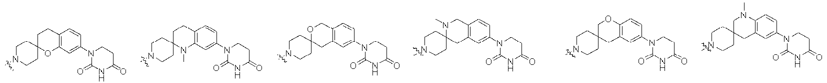 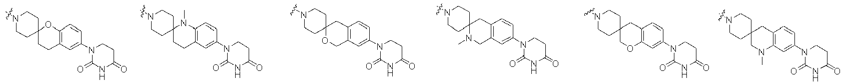 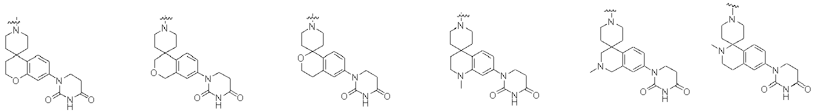 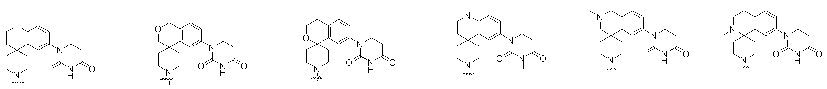 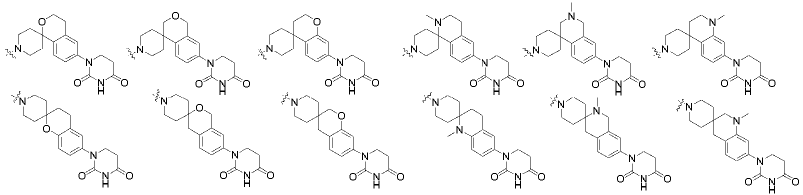 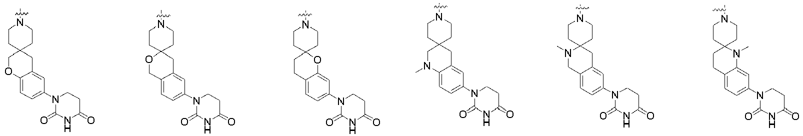 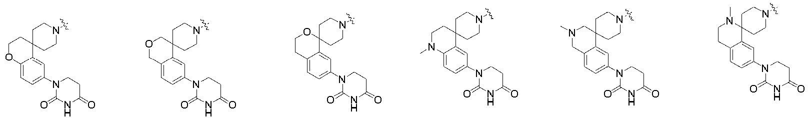 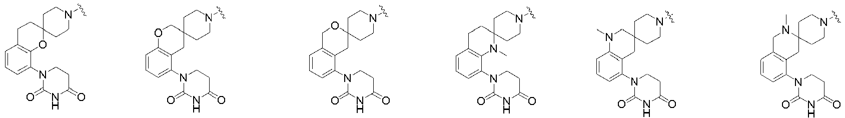 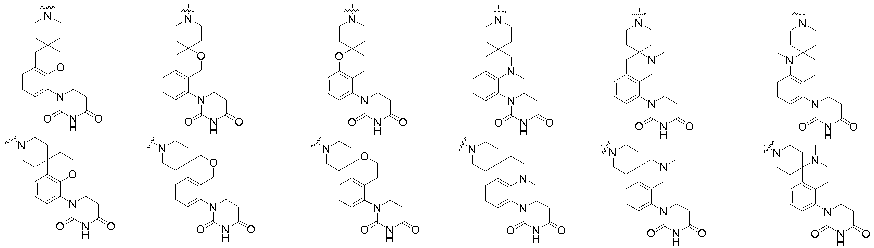 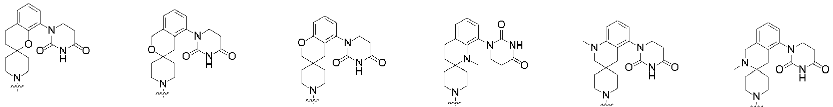 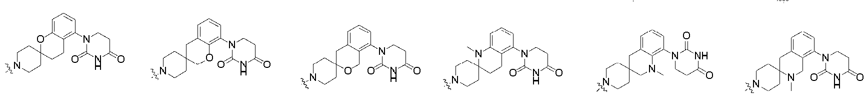 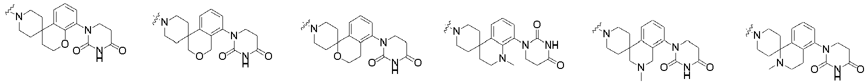 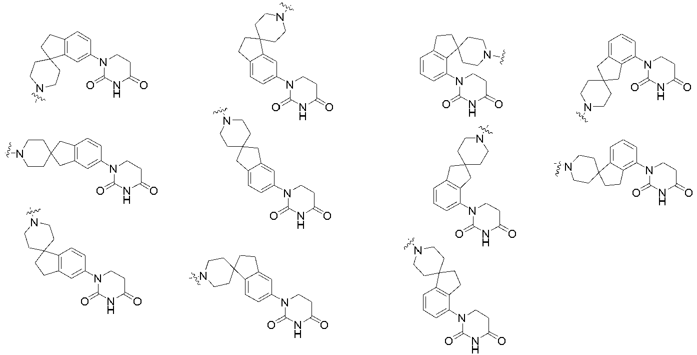 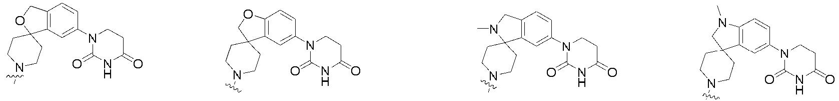 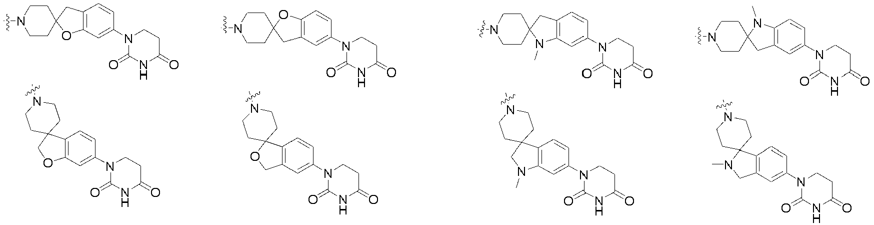 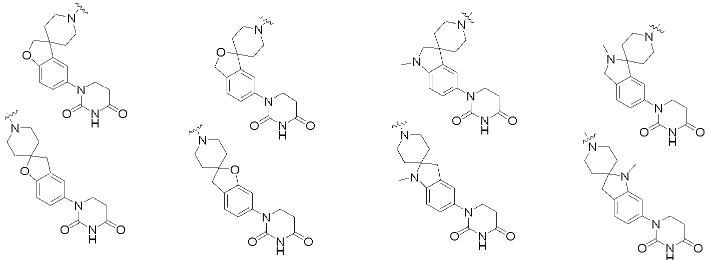 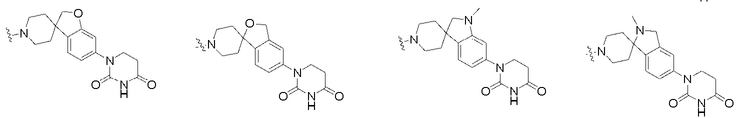 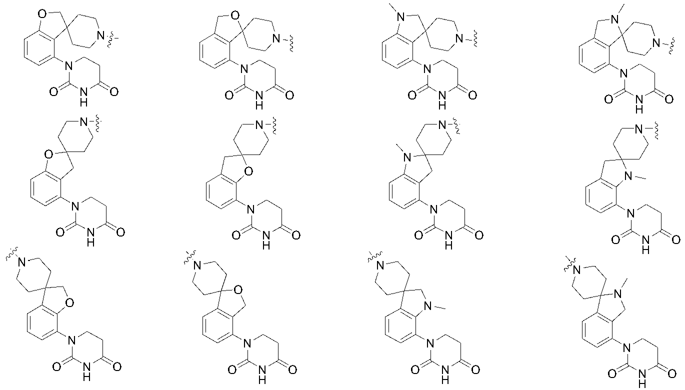 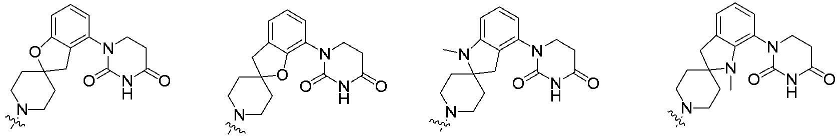 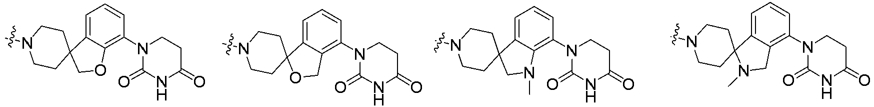 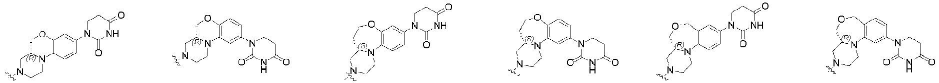 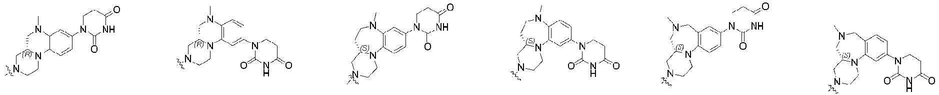 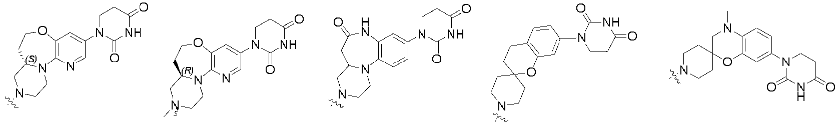 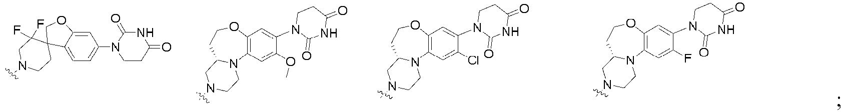 preferably, the CLM is selected from:

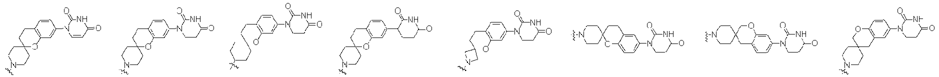

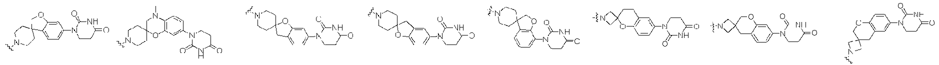

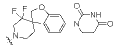

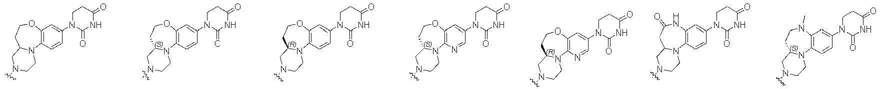

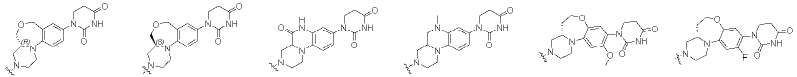

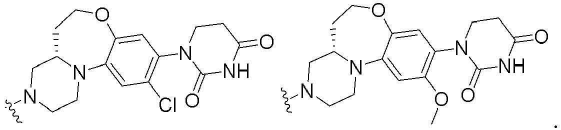

8. The compound according to claim 6 or 7,
wherein, L is a bond or -(B^{L})_{q}-;
B^{L}, at each occurrence, is the same or different and is independently selected from CR^{L1}R^{L2}, O, S, SO, SO₂, NR^{L3}, SO₂NR^{L3}, SONR^{L3}, CONR^{L3}, NR^{L3}CONR^{L4}, NR^{L3}SO₂NR^{L4}, CO, CR^{L1}=CR^{L2}, C≡C, SiR^{L1}R^{L2}, P(O)R^{L1}, P(O)OR^{L1}, NR^{L3}C(=NCN)NR^{L4}, NR^{L3}C(=NCN), NR^{L3}C(=CNO₂)NR^{L4}, cycloalkylene, heterocyclylene, arylene, or heteroarylene, wherein the cycloalkylene, heterocyclylene, arylene, and heteroarylene are optionally substituted with 0-6 R^{L1} and/or R^{L2} groups;
R^{L1}, R^{L2}, R^{L3}, and R^{L4}, at each occurrence, are independently selected from H, halogen, C₁-₈ alkyl, O-C₁-₈ alkyl, S-C₁-₈ alkyl, NH-C₁-₈ alkyl, N(C₁-₈ alkyl)₂, C₃-₁₁ cycloalkyl, aryl, heteroaryl, C₃-₁₁ heterocyclyl, O-C₃-₈ cycloalkyl, O-C₃-₁₁ heterocyclyl, O-aryl, O-heteroaryl, S-C₃-₈ cycloalkyl, NH-C₃-₈ cycloalkyl, N(C₃-₈ cycloalkyl)₂, N(C₃-₈ cycloalkyl)(C₁-₈ alkyl), NH-C₃-₈ heterocyclyl, N(C₃-₈ heterocyclyl)₂, N(C₃-₈ heterocyclyl)(C₁-₈ alkyl), NH-aryl, N(aryl)(C₁-₈ alkyl), NH-heteroaryl, N(heteroaryl)(C₁-₈ alkyl), OH, NH₂, SH, SO₂, P(O)(O-C₁-₈ alkyl)(C₁-₈ alkyl), P(O)(O-C₁-₈ alkyl)₂, C≡C-C₁-₈ alkyl, C≡CH, CH=CH-(C₁-₈ alkyl), C(C₁-₈ alkyl)=CH-(C₁-₈ alkyl), C(C₁-₈ alkyl)= C(C₁-₈ alkyl)₂, Si(OH)₃, Si(C₁-₈ alkyl)₃, Si(OH)(C₁-₈ alkyl)₂, CO-C₁-₈ alkyl, CO₂H, CN, CF₃, CHF₂, CH₂F, NO₂, SF₅, SO₂NH-C₁-₈ alkyl, SO₂N(C₁-₈ alkyl)₂, SONH-C₁-₈ alkyl, SON(C₁-₈ alkyl)₂, CONH-C₁-₈ alkyl, CON(C₁-₈ alkyl)₂, N(C₁-₈ alkyl)CONH(C₁-₈ alkyl), N(C₁-₈ alkyl)CON(C₁-₈ alkyl)₂, NHCONH(C₁-₈ alkyl), NHCON(C₁-₈ alkyl)₂, NHCONH₂, N(C₁-₈ alkyl)SO₂NH(C₁-₈ alkyl), N(C₁-₈ alkyl)SO₂N(C₁-₈ alkyl)₂, NHSO₂NH(C₁-₈ alkyl), NHSO₂N(C₁-₈ alkyl)₂, and NHSO₂NH₂, wherein the C₁-₈ alkyl, C₃-₁₁ cycloalkyl, C₃-₁₁ heterocyclyl, C₆-₁₀ aryl, and C₅-₁₀ heteroaryl are each independently optionally substituted with one or more substituents selected from halogen, alkyl, heteroalkyl, alkenyl, alkynyl, alkoxy, hydroxyl, haloalkyl, hydroxyalkyl, cyano, amino, nitro, cycloalkyl, heterocyclyl, halocycloalkyl, haloheteroalkyl, alkylamino, aryl, heteroaryl, haloaryl, and haloheteroaryl; and
q is an integer greater than or equal to 1; preferably, q is 1, 2, 3, 4, 5, 6, 7, 8, 9, 10, 11, 12, 13, 14, 15, 16, 17, 18, 19, or 20.

9. The compound according to claim 8, wherein B^{L} is selected from one or more of the following structures: -O-, -S-, -SO-, -SO₂-, -CH₂-, -CO-, -NH-, 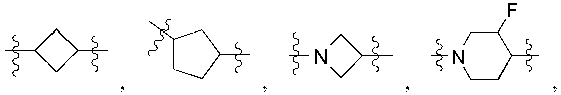 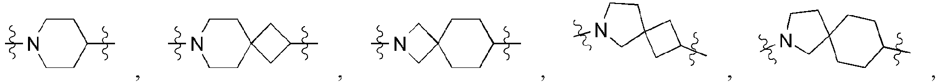 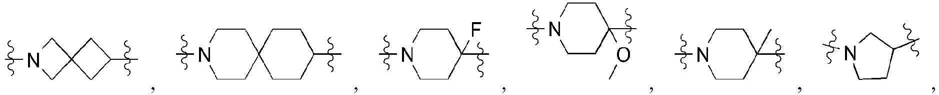 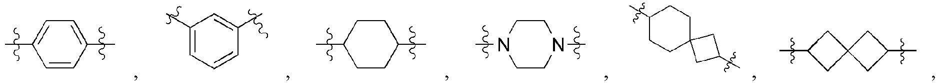 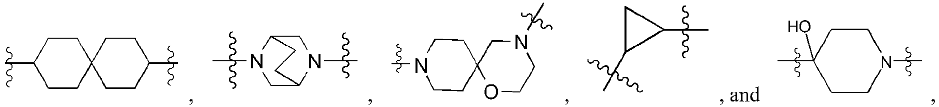 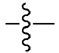 is the point of attachment.

10. The compound according to any one of claims 6 to 9, wherein L is selected from the following structures:
a covalent bond, -(CH₂)ⱼ-, -(CH₂)ₚ-NH-(CH₂)ₛ-, -(CH₂)_{y}-NH-(CH₂)ⱼ-NH-(CH₂)ₛ-, -(CH₂)ₚ-CO-(CH₂)ₛ-, -(CH₂)ₚ-O-(CH₂)ₛ-, -(CH₂)_{y}-CO-(CH₂)ⱼ-CO-(CH₂)ₛ-, -(CH₂)_{y}-O-(CH₂)-O-(CH₂)ₛ-, -(CH₂)y-O-(CH₂)ⱼ-CO-(CH₂)ₛ-, -(CH₂)_{y}-CO-(CH₂)-O-(CH₂)ₛ-, -(CH₂)ₚ-NH-(CH₂)_{y}-O-(CH₂)ⱼ-CO-(CH₂)ₛ-, -(CH₂)_{y}-CO-(CH₂)ⱼ-O-(CH₂)ₛ-NH-(CH₂)ₚ-, 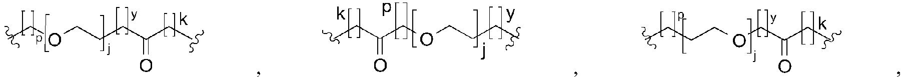 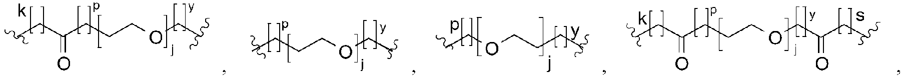 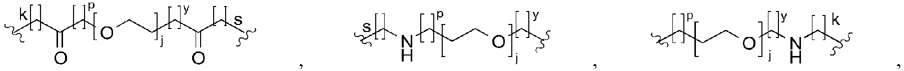 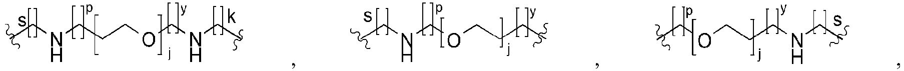 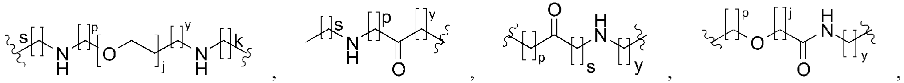 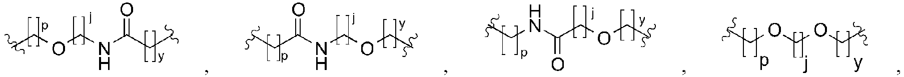 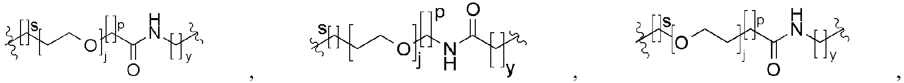 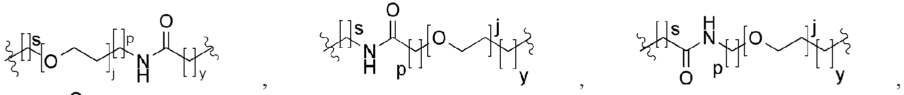 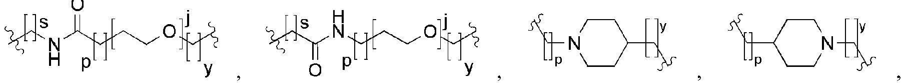 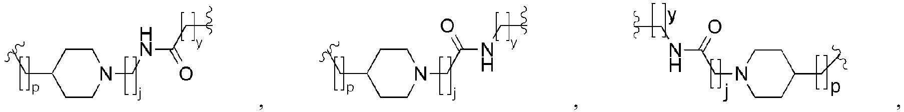 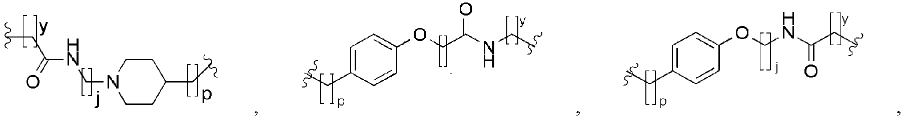 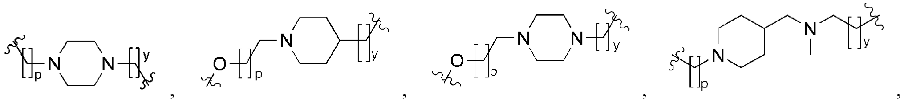 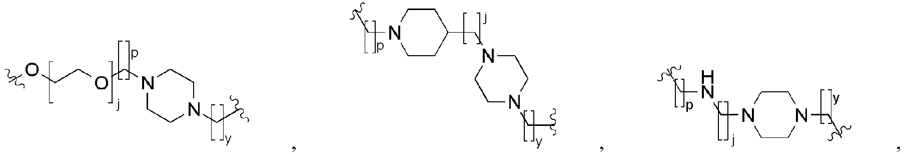 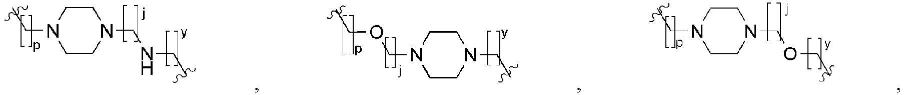 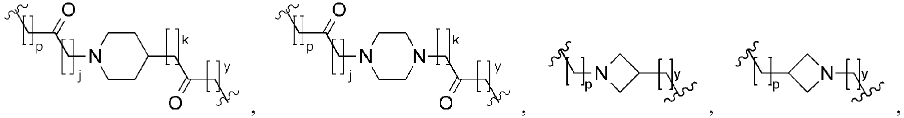 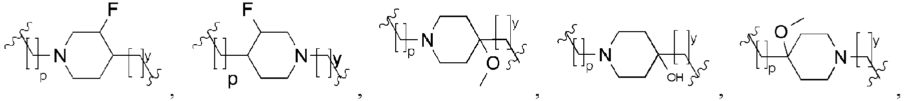 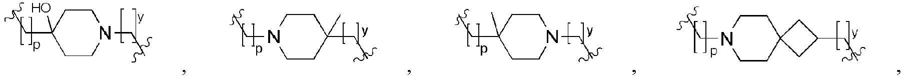 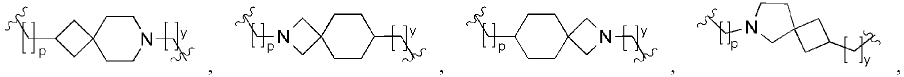 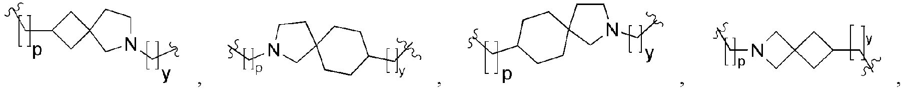 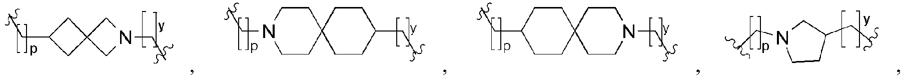 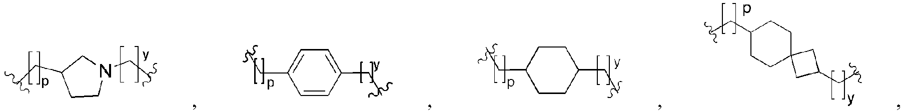 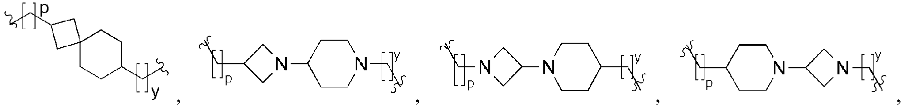 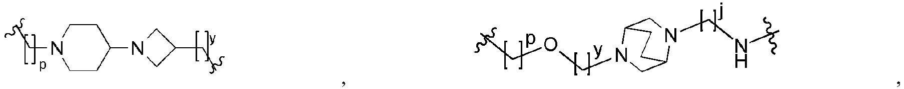 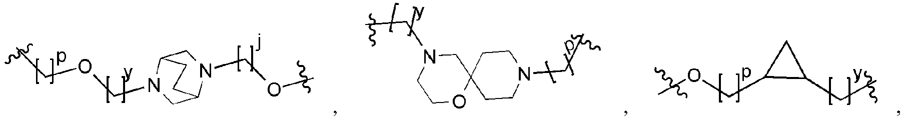 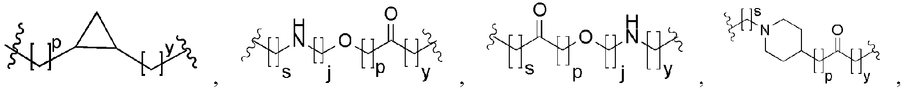 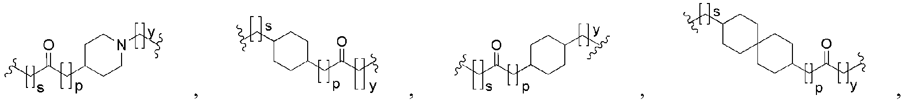 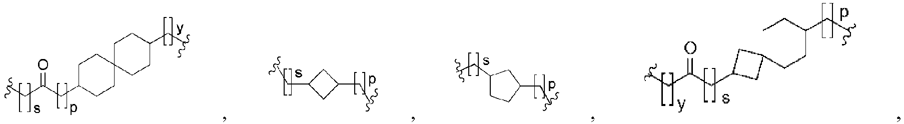 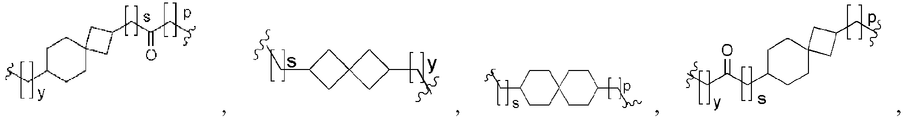 and 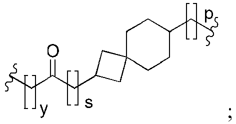
wherein, j is 1, 2, 3, 4, 5, 6, 7, 8, 9, or 10;
k, s, p, and y are 0, 1, 2, 3, 4, 5, 6, 7, 8, 9, or 10; 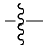 is the point of attachment for the CLM or PTM;
preferably, L is selected from the following structures: a covalent bond, -CH₂-, -(CH₂)₂-, -(CH₂)₃-, -(CH₂)₄-, - (CH₂)₅-, -(CH₂)₆-, -(CH₂)₇-, -(CH₂)₈-, -NH-CH₂-, -NH-(CH₂)₂-, -NH-(CH₂)₃-, -NH-(CH₂)₄-, -NH-(CH₂)₅-, -NH-(CH₂)₆-, -NH-(CH₂)₇-, -NH-(CH₂)₈-, -CO-NH-CH₂-, -CO-NH-(CH₂)₂-, -CO-NH-(CH₂)₃-, -CO-NH-(CH₂)₄-, -CO-NH-(CH₂)₅-, -CO-NH-(CH₂)₆-, -CO-NH-(CH₂)₇-, -CO-NH-(CH₂)₈-, -CH₂-NH-, -(CH₂)₂-NH-, -(CH₂)₃-NH-, - (CH₂)₄-NH-, -(CH₂)₅-NH-, -(CH₂)₆-NH-, -(CH₂)₇-NH-, -(CH₂)₈-NH-, -NH-CH₂-NH-, -NH-(CH₂)₂-NH-, -NH-(CH₂)₃-NH-, -NH-(CH₂)₄-NH-, -NH-(CH₂)₅-NH-, -NH-(CH₂)₆-NH-, -NH-(CH₂)₇-NH-, -NH-(CH₂)₈-NH-, -(CH₂-CH₂-O)-CH₂-CH₂-, -(CH₂-CH₂-O)₂-CH₂-CH₂-, -(CH₂-CH₂-O)₃-CH₂-CH₂-, -NH-(CH₂-CH₂-O)-CH₂-CH₂-, -NH-(CH₂-CH₂-O)₂-CH₂-CH₂-, -NH-(CH₂-CH₂-O)₃-CH₂-CH₂-, -CO-NH-(CH₂-CH₂-O)-CH₂-CH₂-, -CO-NH-(CH₂-CH₂-O)₂-CH₂-CH₂-, -CO-NH-(CH₂-CH₂-O)₃-CH₂-CH₂-, -(CH₂-CH₂-O)-CH₂-CH₂-NH-, -(CH₂-CH₂-O)₂-CH₂-CH₂-NH-, -(CH₂-CH₂-O)₃-CH₂-CH₂-NH-, -NH-(CH₂-CH₂-O)-CH₂-CH₂-NH-, -NH-(CH₂-CH₂-O)₂-CH₂-CH₂-NH-, -NH-(CH₂-CH₂-O)₃-CH₂-CH₂-NH-, -CO-NH-(CH₂-CH₂-O)-CH₂-CH₂-NH-, -CO-NH-(CH₂-CH₂-O)₂-CH₂-CH₂-NH-, -CO-NH-(CH₂-CH₂-O)₃-CH₂-CH₂-NH-, -CH₂-CH₂-(O-CH₂-CH₂)-, -CH₂-CH₂-(O-CH₂-CH₂)₂-, -CH₂-CH₂-(O-CH₂-CH₂)₃-, - NH-CH₂-CH₂-(O-CH₂-CH₂)-, -NH-CH₂-CH₂-(O-CH₂-CH₂)₂-, -NH-CH₂-CH₂-(O-CH₂-CH₂)₃-, -CO-NH-CH₂-CH₂-(O-CH₂-CH₂)-, -CO-NH-CH₂-CH₂-(O-CH₂-CH₂)₂-, -CO-NH-CH₂-CH₂-(O-CH₂-CH₂)₃-, -CH₂-CH₂-(O-CH₂-CH₂)-NH-, -CH₂-CH₂-(O-CH₂-CH₂)₂-NH-, -CH₂-CH₂-(O-CH₂-CH₂)₃-NH-, -NH-CH₂-CH₂-(O-CH₂-CH₂)-NH-, -NH-CH₂-CH₂-(O-CH₂-CH₂)₂-NH-, -NH-CH₂-CH₂-(O-CH₂-CH₂)₃-NH-, -NH-CH₂-CH₂-O-CH₂-CH₂-CO-, -CO-CH₂-CH₂-O-CH₂-CH₂-NH-, -NH-(CH₂)₄-CO-, -NH-(CH₂)₅-CO-, -NH-(CH₂)₆-CO-, -CO-(CH₂)₄-NH-, -CO-(CH₂)₅-NH-, -CO-(CH₂)₆-NH-, -NH-(CH₂-CH₂-O)-(CH₂)₃-, -NH-(CH₂-CH₂-O)-(CH₂)₄-, -NH-(CH₂-CH₂-O)-(CH₂)₅-, -NH-(CH₂-CH₂-O)-(CH₂)₆-, -(CH₂)₃-(O-CH₂-CH₂)-NH-, -(CH₂)₄-(O-CH₂-CH₂)-NH-, -(CH₂)₅-(O-CH₂-CH₂)-NH-, -(CH₂)₆-(O-CH₂-CH₂)-NH-, -CH₂-CH₂-O-(CH₂)₂-CO-, -CH₂-CH₂-O-(CH₂)₃-CO-, -CH₂-CH₂-O-(CH₂)₄-CO-, -CO-(CH₂)₂-O-CH₂-CH₂-, -CO-(CH₂)₃-O-CH₂-CH₂-, -CO-(CH₂)₄-O-CH₂-CH₂-, -CO-(CH₂)₂-, -CO-(CH₂)₃-, -CO-(CH₂)₄-, -CO-(CH₂)₅-, -CO-(CH₂)₆-, -(CH₂)₂-CO-, -(CH₂)₃-CO-, -(CH₂)₄-CO-, -(CH₂)₅-CO-, -(CH₂)₆-CO-, -CO-(CH₂)₂-CO-, -CO-(CH₂)₃-CO-, -CO-(CH₂)₄-CO-, -CO-(CH₂)₅-CO-, -CO-(CH₂)₆-CO-, -CH₂-CO-CH₂-, -CH₂-CO-(CH₂)₂-, -CH₂-CO-(CH₂)₃-, - CH₂-CO-(CH₂)₄-, -(CH₂)₂-CO-CH₂-, -(CH₂)₂-CO-(CH₂)₂-, -(CH₂)₂-CO-(CH₂)₃-, -(CH₂)₂-CO-(CH₂)₄-, -(CH₂)₃-CO-CH₂-, -(CH₂)₃-CO-(CH₂)₂-, -(CH₂)₃-CO-(CH₂)₃-, -(CH₂)₃-CO-(CH₂)₄-, -(CH₂)₄-CO-CH₂-, -(CH₂)₄-CO-(CH₂)₂-, - (CH₂)₄-CO-(CH₂)₃-, -(CH₂)₄-CO-(CH₂)₄-, -CH₂-O-CH₂-, -CH₂-O-(CH₂)₂-, -CH₂-O-(CH₂)₃-, -CH₂-O-(CH₂)₄-, - (CH₂)₂-O-CH₂-, -(CH₂)₂-O-(CH₂)₂-, -(CH₂)₂-O-(CH₂)₃-, -(CH₂)₂-O-(CH₂)₄-, -(CH₂)₃-O-CH₂-, -(CH₂)₃-O-(CH₂)₂-, - (CH₂)₃-O-(CH₂)₃-, -(CH₂)₃-O-(CH₂)₄-, -(CH₂)₄-O-CH₂-, -(CH₂)₄-O-(CH₂)₂-, -(CH₂)₄-O-(CH₂)₃-, -(CH₂)₄-O-(CH₂)₄-,

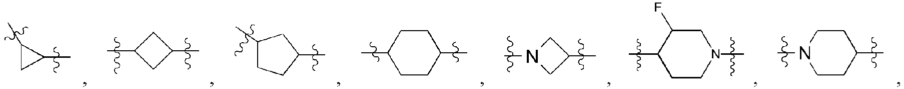

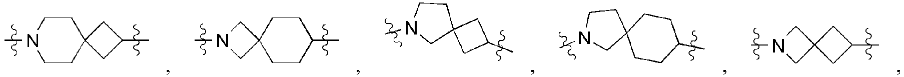

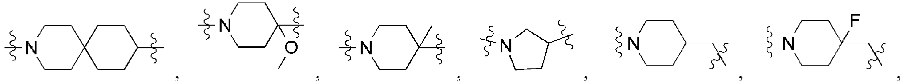

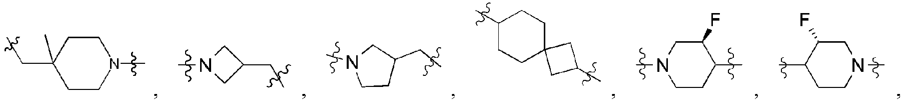

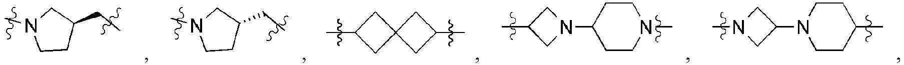

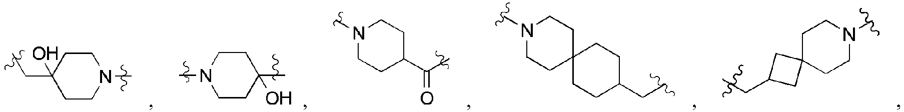

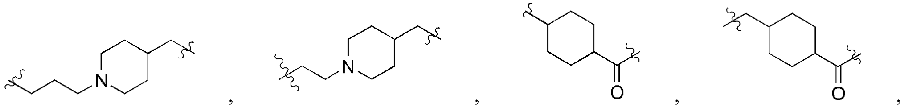

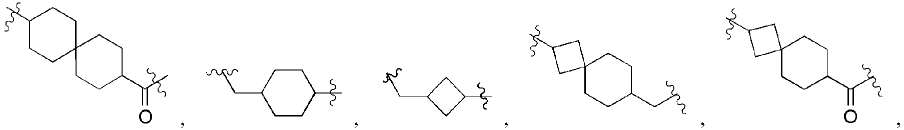

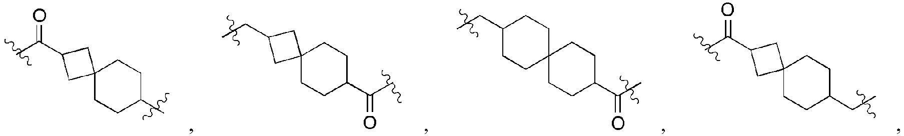

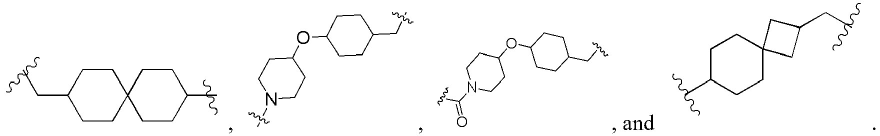

11. The compound according to any one of claims 6 to 10, wherein the PTM is a moiety that binds to an androgen receptor; preferably, the PTM is selected from the following structures: 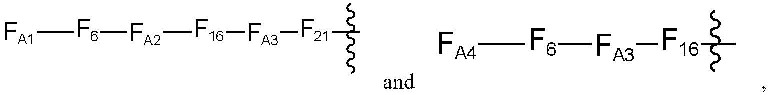 wherein,
F₆, F₁₆, and F₂₁, at each occurrence, are each independently selected from a single bond, NH, SO, S, O, SO₂, alkylene, haloalkylene, heteroalkylene, alkyleneoxy, heteroalkyleneoxy, alkenylene, alkynylene, C(=O), OC(=O), C(=O)O, C(=O)NH, and NHC(=O), or a combination of one or more thereof; wherein the alkylene, alkyleneoxy, and alkenylene are optionally substituted with 0, 1, 2, 3, 4, 5, or 6 R_{c} substituents; preferably, F₆, F₁₆, and F₂₁, at each occurrence, are each independently selected from a single bond, NH, SO, S, O, SO₂, C(=O), OC(=O), and NHC(=O);
F_{A1} and F_{A3} are each independently selected from 6-10-membered aryl and 5-10-membered heteroaryl; the aryl and heteroaryl are optionally substituted with 0, 1, 2, 3, 4, 5, or 6 R_{da} substituents; preferably, F_{A1} is phenyl, optionally substituted with 0, 1, 2, 3, 4, 5, or 6 R_{da} substituents, and F_{A3} is phenyl or a 6-membered heteroaryl containing 1, 2, 3 N, O, S heteroatoms, optionally substituted with 0, 1, 2, 3, 4, 5, or 6 R_{da} substituents;
F_{A2} is 3-15-membered cycloalkylene, 5-15-membered spirocycloalkylene, 3-15-membered heterocycloalkylene, or 5-15-membered spiroheterocycloalkylene, optionally substituted with 0, 1, 2, 3, 4, 5, or 6 R_{ca} substituents; preferably, F_{A2} is 4-6-membered cycloalkylene, 7-11-membered spirocycloalkylene, 4-6-membered heterocycloalkylene, or 7-11-membered spiroheterocycloalkylene, optionally substituted with 0, 1, 2, 3, 4, 5, or 6 R_{ca} substituents; preferably, F_{A2} is 4-, 5-, or 6-membered cycloalkylene, 10-membered spiroheterocycloalkylene containing 1, 2, 3 N atoms, optionally substituted with 0, 1, 2, 3, 4, 5, or 6 R_{ca} substituents;
F_{A4} is 6-10-membered aryl-fused 7-15-membered spiroheterocyclyl, optionally substituted with 0, 1, 2, 3, 4, 5, or 6 R_{da} substituents; preferably, F_{A4} is phenyl-fused 7-15-membered spiroheterocyclyl, the 7-15-membered spiroheterocyclyl containing 1, 2, 3, or 4 heteroatoms each independently selected from N, O, S, optionally substituted with 0, 1, 2, 3, 4, 5, or 6 R_{da} substituents;
R_{da}, at each occurrence, is independently selected from H, halogen, C₁₋₆ alkyl, C₁₋₆ haloalkyl, C₁₋₆ heteroalkyl, C₁₋₆ alkoxy, C₁₋₆ heteroalkoxy, C₃-₇ cycloalkyl, C₃-₇ heterocyclyl, C₆-₈ aryl, C₅-₈ heteroaryl, OH, NH₂, CN, and NO₂; preferably, R_{da}, at each occurrence, is independently selected from H, F, Cl, Br, I, CH₃, OCH₃, CF₃, OH, NH₂, CN, and NO₂;
R_{ca}, at each occurrence, is independently selected from H, halogen, C₁₋₆ alkyl, C₁₋₆ haloalkyl, C₁₋₆ heteroalkyl, C₁₋₆ alkoxy, C₁₋₆ heteroalkoxy, C₃-₇ cycloalkyl, C₃-₇ heterocyclyl, C₆-₈ aryl, C₅-₈ heteroaryl, oxo (=O), thioxo (=S), OH, NH₂, CN, and NO₂; preferably, R_{ca}, at each occurrence, is independently selected from H, F, Cl, Br, I, CH₃, OCH₃, CF₃, OH, NH₂, CN, and NO₂;
preferably, the PTM is selected from:

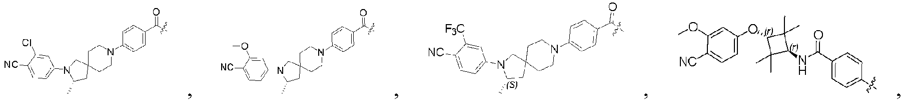

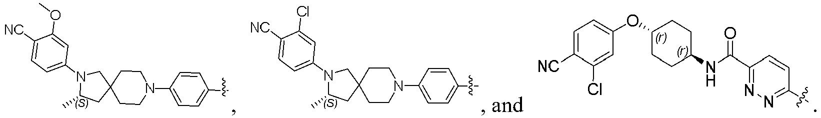

12. The compound according to claim 11, having a structure selected from: 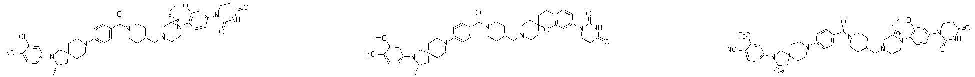 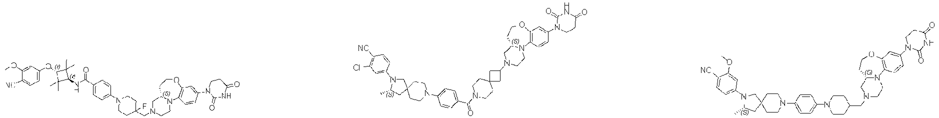 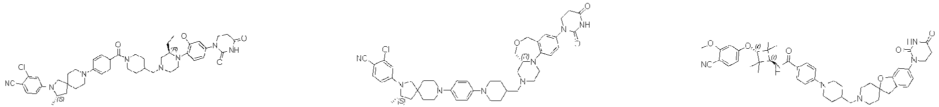 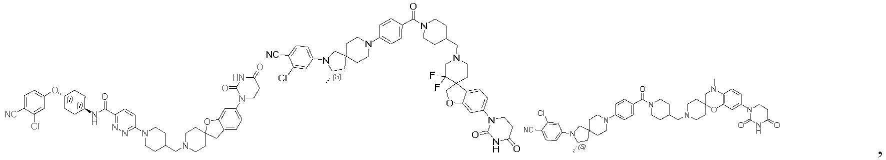 and

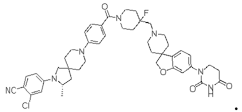

13. The compound according to any one of claims 6 to 10, wherein the PTM is a moiety that binds to an estrogen receptor, preferably, the PTM is selected from the following structures: 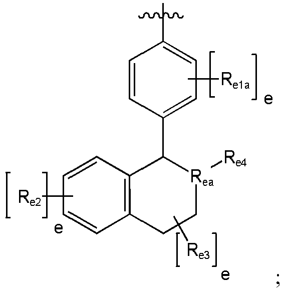
Rₑₐ is selected from CRₑ₁ₐ and N;
Rₑ₄ is selected from 6-10-membered aryl, 5-10-membered heteroaryl, C₁-C₆ alkyl, and C₁-C₆ haloalkyl, wherein the 6-10-membered aryl and 5-10-membered heteroaryl are optionally substituted with 0, 1, 2, 3, 4, or 5 Rₑ₁ₐ substituents; preferably, Rₑ₄ is selected from phenyl and difluoroethyl, wherein the phenyl is optionally substituted with 0, 1, 2, 3, 4, or 5 Rₑ₁ₐ substituents;
Rₑ₁ₐ, Rₑ₂, and Rₑ₃, at each occurrence, are each independently selected from H, F, Cl, Br, I, C₁-C₆ alkyl, C₁-C₆ heteroalkyl, C₂-C₆ alkenyl, C₂-C₆ alkynyl, C₁-C₆ alkoxy, hydroxyl, nitro, cyano, and amino; preferably, Rₑ₁ₐ and Rₑ₂, at each occurrence, are each independently selected from hydroxyl, H, F, Cl, Br, I, and C₁-C₃ alkyl;
e, at each occurrence, is selected from 0, 1, 2, 3, and 4;
preferably, the PTM is selected from:

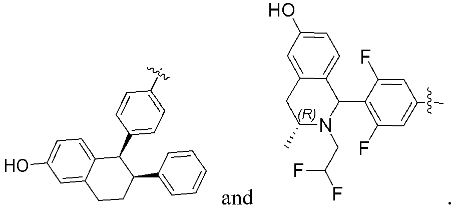

14. The compound according to claim 13, wherein the compound is selected from:

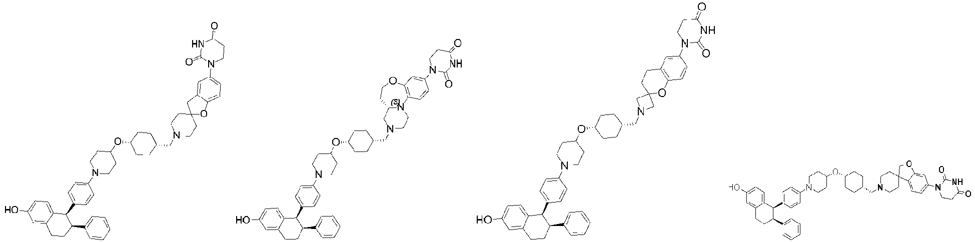

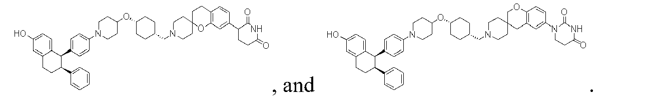

15. The compound according to any one of claims 6 to 10, wherein the PTM is a moiety that binds to the IRAK4 receptor, preferably, the PTM has the structure: 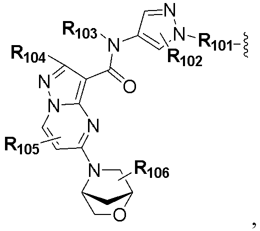 wherein,
R₁₀₁ is selected from a single bond, 3-10-membered heterocycloalkylene, 3-10-membered cycloalkylene, 6-10-membered arylene, and 5-10-membered heteroarylene, wherein the 3-10-membered heterocycloalkyl, 3-10-membered cycloalkyl, 6-10-membered aryl, and 5-10-membered heteroaryl are optionally substituted with one, two, or more substituents selected from halogen, carboxyl, alkoxy, alkyl, hydroxyl, amino, and haloalkyl; preferably, R₁₀₁ is selected from a single bond, 5-7-membered heterocycloalkylene, 5-7-membered cycloalkylene, 6-membered arylene, and 5-8-membered heteroarylene, wherein the 5-7-membered heterocycloalkylene, 5-7-membered cycloalkylene, 6-membered arylene, and 5-8-membered heteroarylene are optionally substituted with one, two, or three substituents selected from F, Cl, Br, I, carboxyl, C₁-₃ alkoxy, C₁-₃ alkyl, hydroxyl, amino, and C₁-₃ haloalkyl;
R₁₀₂, R₁₀₃, R₁₀₄, R₁₀₅, and R₁₀₆ are selected from H, deuterium atom, halogen, C₁₋₆ alkyl, C₁₋₆ deuterated alkyl, carboxyl, C₁₋₆ heteroalkyl, alkenyl, alkynyl, C₁₋₆ alkoxy, C₁₋₆ haloalkyl, C₁₋₆ haloalkoxy, hydroxyl, C₁₋₆ hydroxyalkyl, nitro, cyano, amino, C₁₋₆ alkylamino, C₁₋₆ alkylcarbonyl, C₁₋₆ alkoxycarbonyl, and C₁₋₆ alkylaminocarbonyl; preferably, R₁₀₂, R₁₀₃, R₁₀₄, R₁₀₅, and R₁₀₆ are selected from H, F, Cl, Br, I, C₁-₃ alkyl, carboxyl, C₁-₃ heteroalkyl, C₁-₃ alkoxy, and C₁-₃ haloalkyl; more preferably, R₁₀₂ is C₁-₃ fluoroalkyl;
more preferably, the PTM has the structure:

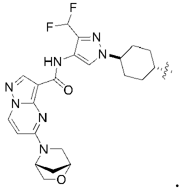

16. The compound according to claim 15, wherein the compound is selected from: 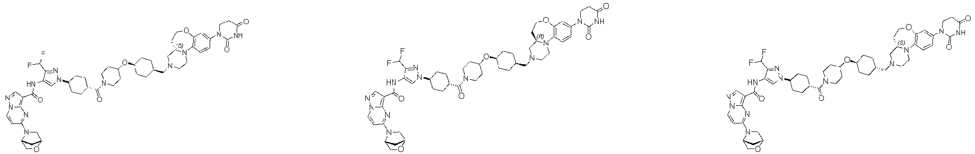 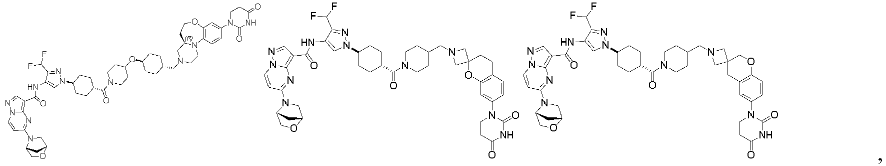 and

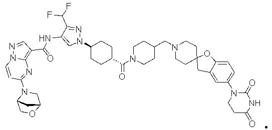

17. A pharmaceutical composition, comprising the compound according to any one of claims 1 to 16.

18. The compound according to any one of claims 6 to 16 or the pharmaceutical composition according to claim 17 for use in treating or preventing a condition treated by degrading a target protein that binds to a target protein ligand; preferably, the target protein is cyclin-dependent kinase 4/6, androgen receptor, estrogen receptor, Aurora kinase A, Bcr-Abl protein, epidermal growth factor receptor, anaplastic lymphoma kinase, Bruton's tyrosine kinase, bromodomain protein 4, or interleukin-1 receptor-associated kinase 4 (IRAK4).

19. The compound according to any one of claims 6 to 16 or the pharmaceutical composition according to claim 17 for use in treating or preventing a condition treated by binding to cereblon in vivo.

20. The compound according to any one of claims 6 to 16 or the pharmaceutical composition according to claim 17 for use in treating or preventing a condition associated with the accumulation and/or aggregation of a target protein; preferably, the target protein is cyclin-dependent kinase 4/6, androgen receptor, estrogen receptor, Aurora kinase A, Bcr-Abl protein, epidermal growth factor receptor, anaplastic lymphoma kinase, Bruton's tyrosine kinase, bromodomain protein 4, or interleukin-1 receptor-associated kinase 4 (IRAK4).

21. The compound or the pharmaceutical composition according to any one of claims 18 to 20, wherein the condition is a tumor or cancer; preferably, the tumor or cancer is breast cancer, ductal breast carcinoma, prostate cancer, mantle cell lymphoma, chronic myeloid leukemia, acute myeloid leukemia, myelomonocytic leukemia, non-small cell lung cancer, lung adenocarcinoma, and/or cervical cancer.

22. Use of the compound according to any one of claims 1 to 5 or the pharmaceutical composition according to claim 17 in the manufacture of a medicament for treating or preventing a condition treated by binding to cereblon in vivo.

23. Use of the compound according to any one of claims 6 to 16 or the pharmaceutical composition according to claim 17 in the manufacture of a medicament for treating or preventing a condition treated by degrading a target protein that binds to a target protein ligand; preferably, the target protein is cyclin-dependent kinase 4/6, androgen receptor, estrogen receptor, Aurora kinase A, Bcr-Abl protein, epidermal growth factor receptor, anaplastic lymphoma kinase, Bruton's tyrosine kinase, bromodomain protein 4, or interleukin-1 receptor-associated kinase 4 (IRAK4).

24. Use of the compound according to any one of claims 6 to 16 or the pharmaceutical composition according to claim 17 in the manufacture of a medicament for treating or preventing a condition associated with the accumulation and/or aggregation of a target protein; preferably, the target protein is cyclin-dependent kinase 4/6, androgen receptor, estrogen receptor, Aurora kinase A, Bcr-Abl protein, epidermal growth factor receptor, anaplastic lymphoma kinase, Bruton's tyrosine kinase, bromodomain protein 4, or interleukin-1 receptor-associated kinase 4 (IRAK4).

25. The use according to any one of claims 23 to 25, wherein the condition is a tumor or cancer; preferably, the tumor or cancer is breast cancer, ductal breast carcinoma, prostate cancer, mantle cell lymphoma, chronic myeloid leukemia, acute myeloid leukemia, myelomonocytic leukemia, non-small cell lung cancer, lung adenocarcinoma, and/or cervical cancer.

26. A method of treating or preventing a condition treated by degrading a target protein that binds to a target protein ligand, comprising administering to a subject in need thereof a therapeutically effective amount of the compound according to any one of claims 6 to 16 or the pharmaceutical composition according to claim 17; preferably, the target protein is cyclin-dependent kinase 4/6, androgen receptor, estrogen receptor, Aurora kinase A, Bcr-Abl protein, epidermal growth factor receptor, anaplastic lymphoma kinase, Bruton's tyrosine kinase, bromodomain protein 4, or interleukin-1 receptor-associated kinase 4 (IRAK4).

27. A method for treating or preventing a condition treated by binding to cereblon in vivo, comprising administering to a subject in need thereof a therapeutically effective amount of the compound according to any one of claims 6 to 16 or the pharmaceutical composition according to claim 17.

28. A method for treating or preventing a condition associated with the accumulation and/or aggregation of a target protein, comprising administering to a subject in need thereof a therapeutically effective amount of the compound according to any one of claims 6 to 16 or the pharmaceutical composition according to claim 17; preferably, the target protein is cyclin-dependent kinase 4/6, androgen receptor, estrogen receptor, Aurora kinase A, Bcr-Abl protein, epidermal growth factor receptor, anaplastic lymphoma kinase, Bruton's tyrosine kinase, bromodomain protein 4, or interleukin-1 receptor-associated kinase 4 (IRAK4).

29. The method according to any one of claims 26 to 28, wherein the condition is a tumor or cancer; preferably, the tumor or cancer is breast cancer, ductal breast carcinoma, prostate cancer, mantle cell lymphoma, chronic myeloid leukemia, acute myeloid leukemia, myelomonocytic leukemia, non-small cell lung cancer, lung adenocarcinoma, and/or cervical cancer.

30. A method for inducing degradation of a target protein in a cell, the method comprising administering to a subject in need thereof an effective amount of the compound according to any one of claims 6 to 16 or the pharmaceutical composition according to claim 17; preferably, the target protein is cyclin-dependent kinase 4/6, androgen receptor, estrogen receptor, Aurora kinase A, Bcr-Abl protein, epidermal growth factor receptor, anaplastic lymphoma kinase, Bruton's tyrosine kinase, bromodomain protein 4, or interleukin-1 receptor-associated kinase 4 (IRAK4).
